(19) Europäisches Patentamt
European Patent Office
Office européen des brevets

(11) EP 4 361 158 B1

(12) **EUROPEAN PATENT SPECIFICATION**

(45) Date of publication and mention of the grant of the patent:
**08.07.2026 Bulletin 2026/28**

(21) Application number: **22828032.7**

(22) Date of filing: **30.03.2022**

(51) International Patent Classification (IPC):
***C07F 5/02*** (2006.01) ***C09K 11/06*** (2006.01)
***C07D 209/86*** (2006.01) ***C07D 519/00*** (2006.01)
***H10K 85/60*** (2023.01) *H10K 101/20* (2023.01)
*H10K 101/30* (2023.01) *H10K 50/11* (2023.01)

(52) Cooperative Patent Classification (CPC):
**C09K 11/06; C07D 209/86; C07D 519/00; H10K 85/658;** H10K 50/11; H10K 85/657; H10K 85/6572; H10K 2101/20; H10K 2101/30

(86) International application number:
**PCT/JP2022/015888**

(87) International publication number:
**WO 2022/270113 (29.12.2022 Gazette 2022/52)**

(54) **ORGANIC ELECTROLUMINESCENT DEVICE**
ORGANISCHE ELEKTROLUMINESZIERENDE VORRICHTUNG
DISPOSITIF ÉLECTROLUMINESCENT ORGANIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.06.2021 JP 2021103702**
**17.09.2021 JP 2021151805**
**19.11.2021 JP 2021188860**
**17.12.2021 JP 2021204983**

(43) Date of publication of application:
**01.05.2024 Bulletin 2024/18**

(73) Proprietor: **Kyulux, Inc.**
**Fukuoka-shi, Fukuoka 819-0388 (JP)**

(72) Inventors:
- **YOSHIZAKI, Makoto**
  **Fukuoka-shi, Fukuoka 8190388 (JP)**
- **MORIMOTO, Kei**
  **Fukuoka-shi, Fukuoka 8190388 (JP)**
- **HWANG, Songhye**
  **Fukuoka-shi, Fukuoka 8190388 (JP)**
- **OZAWA, Hiroaki**
  **Fukuoka-shi, Fukuoka 8190388 (JP)**
- **KAKIZOE, Hayato**
  **Fukuoka-shi, Fukuoka 8190388 (JP)**
- **KODAMA, Yuka**
  **Fukuoka-shi, Fukuoka 8190388 (JP)**

(74) Representative: **Zimmermann & Partner Patentanwälte mbB**
**Postfach 330 920**
**80069 München (DE)**

(56) References cited:
**WO-A1-2018/212169**
**WO-A1-2020/039930**
**WO-A1-2021/245221**
**CN-A- 110 872 316**
**JP-A- 2020 203 875**
**US-A1- 2021 119 145**
**WO-A1-2020/039930**
**WO-A1-2020/040298**
**WO-A1-2022/025248**
**JP-A- 2020 132 636**
**US-A1- 2016 130 225**
**US-A1- 2021 202 851**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 361 158 B1

## Description

Background Art

The present invention relates to an organic electroluminescence device having a high luminous efficiency.

Researches have been actively conducted to improve the luminous efficiency of organic electroluminescence devices (organic EL devices). In particular, various studies for converting the energy in the excited triplet state, which is non-radiatively deactivated at room temperature, into the excited singlet energy to be used in light emission by newly developing a material composition of a light-emitting layer of the organic electroluminescence device have been conducted.

For example, in Patent Document 1, a three-component organic electroluminescence device is suggested in which a light-emitting layer contains a host material, a delayed fluorescence material, and a fluorescence material. In such a light-emitting layer, the excited triplet energy that has been moved to the delayed fluorescence material from the host material, and the excited triplet energy generated in the delayed fluorescence material are converted into the excited singlet energy by inverse intersystem crossing to the excited singlet state from the excited triplet state in the delayed fluorescence material, and are moved to the fluorescence material to be emitted as fluorescence. Accordingly, the excited triplet energy generated in the light-emitting layer is effectively used in the light emission of the fluorescence material such that a high luminous efficiency can be obtained. Patent Document 2 also discloses an OLED comprising three components in the emissive layer, wherein the emitting compound is polycyclic aromatic compound in which a plurality of aromatic rings are linked by a boron atom and a nitrogen atom.

Patent Document 1 JP2015-179809A
Patent Document 2: WO2020/039930

However, the light-emitting wavelength of the fluorescence material depends on an energy gap and a Stokes shift between HOMO-LUMO, and various fluorescence materials having different energy value have been developed. Accordingly, in the three-component light-emitting layer, in accordance with a fluorescence material to be used, the LUMO energy of the fluorescence material may be higher than or may be lower (deeper) than the LUMO energy of the delayed fluorescence material. In such a situation, as a result of configuring a light-emitting layer by combining various delayed fluorescence materials and fluorescence materials with a host material, the present inventors have determined that a phenomenon is observed in which a luminous efficiency decreases in the case of increasing the concentration of the fluorescence material when the LUMO energy of the fluorescence material is lower than the LUMO energy of the delayed fluorescence material, and thus, the luminous efficiency is not capable of being sufficiently improved.

Therefore, the present inventors have conducted intensive studies in order to provide an organic electroluminescence device containing a host material, a delayed fluorescence material, and a fluorescence material in a light-emitting layer, in which a luminous efficiency can be obtained even when the LUMO energy of the fluorescence material is lower than the LUMO energy of the delayed fluorescence material.

Summary of Invention

As a result of conducting the intensive studies, the present inventor have found that even in a case where the LUMO energy of the fluorescence material is lower than the LUMO energy of the delayed fluorescence material, the luminous efficiency can be improved despite of an increase in the concentration of the fluorescence material when an orientation value S of organic compound molecules used as the fluorescence material is -0.3 or less.

The invention is suggested on the basis of such findings, and specifically has the following configurations.

According to the invention, there is provided an organic electroluminescence device including: an anode; a cathode; and at least one organic layer including a light-emitting layer between the anode and the cathode,

in which the light-emitting layer contains a first organic compound, a second organic compound, and a third organic compound, and satisfy the following formulae (a) and (b),
the second organic compound is a delayed fluorescence material, and
the largest component of light emitted from the device is fluorescence from the third organic compound.

$$E_{LUMO}(2) > E_{LUMO}(3) \qquad \text{Formula (a)}$$

$$S \leq -0.3 \qquad \text{Formula (b)}$$

[Here,

$E_{LUMO}(2)$ represents LUMO energy of the second organic compound,
$E_{LUMO}(3)$ represents LUMO energy of the third organic compound, and
S represents an orientation value of the third organic compound in the light-emitting layer.]

The concentration of the third organic compound in the light-emitting layer may be greater than 0.3% by weight.

The third organic compound is a compound including a boron atom and a nitrogen atom, which may exhibit a multiple resonance effect, and having a condensed ring structure including four or more constituent rings.

The third organic compound is a compound having a structure in which a pyrrole ring and two benzene rings, which share a nitrogen atom, are condensed with a heterocyclic 6-membered ring including a boron atom and a nitrogen atom.

The third organic compound is a compound represented by the following formula (16).

Formula (16)

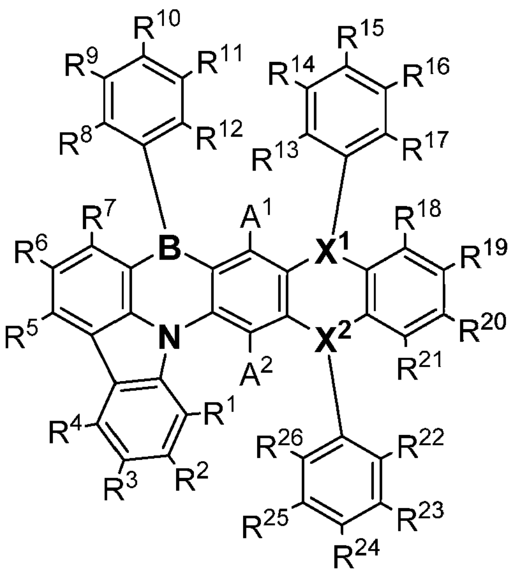

[In the formula (16), one of $X^1$ and $X^2$ is a nitrogen atom, and the other is a boron atom. Each of $R^1$ to $R^{26}$, $A^1$, and $A^2$ independently represents a hydrogen atom, a deuterium atom, or a substituent. $R^1$ and $R^2$, $R^2$ and $R^3$, $R^3$ and $R^4$, $R^4$ and $R^5$, $R^5$ and $R^6$, $R^6$ and $R^7$, $R^7$ and $R^8$, $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{17}$ and $R^{18}$, $R^{18}$ and $R^{19}$, $R^{19}$ and $R^{20}$, $R^{20}$ and $R^{21}$, $R^{21}$ and $R^{22}$, $R^{22}$ and $R^{23}$, $R^{23}$ and $R^{24}$, $R^{24}$ and $R^{25}$, and $R^{25}$ and $R^{26}$ may be bonded to each other to form ring structures. Here, when $X^1$ is a nitrogen atom, $R^{17}$ and $R^{18}$ are bonded to each other to form a single bond and to form a pyrrole ring, and when $X^2$ is a nitrogen atom, $R^{21}$ and $R^{22}$ are bonded to each other to form a single bond and to form a pyrrole ring. Here, when $X^1$ is a nitrogen atom, $R^7$ and $R^8$ and $R^{21}$ and $R^{22}$ are bonded via nitrogen atoms to form 6-membered rings, and $R^{17}$ and $R^{18}$ are bonded to each other to form a single bond, at least one of $R^1$ to $R^6$ is a substituted or unsubstituted aryl group, or any of $R^1$ and $R^2$, $R^2$ and $R^3$, $R^3$ and $R^4$, $R^4$ and $R^5$, and $R^5$ and $R^6$ are bonded to each other to form an aromatic ring or a heteroaromatic ring.]

The concentration of the second organic compound in the light-emitting layer may be 25% by weight or more.

The second organic compound may have a structure in which 1 to 2 cyano groups and at least one donor group are bonded to a benzene ring.

The donor group may have a structure in which a substituted or unsubstituted benzofuran ring is condensed with a benzene ring constituting a carbazole-9-yl group.

The donor group may be a substituted or unsubstituted 5H-benzofuro[3,2-c]carbazole-5-yl group.

Furthermore, three or more donor groups may be bonded to the benzene ring.

The first organic compound, the second organic compound, and the third organic compound may satisfy (a1) described below.

$$E_{LUMO}(1) > E_{LUMO}(2) > E_{LUMO}(3) \quad \text{Formula (a1)}$$

[Here,

$E_{LUMO}(1)$ represents LUMO energy of the first organic compound,
$E_{LUMO}(2)$ represents LUMO energy of the second organic compound,
$E_{LUMO}(3)$ represents LUMO energy of the third organic compound, and
S represents an orientation value of the third organic compound in the light-emitting layer.]

According to the invention, in the organic electroluminescence device containing the first organic compound, the second organic compound that is the delayed fluorescence material, and the third organic compound emitting the fluorescence in the light-emitting layer, even when the LUMO energy of the third organic compound is lower than the LUMO energy of the second organic compound, the luminous efficiency can be improved by increasing the concentration of the third organic compound.

Description of Embodiments

Hereinafter, the contents of the invention will be described in detail. The descriptions on constituent elements to be described below may be made on the basis of representative embodiments or specific examples of the invention, but the invention is not limited to such embodiments or specific examples. The numerical value range represented by using "to" in this application means a range including numerical values described before and after "to", as the lower limit value and the upper limit value. Further, "consisting of" in this application means consisting only of a component described after "consisting of" but not containing other components. Further, a part or all of hydrogen atoms present in the molecule of the compound used in the invention may be replaced with deuterium atoms ($^2H$, deuterium D). In the chemical structural formula of the present specification, the hydrogen atom is indicated by H, or the indication thereof is omitted. For example, when the indication of an atom bonded to a ring skeleton forming carbon atom of a benzene ring is omitted, it is assumed that, at a location where the indication is omitted, H is bonded to the ring skeleton forming carbon atom. In the present specification, the term of "substituent" means an atom or a group of atoms other than a hydrogen atom and a deuterium atom. Meanwhile, the term of "substituted or unsubstituted" or "may be substituted" means that a hydrogen atom may be substituted with a deuterium atom or a substituent. Further, "transparent" in the invention means that a visible light transmission is 50% or more, preferably 80% or more, more preferably 90% or more, and further preferably 99% or more. The visible light transmission can be measured by a UV-visual spectrophotometer.

An organic electroluminescence device of the invention is an organic electroluminescence device including an anode, a cathode, and at least one organic layer including a light-emitting layer between the anode and the cathode. The organic layer may include only the light-emitting layer, or may include an organic layer other than the light-emitting layer. For example, the organic layer may be or may not be interposed between the anode and the light-emitting layer and between the light-emitting layer and the cathode, respectively. In other words, the anode and the light-emitting layer may be laminated to be directly in contact with each other, or may be laminated not to be directly in contact with each other. Further, the light-emitting layer and the cathode may be laminated to be directly in contact with each other, or may be laminated not to be directly in contact with each other. The light-emitting layer is positioned between the anode and the cathode, and it is preferable that the entire light-emitting layer is arranged in a region between the anode and the cathode without protruding.

The organic electroluminescence device of the invention may include a substrate that supports the anode, the cathode, and at least one organic layer including the light-emitting layer. In this case, the substrate may be arranged on the anode on a side opposite to the light-emitting layer, or may be arranged on the cathode on a side opposite to the light-emitting layer. Further, the organic electroluminescence device of the invention may be a top emission type device in which most of the light is emitted from a side opposite to the substrate, or may be a bottom emission type device in which most of the light is emitted from the substrate side. Here, "most of the light" means that the amount of light emitted from the device is 60% or more.

The organic electroluminescence device of the invention contains a first organic compound, a second organic compound, and a third organic compound in the light-emitting layer. Here, the second organic compound is a delayed fluorescence material. Further, the third organic compound is a compound emitting fluorescence. Then, in the organic electroluminescence device of the invention, the largest component of the light emitted from the device is fluorescence from the third organic compound.

The second organic compound and the third organic compound contained in the light-emitting layer satisfy the following formula (a) and formula (b).

$$E_{LUMO}(2) > E_{LUMO}(3) \quad \text{Formula (a)}$$

$$S \leq -0.3 \quad \text{Formula (b)}$$

In one aspect of the invention, the first organic compound, the second organic compound, and the third organic compound contained in the light-emitting layer satisfy the following formula (a1) and formula (b).

$$E_{LUMO}(1) > E_{LUMO}(2) > E_{LUMO}(3) \quad \text{Formula (a1)}$$

$$S \leq -0.3 \quad \text{Formula (b)}$$

In the formula (a) and the formula (a1), $E_{LUMO}(1)$ represents the LUMO energy of the first organic compound, $E_{LUMO}(2)$ represents the LUMO energy of the second organic compound, and $E_{LUMO}(3)$ represents the LUMO energy of the third organic compound. LUMO is an abbreviation for the lowest unoccupied molecular orbital, and is obtained by photo-electron spectroscopy in air (available from RKI INSTRUMENTS, INC., AC-3 or the like).

The invention satisfies the relationship of the formula (a), and thus, the LUMO energy of the second organic compound contained in the light-emitting layer is higher than the LUMO energy of the third organic compound. In the case of satisfying the relationship of the formula (a1), among the first organic compound, the second organic compound, and the third organic compound contained in the light-emitting layer, the LUMO energy of the first organic compound is the highest, the LUMO energy of the second organic compound is the next highest, and the third organic compound is the lowest. A LUMO energy difference [$E_{LUMO}(1)$-$E_{LUMO}(2)$], for example, may be in a range of 0.1 eV or more, may be in a range of 0.5 eV or more, may be in a range of 0.8 eV or more, or may be in a range of 1.0 eV or more, and may be in a range of 2.0 eV or less, may be in a range of 1.5 eV or less, may be in a range of 1.3 eV or less, or may be in a range of 1.1 eV or less. A LUMO energy difference [$E_{LUMO}(2)$-$E_{LUMO}(3)$], for example, may be in a range of 0.01 eV or more, may be in a range of 0.05 eV or more, may be in a range of 0.1 eV or more, may be in a range of 0.15 eV or more, or may be in a range of 0.2 eV or more, and may be in a range of 0.7 eV or less, may be in a range of 0.5 eV or less, may be in a range of 0.4 eV or less, or may be in a range of 0.3 eV or less. In one aspect of the invention, as the first organic compound, a compound having LUMO energy in a range of -2.0 to -5.0 eV, or a compound having LUMO energy in a range of - 2.5 to -4.0 eV can be adopted. In one aspect of the invention, as the second organic compound, a compound having LUMO energy in a range of -2.0 to -5.0 eV, or a compound having LUMO energy in a range of -2.5 to -4.0 eV can be adopted. Further, in one aspect of the invention, as the third organic compound, a compound having LUMO energy in a range of -2.0 to -5.0 eV, or a compound having LUMO energy in a range of - 2.5 to -4.0 eV can be adopted.

The relationship between the HOMO energy of the first organic compound, the HOMO energy of the second organic compound and the HOMO energy of the third organic compound is not particularly limited. For example, the HOMO energy of the second organic compound may be lower than or higher than the HOMO energy of the first organic compound, or may be the same as the HOMO energy of the first organic compound. Further, the HOMO energy of the third organic compound may be lower than or higher than the HOMO energy of the second organic compound, or may be the same as the HOMO energy of the second organic compound. HOMO is an abbreviation for the highest occupied molecular orbital, and can be obtained by photo-electron spectroscopy in air (available from RKI INSTRUMENTS, INC., AC-3 or the like). In one aspect of the invention, as the first organic compound, a compound having HOMO energy in a range of -4.0 to -6.5 eV, or a compound having HOMO energy in a range of - 5.5 to -6.2 eV can be adopted. In one aspect of the invention, as the second organic compound, a compound having HOMO energy in a range of -4.0 to -6.5 eV, a compound having HOMO energy in a range of -5.5 to -6.2 eV can be adopted. Further, in one aspect of the invention, as the third organic compound, a compound having HOMO energy in a range of -4.0 to -6.5 eV, or a compound having HOMO energy in a range of - 5.0 to -6.0 eV can be adopted.

S in the formula (b) represents an orientation value of the third organic compound in the light-emitting layer. The invention satisfies the formula (b), and thus, the orientation value of the third organic compound in the light-emitting layer is -0.3 or less. The orientation value is also referred to as a S value, and is an index indicating the degree of orientation of the third organic compound in the light-emitting layer. A larger negative value (a smaller numerical value) indicates that the orientation is higher. The orientation value (S value) may be determined by the method described in Scientific Reports 2017, 7, 8405. In the organic electroluminescence device of the invention, by setting the orientation value of the third organic compound to -0.3 or less, it is possible to realize a high external quantum yield while satisfying the relationship of the LUMO energy of the formula (a). That is, in the organic electroluminescence device of the related art in which the orientation value of the fluorescence material is not considered, when the LUMO energy of the fluorescence material is lower than the LUMO energy of the delayed fluorescence material, and the concentration of the fluorescence material is increased, a problem that the luminous efficiency decreases occurs. In contrast, in the organic electroluminescence

device of the invention, the LUMO energy of the third organic compound emitting fluorescence is set to be lower than the LUMO energy of the second organic compound that is the delayed fluorescence material, but by defining the orientation value of the third organic compound in the light-emitting layer to -0.3 or less, it is possible to improve the luminous efficiency while increasing the concentration of the third organic compound. Further, the third organic compound of which the orientation value is -0.3 or less has high stability, and by using the third organic compound as a light-emitting material, an effect that device lifetime is improved can be obtained. The orientation value of the third organic compound in the light-emitting layer is preferably - 0.38 or less, more preferably -0.40 or less, further preferably -0.41 or less, and still further preferably -0.42 or less.

It is preferable that the first organic compound, the second organic compound, and the third organic compound contained in the light-emitting layer satisfy the following formula (c).

$$E_{S1}(1) > E_{S1}(2) > E_{S1}(3) \quad \text{Formula (c)}$$

In the formula (c), $E_{S1}(1)$ represents the lowest excited singlet energy of the first organic compound, $E_{S1}(2)$ represents the lowest excited singlet energy of the second organic compound, and $E_{S1}(3)$ represents the lowest excited singlet energy of the third organic compound. In the present specification, eV is adopted as unit. The lowest excited singlet energy can be obtained by preparing a thin film or a toluene solution (a concentration of $10^{-5}$ mol/L) of a measurement target compound, and measuring a fluorescence spectrum at room temperature (300 K) (for the details, a method for measuring the lowest excited singlet energy in the description for the second organic compound can be referred to).

As represented in the formula (c), it is preferable that among the first organic compound, the second organic compound, and the third organic compound contained in the light-emitting layer, the lowest excited singlet energy of the first organic compound is the highest, the lowest excited singlet energy of the second organic compound is the second highest, and the lowest excited singlet energy of the third organic compound is the lowest. $E_{S1}(1)$-$E_{S1}(2)$, for example, can be in a range of 0.20 eV or more, can be in a range of 0.40 eV or more, or can be in a range of 0.60 eV or more, and can be in a range of 1.50 eV or less, can be in a range of 1.20 eV or less, or can be in a range of 0.80 eV or less. $E_{S1}(2)$-$E_{S1}(3)$, for example, can be in a range of 0.05 eV or more, can be in a range of 0.10 eV or more, or can be in a range of 0.15 eV or more, and can be in a range of 0.50 eV or less, can be in a range of 0.30 eV or less, or can be in a range of 0.20 eV or less. $E_{S1}(1)$-$E_{S1}(3)$, for example, can be in a range of 0.25 eV or more, can be in a range of 0.45 eV or more, or can be in a range of 0.65 eV or more, and can be in a range of 2.00 eV or less, can be in a range of 1.70 eV or less, or can be in a range of 1.30 eV or less.

In the organic electroluminescence device of the invention, the largest component of the light emitted from the device is the fluorescence from the third organic compound. In the invention, the "light emitted from the device" means light emitted from the device when driving the device at 20°C. Insofar as the largest component of the light emitted from the device is the fluorescence from the third organic compound, the light emitted from the organic electroluminescence device of the invention may include phosphorescence from the third organic compound, and light emitted from the first organic compound or the second organic compound, but it is preferable that such emitted light is slight compared to the fluorescence from the third organic compound. In the invention, 70% or more of the light emitted from the device may be the fluorescence from the third organic compound, 90% or more of the light emitted from the device may be the fluorescence from the third organic compound, or 99% or more of the light emitted from the device may be the fluorescence from the third organic compound.

It is preferable that the concentration of the third organic compound in the light-emitting layer of the organic electroluminescence device of the invention is greater than 0.3% by weight. The concentration of the third organic compound in the light-emitting layer can be in a range of 0.35% by weight or more, can be in a range of 0.5% by weight or more, can be in a range of 1% by weight or more, or can be in a range of 2% by weight or more. The concentration of the third organic compound in the light-emitting layer can be in a range of 10% by weight or less, can be in a range of 5% by weight or less, or can be in a range of 3% by weight or less.

Further, when the concentrations of the first organic compound, the second organic compound, and the third organic compound in the light-emitting layer of the organic electroluminescence device of the invention are set as Conc(1), Conc(2), and Conc(3), respectively, it is preferable to satisfy the relationship of the following formula (d).

$$\text{Conc}(1) > \text{Conc}(2) > \text{Conc}(3) \qquad \text{Formula (d)}$$

Conc(1) is preferably 30% by weight or more, can be in a range of 50% by weight or more, or can be in a range of 60% by weight or more, and can be in a range of 99% by weight or less, can be in a range of 85% by weight or less, or can be in a range of 70% by weight or less.

Conc(2) is preferably 5% by weight or more, can be in a range of 15% by weight or more, can be in a range of 25% by weight or more, or can be in a range of 30% by weight or more, and can be in a range of 45% by weight or less, can be in a

range of 40% by weight or less, or can be in a range of 35% by weight or less. In a preferable aspect of the invention, Conc(2) is 25 to 45% by weight.

For a preferable range of Conc(3), the description on the concentration of the third organic compound in the light-emitting layer can be referred to.

Conc(1)/Conc(3) can be in a range of 10 or more, can be in a range of 50 or more, or can be in a range of 90 or more, and can be in a range of 10000 or less, can be in a range of 1000 or less, or can be in a range of 200 or less.

Conc(2)/Conc(3) can be in a range of 10 or more, can be in a range of 50 or more, or can be in a range of 90 or more, and can be in a range of 10000 or less, can be in a range of 1000 or less, or can be in a range of 200 or less.

It is preferable that the light-emitting layer of the organic electroluminescence device of the invention does not contain a metal element other than boron. Further, a light-emitting layer that does not contain a metal element including boron can also be adopted. For example, the light-emitting layer can be made of only a compound containing atoms selected from the group consisting of a carbon atom, a hydrogen atom, a nitrogen atom, an oxygen atom, a sulfur atom, a fluorine atom and a boron atom. For example, the light-emitting layer can be made of only a compound containing atoms selected from the group consisting of a carbon atom, a hydrogen atom, a nitrogen atom, an oxygen atom, a sulfur atom, a fluorine atom and a boron atom. For example, the light-emitting layer can be made of only a compound containing atoms selected from the group consisting of a carbon atom, a hydrogen atom, a nitrogen atom, an oxygen atom and a sulfur atom. For example, the light-emitting layer can be made of only a compound containing atoms selected from the group consisting of a carbon atom, a hydrogen atom, a nitrogen atom and an oxygen atom.

(First organic compound)

The first organic compound used in the light-emitting layer of the organic electroluminescence device of the invention is selected from compounds having LUMO energy higher than that of the second organic compound or the third organic compound. It is preferable that the first organic compound is selected from compounds that have LUMO energy higher than that of the second organic compound or the third organic compound, and have lowest excited singlet energy higher than that of the second organic compound or the third organic compound. It is preferable that the first organic compound has a function as a host material for transporting carriers. Further, it is preferable that the first organic compound has a function of confining the energy of the third organic compound within the compound. Accordingly, the third organic compound is capable of efficiently converting energy generated by recombination between holes and electrons in the molecule and energy received from the first organic compound and the second organic compound into light emission.

As the first organic compound, an organic compound that has hole transport performance and electron transport performance, prevents the emitted light from having a long wavelength, and has a high glass transition temperature is preferable. Further, in a preferred aspect of the invention, the first organic compound is selected from compounds that do not emit delayed fluorescence. The light emitted from the first organic compound is preferably less than 1%, and more preferably less than 0.1% of the light emitted from the organic electroluminescence device of the invention, and for example, may be less than 0.01%, or a detection limit or less.

It is preferable that the first organic compound does not contain metal atoms. For example, as the first organic compound, a compound containing atoms selected from the group consisting of a carbon atom, a hydrogen atom, a nitrogen atom, an oxygen atom, and a sulfur atom can be selected. For example, as the first organic compound, a compound containing atoms selected from the group consisting of a carbon atom, a hydrogen atom, a nitrogen atom, and an oxygen atom can be selected. For example, as the first organic compound, a compound consisting of carbon atoms, hydrogen atoms, and nitrogen atoms can be selected.

Hereinafter, preferable compounds that can be used as the first organic compound will be given.

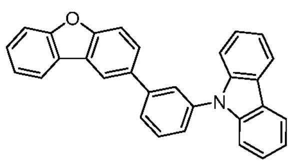

H1

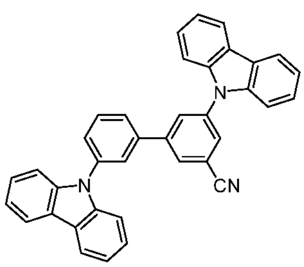

H2

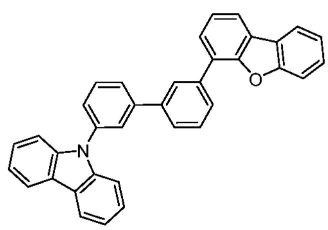

H3

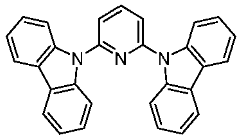

**H4**

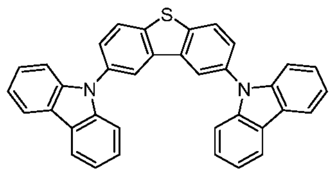

**H5**

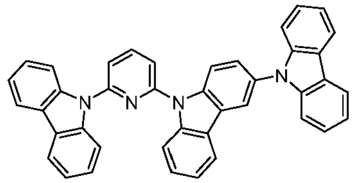

**H6**

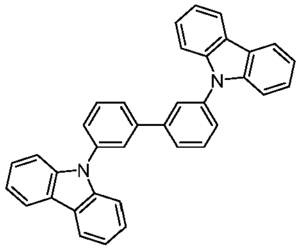

**H7**

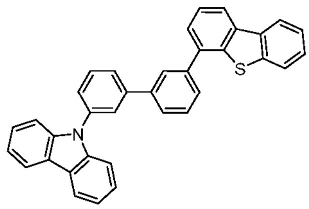

**H8**

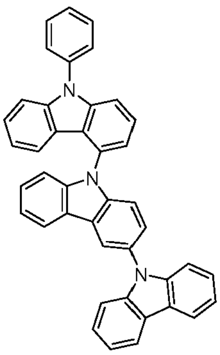

**H9**

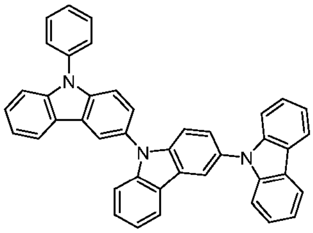

**H10**

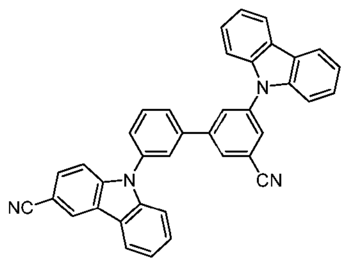

**H11**

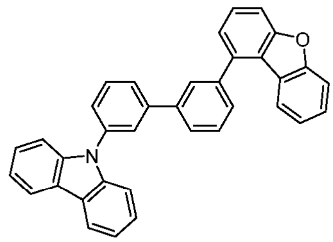

**H12**

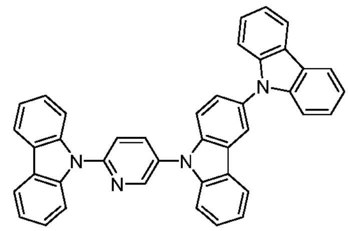

**H13**

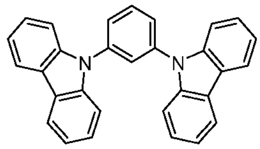

**H14**

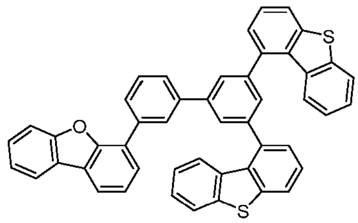

**H15**

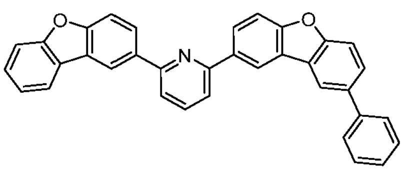

**H16**

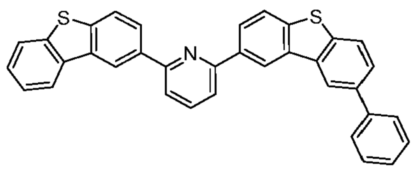

**H17**

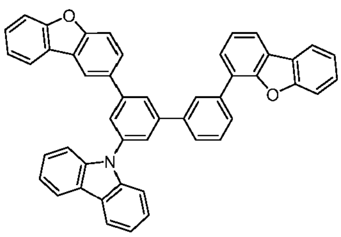
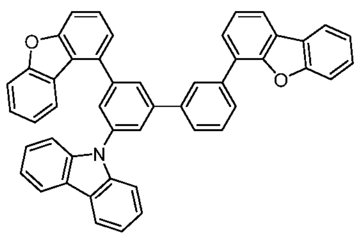
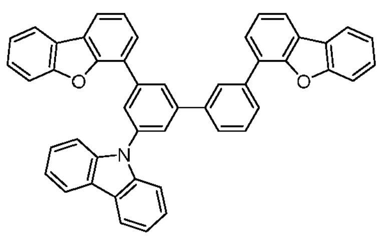

**H18** **H19** **H20**

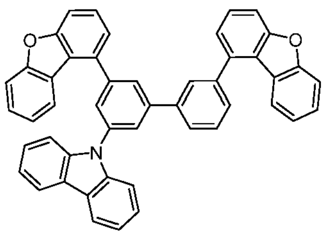
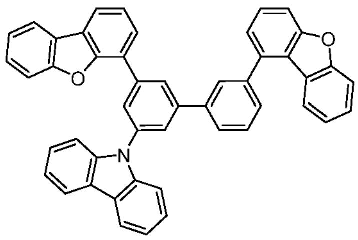
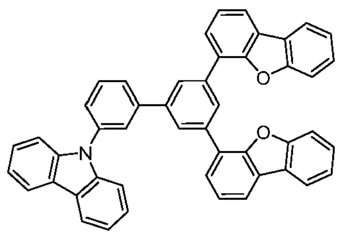

**H21** **H22** **H23**

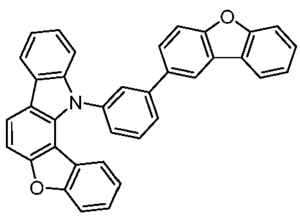
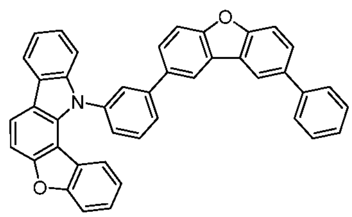
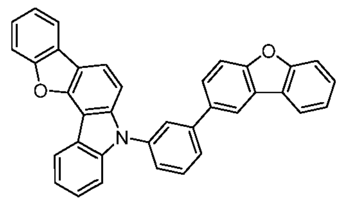

**H24** **H25** **H26**

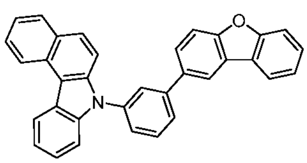
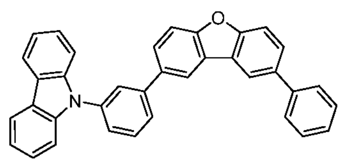
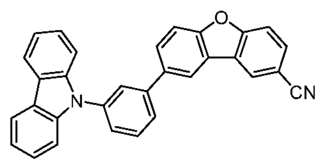

**H27** **H28** **H29**

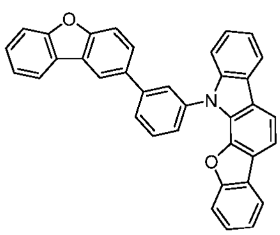
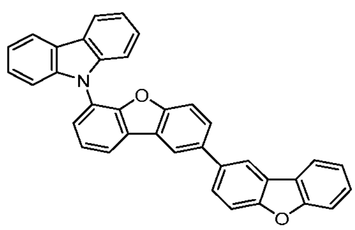
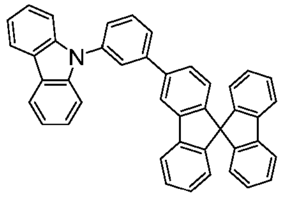

**H30** **H31** **H32**

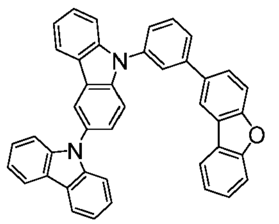

**H33**

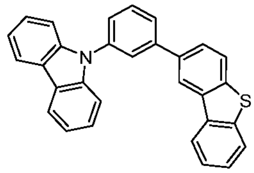

**H34**

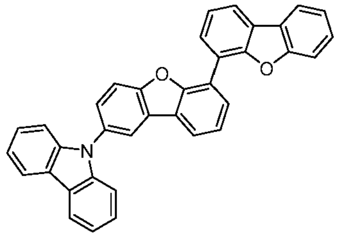

**H35**

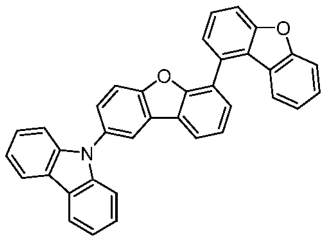

**H36**

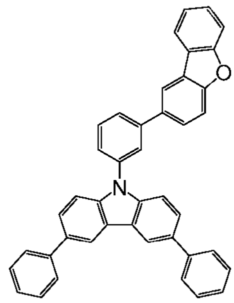

**H37**

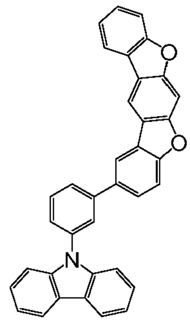

**H38**

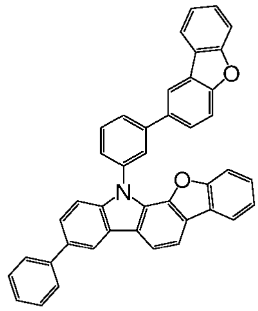

**H39**

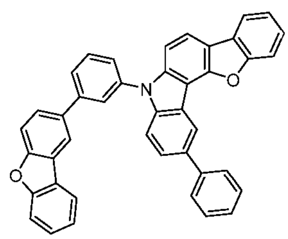

**H40**

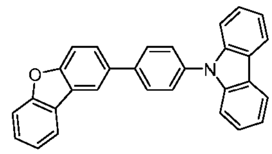

**H41**

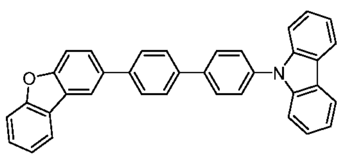

**H42**

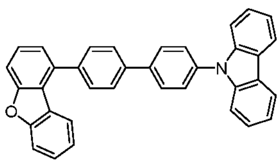

**H43**

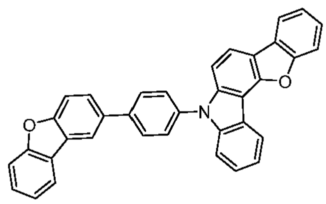

**H44**

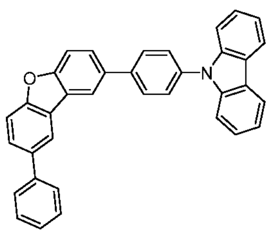

**H45**

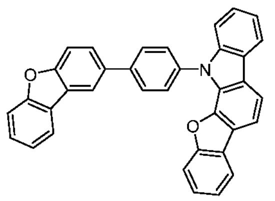

**H46**

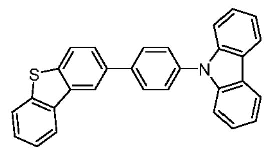

**H47**

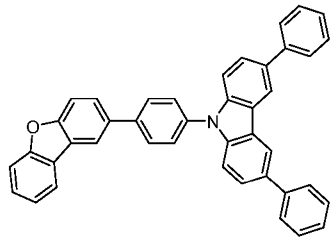

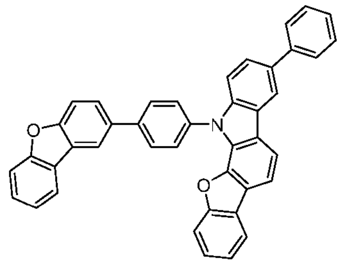

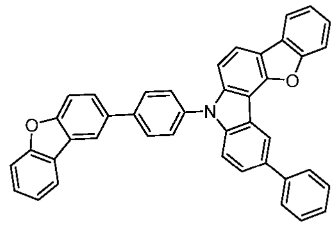

H48 H49 H50

(Second organic compound)

The second organic compound used in the light-emitting layer of the organic electroluminescence device of the invention is a delayed fluorescence material having LUMO energy lower than that of the first organic compound and higher than that of the third organic compound. It is preferable that the second organic compound is a delayed fluorescence material that has LUMO energy lower than that of the first organic compound and higher than that of the third organic compound and has lowest excited singlet energy lower than that of the first organic compound and higher than that of the third organic compound. The "delayed fluorescence material" in the invention is an organic compound that causes inverse intersystem crossing to the excited singlet state from the excited triplet state in the excited state, and emits fluorescence (delayed fluorescence) when returning to the ground state from the excited singlet state. In the invention, when emission lifetime is measured by a fluorescence lifetime measurement system (available from Hamamatsu Photonics K.K., a streak camera system or the like), a material in which fluorescence having emission lifetime of 100 ns (nanoseconds) or more is observed is referred to as a delayed fluorescence material. The second organic compound is a material that is capable of emitting delayed fluorescence, but it is not essential that the second organic compound emits delayed fluorescence derived from the second organic compound when used in the organic electroluminescence device of the invention. The light emitted from the second organic compound is preferably less than 10% of the light emitted from the organic electroluminescence device of the invention, and for example, may be less than 1%, less than 0.1%, less than 0.01%, or a detection limit or less.

In the organic electroluminescence device of the invention, the second organic compound receives energy from the first organic compound in the excited singlet state to transition to the excited singlet state. Further, the second organic compound may receive energy from the first organic compound in the excited triplet state to transition to the excited triplet state. Since the second organic compound has a small difference ($\Delta E_{ST}$) between the excited singlet energy and the excited triplet energy, inverse intersystem crossing to the second organic compound in the excited singlet state from the second organic compound in the excited triplet state is likely to occur. The second organic compound in the excited singlet state generated through such a route imparts energy to the third organic compound such that the third organic compound is transitioned to the excited singlet state.

In the second organic compound, a difference $\Delta E_{ST}$ between the lowest excited singlet energy and the lowest excited triplet energy at 77 K is preferably 0.3 eV or less, more preferably 0.25 eV or less, more preferably 0.2 eV or less, more preferably 0.15 eV or less, further preferably 0.1 eV or less, still further preferably 0.07 eV or less, still further preferably 0.05 eV or less, still further preferably 0.03 eV or less, and particularly preferably 0.01 eV or less.

When $\Delta E_{ST}$ is small, reverse intersystem crossing to the excited triplet state from the excited singlet state is likely to occur through the absorption of the thermal energy, and thus, the second organic compound functions as a thermally activated delayed fluorescence material. The thermally activated delayed fluorescence material absorbs the heat emitted from the device to relatively easily cause the reverse intersystem crossing to the excited singlet from the excited triplet state, which may contribute to efficient emission of the excited triplet energy.

In the invention, the lowest excited singlet energy ($E_{S1}$) and the lowest excited triplet energy ($E_{T1}$) of the compound is a value obtained by the following procedure. $\Delta E_{ST}$ is a value obtained by calculating $E_{S1}$-$E_{T1}$.

(1) Lowest Excited Singlet Energy ($E_{S1}$)

A thin film or a toluene solution (a concentration of $10^{-5}$ mol/L) of a measurement target compound is prepared as a spectrum. The fluorescence spectrum of the specimen is measured at room temperature (300 K). In the fluorescence spectrum, a vertical axis is set as light emission, and a horizontal axis is set as a wavelength. A tangential line is drawn to the rise of the emission spectrum on the short wavelength side, and a wavelength value $\lambda$edge [nm] at an intersection between the tangential line and the horizontal axis is obtained. A value obtained by converting the wavelength value into an energy value through the following conversion formula is set as $E_{S1}$.

$$\text{Conversion Formula: } E_{S1}[\text{eV}]=1239.85/\lambda\text{edge}$$

The emission spectrum in Examples described below was measured by a detector (available from Hamamatsu Photonics K.K., PMA-12 multichannel spectroscope C10027-01) using a LED light source (available from Thorlabs, Inc., M300L4) as an excitation light source.

(2) Lowest Excited Triplet Energy ($E_{T1}$)

The same specimen as that used for the measurement of the lowest excited singlet energy ($E_{S1}$) is cooled to 77 [K] by liquid nitrogen, and a specimen for measuring phosphorescence is irradiated with excitation light (300 nm) to measure the phosphorescence with a detector. Emission after 100 milliseconds after the irradiation of the excitation light is set as a phosphorescence spectrum. A tangential line is drawn to the rise of the phosphorescent spectrum on the short wavelength side, and a wavelength value λedge [nm] at an intersection between the tangential line and the horizontal axis is obtained. A value obtained by converting the wavelength value into an energy value through the following conversion formula is set as $E_{T1}$.

$$\text{Conversion Formula: } E_{T1}[\text{eV}]=1239.85/\lambda\text{edge}$$

The tangential line to the rise of the phosphorescent spectrum on the short wavelength side is drawn as follows. When moving on a spectrum curve from the short wavelength side of the phosphorescence spectrum to the maximum value on the shortest wavelength side among the maximum values of the spectrum, a tangential line at each point on the curve toward the long wavelength side is taken into consideration. The slope of the tangential line increases as the curve rises (that is, as the vertical axis increases). A tangential line drawn at a point where the value of the slope is the maximum value is set as the tangential line to the rise of the phosphorescent spectrum on the short wavelength side.

The maximum point with a peak intensity of 10% or less of the largest peak intensity of the spectrum is not included in the maximum value on the shortest wavelength side, but is closest to the maximum value on the shortest wavelength side, and a tangential line drawn at a point where the value of the slope is the maximum value is set as the tangential line to the rise of the phosphorescent spectrum on the short wavelength side.

It is preferable that the second organic compound does not contain metal atoms. For example, as the second organic compound, a compound containing atoms selected from the group consisting of a carbon atom, a hydrogen atom, a nitrogen atom, an oxygen atom, and a sulfur atom can be selected. For example, as the second organic compound, a compound containing atoms selected from the group consisting of a carbon atom, a hydrogen atom, a nitrogen atom, and an oxygen atom can be selected. For example, as the second organic compound, a compound consisting of carbon atoms, hydrogen atoms, and nitrogen atoms can be selected.

Examples of a typical second organic compound include a compound having a structure in which 1 to 2 cyano groups and at least one donor group are bonded to a benzene ring. As the donor group, for example, a substituted or unsubstituted carbazole-9-yl group can be preferably exemplified. For example, a compound in which three or more substituted or unsubstituted carbazole-9-yl groups are bonded to the benzene ring, a compound in which each 5-membered ring portion of a substituted or unsubstituted benzofuran ring, a substituted or unsubstituted benzothiophene ring, a substituted or unsubstituted indole ring, a substituted or unsubstituted indene ring, and a substituted or unsubstituted silaindene ring is condensed with at least one of two benzene rings constituting a carbazole-9-yl group, and the like can be exemplified. Specific examples of a group having a structure in which a substituted or unsubstituted benzofuran ring is condensed with a benzene ring constituting a carbazole-9-yl group include a substituted or unsubstituted SH-benzofuro[3,2-c]carbazole-5-yl group.

As the second organic compound, a compound that is represented by the following formula (1) and emits delayed fluorescence can be preferably used.

Formula (1)

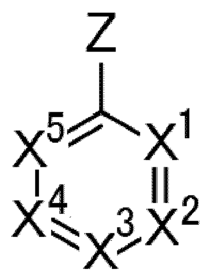

In the formula (1), $X^1$ to $X^5$ represent N or C-R. R represents a hydrogen atom, a deuterium atom, or a substituent. When two or more of $X^1$ to $X^5$ represent C-R, such C-R may be the same or different from each other. Here, at least one of $X^1$ to $X^5$ is C-D (D mentioned herein represents a donor group). When all of $X^1$ to $X^5$ are C-R, Z represents an acceptor group.

Among the compounds represented by the formula (1), a particularly preferable compound is a compound represented by the following formula (2).

Formula (2)

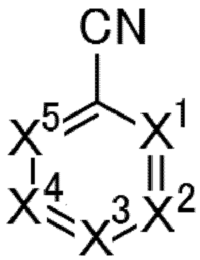

In the formula (2), $X^1$ to $X^5$ represent N or C-R. R represents a hydrogen atom, a deuterium atom, or a substituent. When two or more of $X^1$ to $X^5$ represent C-R, such C-R may be the same or different from each other. Here, at least one of $X^1$ to $X^5$ is C-D (D mentioned herein represents a donor group).

In a preferred aspect of the invention, all of $X^1$ to $X^5$ are not C-CN. That is, the compound is a compound having a structure in which 1 to 2 cyano groups and at least one donor group are bonded to a benzene ring. In another preferred aspect of the invention, only $X^2$ represents C-CN, and $X^1$ and $X^3$ to $X^5$ are not C-CN. That is, the compound is a compound having a structure in which at least one donor group is bonded to a benzene ring of isophthalonitrile. In another aspect of the invention, only $X^3$ represents C-CN, and $X^1$, $X^2$, $X^4$, and $X^5$ are not C-CN. That is, the compound is a compound having a structure in which at least one donor group is bonded to a benzene ring of terephthalonitrile.

The acceptor group represented by X in the formula (1) is a group having properties of donating electrons with respect to a ring to which Z is bonded, and for example can be selected from groups having a positive Hammett σp value. The donor group represented by D in the formula (1) and the formula (2) is a group having properties of suctioning electrons with respect to a ring to which D is bonded, and for example can be selected from groups having a negative Hammett σp value. Hereinafter, the acceptor group may be referred to as A.

Here, the "Hammett σp value", which is proposed by L.P. Hammett, indicates the quantified effect of a substituent on the reaction rate or equilibrium of a para-substituted benzene derivative. Specifically, it is a constant (σp) peculiar to the substituent in the following equation, which is established between the substituent in the para-substituted benzene derivative and the reaction rate constant or the equilibrium constant:

$$\log(k/k_0)=\rho\sigma p$$

or

$$\log(K/K_0)=\rho\sigma p$$

In the equation, $k_0$ represents a rate constant of a benzene derivative having no substituent, k represents a rate constant of a benzene derivative substituted with a substituent, $K_0$ represents an equilibrium constant of a benzene

derivative having no substituent, K represents an equilibrium constant of a benzene derivative substituted with a substituent, and ρ represents a reaction constant determined by the type and condition of the reaction. In relation to descriptions on "the Hammett σp value" and the numerical value of each substituent in the invention, the description on the σp value may be referred to in Hansch, C. et. al., Chem. Rev., 91, 165-195(1991).

For specific examples of the acceptor group, a cyano group, or acceptor groups preferable as A in the following formulae (12) to (14) can be referred to. Further, for specific examples of the donor group, donor groups preferable as D in the following formulae (12) to (14) can be referred to.

In the formula (1) and the formula (2), $X^1$ to $X^5$ represent N or C-R, but at least one is C-D. The number of N's in $X^1$ to $X^5$ is 0 to 4, and for example, a case where $X^1$, $X^3$ and $X^5$, $X^1$ and $X^3$, $X^1$ and $X^4$, $X^2$ and $X^3$, $X^1$ and $X^5$, $X^2$ and $X^4$, only $X^1$, only $X^2$, and only $X^3$ are N can be exemplified. The number of C-D's in $X^1$ to $X^5$ is 1 to 5, and is preferably 2 to 5. For example, a case where $X^1$, $X^2$, $X^3$, $X^4$ and $X^5$, $X^1$, $X^2$, $X^4$ and $X^5$, $X^1$, $X^2$, $X^3$ and $X^4$, $X^1$, $X^3$, $X^4$ and $X^5$, $X^1$, $X^3$ and $X^5$, $X^1$, $X^2$ and $X^5$, $X^1$, $X^2$ and $X^4$, $X^1$, $X^3$ and $X^4$, $X^1$ and $X^3$, $X^1$ and $X^4$, $X^2$ and $X^3$, $X^1$ and $X^5$, $X^2$ and $X^4$, only $X^1$, only $X^2$, only $X^3$ are C-D can be exemplified. At least one of $X^1$ to $X^5$ may be C-A. A mentioned herein represents an acceptor group. The number of C-A's in $X^1$ to $X^5$ is preferably 0 to 2, and more preferably 0 or 1. Preferable examples of A in C-A include a cyano group and an unsaturated heterocyclic aromatic group having a nitrogen atom. Further, each of $X^1$ to $X^5$ may be independently C-D or C-A.

When adjacent two of $X^1$ to $X^5$ represent C-R, two R's may be bonded to each other to form a ring structure. The ring structure formed by the bonding may be an aromatic ring or an adipose ring, and may be a ring having hetero atoms, further, the ring structure may be a condensed ring of two or more rings. The hetero atom mentioned herein is preferably one selected from the group consisting of a nitrogen atom, an oxygen atom, and a sulfur atom. Examples of the ring structure to be formed include a benzene ring, a naphthalene ring, a pyridine ring, a pyridazine ring, a pyrimidine ring, a pyrazine ring, a pyrrole ring, an imidazole ring, a pyrazole ring, an imidazoline ring, an oxazole ring, an isooxazole ring, a thiazole ring, an isothiazole ring, a cyclohexadiene ring, a cyclohexene ring, a cyclopentene ring, a cycloheptatriene ring, a cycloheptadiene ring, a cycloheptene ring, a furan ring, a thiophene ring, a naphthyridine ring, a quinoxaline ring, a quinoline ring, and the like. For example, a ring in which a plurality of rings are condensed, such as a phenanthrene ring or a triphenylene ring, may be formed.

It is preferable that the donor group D in the formula (1) and the formula (2), for example, is a group represented by the following formula (3).

Formula (3)

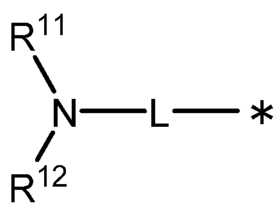

In the formula (3), each of $R^{11}$ and $R^{12}$ independently represents a substituted or unsubstituted alkyl group, a substituted or unsubstituted alkenyl group, a substituted or unsubstituted aryl group, or a substituted or unsubstituted heteroaryl group. $R^{11}$ and $R^{12}$ may be bonded to each other to form a ring structure. L represents a single bond, a substituted or unsubstituted arylene group, or a substituted or unsubstituted heteroarylene group. A substituent that can be introduced into an arylene group or a heteroarylene group of L may be the group represented by the formula (1) or the formula (2), or may be a group represented by the following formulae (3) to (6). Such groups represented by (1) to (6) may be introduced up to the largest number of substituents that can be introduced into L. Further, in a case where a plurality of groups represented by the formulae (1) to (6) are introduced, the substituents may be the same or different from each other. * represents a bond position to carbon atoms (C) constituting the ring skeleton of the ring in the formula (1) or the formula (2).

In the present specification, the "alkyl group" may take any of linear, branched, and cyclic shapes. Further, two or more types of the linear portion, the cyclic portion, and the branched portion may be mixed. The number of carbon atoms of the alkyl group may be, for example, 1 or more, 2 or more, or 4 or more. Further, the number of carbon atoms may be 30 or less, 20 or less, 10 or less, 6 or less, or 4 or less. Specific examples of the alkyl group include a methyl group, an ethyl group, a n-propyl group, an isopropyl group, a n-butyl group, an isobutyl group, a tert-butyl group, a n-pentyl group, an isopentyl group, a n-hexyl group, an isohexyl group, a 2-ethyl hexyl group, a n-heptyl group, an isoheptyl group, a n-octyl group, an isooctyl group, a n-nonyl group, an isononyl group, a n-decanyl group, an isodecanyl group, a cyclopentyl group, a cyclohexyl group, and a cycloheptyl group. The alkyl group as a substituent may be further substituted with an aryl group.

The "alkenyl group" may take any of linear, branched, and cyclic shapes. Further, two or more types of the linear

portion, the cyclic portion, and the branched portion may be mixed. The number of carbon atoms of the alkenyl group may be, for example, 2 or more, or 4 or more. Further, the number of carbon atoms may be 30 or less, 20 or less, 10 or less, 6 or less, or 4 or less. Specific examples of the alkenyl group include an ethenyl group, a n-propenyl group, an isopropenyl group, a n-butenyl group, an isobutenyl group, a n-pentenyl group, an isopentenyl group, a n-hexenyl group, an isohexenyl group, and a 2-ethyl hexenyl group. The alkenyl group as a substituent may be further substituted with a substituent.

The "aryl group" and the "heteroaryl group" may be monocycles, or may be condensed rings in which two or more rings are condensed. In the case of a condensed ring, the number of rings that are condensed is preferably 2 to 6, and for example, can be selected from 2 to 4. Specific examples of the ring include a benzene ring, a pyridine ring, a pyrimidine ring, a triazine ring, a naphthalene ring, an anthracene ring, a phenanthrene ring, a triphenylene ring, a quinoline ring, a pyrazine ring, a quinoxaline ring, and a naphthyridine ring. Specific examples of the aryl group or the heteroaryl group include a phenyl group, a 1-naphthyl group, a 2-naphthyl group, a 1-anthrasenyl group, a 2-anthrasenyl group, a 9-anthrasenyl group, a 2-pyridyl group, a 3-pyridyl group, and a 4-pyridyl group. The "arylene group" and the "heteroaryl group" may be those obtained by changing the valence in the descriptions for the aryl group and the heteroaryl group, from 1 to 2.

The substituent means a monovalent group that can be substituted with a hydrogen atom, and is not a concept including a group to be condensed. For the description and the preferable range of the substituent, the description and the preferable range of a substituent of the following formula (7) can be referred to.

It is preferable that the compound represented by the formula (3) is a compound represented by any of the following formulae (4) to (6).

Formula (4)

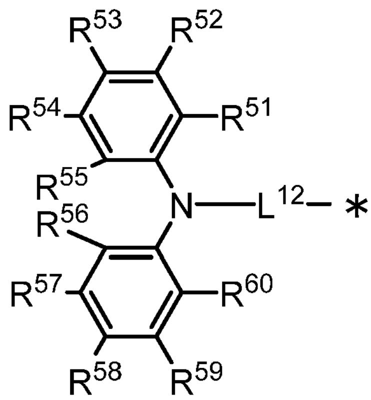

Formula (5)

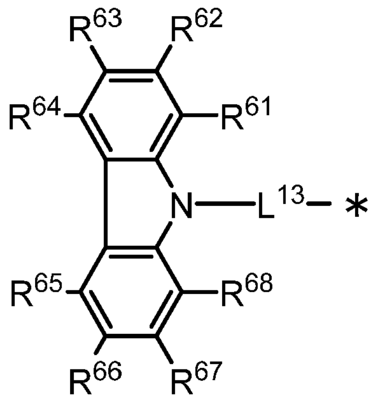

Formula (6)

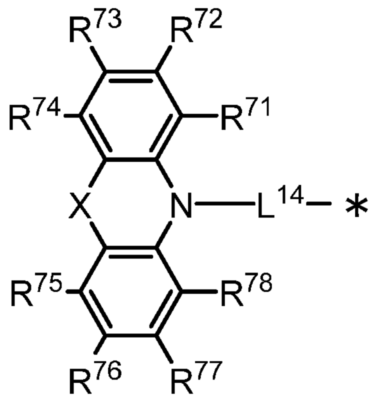

In the formulae (4) to (6), each of $R^{51}$ to $R^{60}$, $R^{61}$ to $R^{68}$, and $R^{71}$ to $R^{78}$ independently represents hydrogen atoms, deuterium atoms or substituents. For the description and the preferable range of the substituent mentioned herein, the description and the preferable range of the substituent in the following formula (7) can be referred to. It is preferable that each of $R^{51}$ to $R^{60}$, $R^{61}$ to $R^{68}$, and $R^{71}$ to $R^{78}$ is independently a group represented by any of the formulae (4) to (6). The number of substituents in the formulae (4) to (6) is not particularly limited. It is also preferable that all of the groups are unsubstituted (that is, hydrogen atoms or deuterium atoms). Further, in a case where there are two or more substituents in each of the formulae (4) to (6), such substituents may be the same or different from each other. In a case where there is a substituent in the formulae (4) to (6), as the substituent, any of $R^{52}$ to $R^{59}$ is preferable in the formula (4), any of $R^{62}$ to $R^{67}$ is preferable in the formula (5), and any of $R^{72}$ to $R^{77}$ is preferable in the formula (6).

In the formula (6), X represents a divalent oxygen atom, a sulfur atom, a substituted or unsubstituted nitrogen atom, a substituted or unsubstituted carbon atom, a substituted or unsubstituted silicon atom, or a carbonyl group, in which the length of a linking chain is one atom, or a divalent substituted or unsubstituted ethylene group, a substituted or unsubstituted vinylene group, a substituted or unsubstituted o-arylene group, or a substituted or unsubstituted o-heteroarylene group, in which the length of a bonding chain is two atoms. For the specific examples and the preferable range of substituent, the description on the substituent in the formula (1) and the formula (2) can be referred to.

In the formulae (4) to (6), $L^{12}$ to $L^{14}$ represent single bonds, substituted or unsubstituted arylene groups, or substituted or unsubstituted heteroarylene groups. For the description and the preferable range of the arylene group or the heteroarylene group represented by $L^{12}$ to $L^{14}$, the description and the preferable range of the arylene group or the heteroarylene group represented by L can be referred to. It is preferable that $L^{12}$ to $L^{14}$ are single bonds, or substituted or unsubstituted arylene groups. The substituent of the arylene group or the heteroarylene group mentioned herein may be the groups represented by the formulae (1) to (6). The groups represented by the formulae (1) to (6) may be introduced up to the largest number of substituents that can be introduced into $L^{11}$ to $L^{14}$. Further, in a case where a plurality of groups represented by the formulae (1) to (6) are introduced, the substituent may be may be the same or different from each other. * represents a bond position to carbon atoms (C) constituting the ring skeleton of the ring in the formula (1) or the formula (2).

In the formulae (4) to (6), $R^{51}$ and $R^{52}$, $R^{52}$ and $R^{53}$, $R^{53}$ and $R^{54}$, $R^{54}$ and $R^{55}$, $R^{55}$ and $R^{56}$ , $R^{56}$ and $R^{57}$, $R^{57}$ and $R^{58}$, $R^{58}$ and $R^{59}$, $R^{59}$ and $R^{60}$, $R^{61}$ and $R^{62}$, $R^{62}$ and $R^{63}$, $R^{63}$ and $R^{64}$, $R^{65}$ and $R^{66}$, $R^{66}$ and $R^{67}$, $R^{67}$ and $R^{68}$, $R^{71}$ and $R^{72}$, $R^{72}$ and $R^{73}$, $R^{73}$ and $R^{74}$, $R^{75}$ and $R^{76}$, $R^{76}$ and $R^{77}$, and $R^{77}$ and $R^{78}$ may be bonded to each other to form ring structures. For the description and the preferable examples of the ring structure, the description and the preferable examples of the ring structure in $X^1$ to $X^5$ in the formula (1) and the formula (2) can be referred to.

Among the ring structures, a structure is preferable in which a substituted or unsubstituted benzofuran ring, a substituted or unsubstituted benzothiophene ring, a substituted or unsubstituted indole ring, a substituted or unsubstituted indene ring, or a substituted or unsubstituted silaindene ring is condensed with at least one benzene ring of the formulae (4) to (6). Groups represented by the following formulae (5a) to (5f), which are condensed with the formula (5), are more preferable.

Formula (5a)

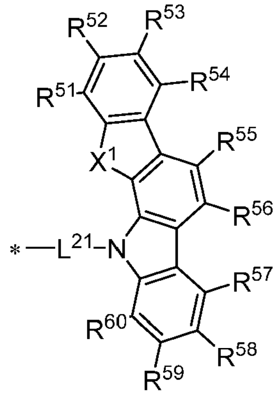

Formula (5b)

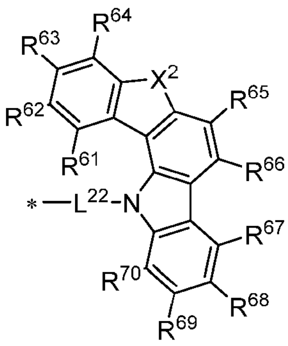

Formula (5c)

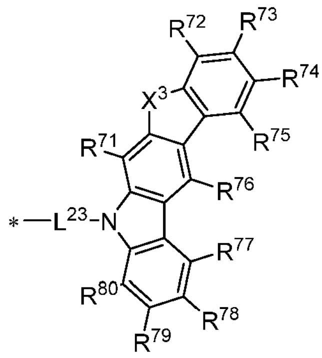

Formula (5d)

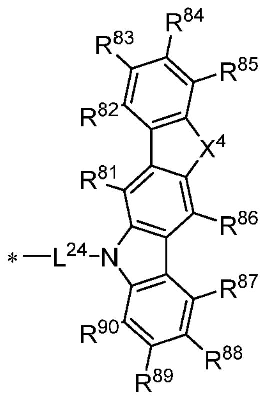

Formula (5e)

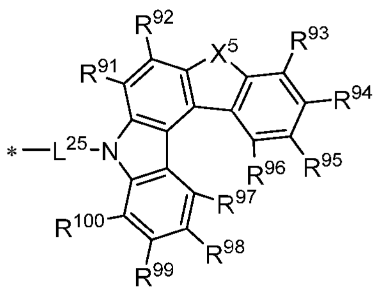

Formula (5f)

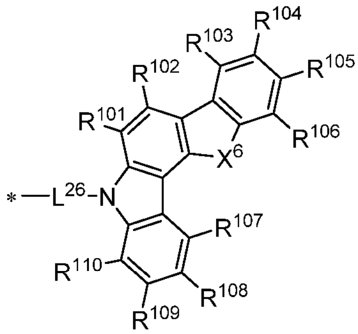

In the formulae (5a) to (5f), $L^{11}$ and $L^{21}$ to $L^{26}$ represent single bonds or divalent linking groups. For the description and the preferable range of $L^{11}$ and $L^{21}$ to $L^{26}$, the description and the preferable range of $L^2$ can be referred to.

In the formulae (5a) to (5f), each of $R^{41}$ to $R^{110}$ independently represents a hydrogen atom or a substituent. $R^{41}$ and $R^{42}$, $R^{42}$ and $R^{43}$, $R^{43}$ and $R^{44}$, $R^{44}$ and $R^{45}$, $R^{45}$ and $R^{46}$, $R^{46}$ and $R^{47}$, $R^{47}$ and $R^{48}$, $R^{51}$ and $R^{52}$, $R^{52}$ and $R^{53}$, $R^{53}$ and $R^{54}$, $R^{54}$ and $R^{55}$, $R^{55}$ and $R^{56}$, $R^{56}$ and $R^{57}$, $R^{57}$ and $R^{58}$, $R^{58}$ and $R^{59}$, $R^{59}$ and $R^{60}$, $R^{61}$ and $R^{62}$, $R^{62}$ and $R^{63}$, $R^{63}$ and $R^{64}$, $R^{65}$ and $R^{66}$, $R^{66}$ and $R^{67}$, $R^{67}$ and $R^{68}$, $R^{68}$ and $R^{69}$, $R^{69}$ and $R^{70}$, $R^{72}$ and $R^{73}$, $R^{73}$ and $R^{74}$, $R^{74}$ and $R^{75}$, $R^{75}$ and $R^{76}$, $R^{76}$ and $R^{77}$, $R^{77}$ and $R^{78}$, $R^{78}$ and $R^{79}$, $R^{79}$ and $R^{80}$, $R^{81}$ and $R^{82}$, $R^{82}$ and $R^{83}$, $R^{83}$ and $R^{84}$, $R^{84}$ and $R^{85}$, $R^{86}$ and $R^{87}$, $R^{87}$ and $R^{88}$, $R^{88}$ and $R^{89}$, $R^{89}$ and $R^{90}$, $R^{91}$ and $R^{92}$, $R^{93}$ and $R^{94}$, $R^{94}$ and $R^{95}$, $R^{95}$ and $R^{96}$, $R^{96}$ and $R^{97}$, $R^{97}$ and $R^{98}$, $R^{99}$ and $R^{100}$, $R^{101}$ and $R^{102}$, $R^{102}$ and $R^{103}$, $R^{103}$ and $R^{104}$, $R^{104}$ and $R^{105}$, $R^{105}$ and $R^{106}$, $R^{107}$ and $R^{108}$, $R^{108}$ and $R^{109}$, and $R^{109}$ and $R^{110}$ may be bonded to each other to form ring structures. The ring structure to be formed by the bonding may be an aromatic ring or an adipose ring, and may be a ring having hetero atoms, further, the ring structure may be a condensed ring of two or more rings. The hetero atom mentioned herein is preferably one selected from the group consisting of a nitrogen atom, an oxygen atom, and a sulfur atom. Examples of the ring structure to be formed include a benzene ring, a naphthalene ring, a pyridine ring, a pyridazine ring, a pyrimidine ring, a pyrazine ring, a pyrrole ring, an imidazole ring, a pyrazole ring, an imidazoline ring, an oxazole ring, an isooxazole ring, a thiazole ring, an isothiazole ring, a cyclohexadiene ring, a cyclohexene ring, a cyclopentene ring, a cycloheptatriene ring, a cycloheptadiene ring, a cycloheptene ring, a furan ring, a thiophene ring, a naphthyridine ring, a quinoxaline ring, a quinoline ring, and the like. For example, a ring in which a plurality of rings are condensed, such as a phenanthrene ring or a triphenylene ring, may be formed. The number of rings included in the group represented by the formula (6) may be selected from a range of 3 to 5, or may be selected from a range of 5 to 7. The number of rings included in the groups represented by the formulae (5a) to (5f) may be selected from a range of 5 to 7, or may be 5.

Examples of the substituent that may be possessed by $R^{41}$ to $R^{110}$ include a group of a substituent group B, and preferably an unsubstituted alkyl group having 1 to 10 carbon atoms, or an aryl group having 6 to 10 carbon atoms that may be substituted with an unsubstituted alkyl group having 1 to 10 carbon atoms. In a preferred aspect of the invention, $R^{41}$ to $R^{110}$ are hydrogen atoms or unsubstituted alkyl groups having 1 to 10 carbon atoms. In a preferred aspect of the invention, $R^{41}$ to $R^{110}$ are hydrogen atoms or unsubstituted aryl groups having 6 to 10 carbon atoms. In a preferred aspect of the invention, all of $R^{41}$ to $R^{110}$ are hydrogen atoms.

In the formulae (5a) to (5f), each of carbon atoms (ring skeleton forming carbon atoms) to which $R^{41}$ to $R^{110}$ are bonded may be independently substituted with a nitrogen atom. That is, in the formulae (5a) to (5f), each of C-$R^{41}$ to C-$R^{110}$ may be independently substituted with N. In the groups represented by the formulae (5a) to (5f), the number of nitrogen atoms that are substituted is preferably 0 to 4, and more preferably 1 to 2. In one aspect of the invention, the number of nitrogen atoms that are substituted is 0. Further, in a case where two or more nitrogen atoms are substituted, it is preferable that the number of nitrogen atoms that are substituted in one ring is 1.

In the formulae (5a) to (5f), $X^1$ to $X^6$ represent oxygen atoms, sulfur atoms or N-R. In one aspect of the invention, $X^1$ to $X^6$ are oxygen atoms. In one aspect of the invention, $X^1$ to $X^6$ are sulfur atoms. In one aspect of the invention, $X^1$ to $X^6$ are N-R. R represents a hydrogen atom or a substituent, and is preferably a substituent. As the substituent, a substituent selected from a substituent group A can be exemplified. For example, a phenyl group that is substituted with one group selected from the group consisting of an unsubstituted phenyl group, an alkyl group and an aryl group, or a group formed by combining two or more thereof can be preferably adopted.

In the formulae (5a) to (5f), * represents a bond position.

In the invention, a compound that is represented by the following formula (7) and emits delayed fluorescence can be particularly preferably used as a delayed fluorescence material. In a preferable embodiment of the invention, as the second organic compound, a compound represented by the formula (7) can be adopted.

Formula (7)

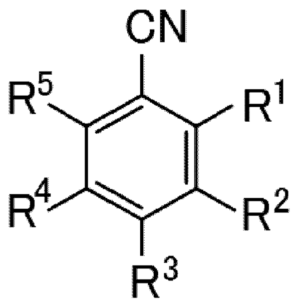

In the formula (7), 0 to 4 of $R^1$ to $R^5$ represent cyano groups, at least one of $R^1$ to $R^5$ represents a substituted amino group, and the rest of $R^1$ to $R^5$ represent hydrogen atoms, deuterium atoms, or substituents other than the cyano group and the substituted amino group.

The substituted amino group mentioned herein is preferably a substituted or unsubstituted diaryl amino group, and two aryl groups constituting the substituted or unsubstituted diaryl amino group may be linked to each other. The aryl groups may be linked to each other via a single bond (in this case, a carbazole ring is formed), or via a linking group such as -O-, -S-, -N($R^6$)-, -C($R^7$)($R^8$)-, and -Si($R^9$)($R^{10}$). Here, $R^6$ to $R^{10}$ represent hydrogen atoms, deuterium atoms or substituents, and $R^7$ and $R^8$, and $R^9$ and $R^{10}$ may be linked to each other to form ring structures.

The substituted amino group may be any of $R^1$ to $R^5$, and for example, $R^1$ and $R^2$, $R^1$ and $R^3$, $R^1$ and $R^4$, $R^1$ and $R^5$, $R^2$ and $R^3$, $R^2$ and $R^4$, $R^1$, $R^2$, and $R^3$, $R^1$, $R^2$, and $R^4$, $R^1$, $R^2$, and $R^5$, $R^1$, $R^3$, and $R^4$, $R^1$, $R^3$, and $R^5$, $R^2$, $R^3$, and $R^4$, $R^1$, $R^2$, $R^3$, and $R^4$, $R^1$, $R^2$, $R^3$, and $R^5$, $R^1$, $R^2$, $R^4$, and $R^5$, and $R^1$, $R^2$, $R^3$, $R^4$, and $R^5$ can be substituted amino groups. The cyano group may be any of $R^1$ to $R^5$, and for example $R^1$, $R^2$, $R^3$, $R^1$, and $R^2$, $R^1$ and $R^3$, $R^1$ and $R^4$, $R^1$ and $R^5$, $R^2$ and $R^3$, $R^2$ and $R^4$, $R^1$, $R^2$, and $R^3$, $R^1$, $R^2$, and $R^4$, $R^1$, $R^2$, and $R^5$, $R^1$, $R^3$, and $R^4$, $R^1$, $R^3$, and $R^5$, and $R^2$, $R^3$, and $R^4$ may be cyano groups.

$R^1$ to $R^5$, which are neither the cyano group nor the substituted amino group, represent hydrogen atoms, deuterium atoms or substituents. Examples of the substituent mentioned herein include the substituent group A including a hydroxy group, a halogen atom (e.g., a fluorine atom, a chlorine atom, a bromine atom, and an iodine atom), an alkyl group (e.g., 1 to 40 carbon atoms), an alkoxy group (e.g., 1 to 40 carbon atoms), an alkylthio group (e.g., 1 to 40 carbon atoms), an aryl group (e.g., 6 to 30 carbon atoms), an aryl oxy group (e.g., 6 to 30 carbon atoms), an arylthio group (e.g., 6 to 30 carbon atoms), a heteroaryl group (e.g., 5 to 30 ring skeleton forming atoms), a heteroaryl oxy group (e.g., 5 to 30 ring skeleton forming atoms), a heteroarylthio group (e.g., 5 to 30 ring skeleton forming atoms), an acyl group (e.g., 1 to 40 carbon atoms), an alkenyl group (e.g., 1 to 40 carbon atoms), an alkynyl group (e.g., 1 to 40 carbon atoms), an alkoxycarbonyl group (e.g., 1 to 40 carbon atoms), an aryl oxycarbonyl group (e.g., 1 to 40 carbon atoms), a heteroaryl oxycarbonyl group (e.g., 1 to 40 carbon atoms), a silyl group (e.g., a trialkyl silyl group having 1 to 40 carbon atoms), a nitro group, and groups in which the groups exemplified herein are further substituted with one or more groups exemplified herein. Preferable examples of the substituent when the aryl group of the diaryl amino group is substituted also include the substituent of the substituent group A, further include a cyano group and a substituted amino group.

For the specific examples of the compound group and the compound included in the formula (7), WO2013/154064, paragraphs 0008 to 0048, WO2015/080183, paragraphs 0009 to 0030, WO2015/129715, paragraphs

0006 to 0019, JP 2017-119663 A, paragraphs 0013 to 0025, and JP 2017-119664 A, paragraphs 0013 to 0026, can be referred to.

Further, a compound that is represented by the following formula (8) and emits delayed fluorescence can be also particularly preferably used as the delayed fluorescence material of the invention. In a preferable embodiment of the invention, as the second organic compound, a compound represented by the formula (8) can be adopted.

Formula (8)

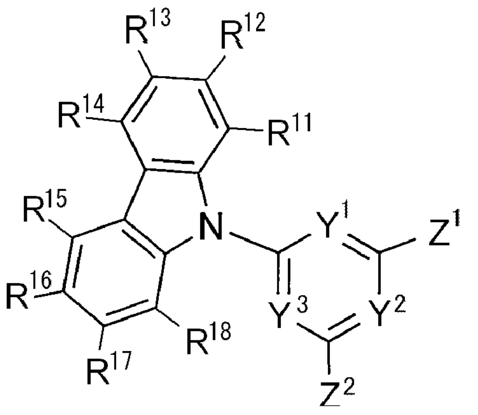

In the formula (8), any two of $Y^1$, $Y^2$ and $Y^3$ represent nitrogen atoms, and the rest one represents a methyl group, or all of $Y^1$, $Y^2$ and $Y^3$ represent nitrogen atoms. Each of $Z^1$ and $Z^2$ independently represents a hydrogen atom, a deuterium atom, or a substituent. Each of $R^{11}$ to $R^{18}$ independently represents a hydrogen atom, a deuterium atom, or a substituent, and it is preferable that at least one of $R^{11}$ to $R^{18}$ is a substituted or unsubstituted aryl amino group, or a substituted or unsubstituted carbazolyl group. The benzene ring constituting the aryl amino group and the benzene ring constituting the carbazolyl group are combined with $R^{11}$ to $R^{18}$, respectively, to form a single bond or a linking group. Further, the compound represented by the formula (8) has at least two carbazole structures in the molecules. Examples of the substituent that may be possessed by $Z^1$ and $Z^2$ include the substituent of the substituent group A. Further, specific examples of the substituents that may be possessed by $R^{11}$ to $R^{18}$, the aryl amino group, and the carbazolyl group include the substituent of the substituent group A, a cyano group, a substituted aryl amino group, and a substituted alkyl amino group. $R^{11}$ and $R^{12}$, $R^{12}$ and $R^{13}$, $R^{13}$ and $R^{14}$, $R^{15}$ and $R^{16}$, $R^{16}$ and $R^{17}$, and $R^{17}$ and $R^{18}$ may be bonded to each other to form ring structures.

Among the compounds represented by the formula (8), a compound represented by the following formula (9) is particularly useful.

Formula (9)

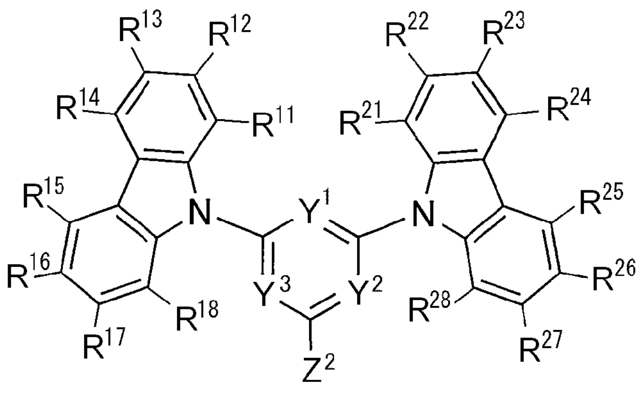

In the formula (9), any two of $Y^1$, $Y^2$ and $Y^3$ represent nitrogen atoms, and the rest one represents a methyl group, or all of $Y^1$, $Y^2$ and $Y^3$ represent nitrogen atoms. $Z^2$ represents a hydrogen atom, a deuterium atom, or a substituent. Each of $R^{11}$ to $R^{18}$ and $R^{21}$ to $R^{28}$ independently represents a hydrogen atom, a deuterium atom, or a substituent. It is preferable that at least one of $R^{11}$ to $R^{18}$, and/or, at least one of $R^{21}$ to $R^{28}$ represent a substituted or unsubstituted aryl amino group, or a substituted or unsubstituted carbazolyl group. The benzene ring constituting the aryl amino group and the benzene ring constituting the carbazolyl group may be combined with $R^{11}$ to $R^{18}$ or $R^{21}$ to $R^{28}$, respectively, to form a single bond or a

linking group. Examples of the substituent that may be possessed by $Z^2$ include the substituent of the substituent group A. Further, specific examples of the substituents that may be possessed by $R^{11}$ to $R^{18}$, $R^{21}$ to $R^{28}$, the aryl amino group, and the carbazolyl group include the substituent of the substituent group A, a cyano group, a substituted aryl amino group, and a substituted alkyl amino group. $R^{11}$ and $R^{12}$, $R^{12}$ and $R^{13}$, $R^{13}$ and $R^{14}$, $R^{15}$ and $R^{16}$, $R^{16}$ and $R^{17}$, $R^{17}$ and $R^{18}$, $R^{21}$ and $R^{22}$, $R^{22}$ and $R^{23}$, $R^{23}$ and $R^{24}$, $R^{25}$ and $R^{26}$, $R^{26}$ and $R^{27}$, and $R^{27}$ and $R^{28}$ may be bonded to each other to form ring structures.

For the specific examples of the compound group and the compound included in the formula (9), compounds, as described in WO2013/081088, paragraphs 0020 to 0062, or Appl. Phys. Let, 98, 083302(2011), can be referred to.

Further, a compound that is represented by the following formula (10) and emits delayed fluorescence can also be particularly preferably used as the delayed fluorescence material of the invention.

Formula (10)

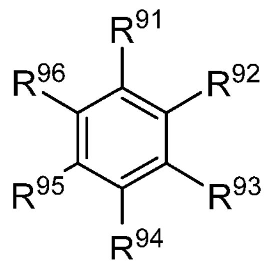

In the formula (10), each of $R^{91}$ to $R^{96}$ independently represents a hydrogen atom, a deuterium atom, a donor group, or an acceptor group, in which at least one thereof is the donor group, and at least two thereof are the acceptor groups. Substitution positions of at least two acceptor groups are not particularly limited, but it is preferable to include two acceptor groups in a meta-position relationship. For example, when $R^{91}$ is a donor group, a structure in which at least $R^{92}$ and $R^{94}$ are acceptor groups, or a structure in which at least $R^{92}$ and $R^{96}$ are acceptor groups can be preferably exemplified. All of the acceptor groups present in the molecules may be the same or different from each other, but for example, a structure in which all of the acceptor groups are the same can be selected. The number of acceptor groups is preferably 2 to 3, and for example, 2 can be selected. Further, there may be two or more donor groups, and in this case, all of the donor groups may be the same or different from each other. The number of donor groups is preferably 1 to 3, and for example only 1, or 2. For the descriptions and the preferable ranges of the donor group and the acceptor group, the descriptions and the preferable ranges of D and Z in the formula (1) can be referred to. In particular, in the formula (10), it is preferable that the donor group is represented by the formula (3), and it is preferable that the acceptor group is a cyano group or is represented by the following formula (11).

Formula (11)

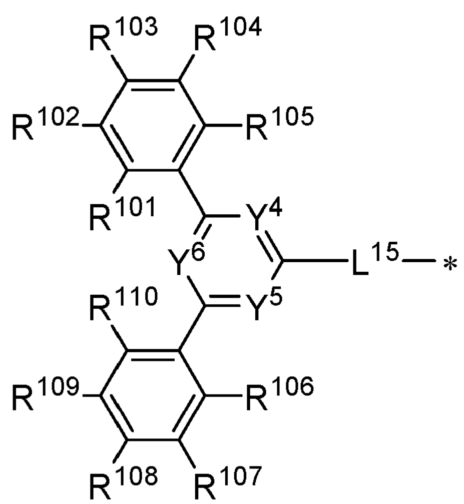

In the formula (11), $Y^4$ to $Y^6$ represent nitrogen atoms or represent methyl groups, but at least one thereof represents a nitrogen atom, and preferably, all thereof represent nitrogen atoms. Each of $R^{101}$ to $R^{110}$ independently represents a hydrogen atom, a deuterium atom, or a substituent, but it is preferable that at least one is an alkyl group. For

the description and the preferable range of the substituent mentioned herein, the description and the preferable range of the substituent in the formula (7) can be referred to. $L^{15}$ represents a single bond or a linking group, and the description and the preferable range of L in the formula (3) can be referred to. In a preferred aspect of the invention, $L^{15}$ in the formula (11) is a single bond. * represents a bond position to carbon atoms (C) constituting the ring skeleton of the ring in the formula (10).

In another preferable embodiment of the invention, as the second organic compound, a compound represented by the following formula (12) can be adopted. The compound represented by the formula (12) includes a compound represented by the following formula (12a).

Formula (12)

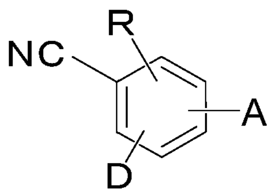

Formula (12a)

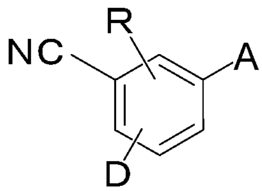

Among the compounds represented by the formula (12), a compound represented by the following formula (13) or a compound represented by the following formula (14) is particularly preferable.

Formula (13)

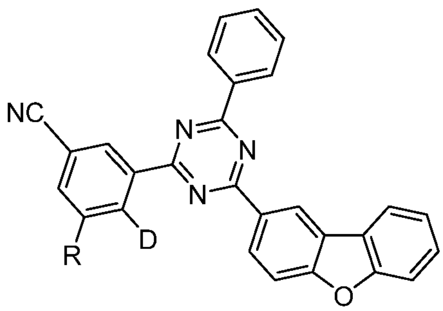

Formula (14)

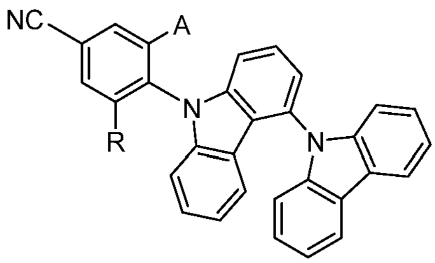

In another preferable embodiment of the invention, as the second organic compound, a compound represented by the following formula (15) can be adopted.

Formula (15)

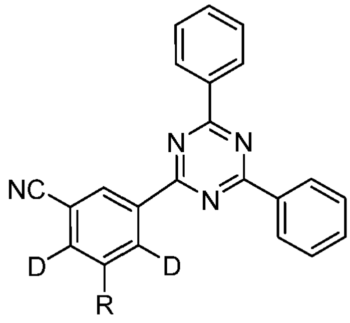

In the formulae (12) to (15), D represents a donor group, A represents an acceptor group, and R represents a hydrogen atom, a deuterium atom, or a substituent. Two D's in the formula (15) may be the same or different from each other. For the descriptions and the preferable ranges of the donor group and the acceptor group, corresponding description and preferable range in the formula (1) can be referred to. As the substituent of R, an alkyl group, or an aryl group that may be substituted with one group selected from the group consisting of an alkyl group and an aryl group, or a group formed by combining two or more thereof can be exemplified.

Specific examples of the donor group preferable as D in the formulae (12) to (15) will be given below. In the following specific examples, * represents a bond position, and "D" represents a deuterium atom. In the following specific examples, a hydrogen atom may be, for example, substituted with an alkyl group. Further, a substituted or unsubstituted benzene ring may be further condensed.

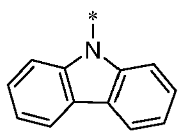
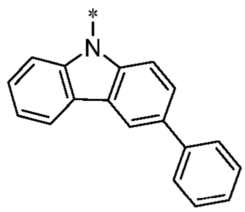
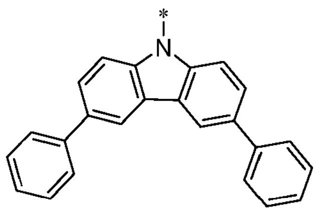
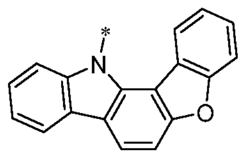

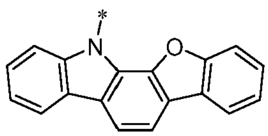
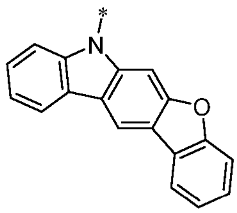
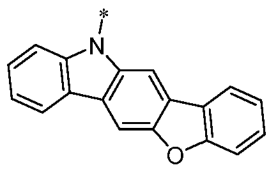
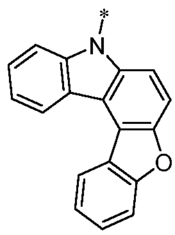

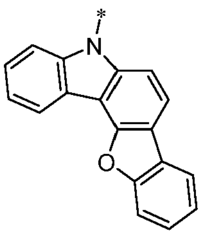
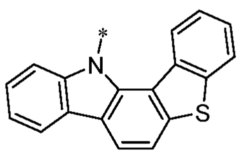
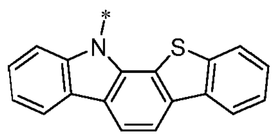
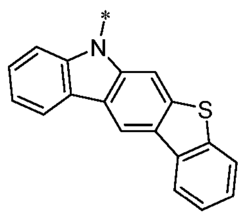

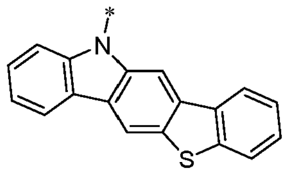
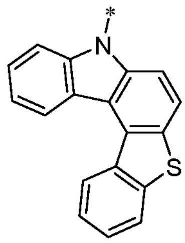
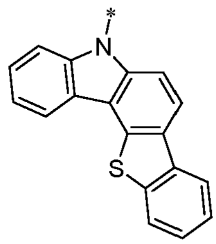
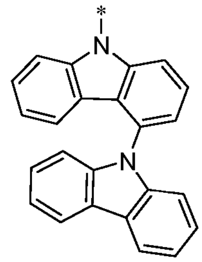
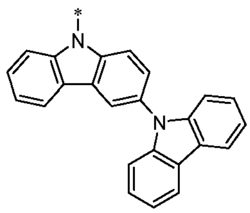
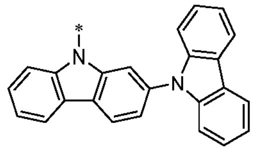
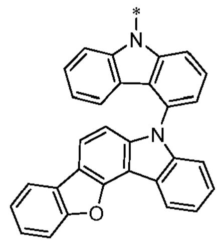
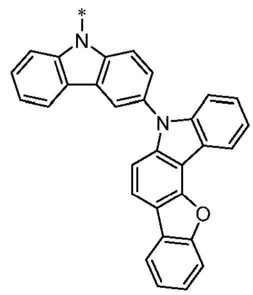
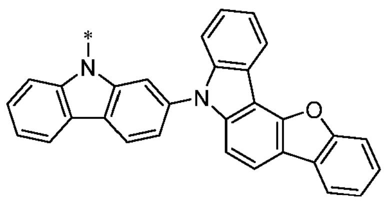
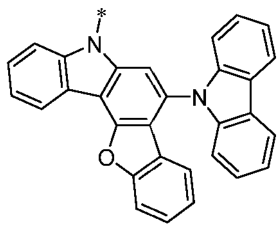
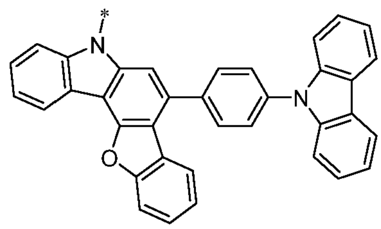
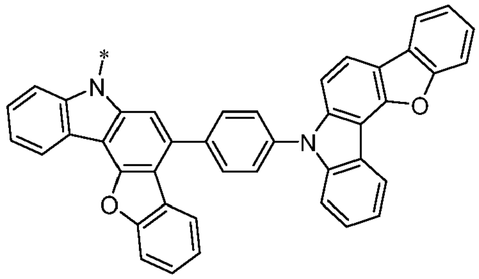
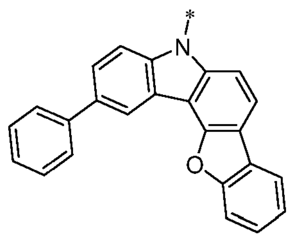
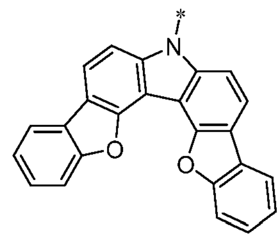
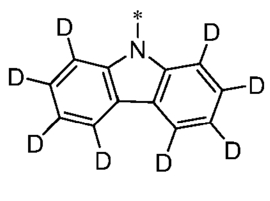
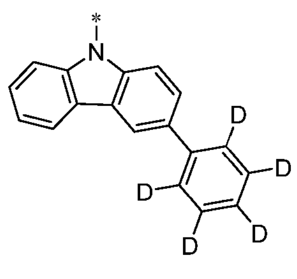
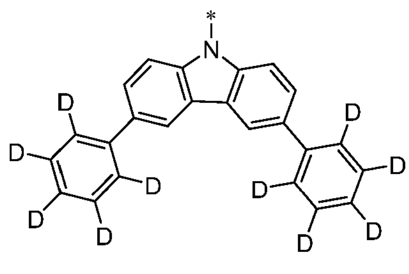

Specific examples of the acceptor group preferable as A in the formulae (12) to (14) will be given below. In the following specific examples, * represents a bond position, and "D" represents deuterium.

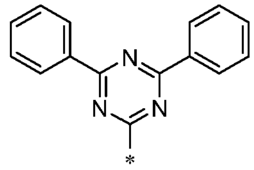
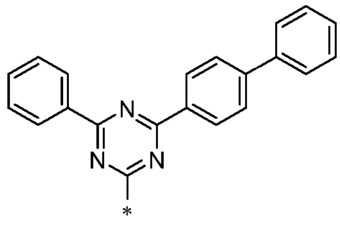
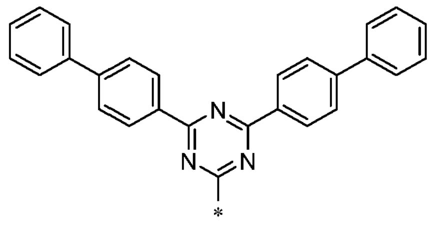

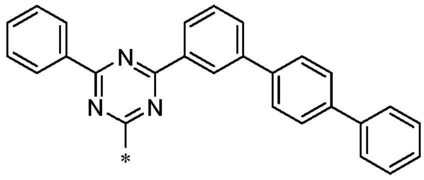
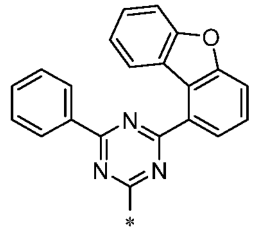
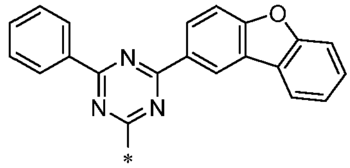

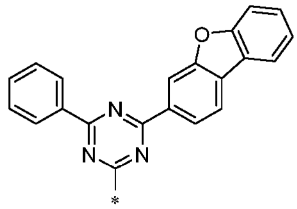
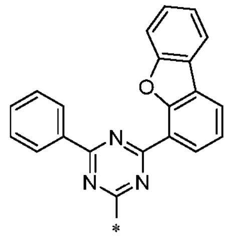
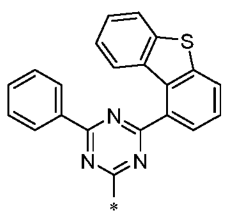
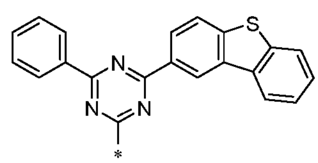

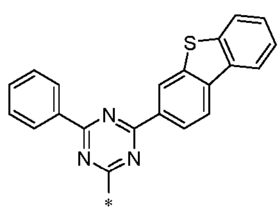
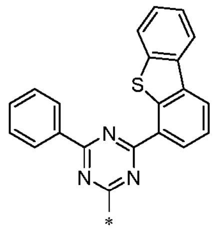
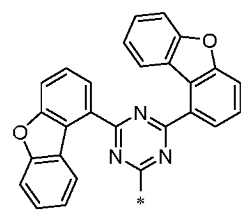
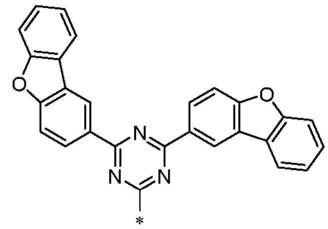

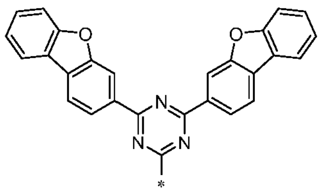
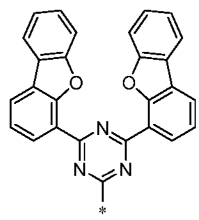
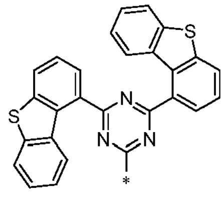
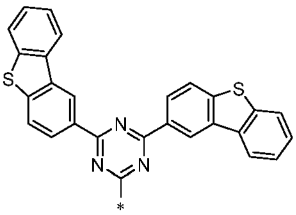

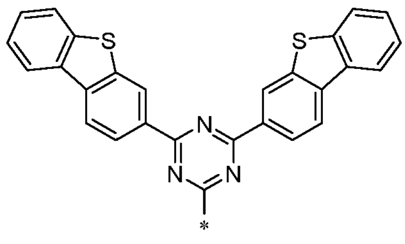
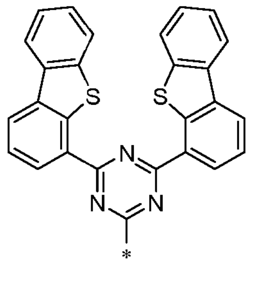
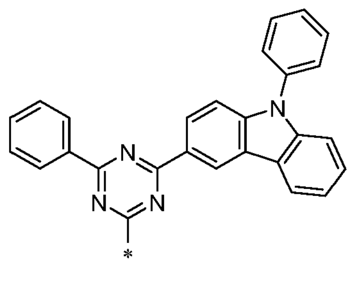

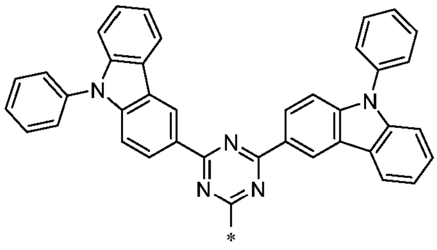

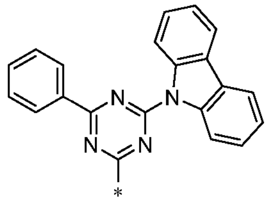

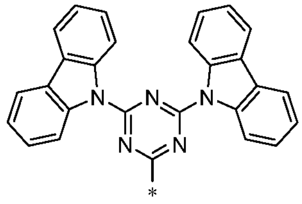

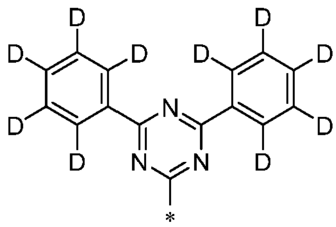

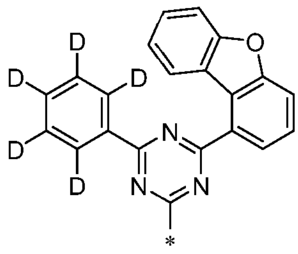

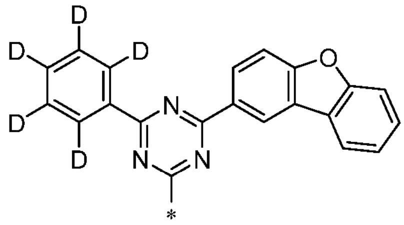

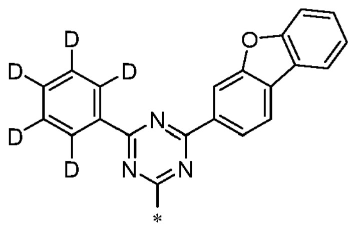

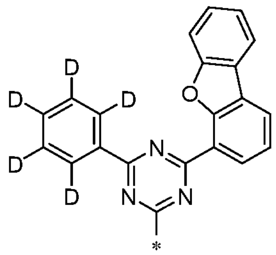

Examples preferable as R in the formulae (12) to (15) will be given below. In the following specific examples, * represents a bond position, and "D" represents deuterium. *-H

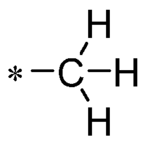

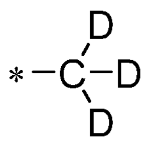

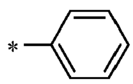

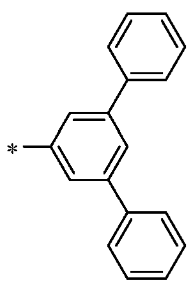

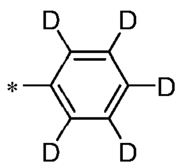

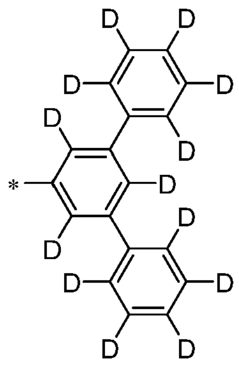

Among the compounds represented by the formula (15), a compound represented by the following formula (15a) is particularly preferable.

Formula (15a)

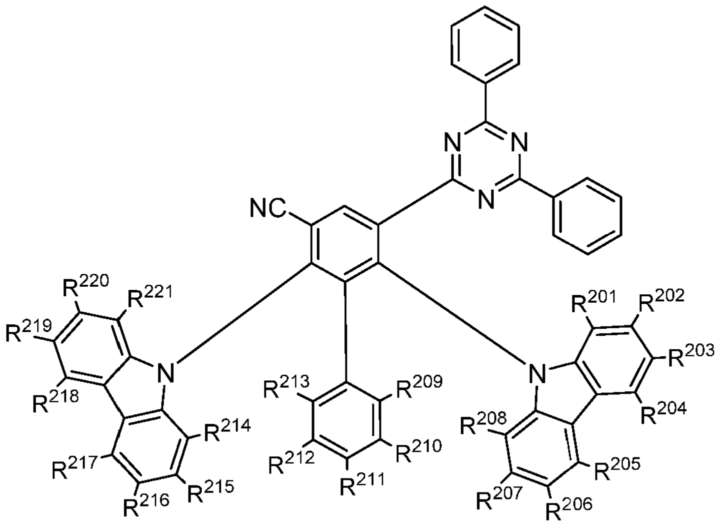

In the formula (15a), each of $R^{201}$ to $R^{221}$ independently represents a hydrogen atom or a substituent, and preferably represents a hydrogen atom, an alkyl group, an aryl group, or a group to which an alkyl group and an aryl group are bonded. At least one set of $R^{201}$ and $R^{202}$, $R^{202}$ and $R^{203}$, $R^{203}$ and $R^{204}$, $R^{205}$ and $R^{206}$, $R^{206}$ and $R^{207}$, $R^{207}$ and $R^{208}$, $R^{214}$ and $R^{215}$, $R^{215}$ and $R^{216}$, $R^{216}$ and $R^{217}$, $R^{218}$ and $R^{219}$, $R^{219}$ and $R^{220}$, and $R^{220}$ and $R^{221}$ is bonded to each other to form a benzofuro structure or a benzothieno structure. Preferably, one set or two sets of $R^{201}$ and $R^{202}$, $R^{202}$ and $R^{203}$, $R^{203}$ and $R^{204}$, $R^{205}$ and $R^{206}$, $R^{206}$ and $R^{207}$, and $R^{207}$ and $R^{208}$, and one set or two sets of $R^{214}$ and $R^{215}$, $R^{215}$ and $R^{216}$, $R^{216}$ and $R^{217}$, $R^{218}$ and $R^{219}$, $R^{219}$ and $R^{220}$, and $R^{220}$ and $R^{221}$ are bonded to each other to form benzofuro structures or benzothieno structures. Further preferably, $R^{203}$ and $R^{204}$ are bonded to each other to form a benzofuro structure or a benzothieno structure, and still further preferably $R^{203}$ and $R^{204}$ and $R^{216}$ and $R^{217}$ are bonded to each other to form benzofuro structures or benzothieno structures. Particularly preferably, $R^{203}$ and $R^{204}$ and $R^{216}$ and $R^{217}$ are bonded to each other to form benzofuro structures or benzothieno structures, and $R^{206}$ and $R^{219}$ are substituted or unsubstituted aryl groups (preferably substituted or unsubstituted phenyl groups, and more preferably unsubstituted phenyl groups).

A part or all of hydrogen atoms in the formula (15a) may be substituted with a deuterium atom. For example, a part or all of hydrogen atoms of two phenyl groups bonded to a triazinyl group may be substituted with a deuterium atom. Further, a part or all of hydrogen atoms bonded to two carbazolyl groups may be substituted with a deuterium atom. Further, $R^{209}$ to $R^{213}$ may be deuterium atoms.

Hereinafter, preferable compounds that can be used as the second organic compound will be given. In the structural formulae of the following exemplary compounds, t-Bu represents a tertiary butyl group.

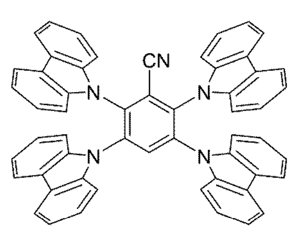

**T1**

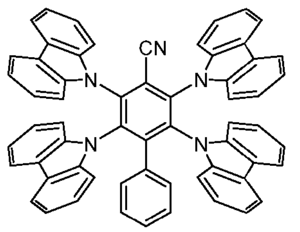

**T2**

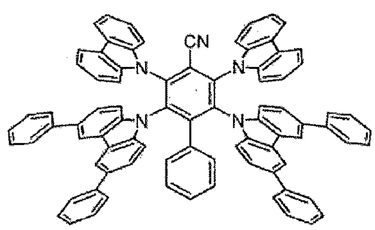

**T3**

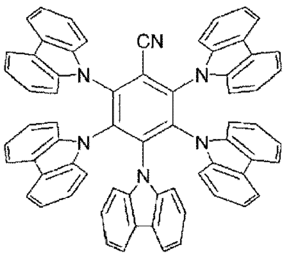

**T4**

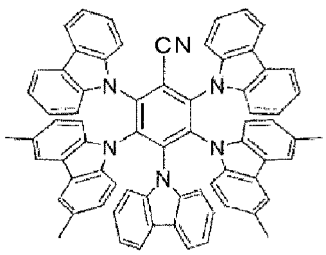

**T5**

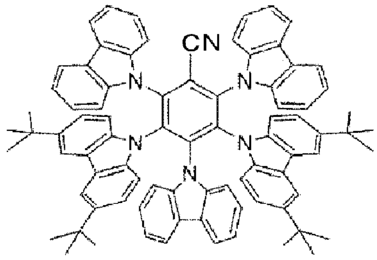

**T6**

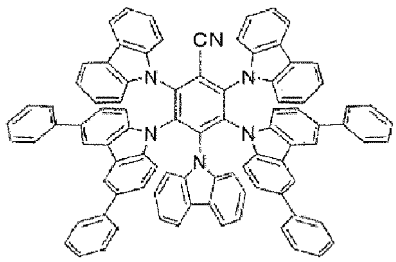

**T7**

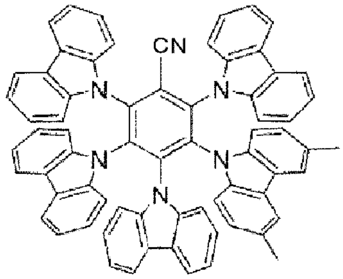

**T8**

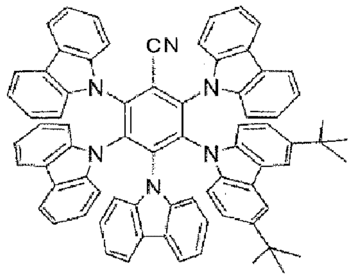

**T9**

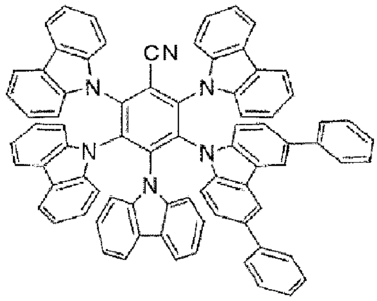

**T10**

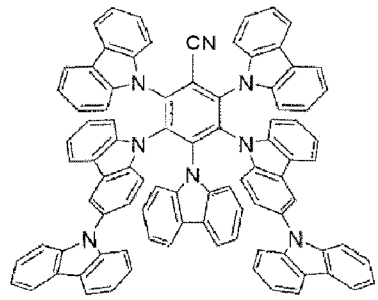

**T11**

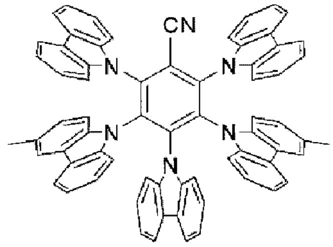

**T12**

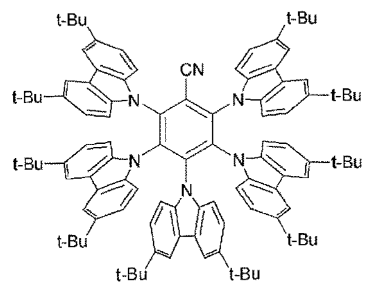

**T13**

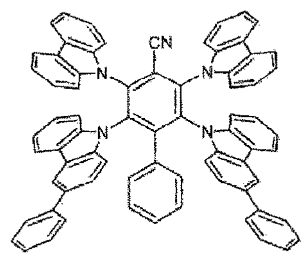

**T14**

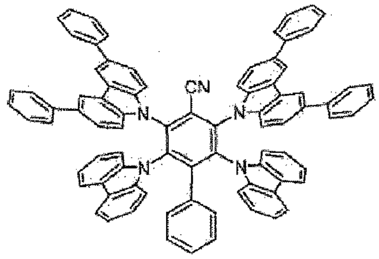

**T15**

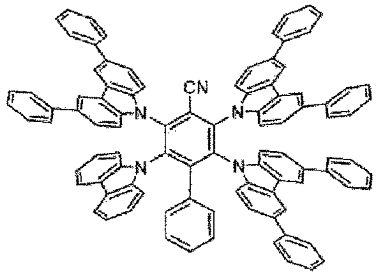

**T16**

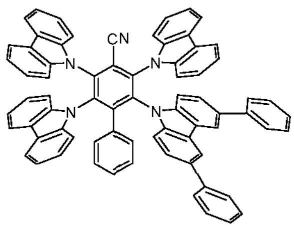

**T17**

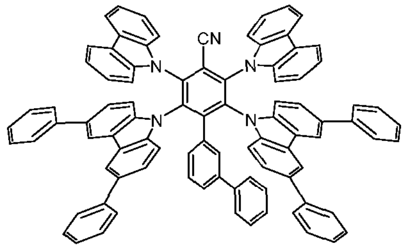

**T18**

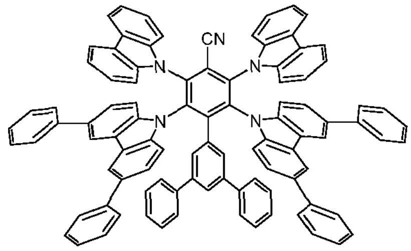

**T19**

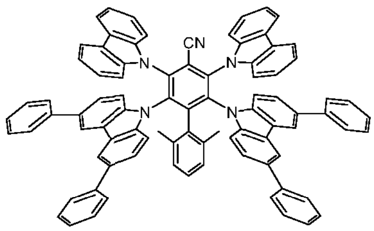

**T20**

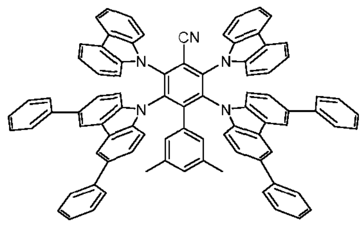

**T21**

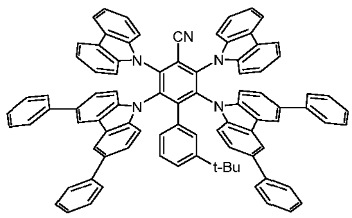

**T22**

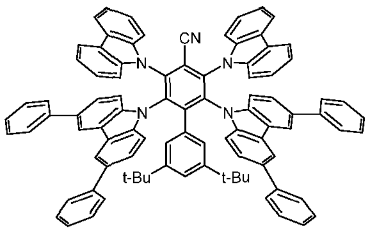

**T23**

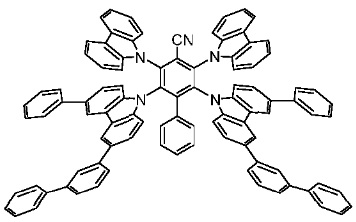

**T24**

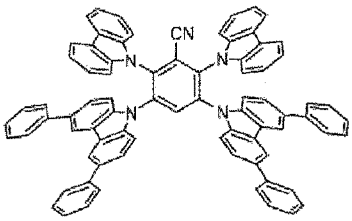

**T25**

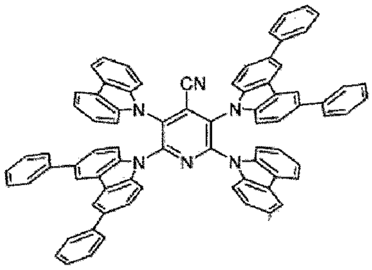

**T26**

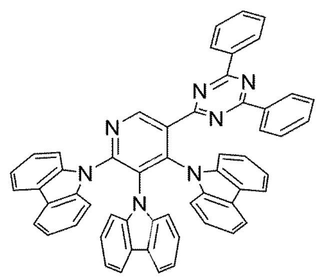

**T27**

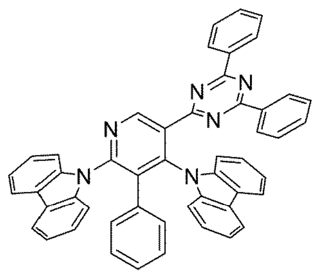

**T28**

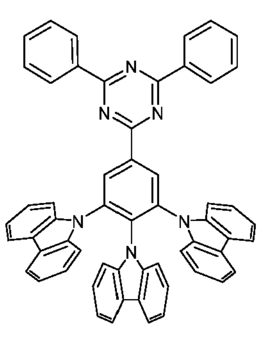
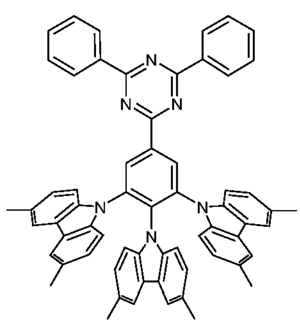
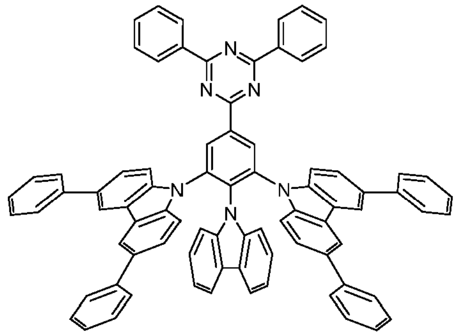

**T29** **T30** **T31**

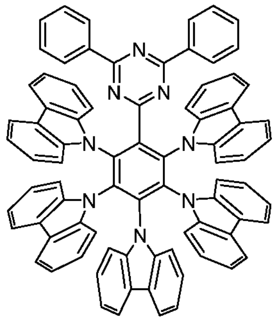
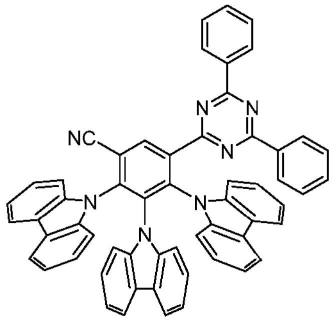
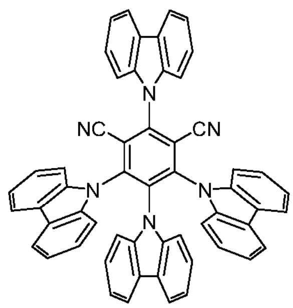

**T32** **T33** **T34**

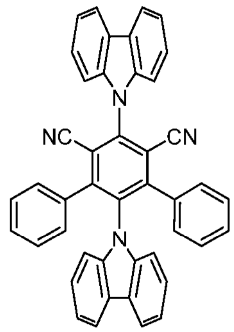
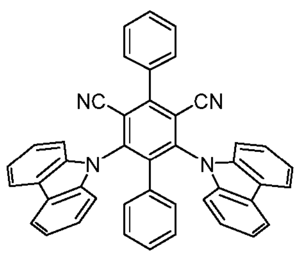
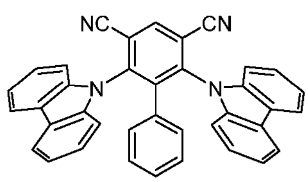

**T35** **T36** **T37**

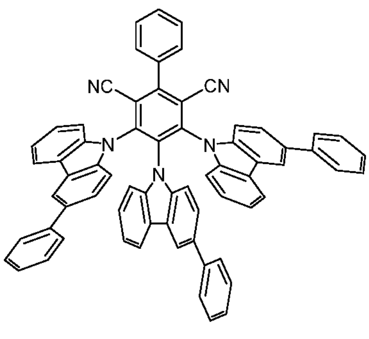
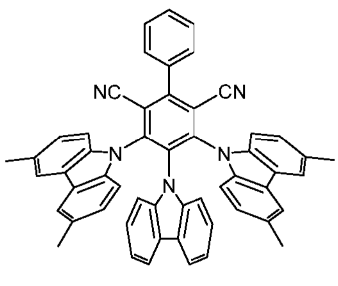
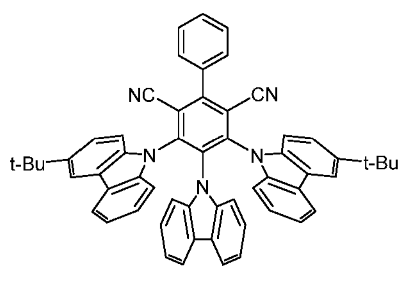

**T38** **T39** **T40**

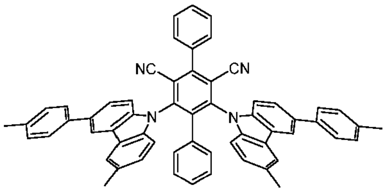

**T41**

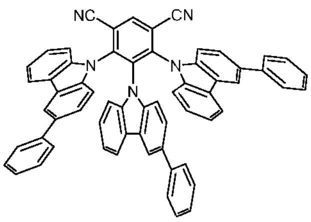

**T42**

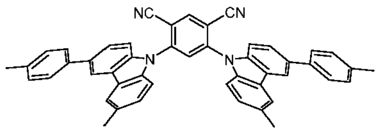

**T43**

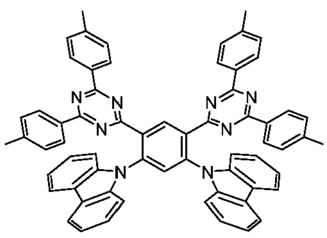

**T44**

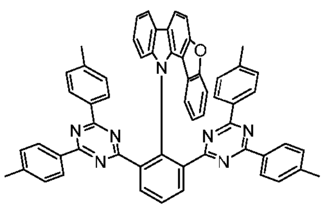

**T45**

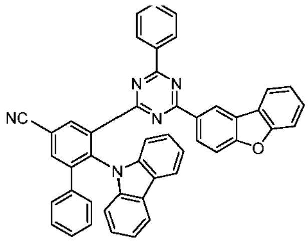

**T46**

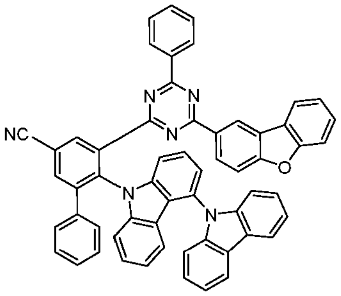

**T47**

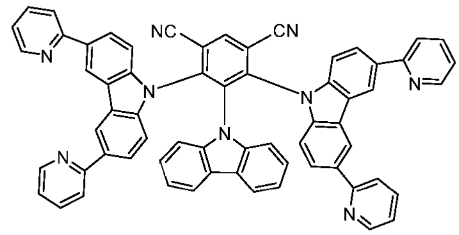

**T48**

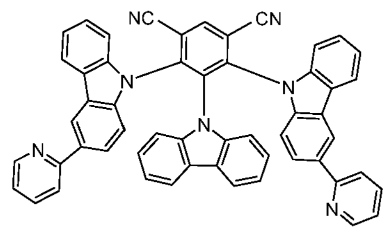

**T49**

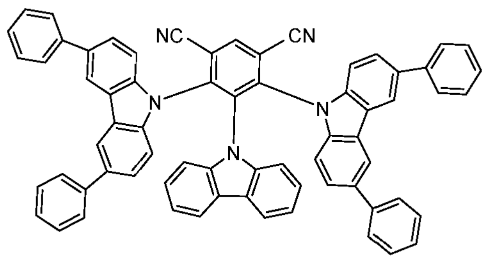

**T50**

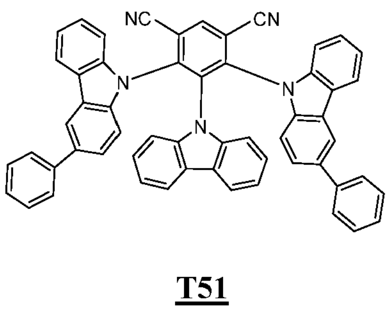

**T51**

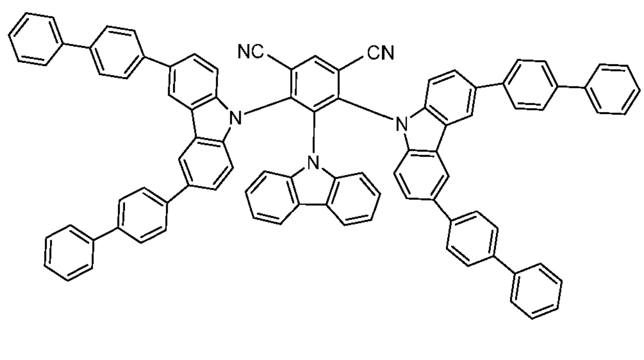

**T52**

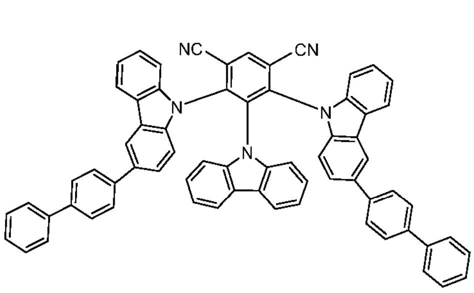

**T53**

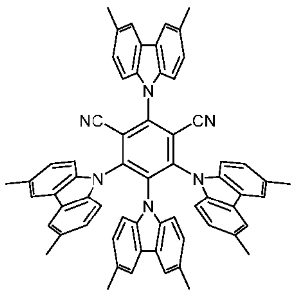

**T54**

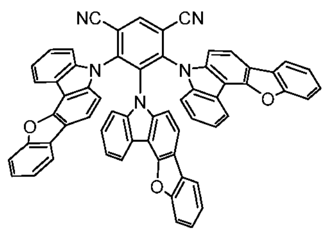

**T55**

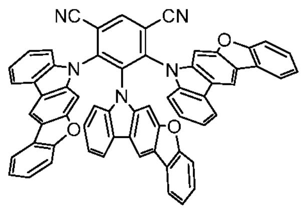

**T56**

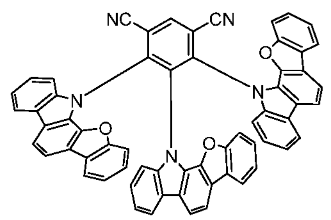

**T57**

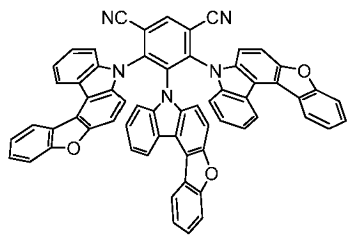

**T58**

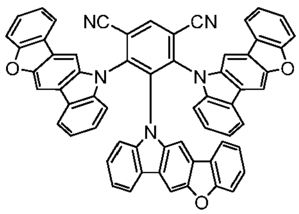

**T59**

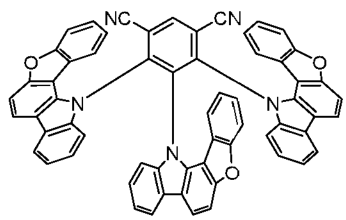

**T60**

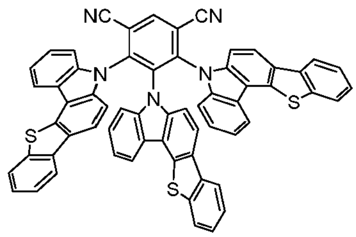

**T61**

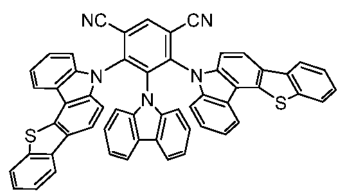

**T62**

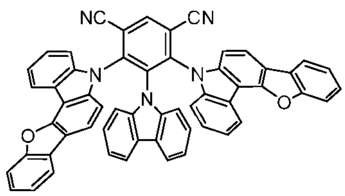

**T63**

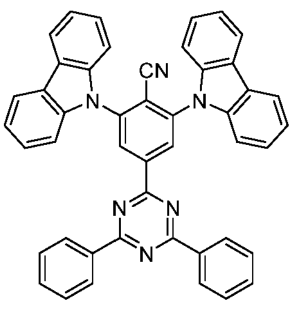

**T64**

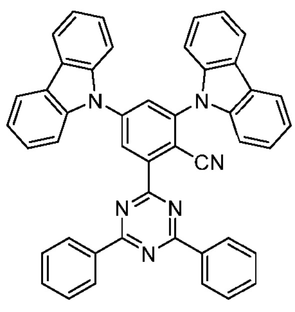

**T65**

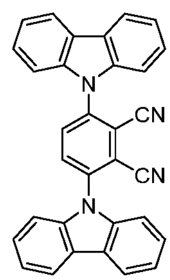

**T66**

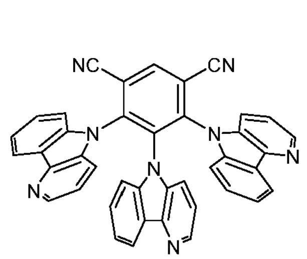

**T67**

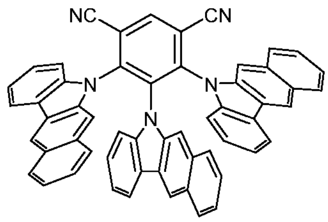

**T68**

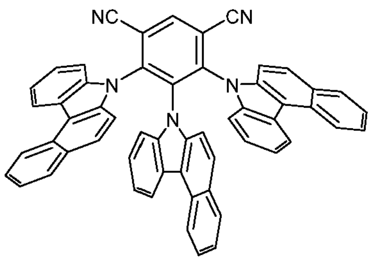

**T69**

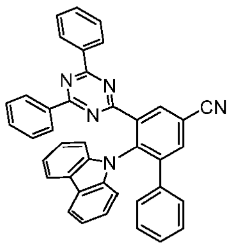

**T70**

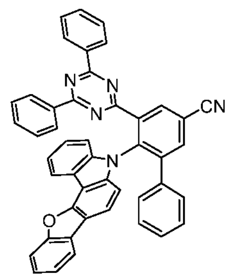

**T71**

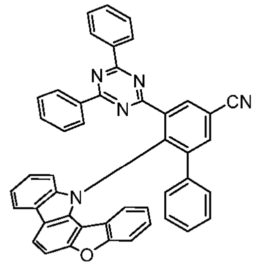

**T72**

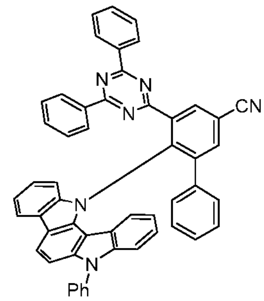

**T73**

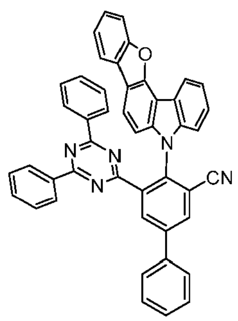

**T74**

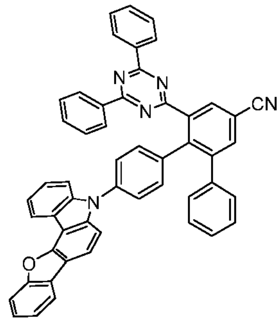

**T75**

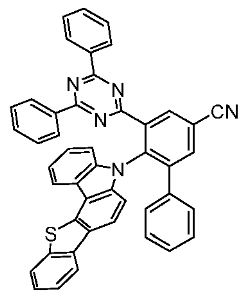

**T76**

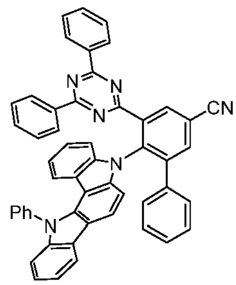

**T77**

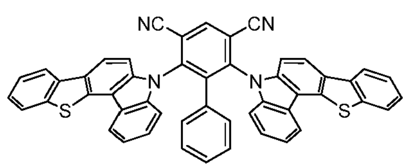

**T78**

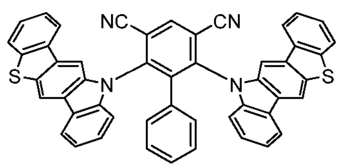

**T79**

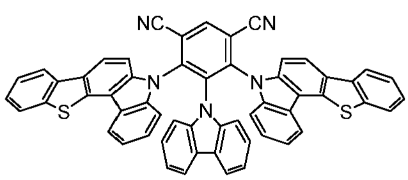

**T80**

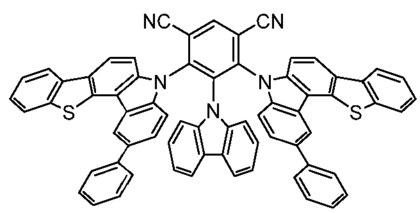

**T81**

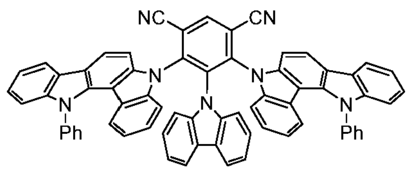

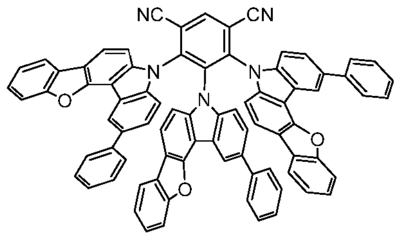

**T82** **T83**

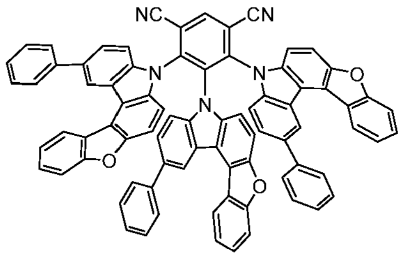

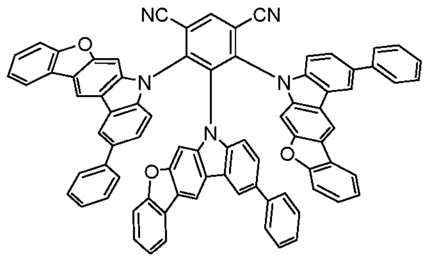

**T84** **T85**

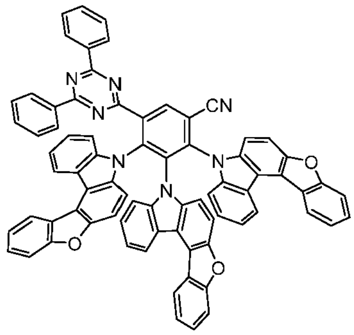

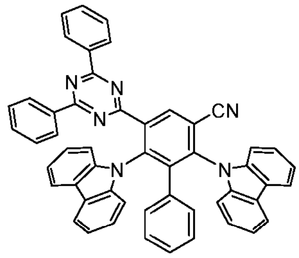

**T86** **T87**

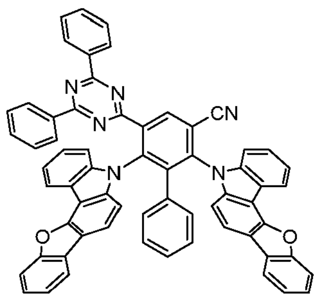

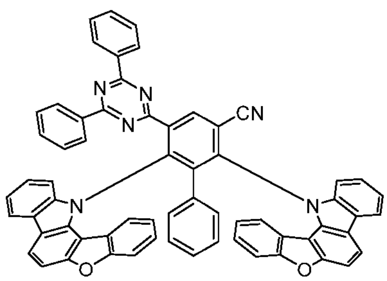

**T88** **T89**

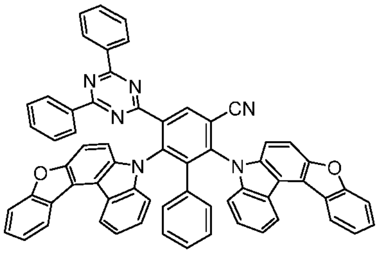

**T90**

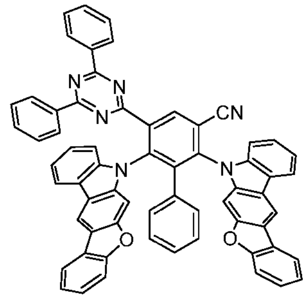

**T91**

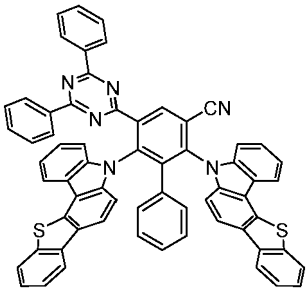

**T92**

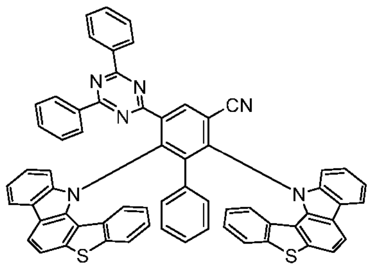

**T93**

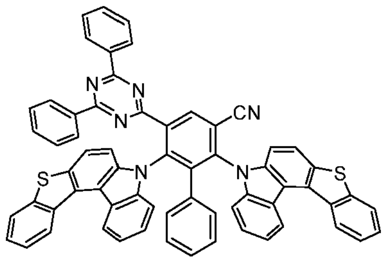

**T94**

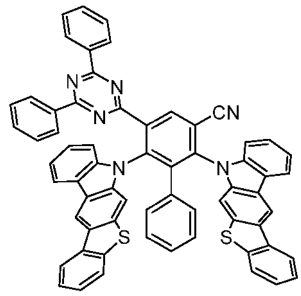

**T95**

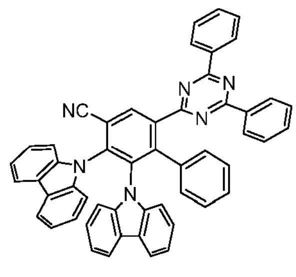

**T96**

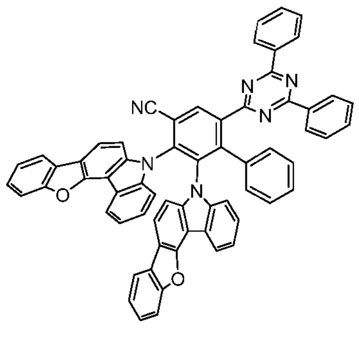

**T97**

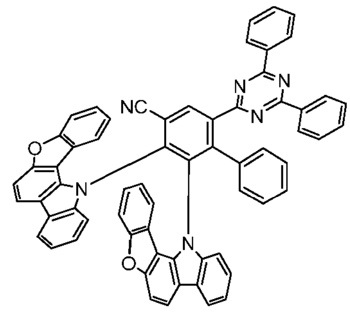

**T98**

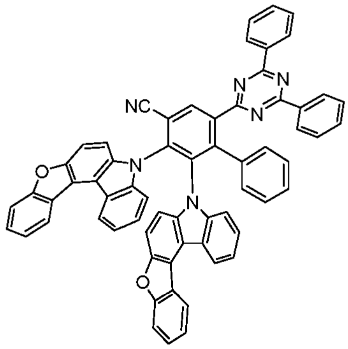
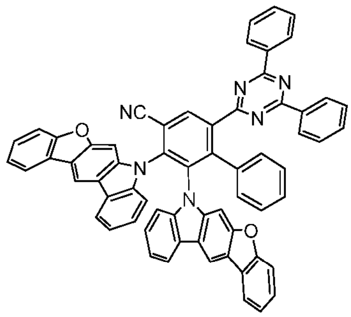

**T99** **T100**

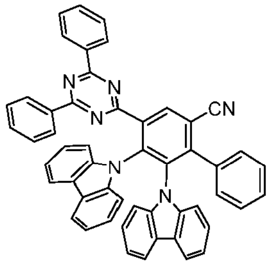
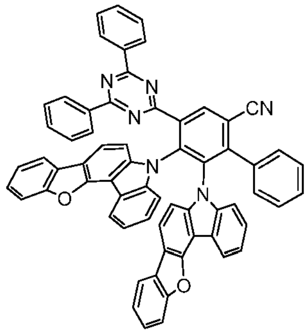
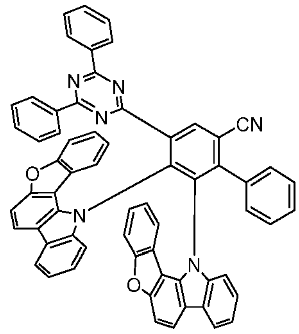

**T101** **T102** **T103**

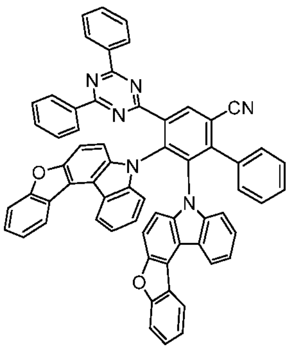
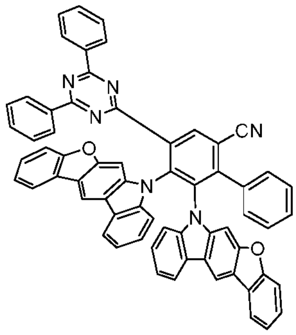

**T104** **T105**

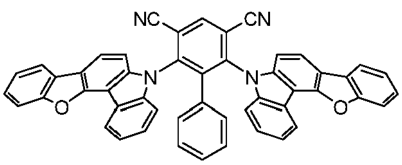
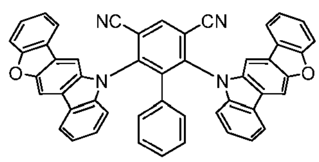
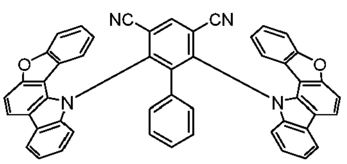

**T106** **T107** **T108**

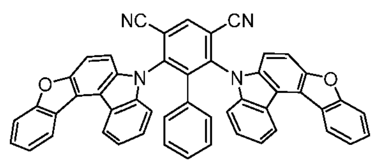

**T109**

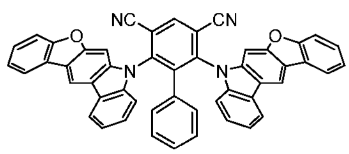

**T110**

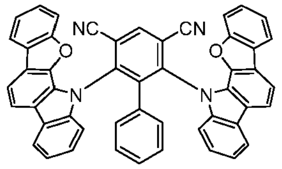

**T111**

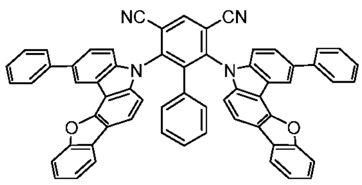

**T112**

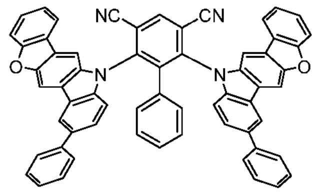

**T113**

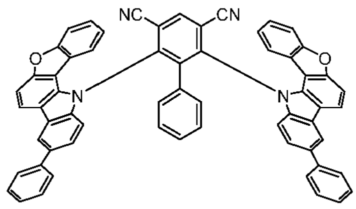

**T114**

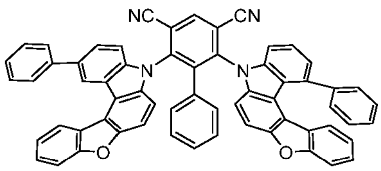

**T115**

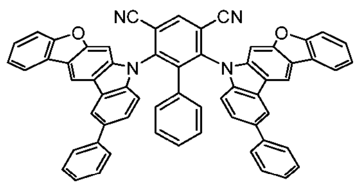

**T116**

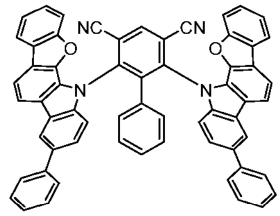

**T117**

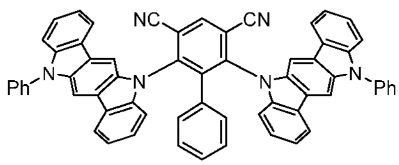

**T118**

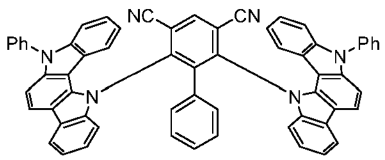

**T119**

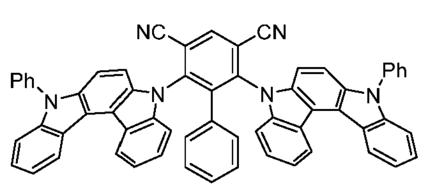

**T120**

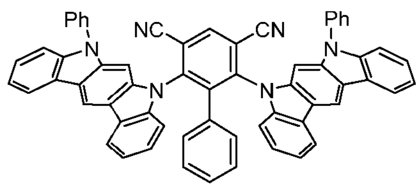

**T121**

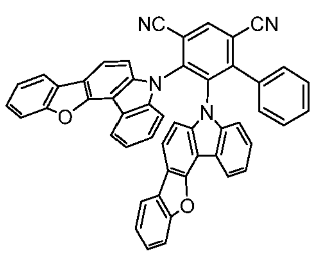

**T122**

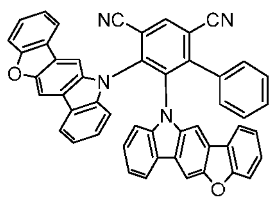

**T123**

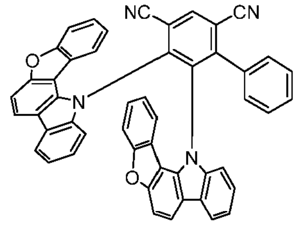

**T124**

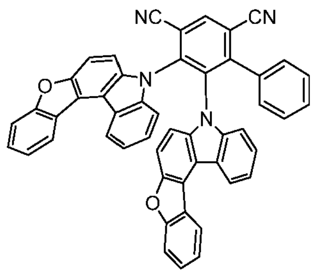
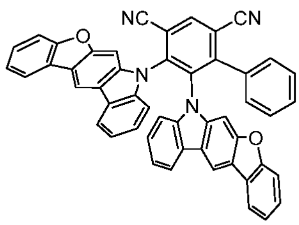
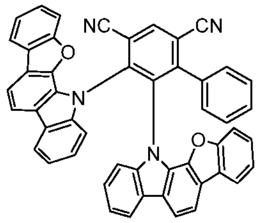

**T125** **T126** **T127**

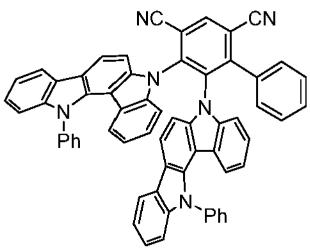
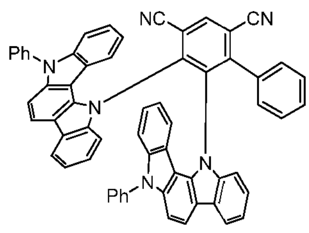
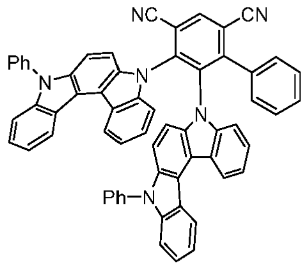

**T128** **T129** **T130**

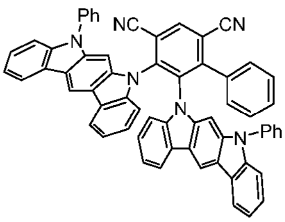
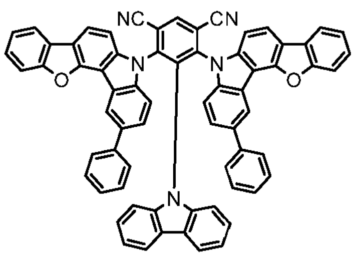
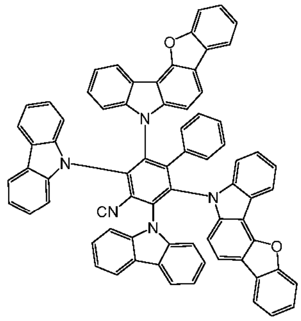

**T131** **T132** **T133**

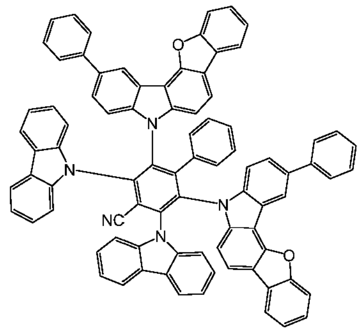
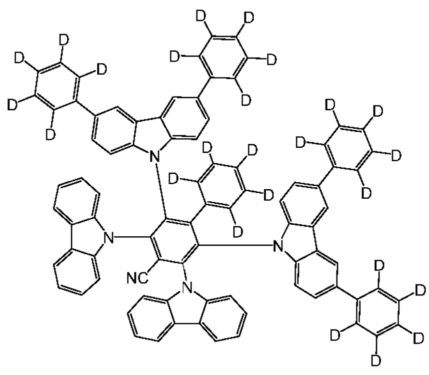

**T134** **T135**

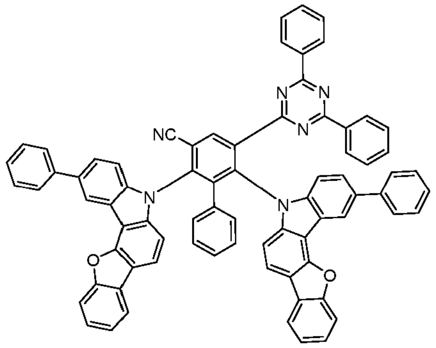

**T136**

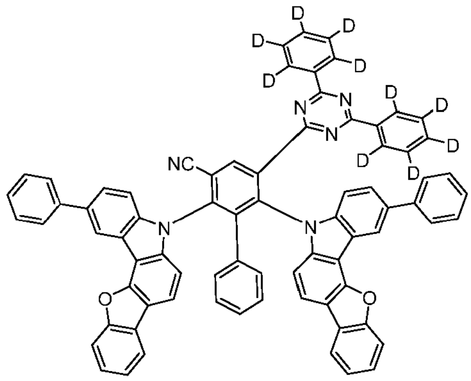

**T137**

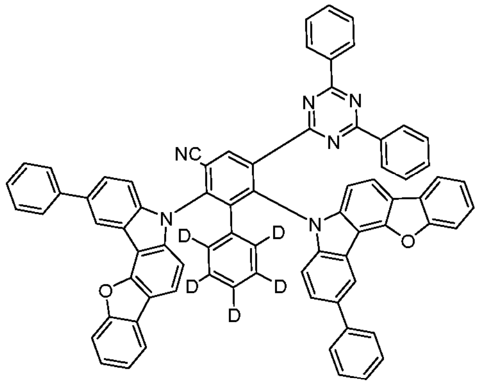

**T138**

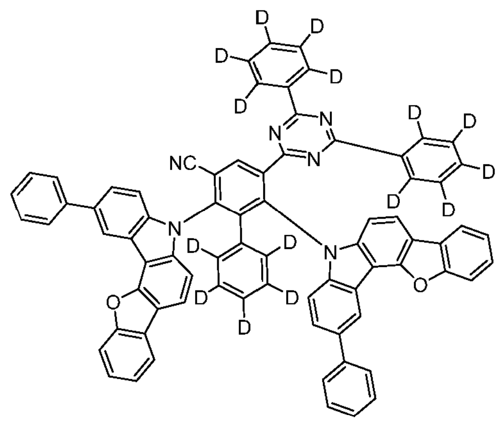

**T139**

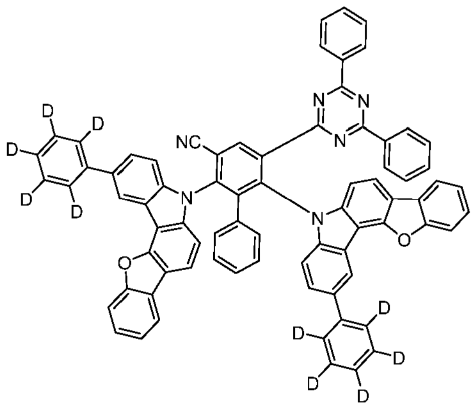

**T140**

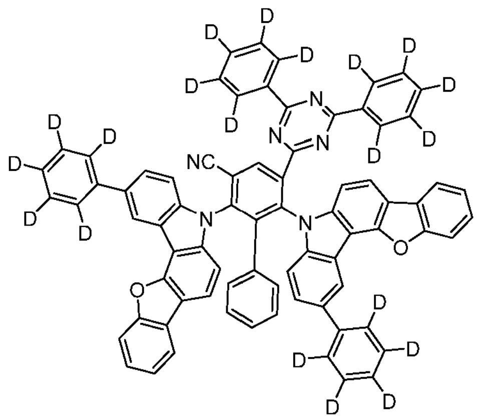

**T141**

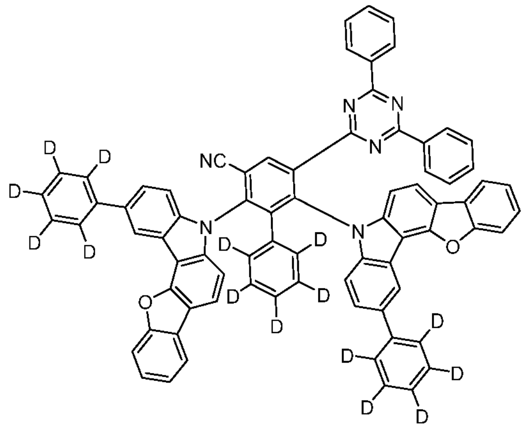

**T142**

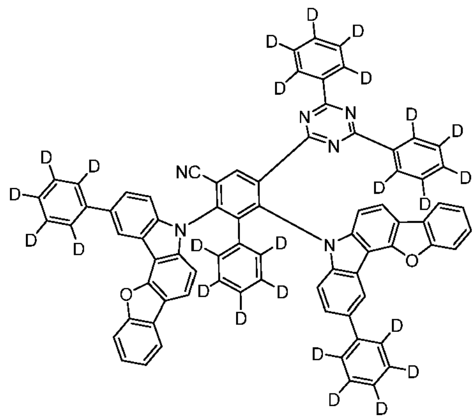

**T143**

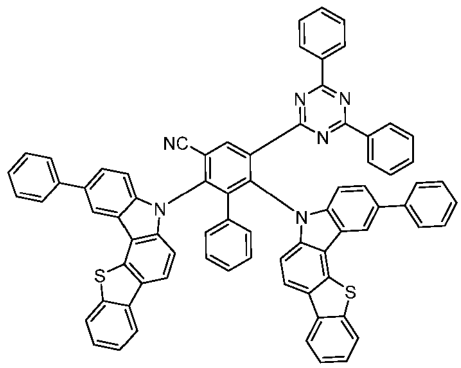

**T144**

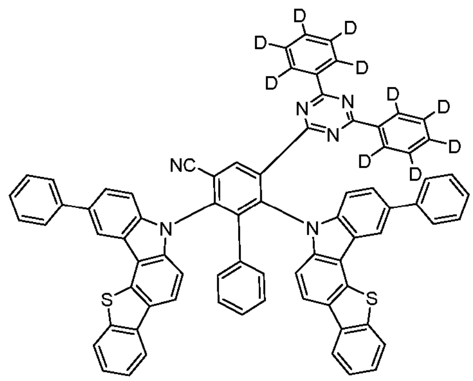

**T145**

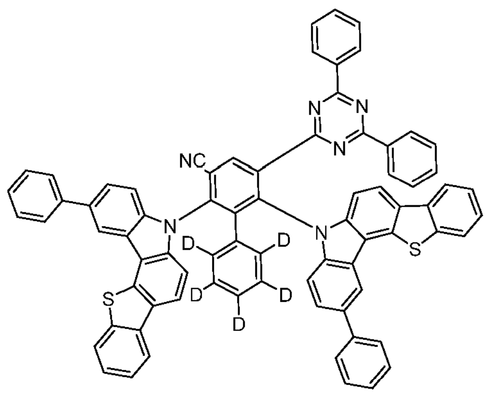

**T146**

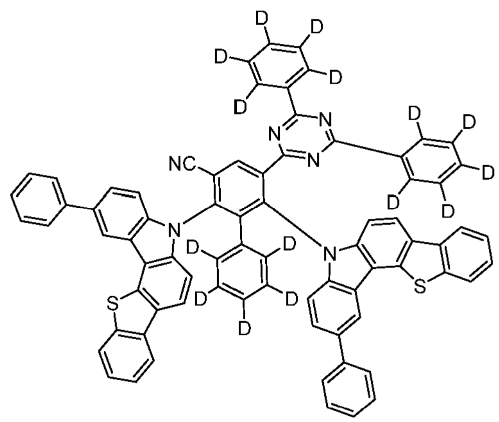

**T147**

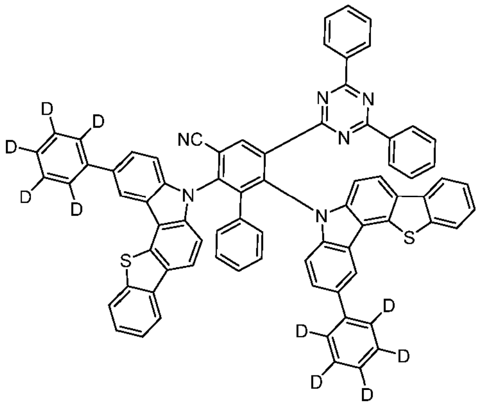

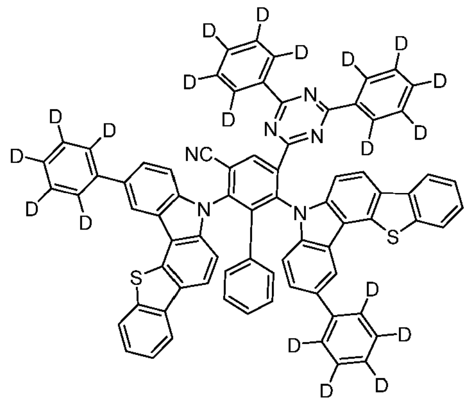

**T148** **T149**

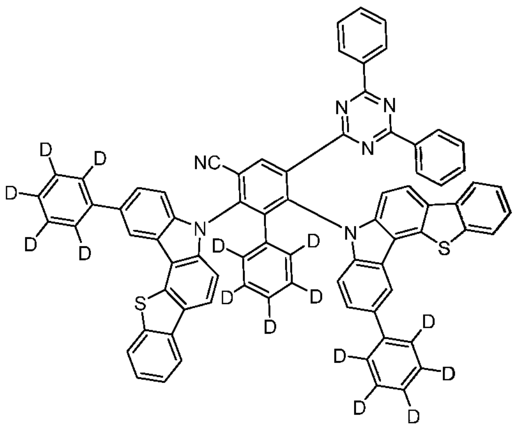

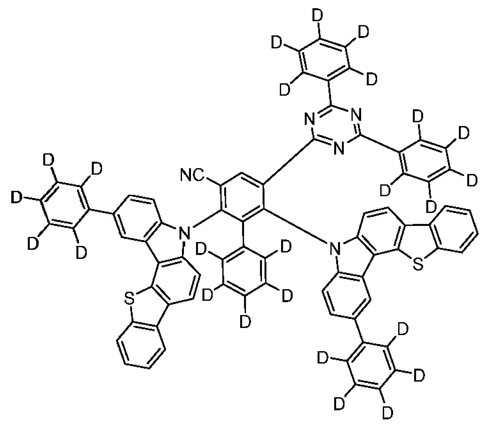

**T150** **T151**

In addition to the above, known delayed fluorescence materials can be used in suitable combination. Further, an unknown delayed fluorescence material can also be used. In particular, the compound represented by the formula (1) described in the specification of Patent Application No. 2021-188860, paragraphs 0013 to 0042, particularly, the compound described in paragraphs 0043 to 0048 can be preferably used.

Examples of the delayed fluorescence material include compounds included in the general formulae described in WO2013/154064, paragraphs 0008 to 0048 and 0095 to 0133, WO2013/011954, paragraphs 0007 to 0047 and 0073 to 0085, WO2013/011955, paragraphs 0007 to 0033 and 0059 to 0066, WO2013/081088, paragraphs 0008 to 0071 and 0118 to 0133, JP 2013-256490 A, paragraphs 0009 to 0046 and 0093 to 0134, JP 2013-116975 A, paragraphs 0008 to 0020 and 0038 to 0040, WO2013/133359, paragraphs 0007 to 0032 and 0079 to 0084, WO2013/161437, paragraphs 0008 to 0054 and 0101 to 0121, JP 2014-9352 A, paragraphs 0007 to 0041 and 0060 to 0069, JP 2014-9224 A, paragraphs 0008 to 0048 and 0067 to 0076, JP 2017-119663 A, paragraphs 0013 to 0025, JP 2017-119664 A, paragraphs 0013 to 0026, JP 2017-222623 A, paragraphs 0012 to 0025, JP 2017-226838 A, paragraphs 0010 to 0050, JP 2018-100411 A, paragraphs 0012 to 0043, and WO2018/047853, paragraphs 0016 to 0044, and exemplary compounds therein capable of emitting delayed fluorescence. Further, light-emitting materials capable of emitting delayed fluorescence, as described in JP 2013-253121 A, WO2013/133359, WO2014/034535, WO2014/115743, WO2014/122895, WO2014/126200, WO2014/136758, WO2014/133121, WO2014/136860, WO2014/196585, WO2014/189122, WO2014/168101, WO2015/008580, WO2014/203840, WO2015/002213, WO2015/016200, WO2015/019725, WO2015/072470, WO2015/108049, WO2015/080182, WO2015/072537, WO2015/080183, JP 2015-129240 A, WO2015/129714, WO2015/129715, WO2015/133501, WO2015/136880, WO2015/137244, WO2015/137202, WO2015/137136, WO2015/146541, and WO2015/159541, can also be adopted.

(Third organic compound)

The third organic compound used in the light-emitting layer of the organic electroluminescence device of the invention is a compound that emits fluorescence and has LUMO energy lower than that of the first organic compound or the second organic compound. It is preferable that the third organic compound is a compound that emits fluorescence, has the

LUMO energy lower than that of the first organic compound or the second organic compound, and has the lowest excited singlet energy lower than that of the first organic compound or the second organic compound. In the organic electroluminescence device of the invention, the orientation value of the third organic compound in the light-emitting layer is -0.3 or less. In the organic electroluminescence device of the invention, fluorescence derived from the third organic compound is emitted. The light emitted from the third organic compound generally includes delayed fluorescence. The largest component of the light emitted from the device is the fluorescence from the third organic compound. That is, in the light emitted from the organic electroluminescence device of the invention, the amount of fluorescence emitted from the third organic compound is the largest.

In a preferable aspect of the invention, the third organic compound receives the energy from the first organic compound in the excited singlet state, the second organic compound in the excited singlet state, and the second organic compound in the excited singlet state from the excited triplet state by the inverse intersystem crossing to transition to the excited singlet state. Further, in a more preferable aspect of the invention, the third organic compound receives the energy from the second organic compound in the excited singlet state, and the second organic compound in the excited singlet state from the excited triplet state by the inverse intersystem crossing to transition to the excited singlet state. In the generated excited singlet state of the third organic compound, fluorescence is emitted afterward when returning to the ground state.

The third organic compound can be used without any particular limitation insofar as the third organic compound is a fluorescence material (fluorescence-emitting compound) satisfying a predetermined condition. Here, the "fluorescence material" indicates that when the emission lifetime is measured by a fluorescence lifetime measurement system (available from Hamamatsu Photonics K.K., a streak camera system or the like), fluorescence having emission lifetime of less than 100 ns (nanoseconds) is observed. The light emitted from the third organic compound may include delayed fluorescence or phosphorescence, but the largest component of the light emitted from the third organic compound is the fluorescence. In one aspect of the invention, the organic electroluminescence device does not emit phosphorescence, or the amount of phosphorescence emitted is 1% or less of the fluorescence.

Two or more types of third organic compounds may be used insofar as the condition of the invention is satisfied. For example, by using two or more types of third organic compounds with different luminescent colors, it is possible to emit light with a desired color. Further, light with a single color may be emitted from the third organic compound by using one type of third organic compound.

In the invention, the largest light-emitting wavelength of the compound that can be used as the third organic compound is not particularly limited. Thus, a light-emitting material having the largest light-emitting wavelength in a visible region (380 to 780 nm), a light-emitting material having the largest light-emitting wavelength in an IR region (780 nm to 1 mm), a compound having the largest light-emitting wavelength in a UV region (e.g., 280 to 380 nm), or the like can be suitably selected and used. A fluorescence material having the largest light-emitting wavelength in a visible region is preferable. For example, a light-emitting material of which the largest light-emitting wavelength in a region of 380 to 780 nm is in a range of 380 to 570 nm may be selected and used, a light-emitting material of which the largest light-emitting wavelength is in a range of 570 to 650 nm may be selected and used, a light-emitting material of which the largest light-emitting wavelength is in a range of 650 to 700 nm may be selected and used, or a light-emitting material of which the largest light-emitting wavelength is in a range of 700 to 780 nm may be selected and used.

In a preferable aspect of the invention, each compound is selected and combined such that there is an overlap between the light-emitting wavelength region of the second organic compound and the absorption wavelength region of the third organic compound. In particular, it is preferable that an edge on the short wavelength side of the emission spectrum of the second organic compound overlaps with an edge on the long wavelength side of the absorption spectrum of the third organic compound.

It is preferable that the third organic compound does not contain a metal atom other than a boron atom. For example, the third organic compound may be a compound containing both of boron atoms and fluorine atoms. Further, the third organic compound may be a compound containing boron atoms but not fluorine atoms. Further, the third organic compound may not contain a metal atom at all. For example, as the third organic compound, a compound containing atoms selected from the group consisting of a carbon atom, a hydrogen atom, a deuterium atom, a nitrogen atom, an oxygen atom, a sulfur atom, a fluorine atom, and a boron atom can be selected. For example, as the third organic compound, a compound containing atoms selected from the group consisting of a carbon atom, a hydrogen atom, a deuterium atom, a nitrogen atom, an oxygen atom, a fluorine atom, and a boron atom can be selected. For example, as the third organic compound, a compound containing atoms selected from the group consisting of a carbon atom, a hydrogen atom, a deuterium atom, a nitrogen atom, an oxygen atom, a sulfur atom, and a boron atom can be selected. For example, as the third organic compound, a compound containing atoms selected from the group consisting of a carbon atom, a hydrogen atom, a deuterium atom, a nitrogen atom, and a boron atom can be selected. For example, as the third organic compound, a compound containing atoms selected from the group consisting of a carbon atom, a hydrogen atom, a deuterium atom, a nitrogen atom, an oxygen atom, and a sulfur atom can be selected. For example, as the third organic compound, a compound containing atoms selected from the group consisting of a carbon atom, a hydrogen atom, a deuterium atom, a

nitrogen atom, and an oxygen atom can be selected. For example, as the third organic compound, a compound containing carbon atoms and hydrogen atoms can be selected.

As the third organic compound, a compound having a multiple resonance effect of a boron atom and a nitrogen atom, or a compound having a condensed aromatic ring structure, such as anthracene, pyrene, and perylene can be exemplified. Further, as the third organic compound, a compound that contains a boron atom and a nitrogen atom, which exhibit a multiple resonance effect, and has a condensed ring structure including four or more constituent rings can be exemplified. Further, as the third organic compound, a compound having a structure in which a pyrrole ring and two benzene rings, which share a nitrogen atom, are condensed with a heterocyclic 6-membered ring containing a boron atom and a nitrogen atom can also be exemplified.

According to the invention, as the third organic compound, a compound represented by the following formula (16) is used.

Formula (16)

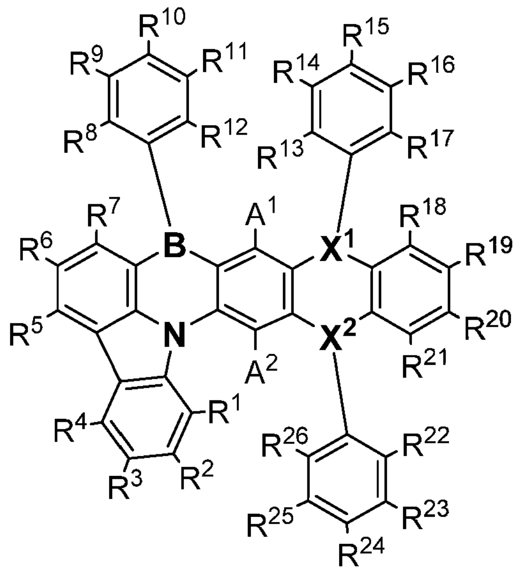

In the formula (16), one of $X^1$ and $X^2$ is a nitrogen atom, and the other is a boron atom. In one aspect of the invention, $X^1$ is a nitrogen atom, and $X^2$ is a boron atom. Here, $R^{17}$ and $R^{18}$ are bonded to each other to form a single bond so as to form a pyrrole ring. In another aspect of the invention, $X^1$ is a boron atom, and $X^2$ is a nitrogen atom. Here, $R^{21}$ and $R^{22}$ are bonded to each other to form a single bond so as to form a pyrrole ring.

In the formula (16), each of $R^1$ to $R^{26}$, $A^1$, and $A^2$ independently represents a hydrogen atom, a deuterium atom, or a substituent.

$R^1$ and $R^2$, $R^2$ and $R^3$, $R^3$ and $R^4$, $R^4$ and $R^5$, $R^5$ and $R^6$, $R^6$ and $R^7$, $R^7$ and $R^8$, $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{17}$ and $R^{18}$, $R^{18}$ and $R^{19}$, $R^{19}$ and $R^{20}$, $R^{20}$ and $R^{21}$, $R^{21}$ and $R^{22}$, $R^{22}$ and $R^{23}$, $R^{23}$ and $R^{24}$, $R^{24}$ and $R^{25}$, and $R^{25}$ and $R^{26}$ may be bonded to each other to form ring structures.

The ring structure formed by combining $R^7$ and $R^8$ includes a boron atom and four carbon atoms as ring skeleton forming atoms. The ring structure formed by combining $R^{17}$ and $R^{18}$ includes a boron atom and four carbon atoms as ring skeleton forming atoms when $X^1$ is a boron atom. When $X^1$ is a nitrogen atom, the ring structure is limited to a pyrrole ring. The ring structure formed by combining $R^{21}$ and $R^{22}$ includes a boron atom and four carbon atoms as ring skeleton forming atoms when $X^2$ is a boron atom. When $X^2$ is a nitrogen atom, the ring structure is limited to a pyrrole ring. When $R^7$ and $R^8$, $R^{17}$ and $R^{18}$, and $R^{21}$ and $R^{22}$ are bonded to each other to form boron atom-containing ring structures, the ring structure is preferably a 5 to 7-membered ring, more preferably a 5 or 6-membered ring, further preferably a 6-membered ring. When $R^7$ and $R^8$, $R^{17}$ and $R^{18}$, and $R^{21}$ and $R^{22}$ are bonded to each other, these preferably form a single bond, -O-, -S-, $-N(R^{27})-$, $-C(R^{28})(R^{29})-$, $-Si(R^{30})(R^{31})-$, $-B(R^{32})-$, -CO-, or -CS- by combining with each other, more preferably form -O-, -S- or $-N(R^{27})-$, further preferably form $-N(R^{27})-$. Here, each of $R^{27}$ to $R^{32}$ independently represents a hydrogen atom, a deuterium atom, or a substituent. As the substituent, a group selected from any of substituent groups A to E to be described below may be employed, but a substituted or unsubstituted alkyl group, a substituted or unsubstituted aryl group, or a substituted or unsubstituted heteroaryl group is preferable. In particular, $R^{27}$ is preferably a substituted or unsubstituted aryl group. When $R^{27}$ to $R^{32}$ are substituents, $R^{27}$ to $R^{32}$ in the ring formed by bonding $R^7$ and $R^8$ to each other may further form a ring structure by bonding to at least one of $R^6$ and $R^9$, $R^{27}$ to $R^{32}$ in the ring formed by bonding $R^{17}$ and $R^{18}$ to each other may further form a ring structure by bonding to at least one of $R^{16}$ and $R^{19}$, and $R^{27}$ to $R^{32}$ in the ring formed by

bonding $R^{21}$ and $R^{22}$ to each other may further form a ring structure by bonding to at least one of $R^{20}$ and $R^{23}$. In one aspect of the invention, in only one set among $R^7$ and $R^8$, $R^{17}$ and $R^{18}$, and $R^{21}$ and $R^{22}$, these are bonded to each other. In one aspect of the invention, in only two sets among $R^7$ and $R^8$, $R^{17}$ and $R^{18}$, and $R^{21}$ and $R^{22}$, these are bonded to each other. In one aspect of the invention, all of $R^7$ and $R^8$, $R^{17}$ and $R^{18}$, and $R^{21}$ and $R^{22}$ are bonded to each other.

The ring structure formed by bonding $R^1$ and $R^2$, $R^2$ and $R^3$, $R^3$ and $R^4$, $R^4$ and $R^5$, $R^5$ and $R^6$, $R^6$ and $R^7$, $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{18}$ and $R^{19}$, $R^{19}$ and $R^{20}$, $R^{20}$ and $R^{21}$, $R^{22}$ and $R^{23}$, $R^{23}$ and $R^{24}$, $R^{24}$ and $R^{25}$, and $R^{25}$ and $R^{26}$ to each other may be an aromatic ring or an adipose ring, or may include a hetero atom. Further, one or more rings, as other rings, may be condensed. The hetero atom mentioned herein is preferably one selected from the group consisting of a nitrogen atom, an oxygen atom, and a sulfur atom. Examples of the ring structure to be formed include a benzene ring, a pyridine ring, a pyridazine ring, a pyrimidine ring, a pyrazine ring, a pyrrole ring, an imidazole ring, a pyrazole ring, a triazole ring, an imidazoline ring, a furan ring, a thiophene ring, an oxazole ring, an isooxazole ring, a thiazole ring, an isothiazole ring, a cyclohexadiene ring, a cyclohexene ring, a cyclopentene ring, a cycloheptatriene ring, a cycloheptadiene ring, a cycloheptene ring, and a ring in which one or more rings selected from the group consisting of these rings are further condensed. In a preferred aspect of the invention, the ring structure is a substituted or unsubstituted benzene ring (further, a ring may be condensed), and is for example, a benzene ring which may be substituted with an alkyl group or an aryl group. In a preferred aspect of the invention, the ring structure is a substituted or unsubstituted heteroaromatic ring, preferably a furan ring of benzofuran, or a thiophene ring of benzothiophene. Among $R^1$ and $R^2$, $R^2$ and $R^3$, $R^3$ and $R^4$, $R^4$ and $R^5$, $R^5$ and $R^6$, $R^6$ and $R^7$, $R^8$ and $R^9$, $R^9$ and $R^{10}$, $R^{10}$ and $R^{11}$, $R^{11}$ and $R^{12}$, $R^{13}$ and $R^{14}$ $R^{14}$ and $R^{15}$, $R^{15}$ and $R^{16}$, $R^{16}$ and $R^{17}$, $R^{18}$ and $R^{19}$, $R^{19}$ and $R^{20}$, $R^{20}$ and $R^{21}$, $R^{22}$ and $R^{23}$, $R^{23}$ and $R^{24}$, $R^{24}$ and $R^{25}$, and $R^{25}$ and $R^{26}$, the number of combinations that are bonded to each other to form ring structures may be 0, or may be, for example, any one of 1 to 6. For example, it may be any one of 1 to 4, 1 may be selected, 2 may be selected, or 3 or 4 may be selected. In one aspect of the invention, one set selected from $R^1$ and $R^2$, $R^2$ and $R^3$, and $R^3$ and $R^4$ is bonded to form a ring structure. In one aspect of the invention, $R^5$ and $R^6$ are bonded to each other to form a ring structure. In one aspect of the invention, one set selected from $R^9$ and $R^{10}$, $R^{10}$ and $R^{11}$, and $R^{11}$ and $R^{12}$ is bonded to each other to form a ring structure. In one aspect of the invention, each of $R^1$ and $R^2$, and $R^{13}$ and $R^{14}$ is bonded to each other to form a ring structure. In one aspect of the invention, one set selected from $R^1$ and $R^2$, $R^2$ and $R^3$, and $R^3$ and $R^4$ is bonded to each other to form a ring structure, and moreover $R^5$ and $R^6$ are bonded to each other to form a ring structure. In one aspect of the invention, in each of $R^5$ and $R^6$, and $R^{19}$ and $R^{20}$ is bonded to each other to form a ring structure.

$R^1$ to $R^{26}$ which are not bonded to adjacent $R^n$(n=1 to 26) together are hydrogen atoms, deuterium atoms, or substituents. As the substituent, a group selected from any of substituent groups A to E to be described below may be employed.

Preferable substituents that may be possessed by $R^1$ to $R^{26}$ are a substituted or unsubstituted alkyl group, a substituted or unsubstituted aryl group, and a substituted or unsubstituted heteroaryl group. For example, the substituent may be a substituted or unsubstituted aryl group, and for example the substituent may be a substituted or unsubstituted alkyl group. As the substituent of the alkyl group, the aryl group, or the heteroaryl group mentioned herein, a group selected from any of substituent groups A to E may also be employed. Meanwhile, one or more groups selected from the group consisting of an alkyl group, an aryl group, and a heteroaryl group are preferred, and a group of a substituent group E is more preferred, and it may be unsubstituted. In a preferred aspect of the invention, at least one of $R^1$ to $R^6$ is a substituent, preferably a group of a substituent group E. For example, at least one of $R^1$ to $R^6$ is a substituent, preferably a group of a substituent group E. For example, at least one of $R^5$ and $R^6$ is a substituent, preferably a group of a substituent group E. In a preferred aspect of the invention, at least one of $R^3$ and $R^6$ is a substituent, more preferably both are substituents, and a group of a substituent group E is preferred. In a preferred aspect of the invention, when $X^1$ is a nitrogen atom, at least one of $R^{15}$ and $R^{20}$ is a substituent, more preferably both are substituents, and a group of a substituent group E is preferred. Here, $R^{16}$ and $R^{17}$ are bonded to each other to form a single bond. In a preferred aspect of the invention, when $X^2$ is a nitrogen atom, at least one of $R^{19}$ and $R^{24}$ is a substituent, more preferably both are substituents, and a group of a substituent group E is preferred. Here, $R^{21}$ and $R^{22}$ are bonded to each other to form a single bond. In one aspect of the invention, at least one of $R^8$ and $R^{12}$ is a substituent, and preferably both are substituents. In one aspect of the invention, $R^8$, $R^{10}$ and $R^{12}$ are substituents. As for the substituent of $R^8$ to $R^{12}$, an unsubstituted alkyl group is preferable. When $X^1$ is a boron atom, at least one of $R^{13}$ and $R^{17}$ is a substituent, and preferably both are substituents. In one aspect of the invention, when $X^1$ is a boron atom, $R^{13}$, $R^{15}$ and $R^{17}$ are substituents. When $X^1$ is a boron atom, as for the substituent of $R^{13}$ to $R^{17}$, an unsubstituted alkyl group is preferable. When $X^2$ is a boron atom, at least one of $R^{22}$ and $R^{26}$ is a substituent, and preferably both are substituents. In one aspect of the invention, when $X^2$ is a boron atom, $R^{22}$, $R^{24}$ and $R^{26}$ are substituents. When $X^2$ is a boron atom, as for the substituent of $R^{22}$ to $R^{26}$, an unsubstituted alkyl group is preferable.

$A^1$ and $A^2$ are hydrogen atoms, deuterium atoms, or substituents. As for the substituent, a group selected from any of substituent groups A to E to be described below may be adopted.

A preferable substituent that may be possessed by $A^1$ and $A^2$ is an acceptor group. The acceptor group is a group having a positive Hammett $\sigma$p value. Here, the "Hammett $\sigma$p value", which is proposed by L.P. Hammett, indicates the

quantified effect of a substituent on the reaction rate or equilibrium of a para-substituted benzene derivative. Specifically, it is a constant (σp) peculiar to the substituent in the following equation, which is established between the substituent in the para-substituted benzene derivative and the reaction rate constant or the equilibrium constant:

$$\log(k/k_0)=\rho\sigma p$$

or

$$\log(K/K_0)=\rho\sigma p$$

In the equation, $k_0$ represents a rate constant of a benzene derivative having no substituent, k represents a rate constant of a benzene derivative substituted with a substituent, $K_0$ represents an equilibrium constant of a benzene derivative having no substituent, K represents an equilibrium constant of a benzene derivative substituted with a substituent, and ρ represents a reaction constant determined by the type and condition of the reaction. In relation to descriptions on "the Hammett σp value" and the numerical value of each substituent in the invention, the description on the σp value may be referred to in Hansch, C.et.al., Chem. Rev., 91, 165-195(1991).

The acceptor group that may be possessed by $A^1$ and $A^2$ is more preferably a group having a Hammett σp value greater than 0.2. Examples of the group having a Hammett σp value greater than 0.2 include a cyano group, an aryl group substituted with at least a cyano group, a fluorine atom-containing group, and a substituted or unsubstituted heteroaryl group containing a nitrogen atom as a ring skeleton forming atom. The aryl group substituted with at least a cyano group, which is mentioned herein, may be substituted with a substituent other than the cyano group (for example, an alkyl group or an aryl group), but may be an aryl group substituted with only a cyano group. The aryl group substituted with at least a cyano group is preferably a phenyl group substituted with at least a cyano group. The number of substitutions of the cyano group is preferably one or two, and, for example, may be one, or may be two. As the fluorine atom-containing group, a fluorine atom, an alkyl fluoride group, and an aryl group substituted with at least a fluorine atom or an alkyl fluoride group may be mentioned. The alkyl fluoride group is preferably a perfluoroalkyl group, and the number of carbon atoms is preferably 1 to 6, more preferably 1 to 3. Further, the heteroaryl group containing a nitrogen atom as a ring skeleton forming atom may be a monocycle, or may be a condensed ring in which two or more rings are condensed. In the case of a condensed ring, the number of rings after condensation is preferably two to six, and, for example, may be selected from two to four, or may be two. Specific examples of the ring forming the heteroaryl group include a pyridine ring, a pyrimidine ring, a pyrazine ring, a triazine ring, a quinoline ring, an isoquinoline ring, a quinazoline ring, a quinoxaline ring, and a naphthyridine ring other than the quinazoline ring or the quinoxaline ring. The ring forming the heteroaryl group may be substituted with a deuterium atom or a substituent, and as for the substituent, for example, one group selected from the group consisting of an alkyl group, an aryl group, and a heteroaryl group or a group formed by two or more thereof may be mentioned. As the acceptor group that $A^1$ and $A^2$ may have, a cyano group is particularly preferable.

In one aspect of the invention, each of $A^1$ and $A^2$ is independently a hydrogen atom or a deuterium atom. In one aspect of the invention, at least one of $A^1$ and $A^2$ is an acceptor group. In one aspect of the invention, at least one of $A^1$ and $A^2$ is an acceptor group. In one aspect of the invention, both $A^1$ and $A^2$ are acceptor groups. In one aspect of the invention, both $A^1$ and $A^2$ are acceptor groups. In one aspect of the invention, $A^1$ and $A^2$ are cyano groups. In one aspect of the invention, $A^1$ and $A^2$ are halogen atoms, e.g., bromine atoms.

Hereinafter, specific examples of the acceptor group that may be adopted in the invention will be illustrated. Meanwhile, the acceptor group that may be used in the invention is not construed as limiting to the following specific examples. In the present specification, indication of $CH_3$ is omitted for a methyl group. Thus, for example, A15 indicates a group including two 4-methyl phenyl groups. Further, "D" represents a deuterium atom. * represents a bond position.

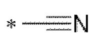 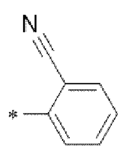 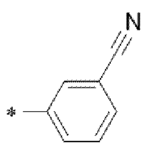 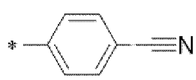 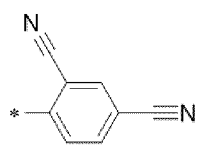 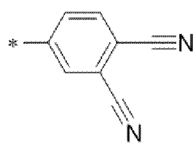

A1 A2 A3 A4 A5 A6

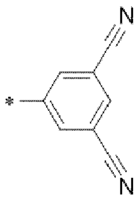 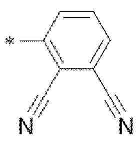 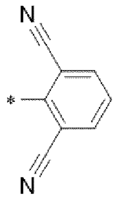 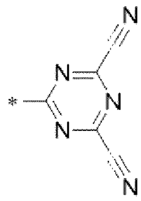 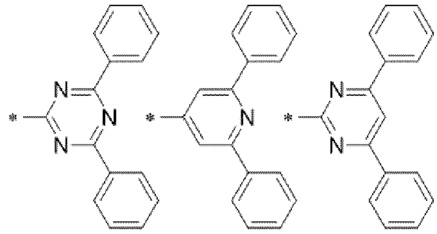

A7 A8 A9 A10 A11 A12 A13

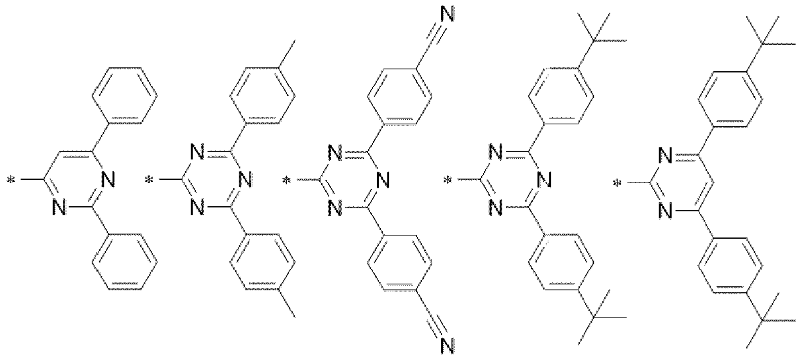

A14 A15 A16 A17 A18

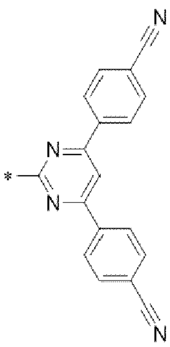 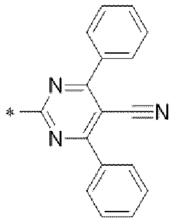 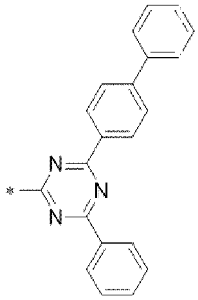 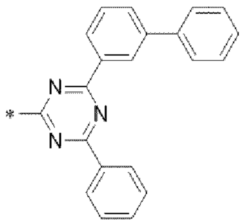

A19 A20 A21 A22

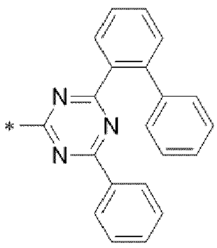 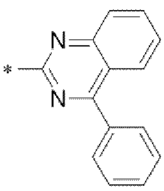 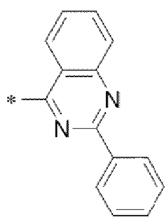

A23 A24 A25

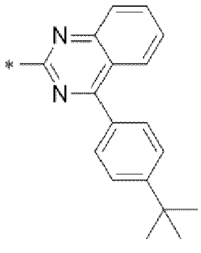 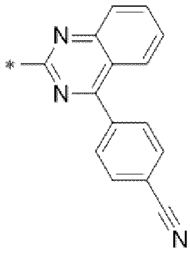 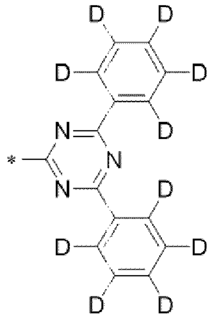 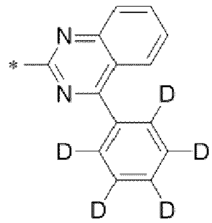

A26 A27 A28 A29

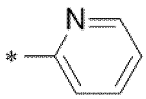 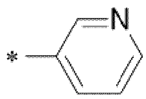 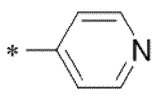 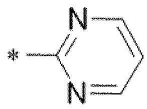 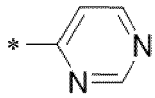

A30 A31 A32 A33 A34

*-$CF_3$ A35

* - $C_2F_5$ A36

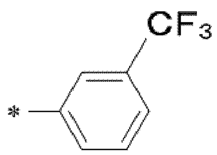 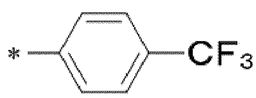

A37 A38

*-F A39

*-Cl A40

*-Br A41

*- I A42

When $X^1$ is a nitrogen atom, $R^7$ and $R^8$ are bonded via a nitrogen atom to form a 6-membered ring, $R^{21}$ and $R^{22}$ are bonded via a nitrogen atom to form a 6-membered ring, and $R^{17}$ and $R^{18}$ are bonded to each other to form a single bond, at least one of $R^1$ to $R^6$ is a substituted or unsubstituted aryl group, or any of $R^1$ and $R^2$, $R^2$ and $R^3$, $R^3$ and $R^4$, $R^4$ and $R^5$, and $R^5$ and $R^6$ is bonded to each other to form an aromatic ring (a substituted or unsubstituted benzene ring which may be condensed) or a heteroaromatic ring (preferably a substituted or unsubstituted furan ring of benzofuran which may be condensed, or a substituted or unsubstituted thiophene ring of benzothiophene which may be condensed).

When $X^1$ of the formula (16) is a nitrogen atom, the compound of the invention has the following skeleton (16a). When $X^2$ of the formula (16) is a nitrogen atom, the compound of the invention has the following skeleton (16b).

Skeleton (16a)

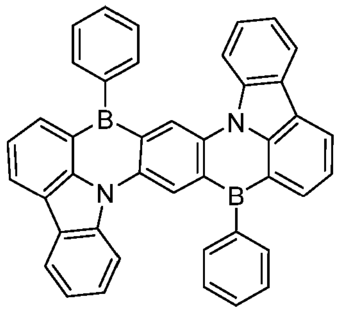

Skeleton (16b)

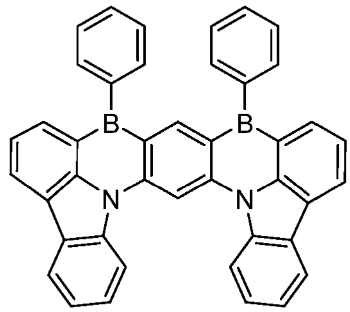

In the skeletons (16a) and (16b), each hydrogen atom may be substituted with a deuterium atom or a substituent. Further, it may be substituted with a linking group together with an adjacent hydrogen atom to form a ring structure. For details, corresponding descriptions on $R^1$ to $R^{26}$, $A^1$, and $A^2$ in the formula (16) may be referred to. Compounds, in which all phenyl groups bonded to boron atoms in the skeletons (16a) and (16b) are substituted with mesityl groups, 2,6-diisopropyl phenyl groups or 2,4,6-triisopropyl phenyl groups, may be exemplified. In one aspect of the invention, each hydrogen atom in the skeletons (16a) and (16b) is not substituted with a linking group together with an adjacent hydrogen atom to form a ring structure.

As one preferable group of compounds having the skeleton (16a), compounds represented by the following formula (16a) may be exemplified.

Formula (16a)

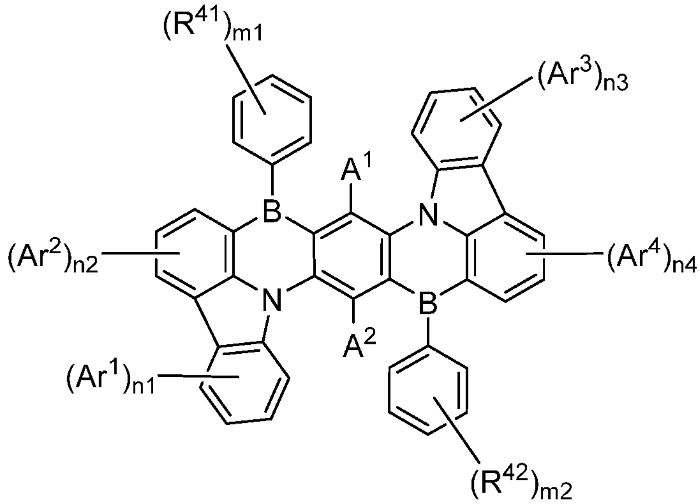

In the formula (16a), each of $Ar^1$ to $Ar^4$ independently represents a substituted or unsubstituted aryl group, or a substituted or unsubstituted heteroaryl group. Each of $R^{41}$ and $R^{42}$ independently represents a substituted or unsubstituted alkyl group. Each of m1 and m2 independently represents an integer of 0 to 5, each of n1 and n3 independently represents an integer of 0 to 4, and each of n2 and n4 independently represents an integer of 0 to 3. Each of $A^1$ and $A^2$ independently represents a hydrogen atom, a deuterium atom, or a substituent. In one aspect of the invention, each of n1 to n4 independently represents an integer of 0 to 2. In a preferred aspect of the invention, at least one of n1 to n4 is 1 or more. Preferably, at least one of n1 and n2 is 1 or more, and at least one of n3 and n4 is 1 or more. In one aspect of the invention, each of n1 and n3 is independently 1 or 2, and n2 and n4 are 0. In one aspect of the invention, each of n2 and n4 is independently 1 or 2, and n1 and n3 are 0. In one aspect of the invention, each of n1 to n4 is independently 1 or 2. In one aspect of the invention, n1 and n3 are the same, and n2 and n4 are the same. In one aspect of the invention, n1 and n3 are 1, and n2 and n4 are 0. In one aspect of the invention, n1 and n3 are 0, and n2 and n4 are 1. In one aspect of the invention, n1 to n4 are all 1. The bond positions of $Ar^1$ to $Ar^4$ may be at least one of 3 and 6 positions in a carbazole ring, may be at least one of 2 and 7 positions, may be at least one of 1 and 8 positions, or may be at least one of 4 and 5 positions. The bond positions of $Ar^1$ to $Ar^4$ may be both of 3 and 6 positions in the carbazole ring, may be both of 2 and 7 positions, may be both of 1 and 8 positions, or may be both of 4 and 5 positions. For example, at least one of 3 and 6 positions may be preferably selected, or both of 3 and 6 positions may be further preferably selected. In a preferred aspect of the invention, $Ar^1$ to $Ar^4$ are all the

same group. In a preferred aspect of the invention, each of $Ar^1$ to $Ar^4$ is independently a substituted or unsubstituted aryl group, more preferably a substituted or unsubstituted phenyl group or naphthyl group, further preferably a substituted or unsubstituted phenyl group. As the substituent, a group selected from any of substituent groups A to E to be described below may be mentioned, but an unsubstituted phenyl group is also preferable. Specific preferable examples of $Ar^1$ to $Ar^4$ include a phenyl group, an o-biphenyl group, a m-biphenyl group, a p-biphenyl group, and a terphenyl group.

In one aspect of the invention, each of m1 and m2 is independently 0. In one aspect of the invention, each of m1 and m2 is independently any integer of 1 to 5. In one aspect of the invention, m1 and m2 are the same. In one aspect of the invention, $R^{41}$ and $R^{42}$ are alkyl groups having 1 to 6 carbon atoms and may be selected from, for example, alkyl groups having 1 to 3 carbon atoms, or a methyl group may be selected. When a carbon atom bonded to a boron atom is the 1-position, as the substitution position of the alkyl group, only the 2-position, only the 3-position, only the 4-position, the 3 and 5 positions, the 2 and 4 positions, the 2 and 6 positions, the 2, 4, and 6 positions, and the like may be exemplified. At least the 2-position is preferable, and at least 2 and 6 positions are more preferable.

For descriptions and preferable ranges of $A^1$ and $A^2$, corresponding descriptions on the formula (16) may be referred to.

Hereinafter, specific examples of the compound represented by the formula (16a) will be given. The compound of the formula (16a) that can be used in the invention is not construed as limiting to the following specific examples.

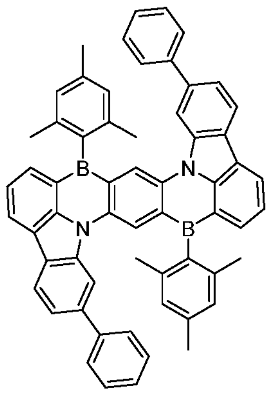
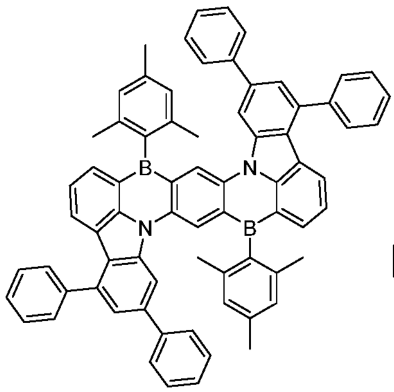
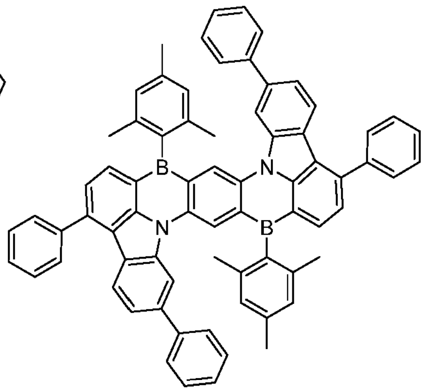
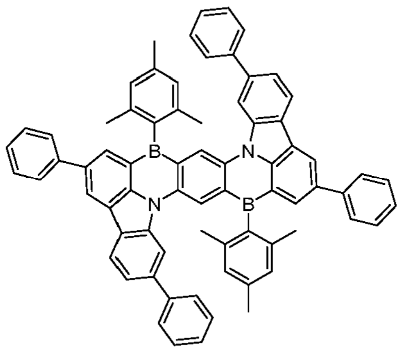
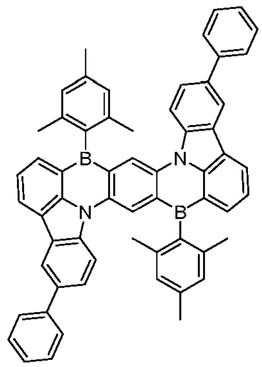
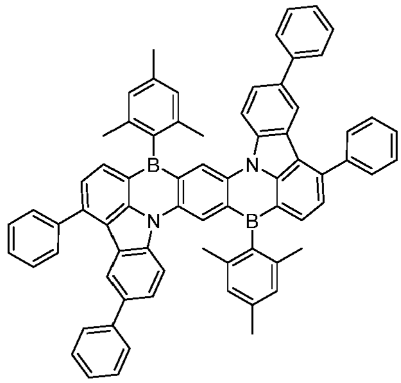
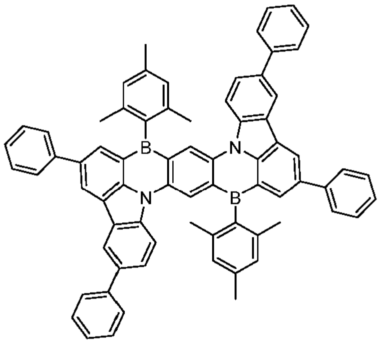
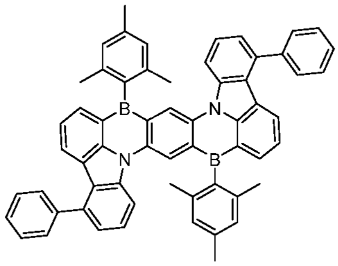
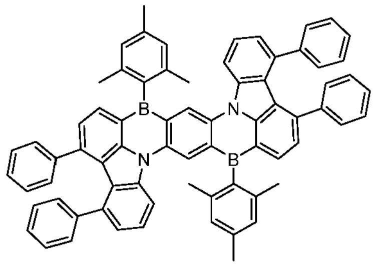

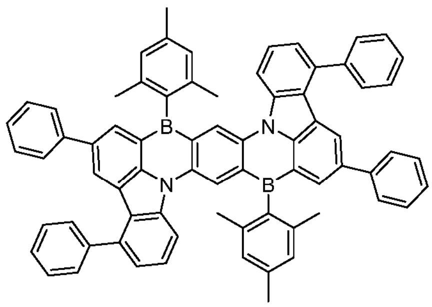
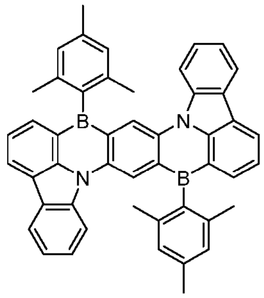
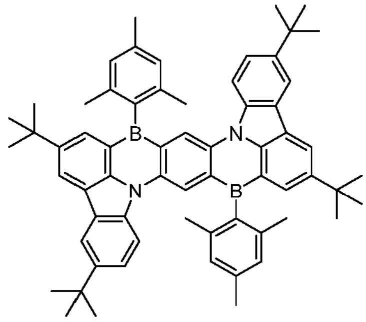

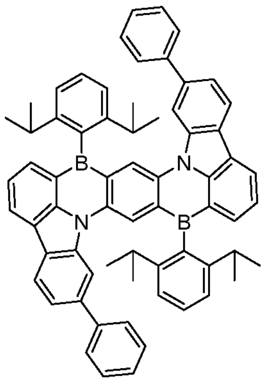
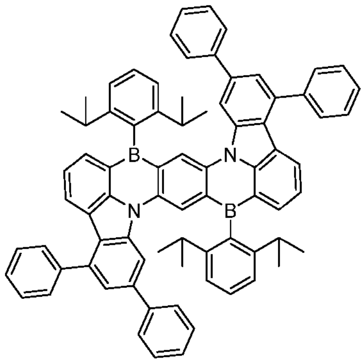
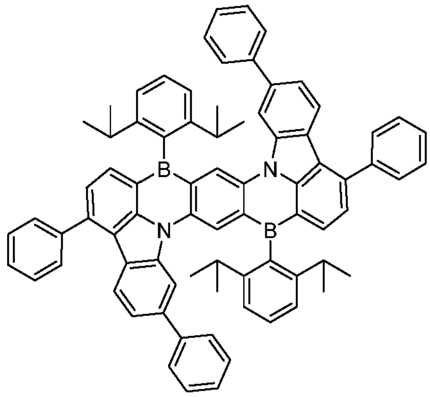

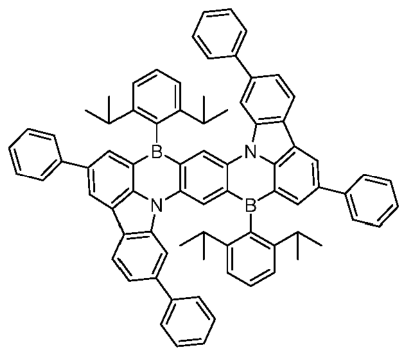
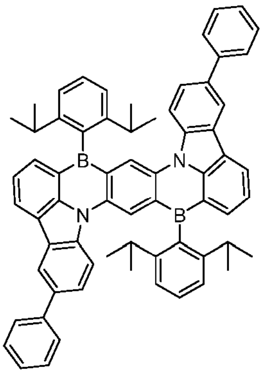
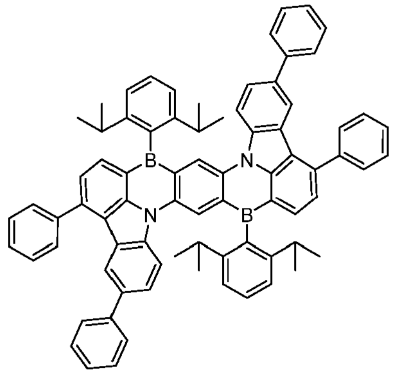

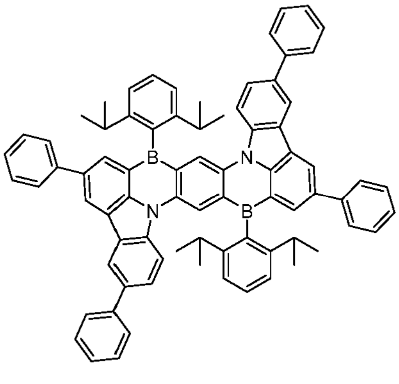
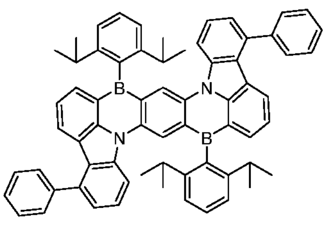
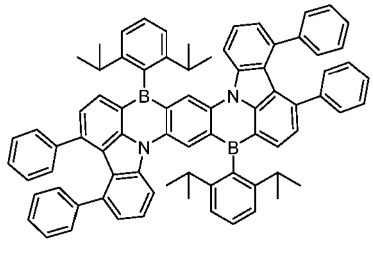

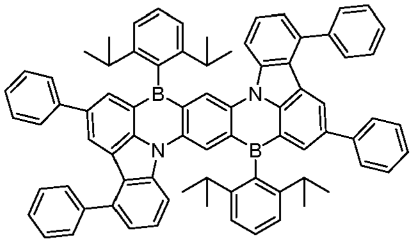
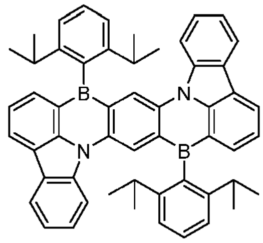
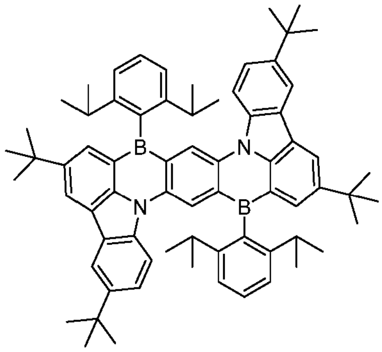

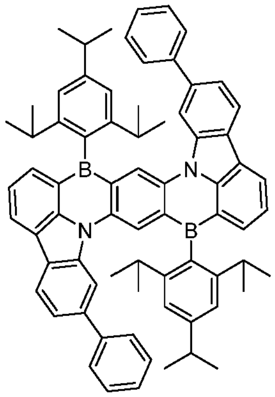
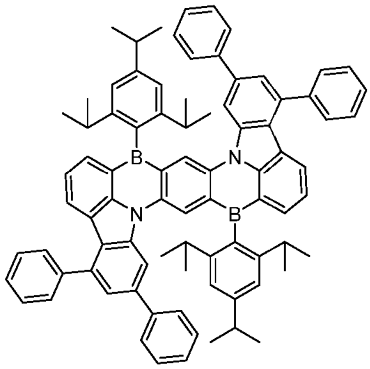
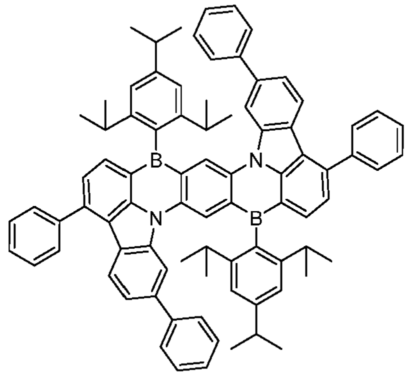

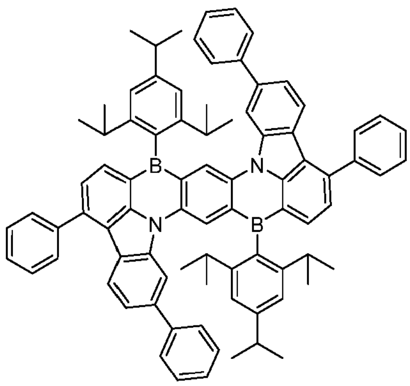
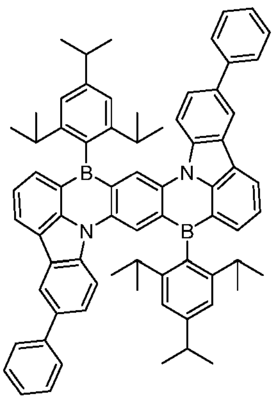
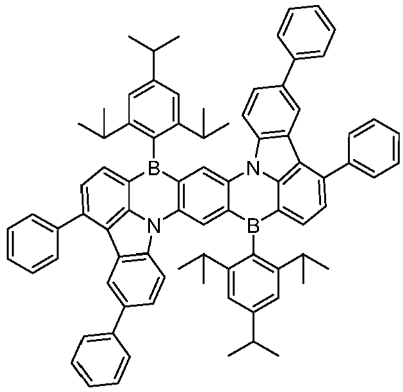

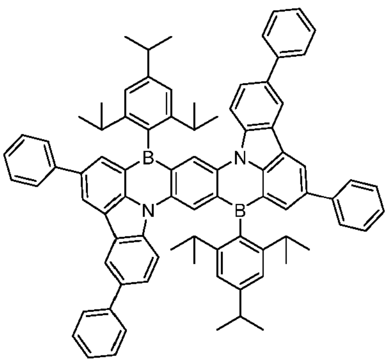
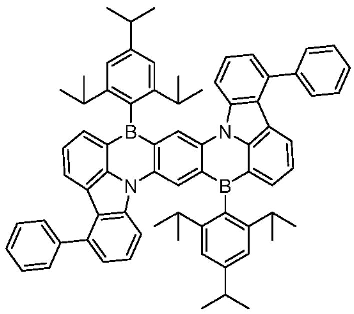
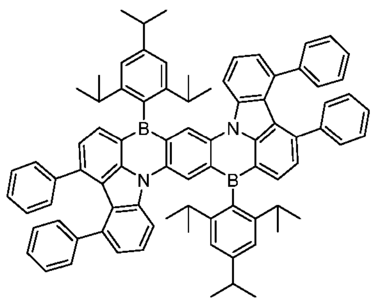

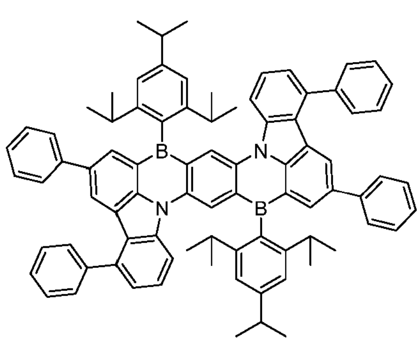 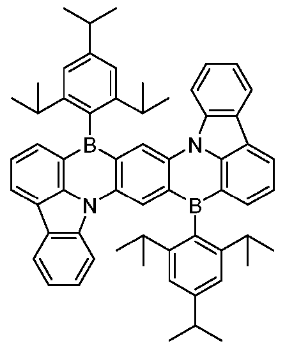 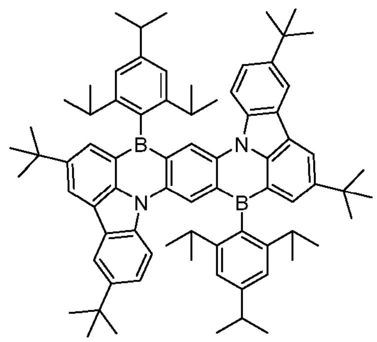

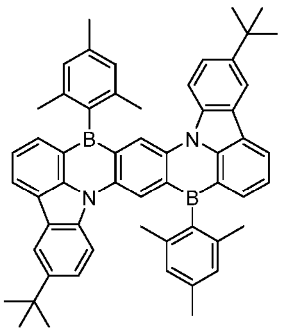 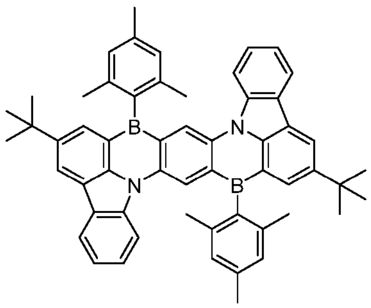 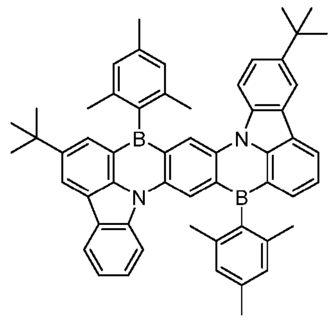

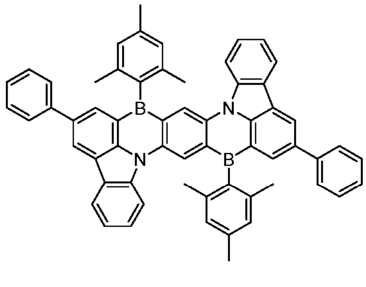 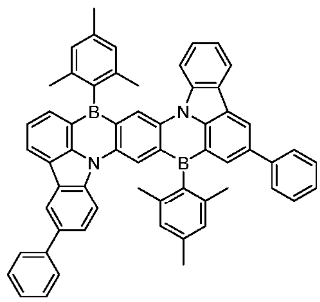 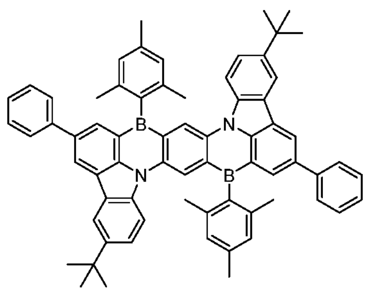

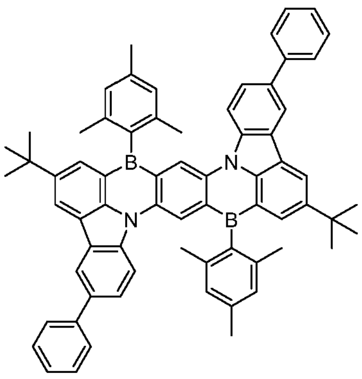 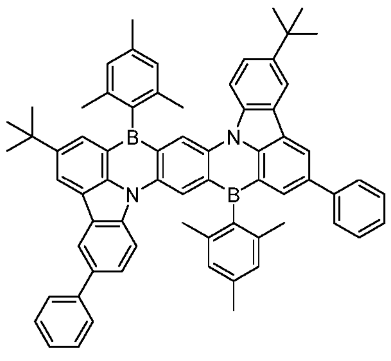 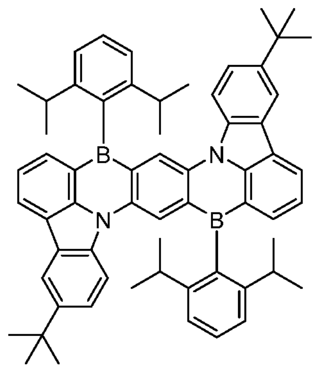

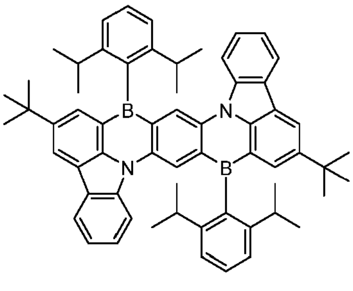 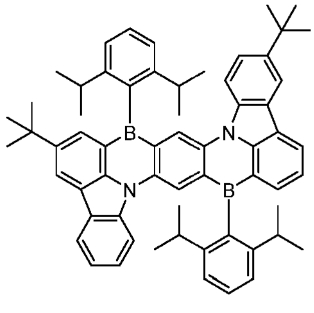 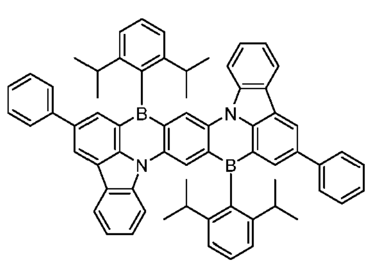

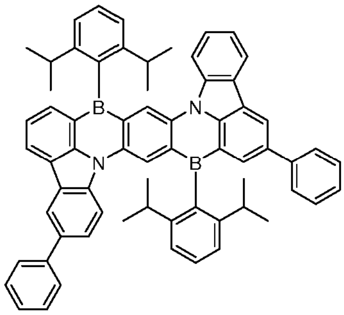
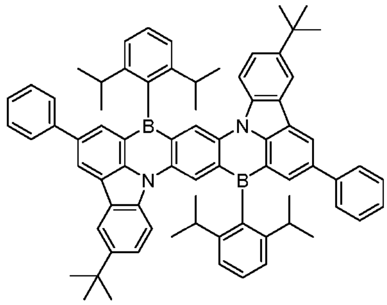
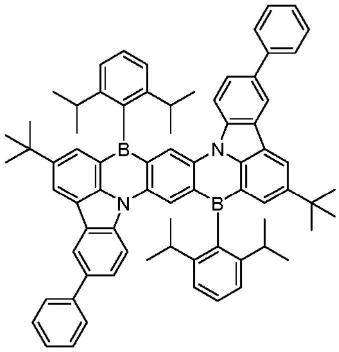
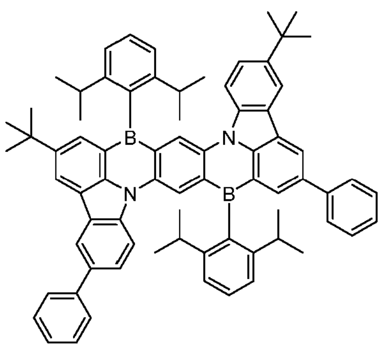
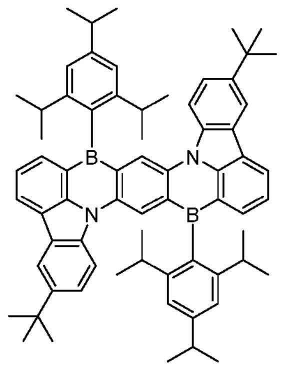
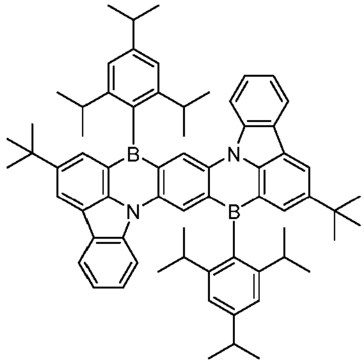
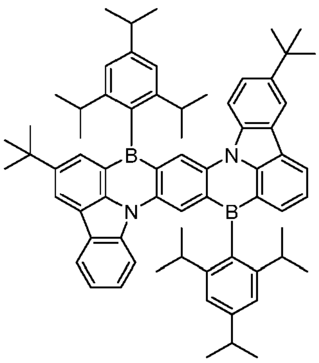
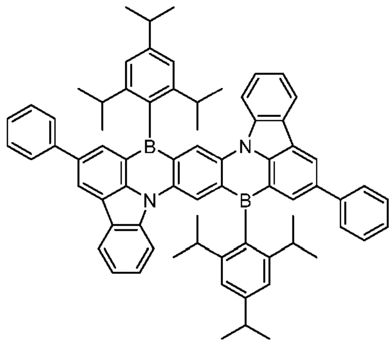
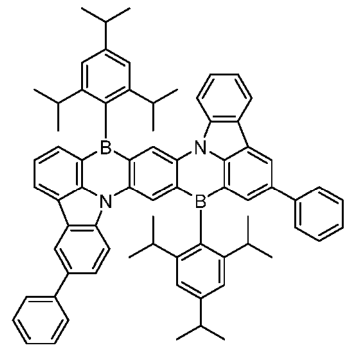
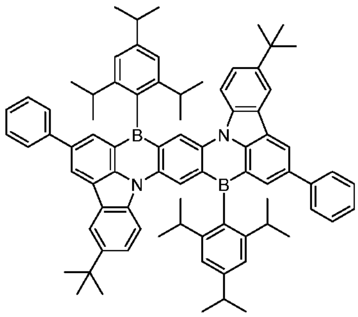
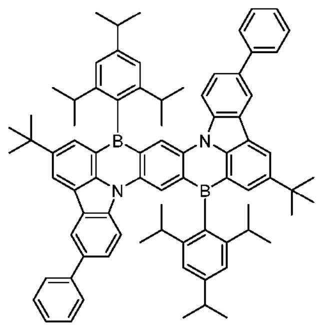
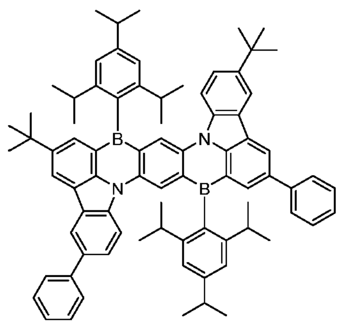

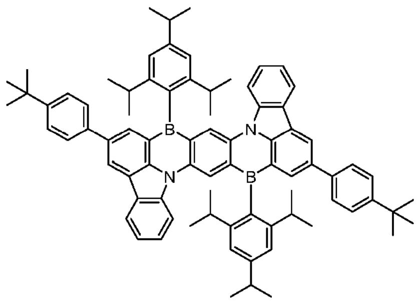
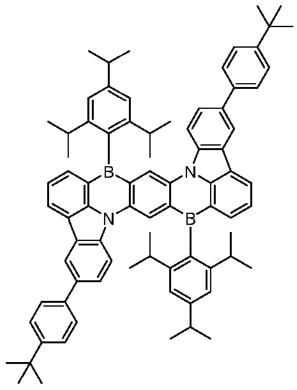
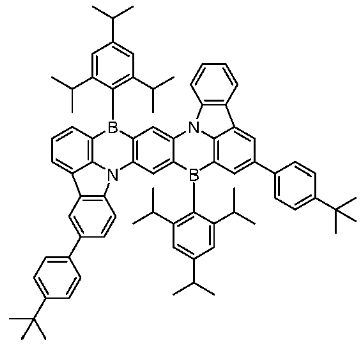
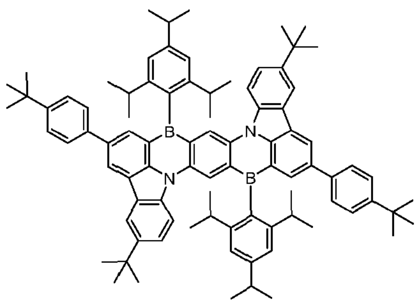
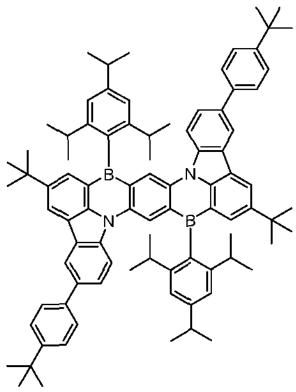
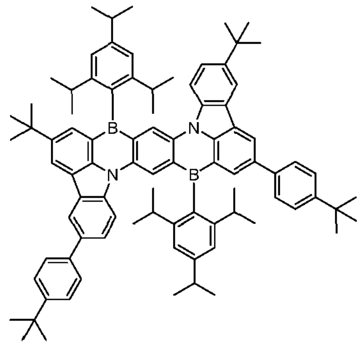
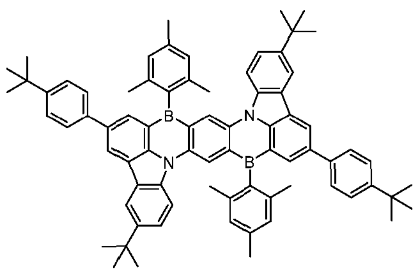
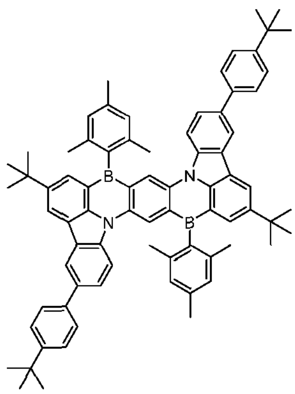
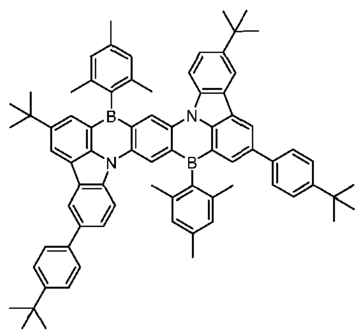
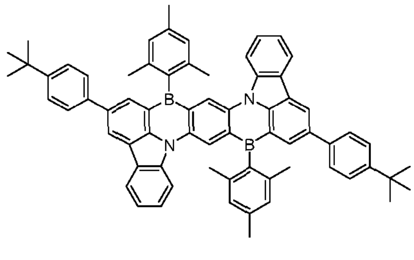
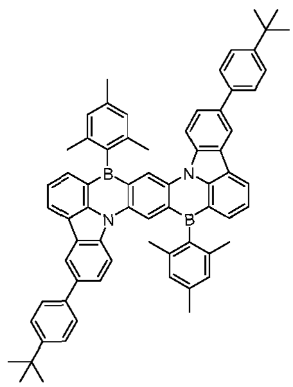
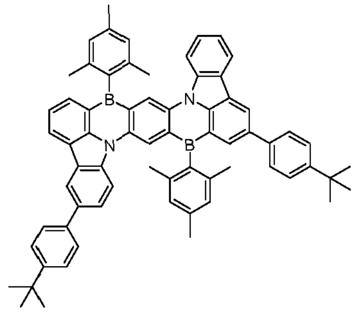

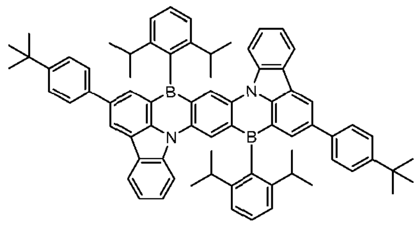

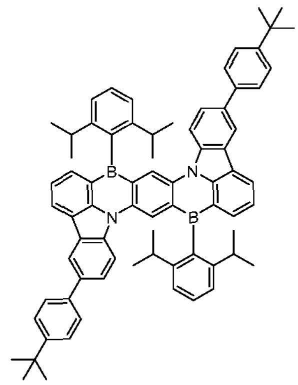

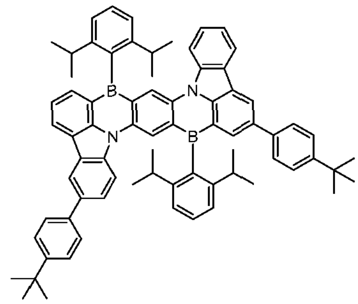

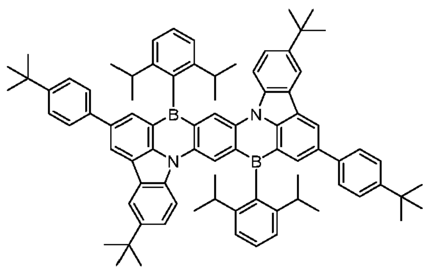

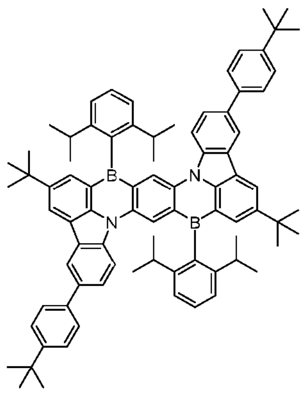

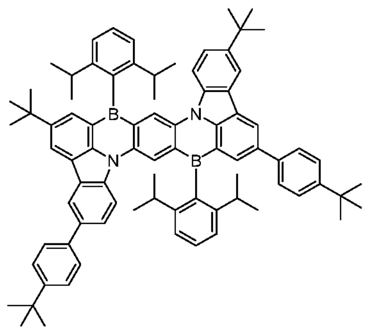

As one preferable group of compounds having the skeleton (16b), compounds represented by the following formula (16b) may be exemplified.

Formula (16b)

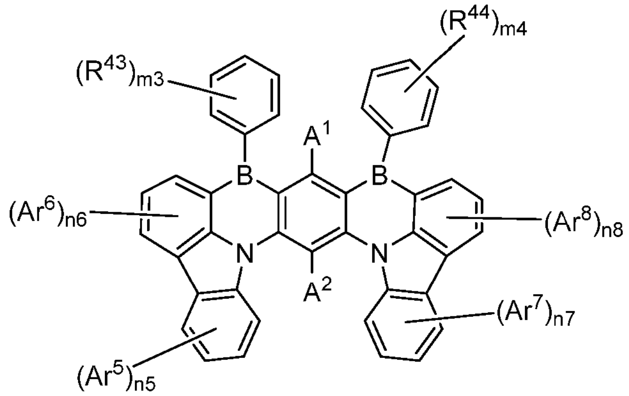

In the formula (16b), each of $Ar^5$ to $Ar^8$ independently represents a substituted or unsubstituted aryl group, or a substituted or unsubstituted heteroaryl group. Each of $R^{43}$ and $R^{44}$ independently represents a substituted or unsubstituted alkyl group. Each of m3 and m4 independently represents an integer of 0 to 5, each of n6 and n8 independently represents an integer of 0 to 3, and each of n5 and n7 independently represents an integer of 0 to 4. Each of $A^1$ and $A^2$ independently represents a hydrogen atom, a deuterium atom, or a substituent. In relation to details of $Ar^5$ to $Ar^8$, $R^{43}$ and $R^{44}$, m3 and m4, n5 to n8, $A^1$, and $A^2$, the descriptions on $Ar^1$ to $Ar^4$, $R^{41}$ and $R^{42}$, m1 and m2, n1 to n4, $A^1$, and $A^2$ in the formula (16a) may be referred to.

Hereinafter, specific examples of the compound represented by the formula (16b) will be given. Compounds of the formula (16b) that may be used in the invention are not construed as limiting to the following specific examples.

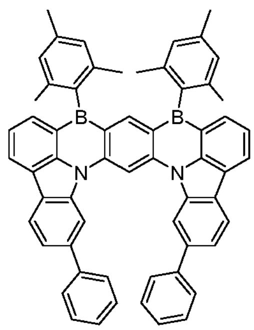 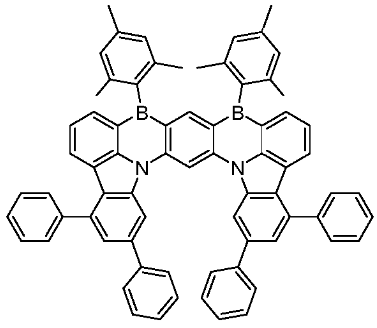 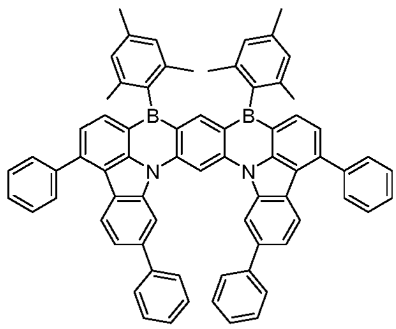

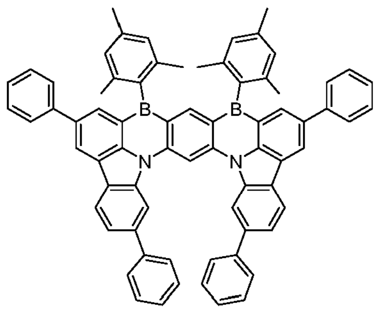 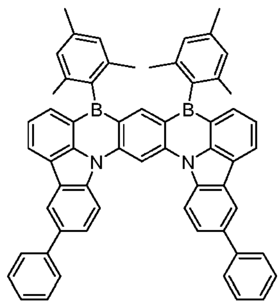 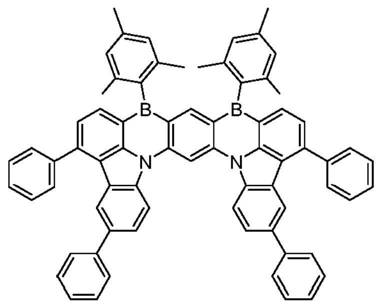

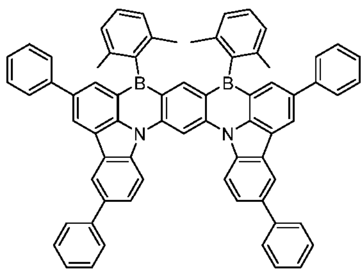 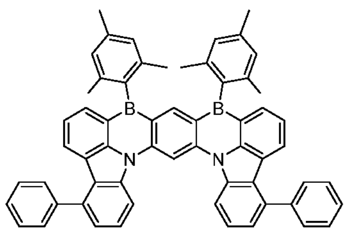 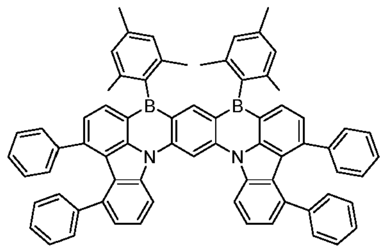

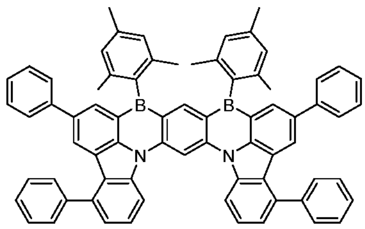 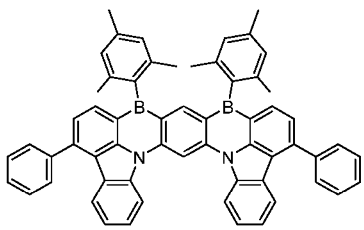 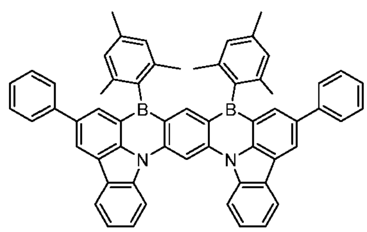

When $R^7$ and $R^8$ in the formula (16) are bonded to each other to form N-Ph, the compound of the invention has, for example, the following skeleton (17a) if $X^1$ is a nitrogen atom, and, has for example, the following skeleton (17b) if $X^2$ is a nitrogen atom. Ph is a phenyl group.

Skeleton (17a)

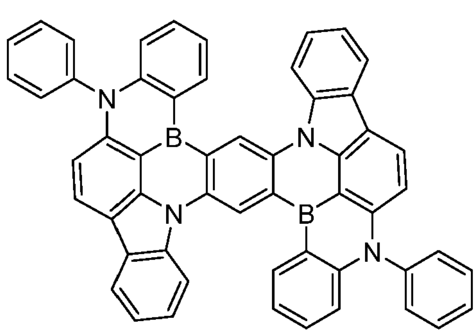

Skeleton (17b)

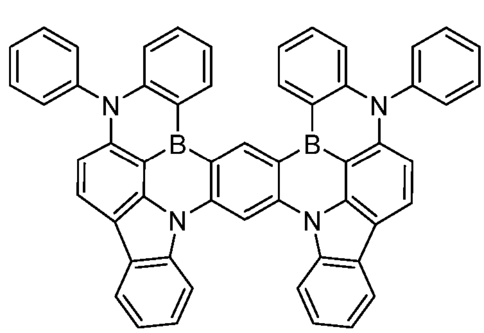

In the skeletons (17a) and (17b), each hydrogen atom may be substituted with a deuterium atom or a substituent. Further, it may be substituted with a linking group together with an adjacent hydrogen atom to form a ring structure. For details, corresponding descriptions on $R^1$ to $R^{26}$, $A^1$, and $A^2$ in the formula (16) may be referred to. At least one hydrogen atom of a benzene ring forming a carbazole partial structure included in the skeleton (17a) is substituted with a substituted or unsubstituted aryl group. In one aspect of the invention, each hydrogen atom in the skeletons (17a) and (17b) is not substituted with a linking group together with an adjacent hydrogen atom to form a ring structure.

As one preferable group of compounds having the skeleton (17a), compounds represented by the following formula (17a) may be exemplified.

Formula (17a)

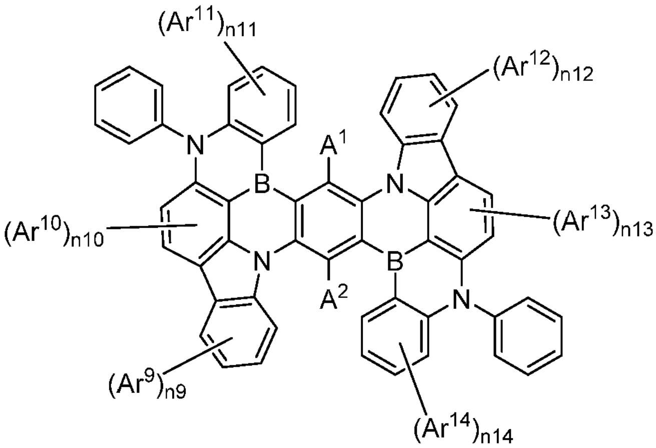

In the formula (17a), each of $Ar^9$ to $Ar^{14}$ independently represents a substituted or unsubstituted aryl group, or a substituted or unsubstituted heteroaryl group. Each of n9, n11, n12, and n14 independently represents an integer of 0 to 4, and each of n10 and n13 independently represents an integer of 0 to 2. Meanwhile, at least one of n9, n10, n12, and n13 is 1 or more. Each of $A^1$, and $A^2$ independently represents a hydrogen atom, a deuterium atom, or a substituent.

In one aspect of the invention, each of n9 to n14 independently represents an integer of 0 to 2. In one aspect of the invention, at least one of n9 to n14 is 1 or more. For example, n9 and n12 may be 1 or more or n10 and n13 may be 1 or more. In a preferred aspect of the invention, at least one of n9, n10, n12, and n13 is 1 or more. In one aspect of the invention, each of n9 and n12 is independently 1 or 2, and n10, n11, n13, and n14 are 0. In one aspect of the invention, each of n10 and n13 is independently 1 or 2, and n9, n11, n12, and n14 are 0. In one aspect of the invention, each of n9 and n12 is independently 1 or 2, each of n10 and n13 is independently 1 or 2, and n11 and n14 are 0. In one aspect of the invention, n9 to n14 are all 1. The bond positions of $Ar^9$ to $Ar^{14}$ may be 3 and 6 positions of a carbazole ring, or may be other positions. In a preferred aspect of the invention, $Ar^9$ to $Ar^{14}$ are all the same group. For preferable groups for $Ar^9$ to $Ar^{14}$, corresponding descriptions on $Ar^1$ to $Ar^4$ may be referred to. For descriptions and preferable ranges of $A^1$ and $A^2$, corresponding descriptions on the formula (16) may be referred to.

Hereinafter, specific examples of the compound represented by the formula (17a) will be given. Compounds of the formula (17a) that may be used in the invention are not construed as limiting to the following specific examples.

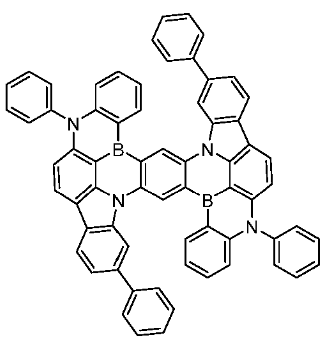

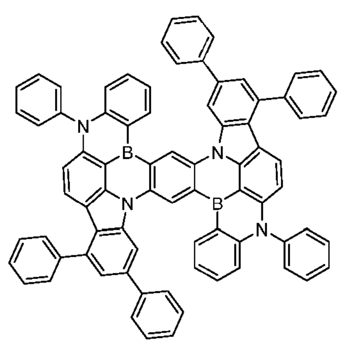

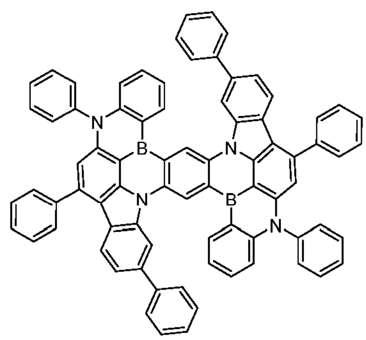

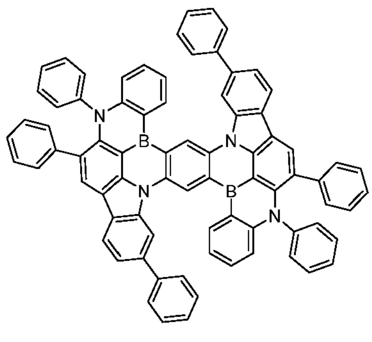
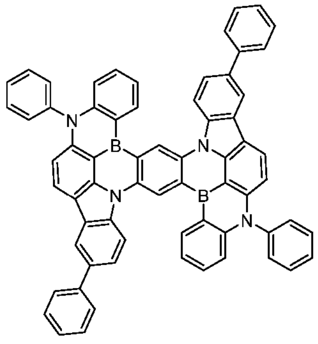
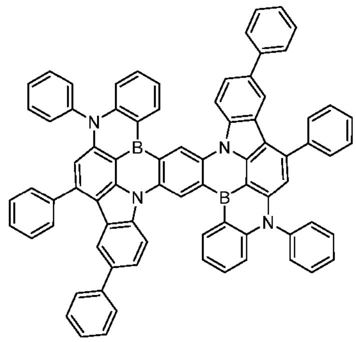

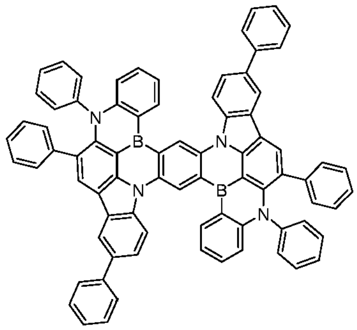
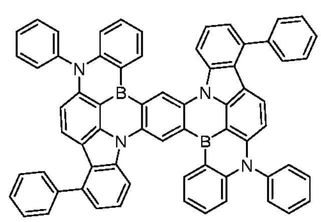
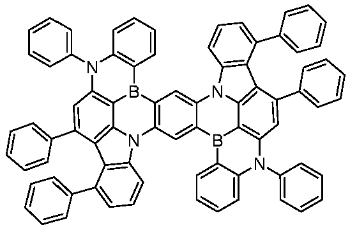

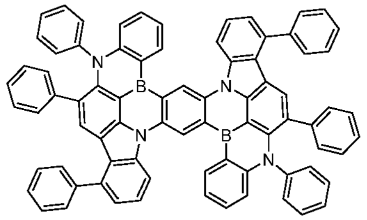
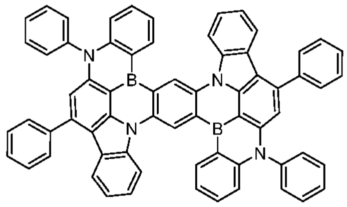
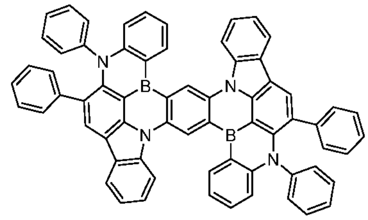

As one preferable group of compounds having the skeleton (17b), compounds represented by the following formula (17b) may be exemplified.

Formula (17b)

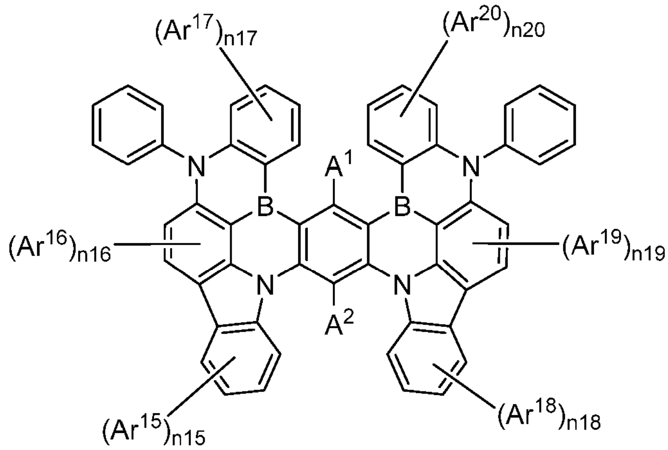

In the formula (17b), each of $Ar^{15}$ to $Ar^{20}$ independently represents a substituted or unsubstituted aryl group, or a substituted or unsubstituted heteroaryl group. Each of n15, n17, n18, and n20 independently represents an integer of 0 to 4, and each of n16 and n19 independently represents an integer of 0 to 2. Each of $A^1$, and $A^2$ independently represents a hydrogen atom, a deuterium atom, or a substituent. For details of $Ar^{15}$ to $Ar^{20}$, n15 to n20, $A^1$, and $A^2$, descriptions on $Ar^9$ to $Ar^{14}$, n9 to n14, $A^1$, and $A^2$ in the formula (17a) may be referred to in this order.

Hereinafter, specific examples of the compound represented by the formula (17b) will be given. Compounds of the formula (17b) that may be used in the invention are not construed as limiting to the following specific examples.

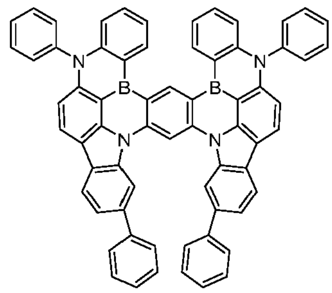
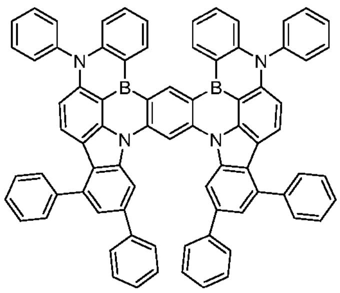
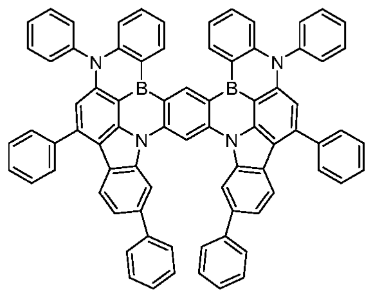

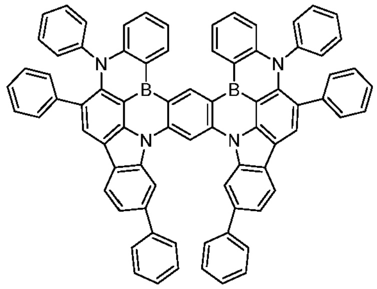
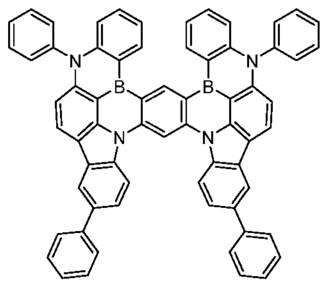
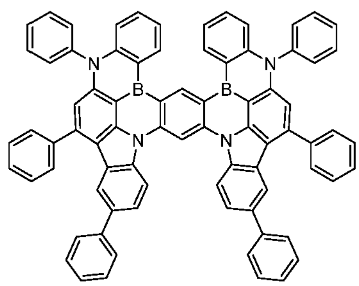

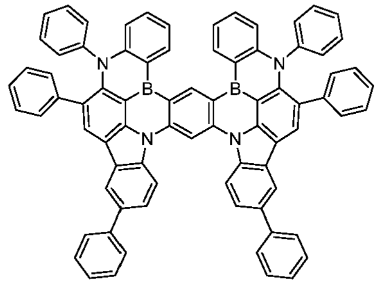
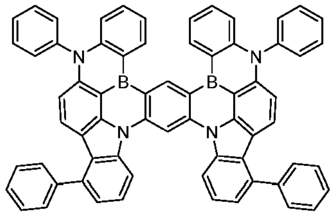
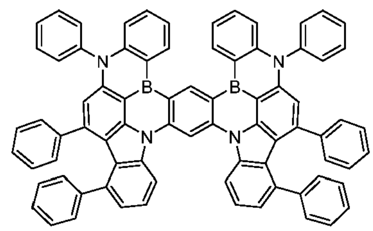

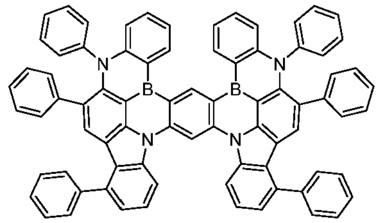
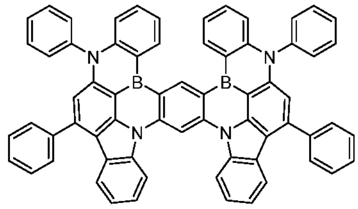
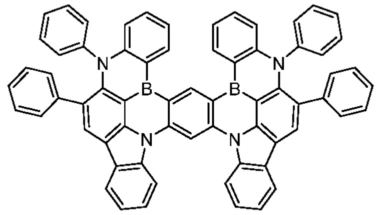

When $R^7$ and $R^8$ in the formula (16) are bonded to each other to form a single bond, the compound of the invention has, for example, the following skeleton (18a) if $X^1$ is a nitrogen atom, and has, for example, the following skeleton (18b) if $X^2$ is a nitrogen atom.

Skeleton (18a)

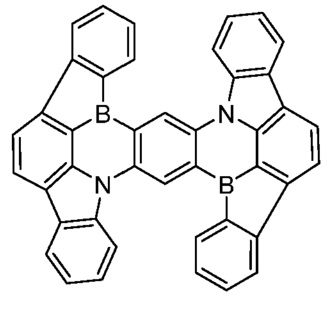

Skeleton (18b)

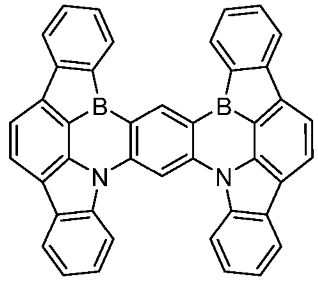

In the skeletons (18a) and (18b), each hydrogen atom may be substituted with a deuterium atom or a substituent. Further, it may be substituted with a linking group together with an adjacent hydrogen atom to form a ring structure. For details, corresponding descriptions on $R^1$ to $R^{26}$, $A^1$, and $A^2$ in the formula (16) may be referred to. In one aspect of the invention, each hydrogen atom in the skeletons (18a) and (18b) is not substituted with a linking group together with an adjacent hydrogen atom to form a ring structure.

As one preferable group of compounds having the skeleton (18a), compounds represented by the following formula (18a) may be exemplified.

Formula (18a)

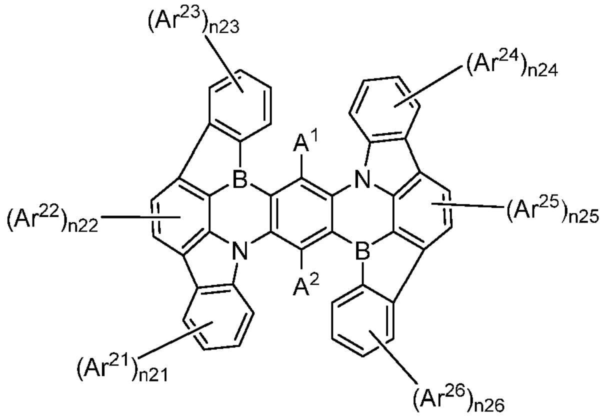

In the formula (18a), each of $Ar^{21}$ to $Ar^{26}$ independently represents a substituted or unsubstituted aryl group, or a substituted or unsubstituted heteroaryl group. Each of n21, n23, n24, and n26 independently represents an integer of 0 to 4, and each of n22 and n25 independently represents an integer of 0 to 2. Each of $A^1$ and $A^2$ independently represents a hydrogen atom, a deuterium atom, or a substituent. For details of $Ar^{21}$ to $Ar^{25}$, and n21 to n25, descriptions on $Ar^9$ to $Ar^{14}$, n9 to n14, $A^1$, and $A^2$ in the formula (17a) may be referred to.

Hereinafter, specific examples of the compound represented by the formula (18a) will be given. Compounds of the formula (18a) that may be used in the invention are not construed as limiting to the following specific examples.

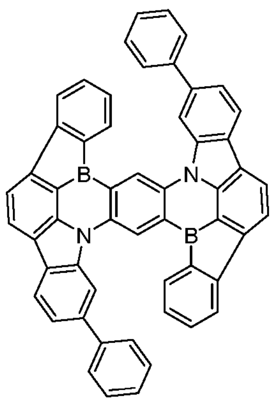

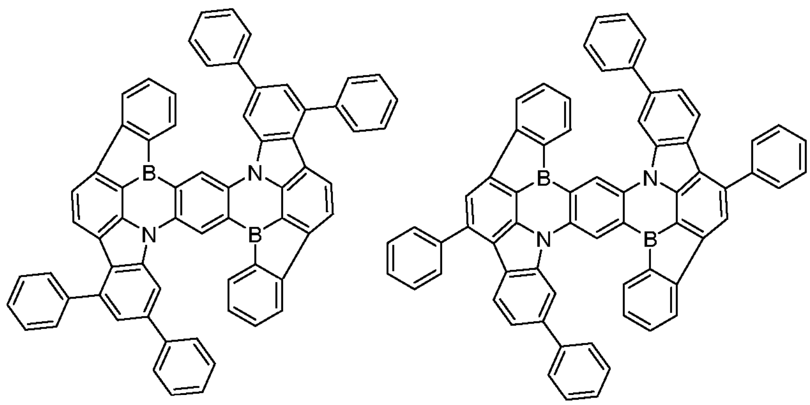

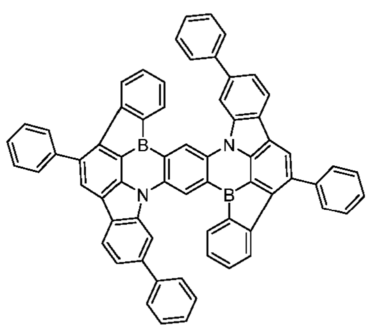

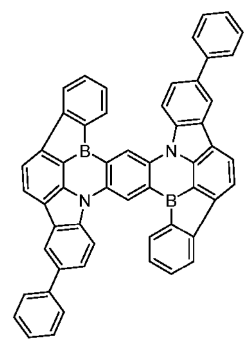

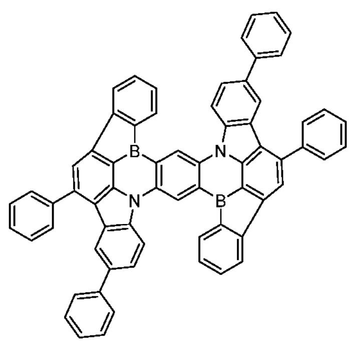

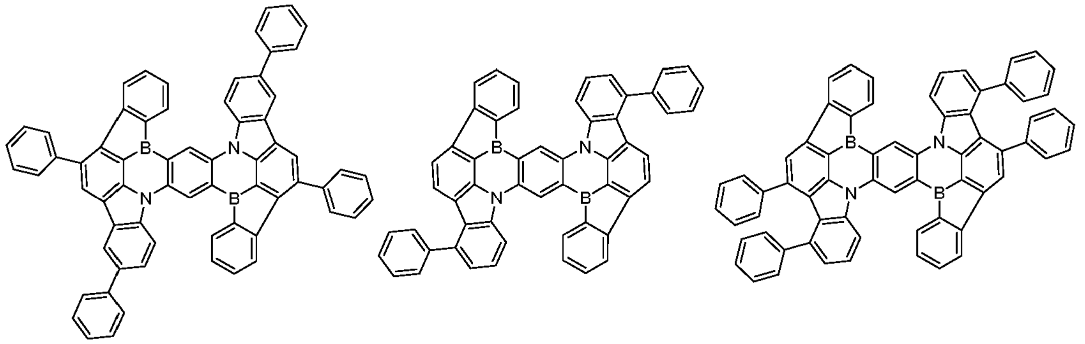

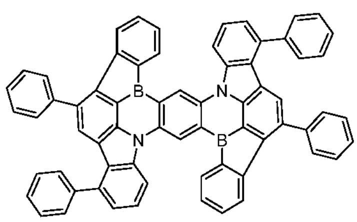

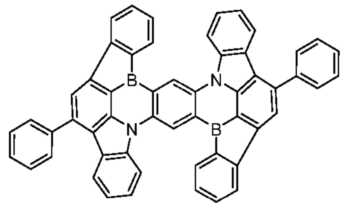

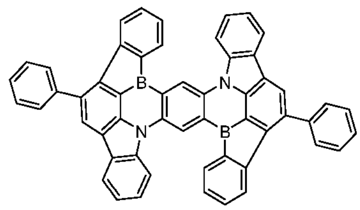

As one preferable group of compounds having the skeleton (18b), compounds represented by the following formula (18b) may be exemplified.

Formula (18b)

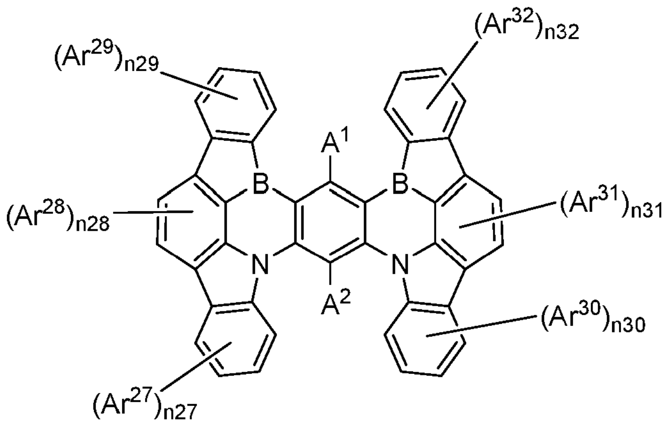

In the formula (18b), each of $Ar^{27}$ to $Ar^{32}$ independently represents a substituted or unsubstituted aryl group, or a substituted or unsubstituted heteroaryl group. Each of n27, n29, n30, and n32 independently represents an integer of 0 to 4, and each of n28 and n31 independently represents an integer of 0 to 2. Each of $A^1$ and $A^2$ independently represents a hydrogen atom, a deuterium atom, or a substituent. For details of $Ar^{27}$ to $Ar^{32}$, n27 to n32, $A^1$, and $A^2$, descriptions on $Ar^{15}$ to $Ar^{20}$, n15 to n20, $A^1$, and $A^2$ in the formula (17b) may be referred to in this order.

Hereinafter, specific examples of the compound represented by the formula (18b) will be given. Compounds of the formula (18b) that may be used in the invention are not construed as limiting to the following specific examples.

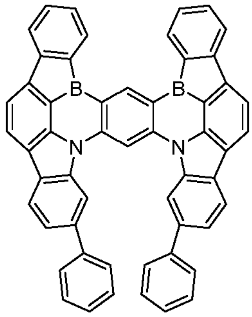
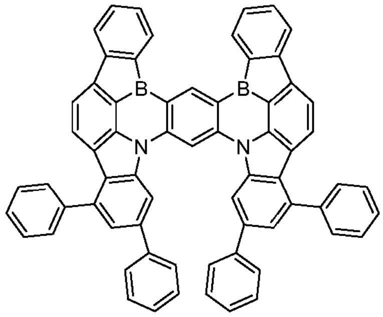
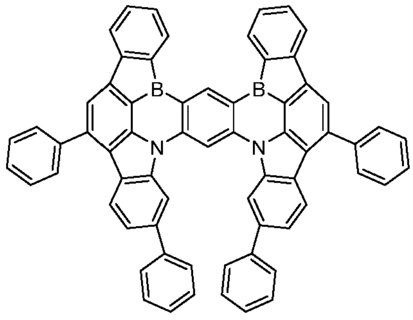
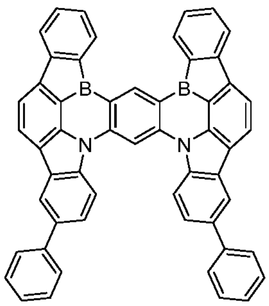
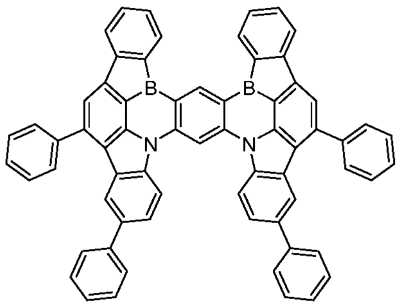
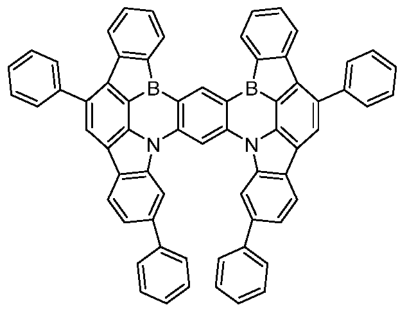
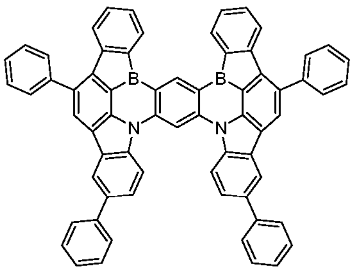
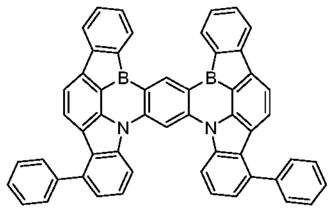
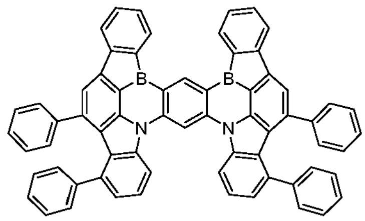
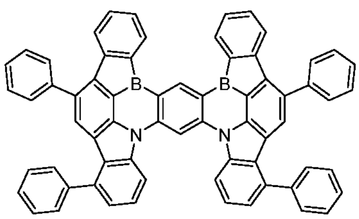
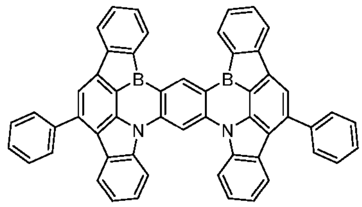
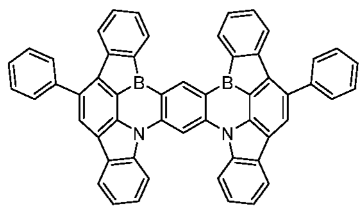

In a preferred aspect of the invention, compounds in which another ring is condensed with two benzene rings forming a carbazole partial structure existing in the formula (16) are selected. Among them, a compound in which a benzofuran ring is condensed, a compound in which a benzothiophene ring is condensed, and a compound in which a benzene ring is condensed may be particularly preferably selected. Hereinafter, compounds in which these rings are condensed will be described with reference to specific examples.

A compound in which a benzofuran ring or a benzothiophene ring is condensed with a benzene ring to which a boron atom is not directly bonded, between two benzene rings forming a carbazole partial structure existing in the formula (16), may be preferably mentioned. Examples of such a compound include a compound having the following skeleton (19a), and a compound having the following skeleton (19b).

Skeleton (19a)

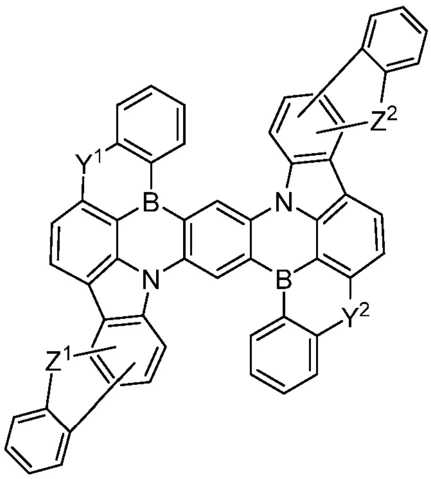

Skeleton (19b)

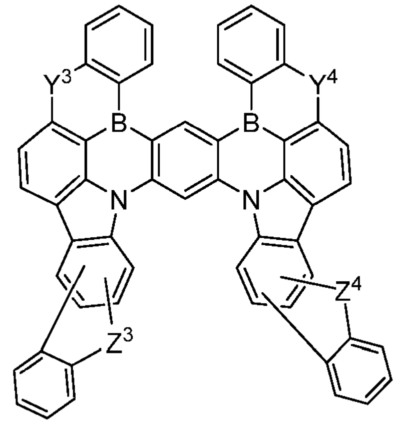

In the skeletons (19a) and (19b), each of $Y^1$ to $Y^4$ independently represents two hydrogen atoms, a single bond or $N(R^{27})$. Two hydrogen atoms mentioned herein indicate a state where two benzene rings bonded to a boron atom are not linked to each other. It is preferable that $Y^1$ and $Y^2$ are the same, and $Y^3$ and $Y^4$ are the same, but they may be different from each other. In one aspect of the invention, $Y^1$ to $Y^4$ are single bonds. In one aspect of the invention, $Y^1$ to $Y^4$ are $N(R^{27})$. $R^{27}$ represents a hydrogen atom, a deuterium atom, or a substituent.

Each of $Z^1$ to $Z^4$ independently represents an oxygen atom or a sulfur atom. It is preferable that $Z^1$ and $Z^2$ are the same, and $Z^3$ and $Z^4$ are the same, but they may be different from each other. In one aspect of the invention, $Z^1$ to $Z^4$ are oxygen atoms. Here, a furan ring of benzofuran is condensed with the benzene ring forming the carbazole partial structure in (19a) and (19b). The orientation of the condensed furan ring is not limited. In one aspect of the invention, $Z^1$ to $Z^4$ are sulfur atoms. Here, a thiophene ring of benzothiophene is condensed with the benzene ring forming the carbazole partial structure in (19a) and (19b). The orientation of the condensed thiophene ring is not limited.

Each hydrogen atom in the skeletons (19a) and (19b) may be substituted with a deuterium atom or a substituent. Further, it may be substituted with a linking group together with an adjacent hydrogen atom to form a ring structure. For details, corresponding descriptions on $R^1$ to $R^{26}$, $A^1$, and $A^2$ in the formula (16) may be referred to. In one aspect of the invention, each hydrogen atom in the skeletons (19a) and (19b) is not substituted with a linking group together with an adjacent hydrogen atom to form a ring structure.

As one preferable group of compounds having the skeleton (19a), compounds represented by the following formula (19a) may be exemplified. It is assumed that X in specific examples is an oxygen atom or a sulfur atom, and a compound in which X is an oxygen atom and a compound in which X is a sulfur atom are disclosed, respectively. Further, in specific examples of compounds represented by other subsequent formulas, X has the same meaning.

Formula (19a)

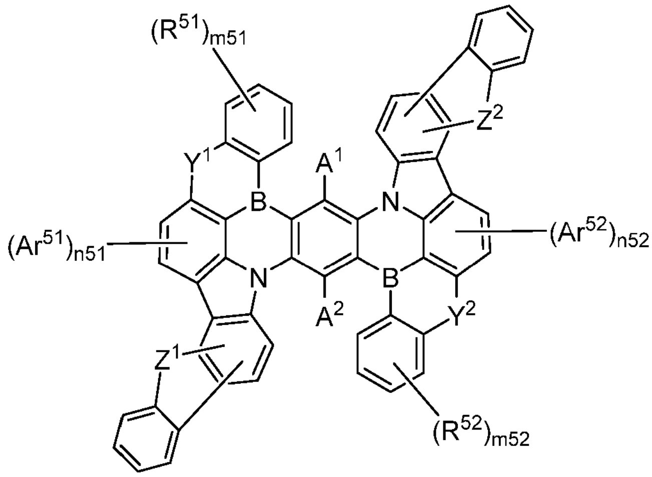

In the formula (19a), each of $Ar^{51}$ and $Ar^{52}$ independently represents a substituted or unsubstituted aryl group, or a substituted or unsubstituted heteroaryl group. Each of $R^{51}$ and $R^{52}$ independently represents a substituted or unsubstituted alkyl group. Each of m51 and m52 independently represents an integer of 0 to 4. Each of n51 and n52 independently represents an integer of 0 to 2. Each of $Y^1$ to $Y^4$ independently represents two hydrogen atoms, a single bond or $N(R^{27})$. $R^{27}$ represents a hydrogen atom, a deuterium atom, or a substituent. Each of $Z^1$ to $Z^4$ independently represents an oxygen atom or a sulfur atom. Each of $A^1$ and $A^2$ independently represents a hydrogen atom, a deuterium atom, or a substituent.

In one aspect of the invention, n51 and n52 are the same number. For example, n51 and n52 may be 0, and n51 and n52 may be 1. In one aspect of the invention, m51 and m52 are the same number. In one aspect of the invention, m51 and m52 are integers of 0 to 3. For example, m51 and m52 may be 0, m51 and m52 may be 1, m51 and m52 may be 2, and m51 and m52 may be 3. In relation to preferable groups for $Ar^{51}$, $Ar^{52}$, $R^{51}$, $R^{52}$, $A^1$, and $A^2$, corresponding descriptions on $Ar^1$ to $Ar^4$, $R^{41}$ to $R^{42}$, $A^1$, and $A^2$ in the formula (16a) may be referred to.

Hereinafter, specific examples of the compound represented by the formula (19a) will be given. Compounds of the formula (19a) that may be used in the invention are not construed as limiting to the following specific examples. In relation to specific examples including X, it is assumed that a compound in which all X's in the molecule are oxygen atoms, and a compound in which all X's in the molecule are sulfur atoms are disclosed, respectively. A compound in which some of X's in the molecule are oxygen atoms, and the rest are sulfur atoms may also be adopted.

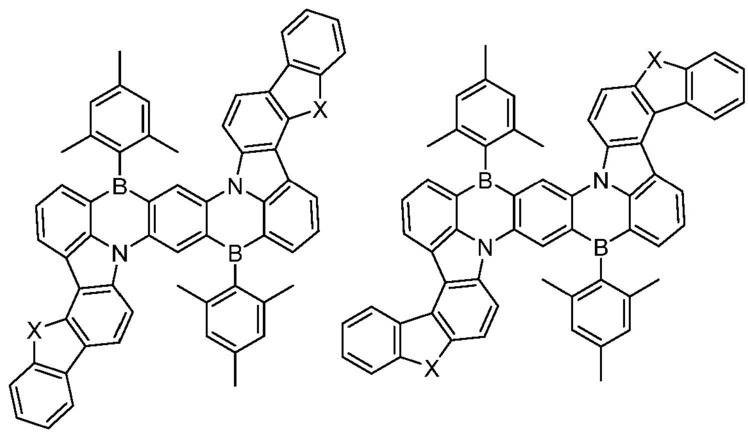

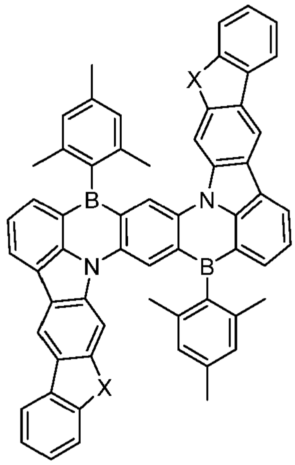

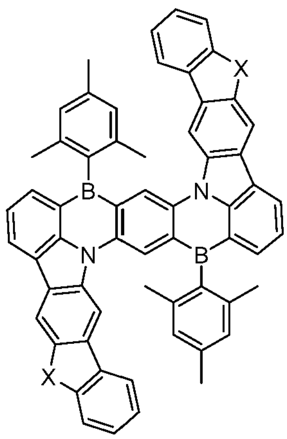

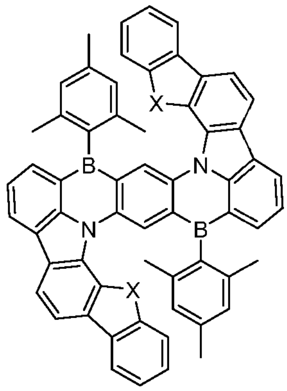

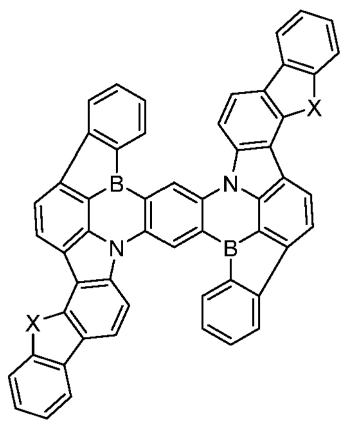

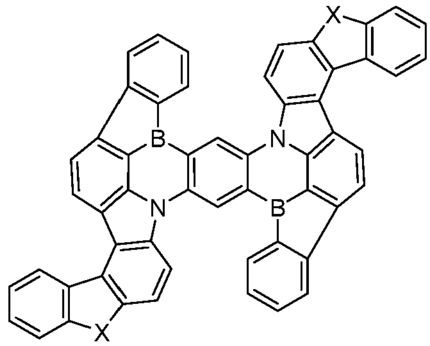
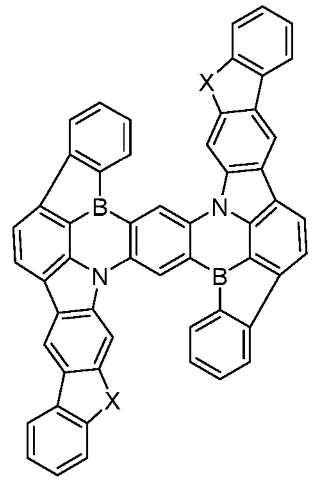
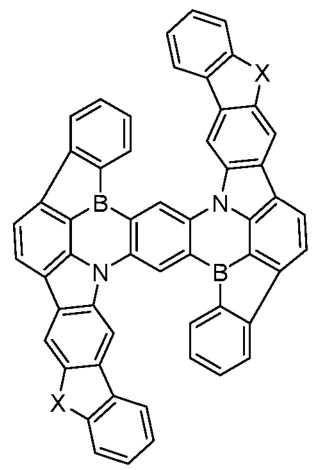

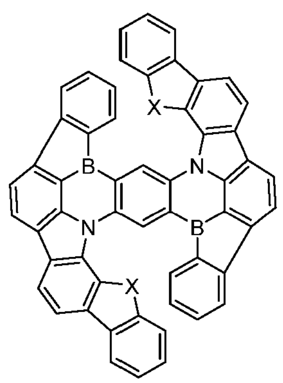
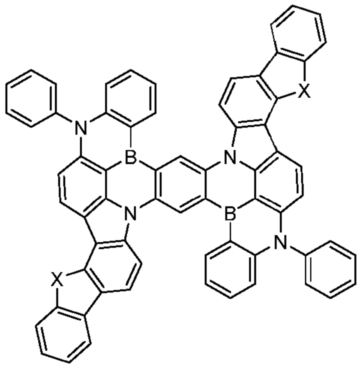
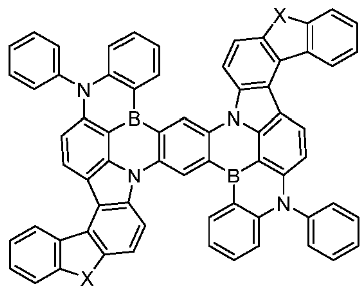

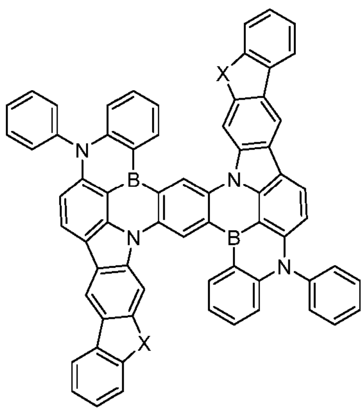
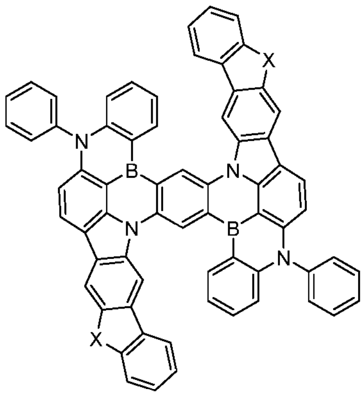
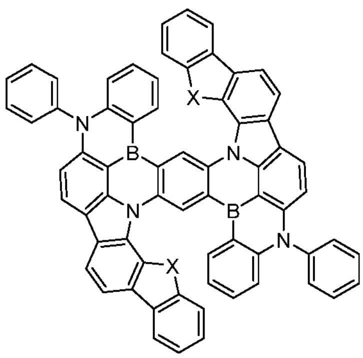

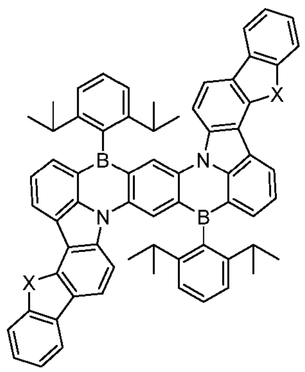
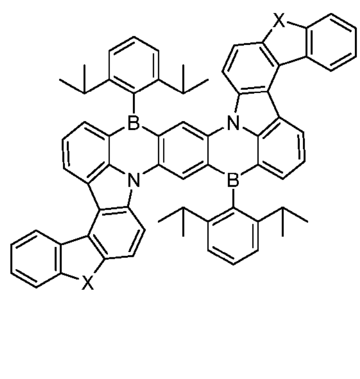
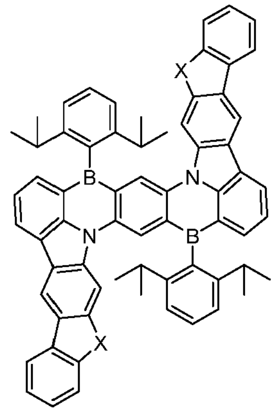

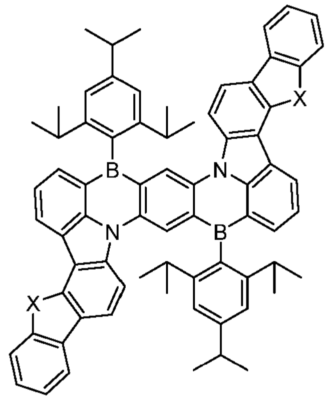
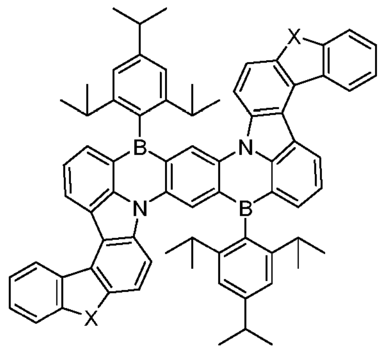
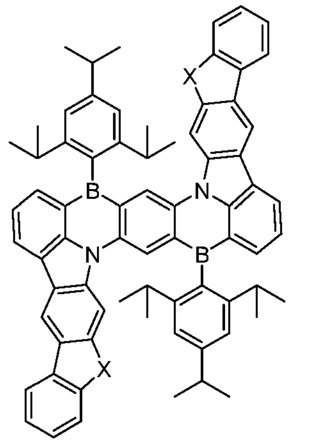

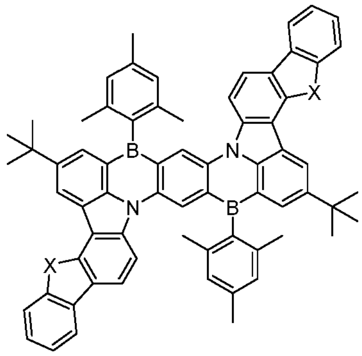
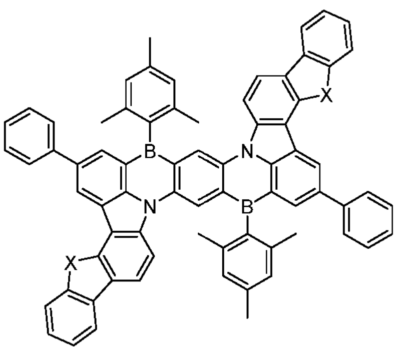
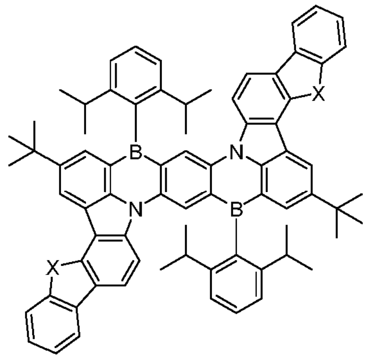

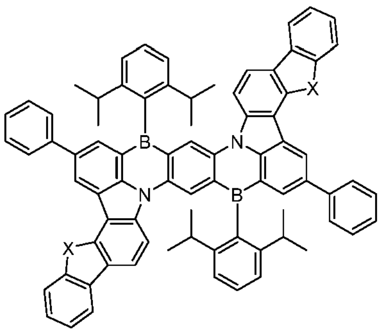
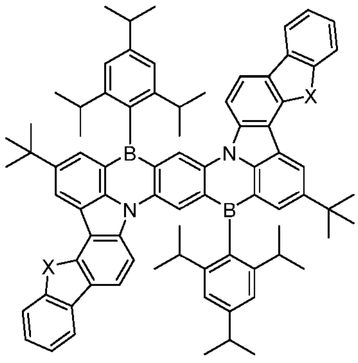
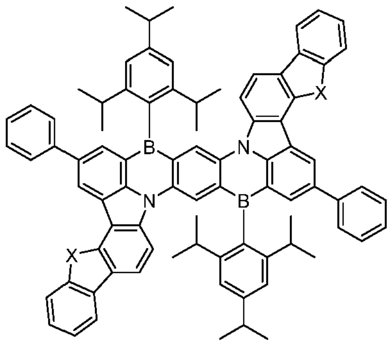

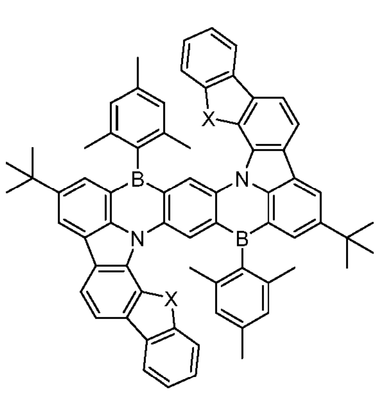
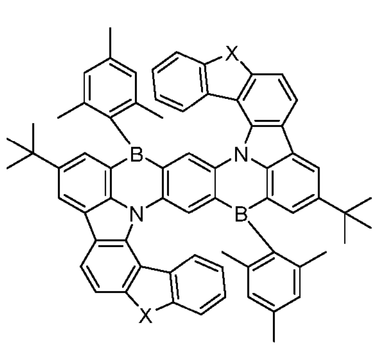
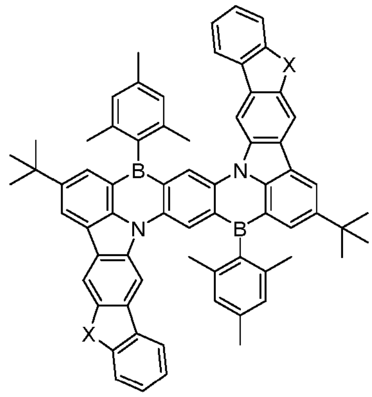

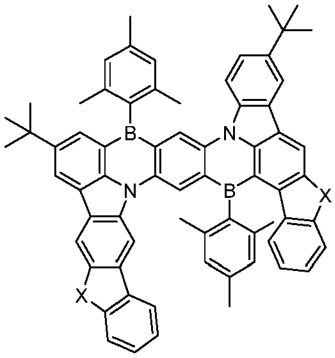
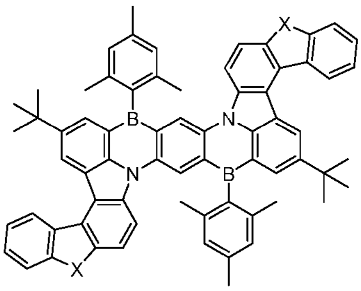
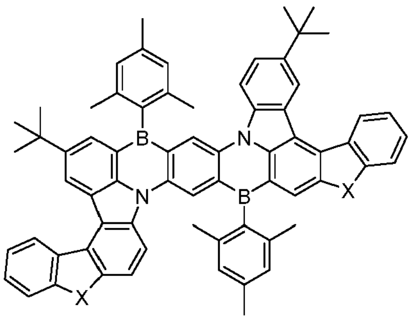

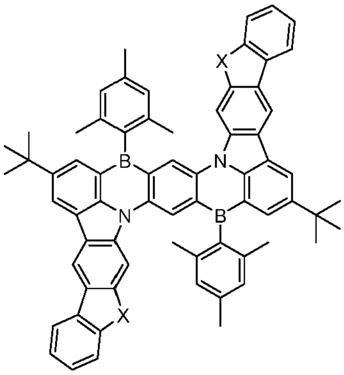
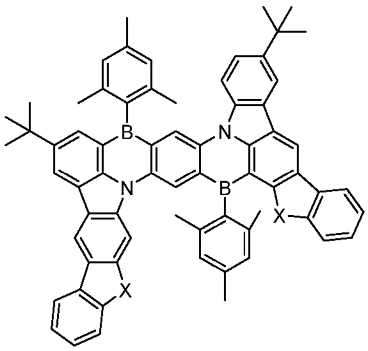
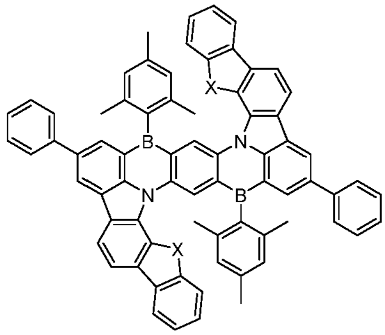

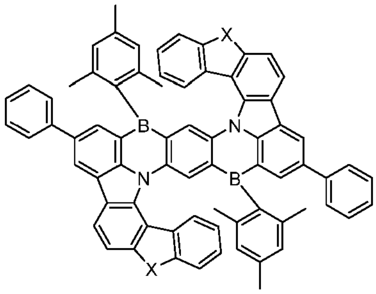
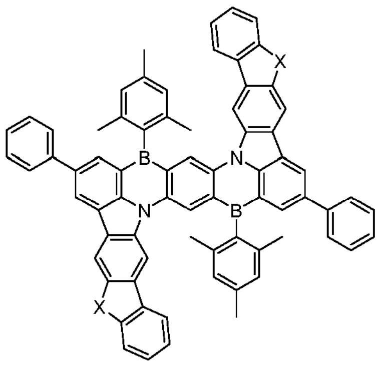
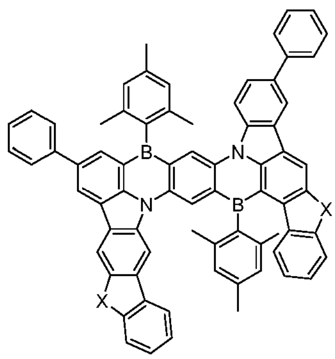

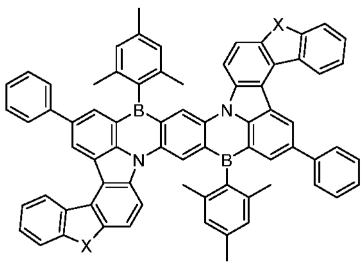
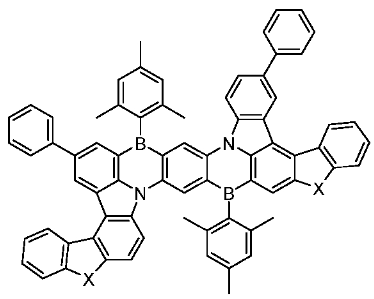
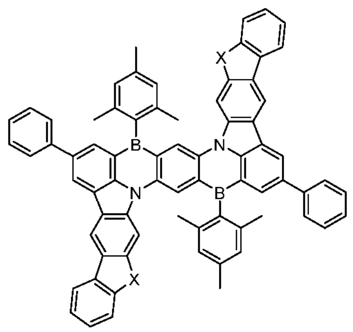

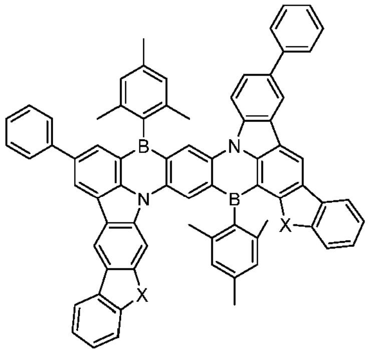
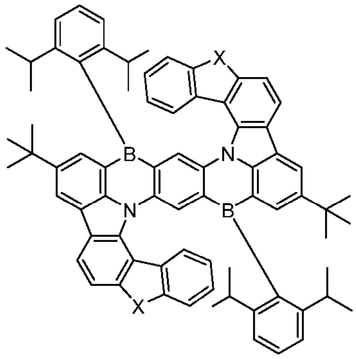
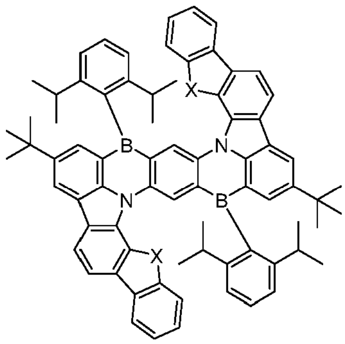
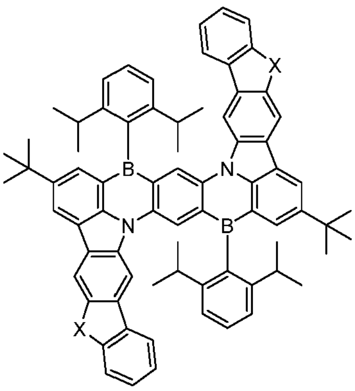
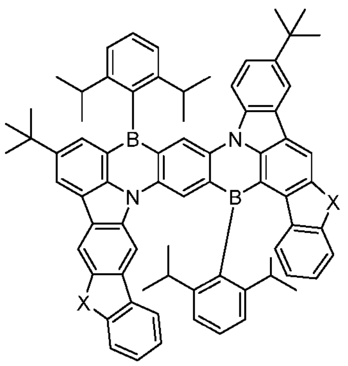
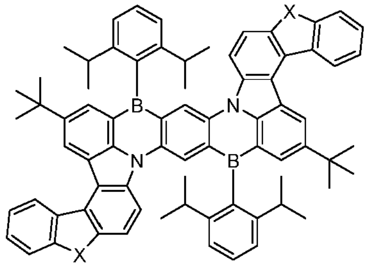
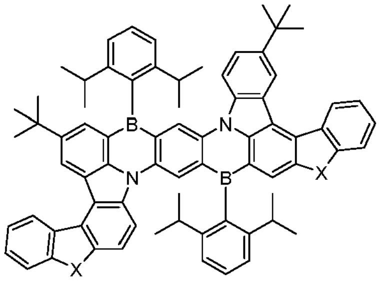
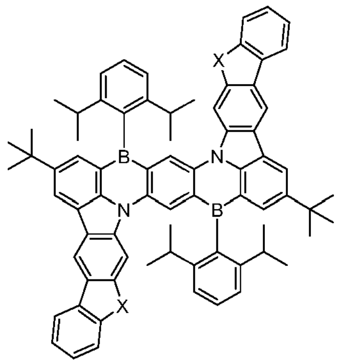
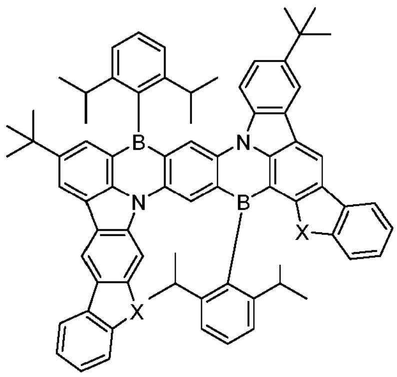
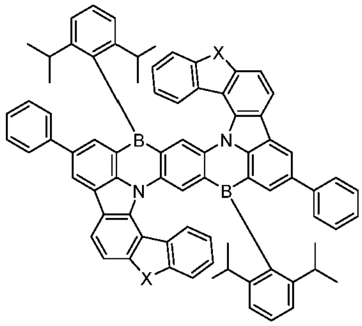
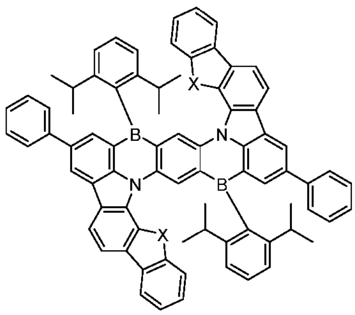
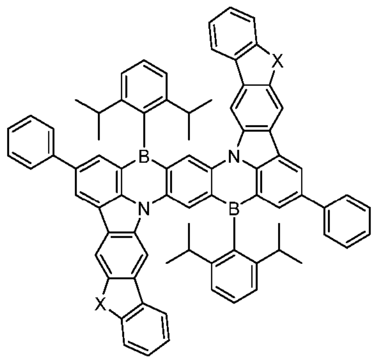

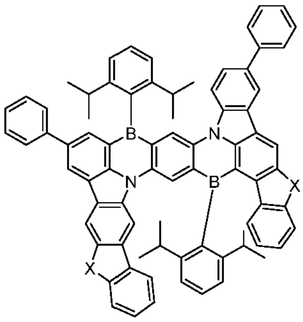

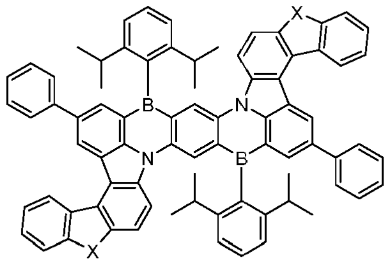

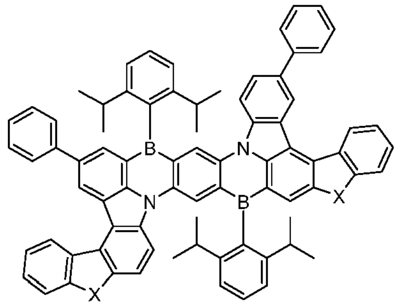

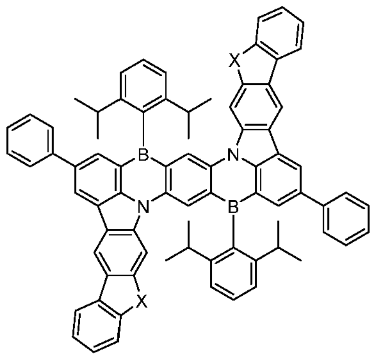

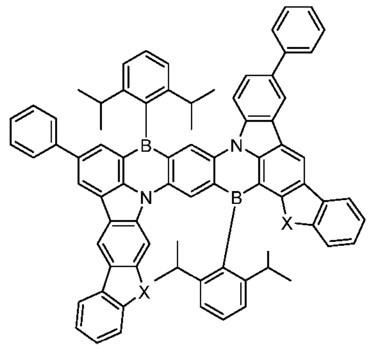

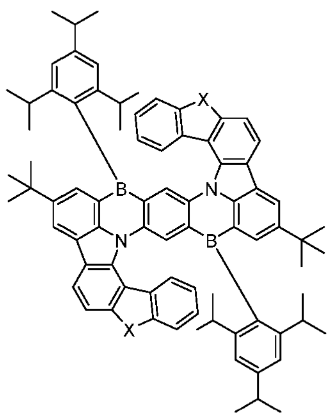

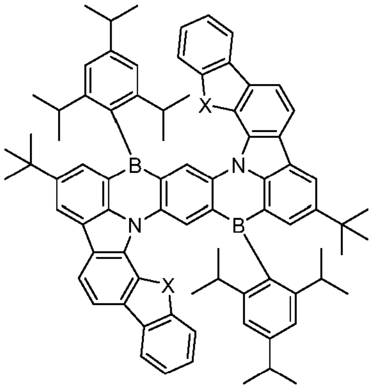

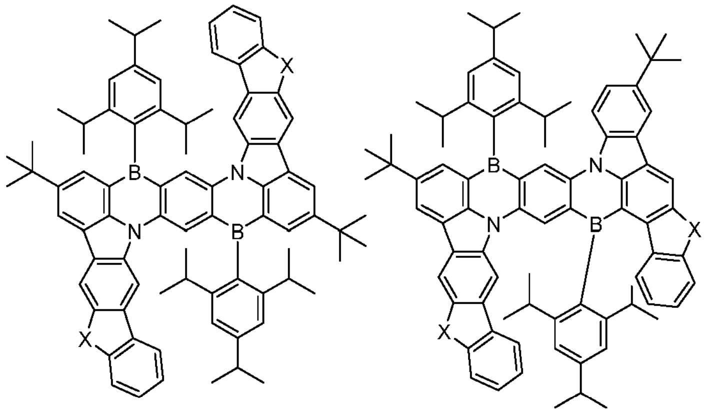

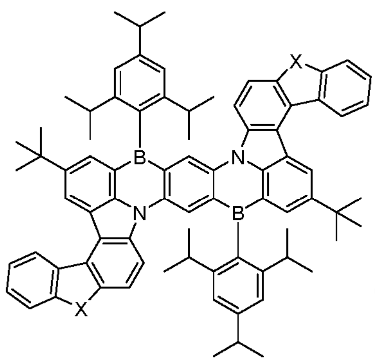

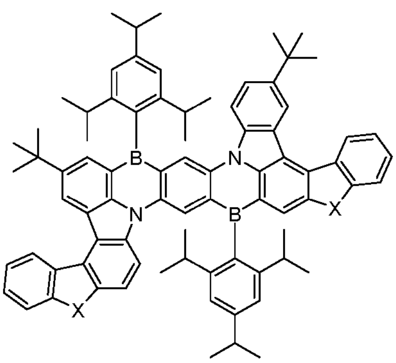

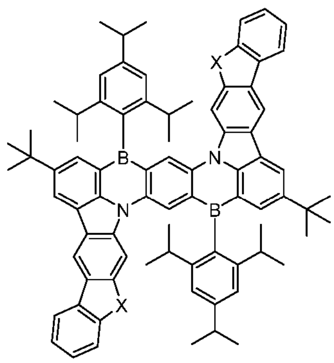

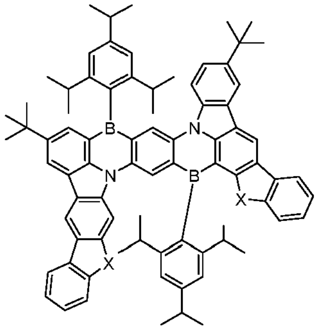
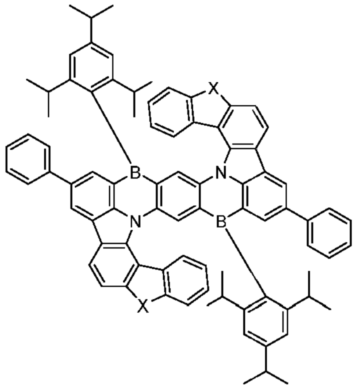
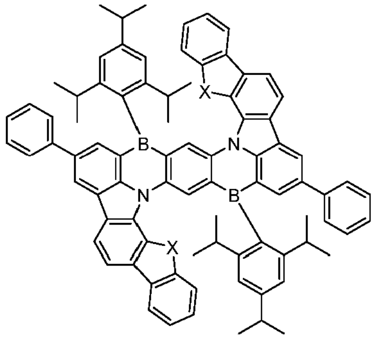

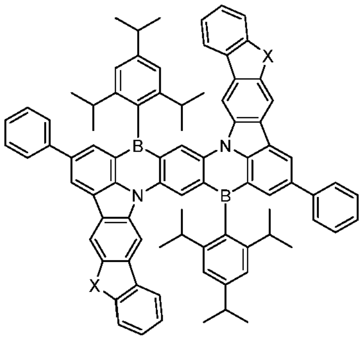
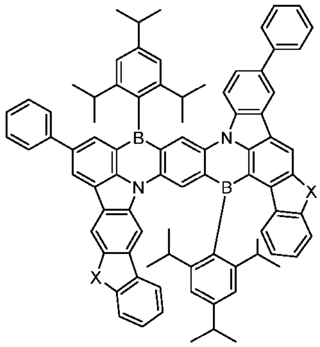
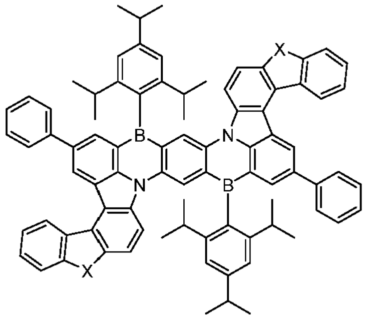

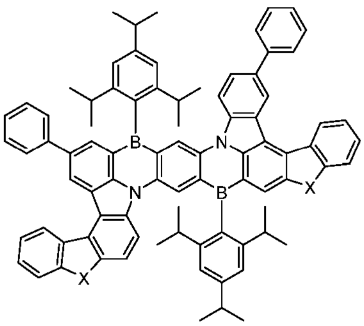
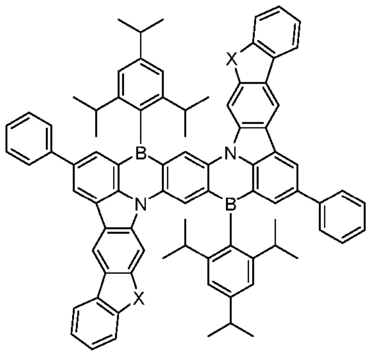
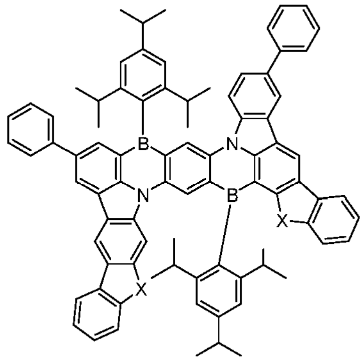

As one preferable group of compounds having the skeleton (19b), compounds represented by the following formula (19b) may be exemplified.

Formula (19b)

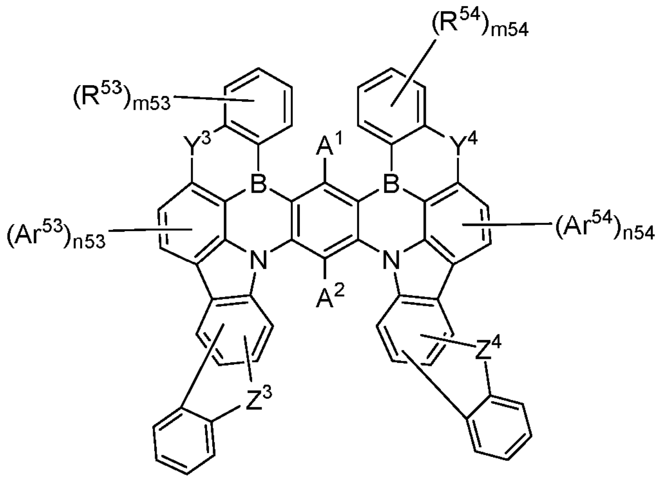

In the formula (19b), each of $Ar^{53}$ and $Ar^{54}$ independently represents a substituted or unsubstituted aryl group, or a substituted or unsubstituted heteroaryl group. Each of $R^{53}$ and $R^{54}$ independently represents a substituted or unsubstituted alkyl group. Each of m53 and m54 independently represents an integer of 0 to 4. Each of n53 and n54 independently represents an integer of 0 to 2. Each of $Y^3$ and $Y^4$ independently represents two hydrogen atoms, a single bond or $N(R^{27})$. $R^{27}$ represents a hydrogen atom, a deuterium atom, or a substituent. Each of $Z^3$ and $Z^4$ independently represents an oxygen atom or a sulfur atom. Each of $A^1$ and $A^2$ independently represents a hydrogen atom, a deuterium atom, or a substituent. In relation to details of $Ar^{53}$, $Ar^{54}$, $R^{53}$, $R^{54}$, m53, m54, n53, n54, $A^1$, and $A^2$, the descriptions on $Ar^{51}$, $Ar^{52}$, $R^{51}$, $R^{52}$, m51, m52, n51, n52, $A^1$, and $A^2$ in the formula (19a) may be referred to.

Hereinafter, specific examples of the compound represented by the formula (19b) will be given. Compounds of the formula (19b) that may be used in the invention are not construed as limiting to the following specific examples. In relation to specific examples including X, it is assumed that a compound in which all X's in the molecule are oxygen atoms, and a compound in which all X's in the molecule are sulfur atoms are disclosed, respectively. A compound in which some of X's in the molecule are oxygen atoms, and the rest are sulfur atoms may also be adopted.

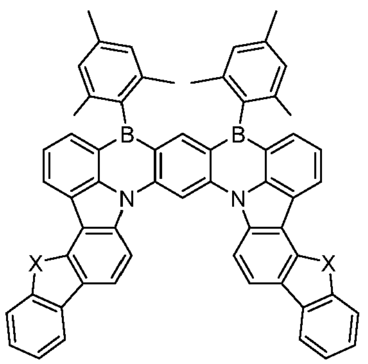

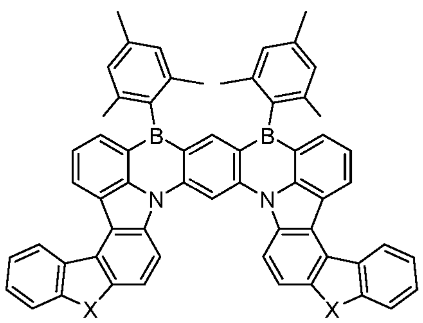

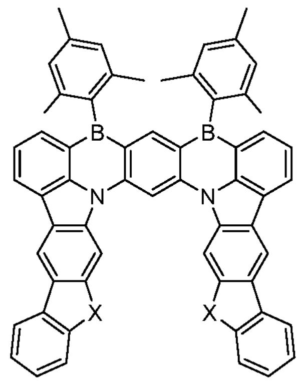

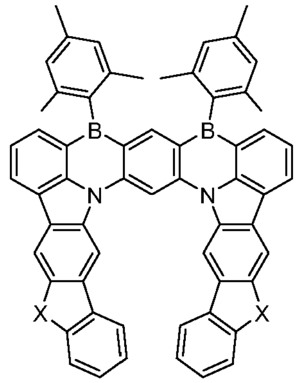
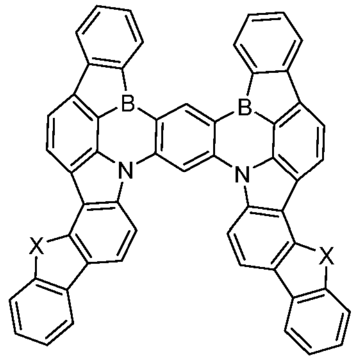
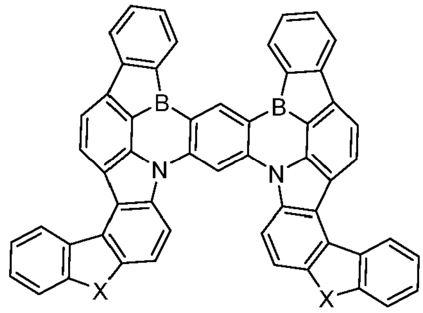
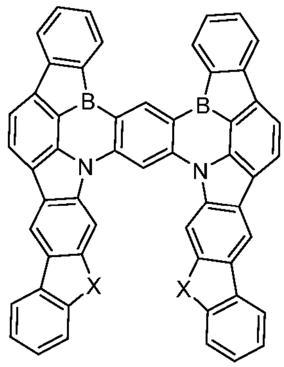
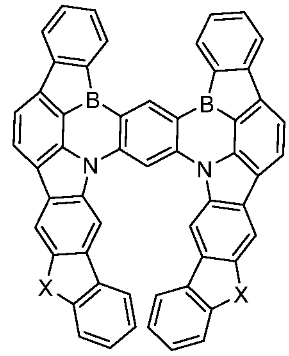
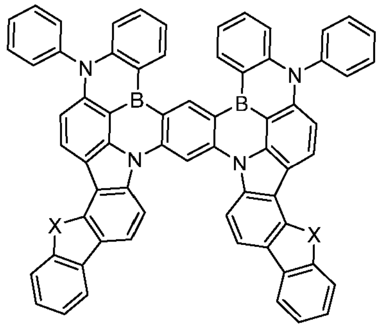
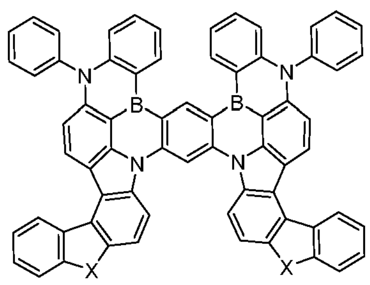
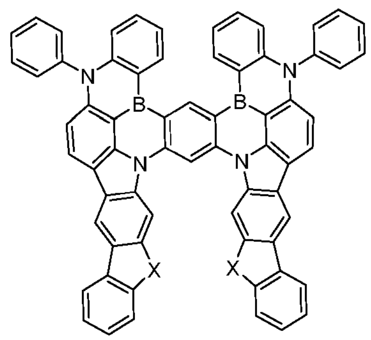
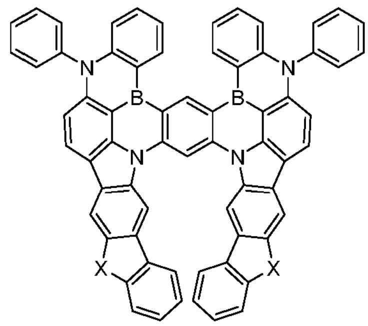

A compound in which a benzofuran ring or a benzothiophene ring is condensed with a benzene ring to which a boron atom is directly bonded, between two benzene rings forming a carbazole partial structure existing in the formula (16), may be preferably mentioned. Examples of such a compound include a compound having the following skeleton (20a) and a compound having the following skeleton (20b).

Skeleton (20a)

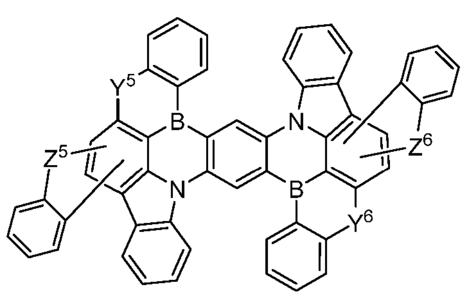

Skeleton (20b)

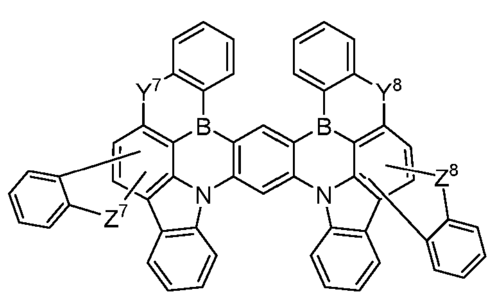

In the skeletons (20a) and (20b), each of $Y^5$ to $Y^8$ independently represents two hydrogen atoms, a single bond or $N(R^{27})$. Each of $Z^5$ to $Z^8$ independently represents an oxygen atom or a sulfur atom. In relation to details of $Y^5$ to $Y^8$, and $Z^5$ to $Z^8$, corresponding descriptions for the skeletons (19a) and (19b) may be referred to. In one aspect of the invention, each

hydrogen atom in the skeletons (20a) and (20b) is not substituted with a linking group together with an adjacent hydrogen atom to form a ring structure.

As one preferable group of compounds having the skeleton (20a), compounds represented by the following formula (20a) may be exemplified.

Formula (20a)

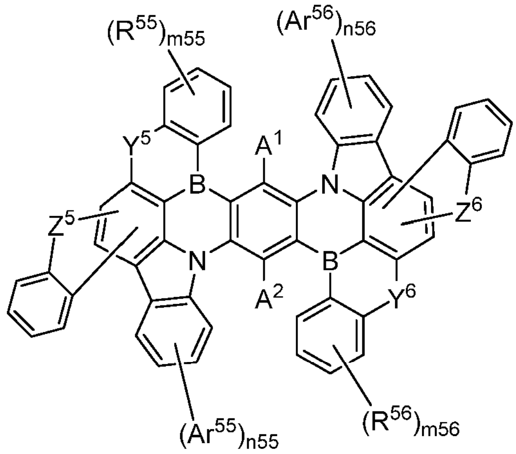

In the formula (20a), each of $Ar^{55}$ and $Ar^{56}$ independently represents a substituted or unsubstituted aryl group, or a substituted or unsubstituted heteroaryl group. Each of $R^{55}$ and $R^{56}$ independently represents a substituted or unsubstituted alkyl group. Each of m55 and m56 independently represents an integer of 0 to 4. Each of n55 and n56 independently represents an integer of 0 to 4. Each of $Y^5$ and $Y^6$ independently represents two hydrogen atoms, a single bond or $N(R^{27})$. $R^{27}$ represents a hydrogen atom, a deuterium atom, or a substituent. Each of $Z^5$ and $Z^6$ independently represents an oxygen atom or a sulfur atom. Each of $A^1$ and $A^2$ independently represents a hydrogen atom, a deuterium atom, or a substituent.

In one aspect of the invention, n55 and n56 are integers of 0 to 2. For example, n55 and n56 may be 0, and n55 and n56 may be 1. In one aspect of the invention, m51 and m52 are the same number. In relation to details of m55 and m56, descriptions on m51 and m52 in the formula (19a) may be referred to. In relation to preferable groups for $Ar^{55}$, $Ar^{56}$, $R^{55}$, $R^{56}$, $A^1$, and $A^2$, corresponding descriptions on $Ar^1$, $Ar^3$, $R^{41}$, $R^{42}$, $A^1$, and $A^2$ in the formula (16a) may be referred to.

Hereinafter, specific examples of the compound represented by the formula (20a) will be given. Compounds of the formula (20a) that may be used in the invention are not construed as limiting to the following specific examples. In relation to specific examples including X, it is assumed that a compound in which all X's in the molecule are oxygen atoms, and a compound in which all X's in the molecule are sulfur atoms are disclosed, respectively. A compound in which some of X's in the molecule are oxygen atoms, and the rest are sulfur atoms may also be adopted.

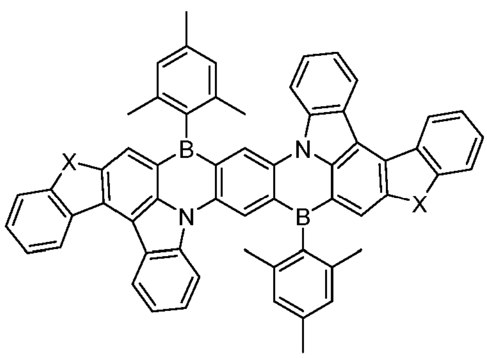

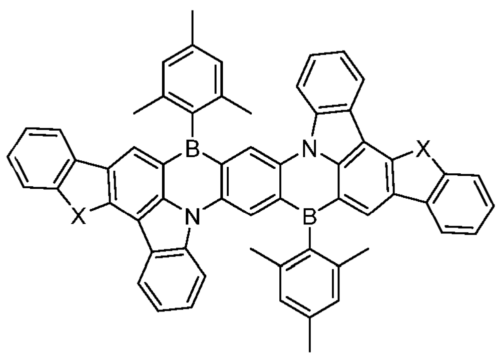

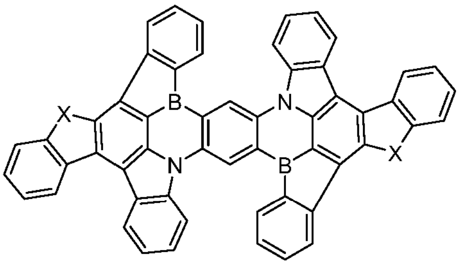
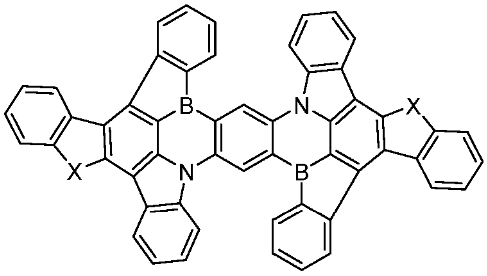

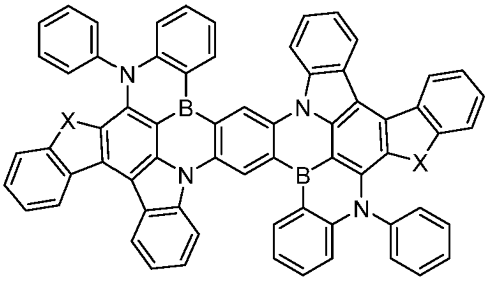
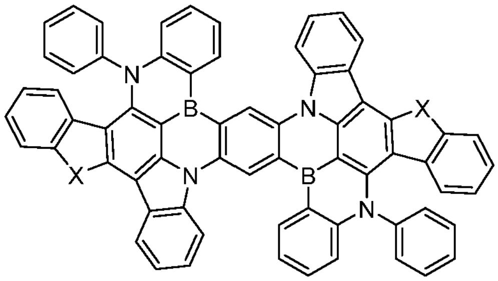

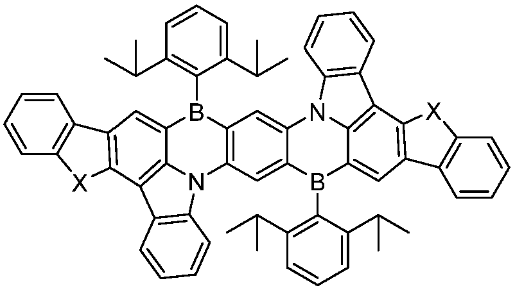
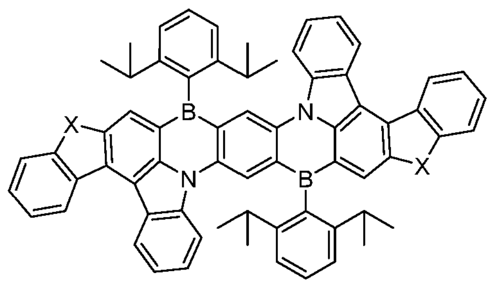

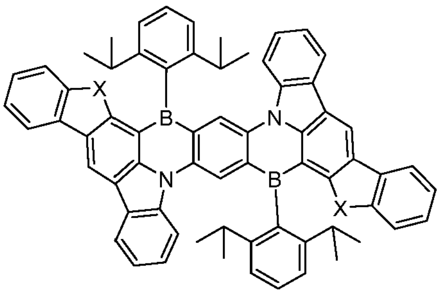
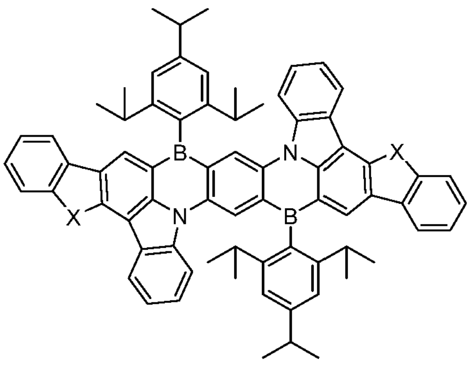

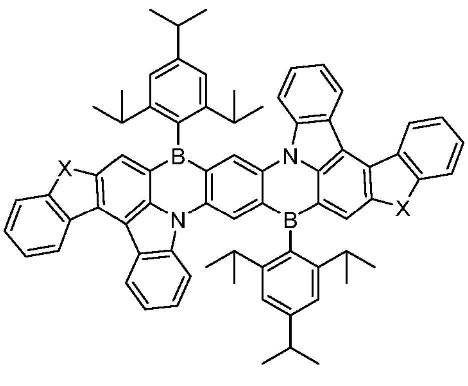
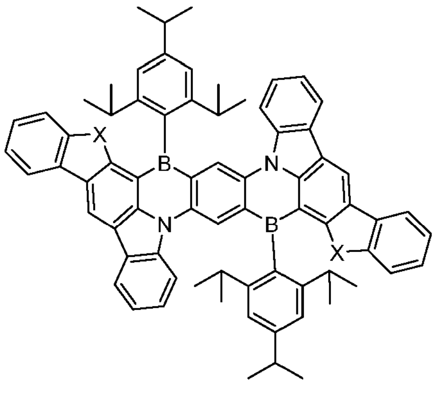

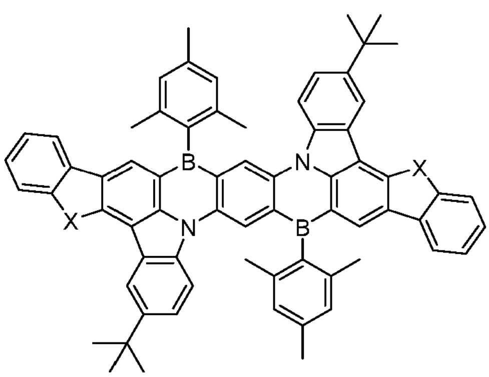

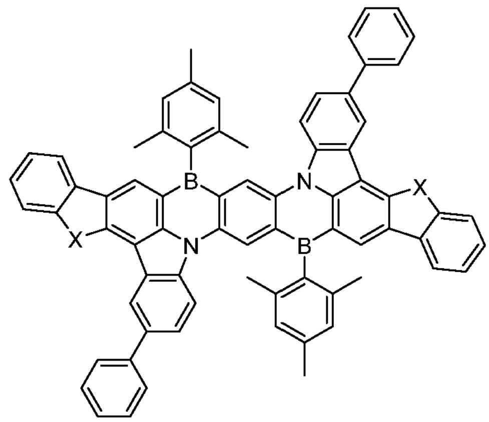

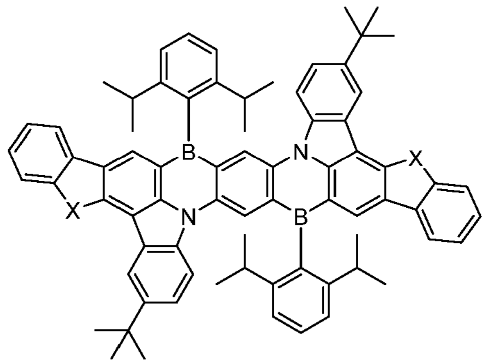

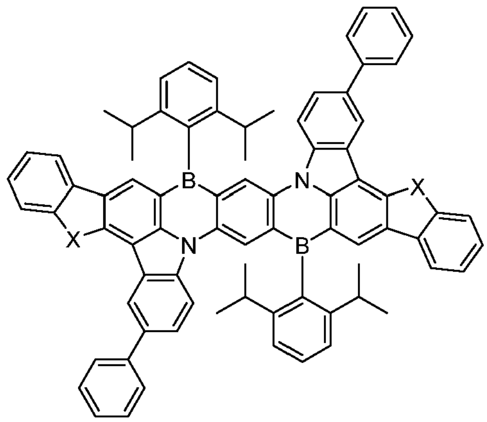

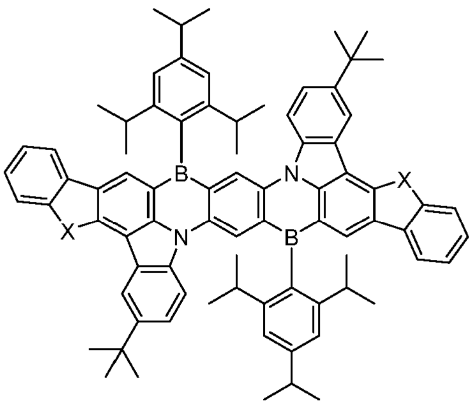

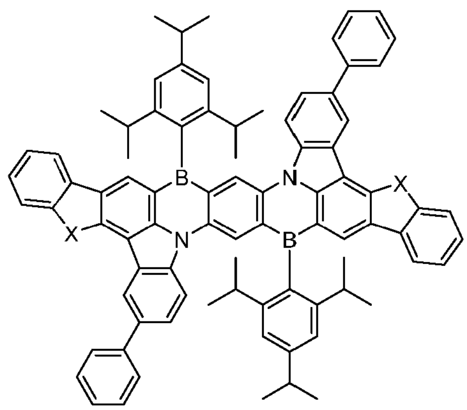

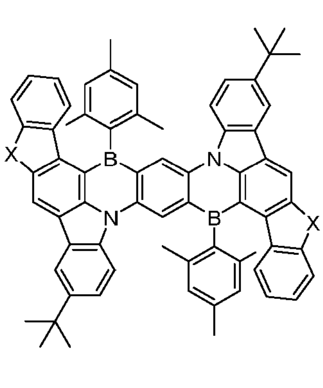

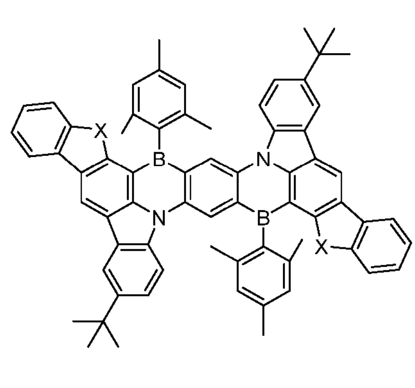

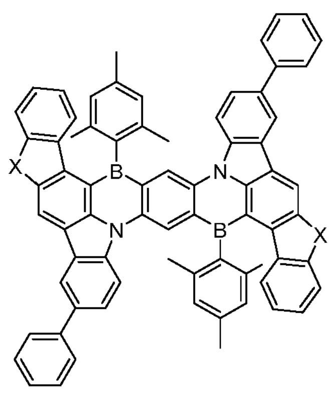

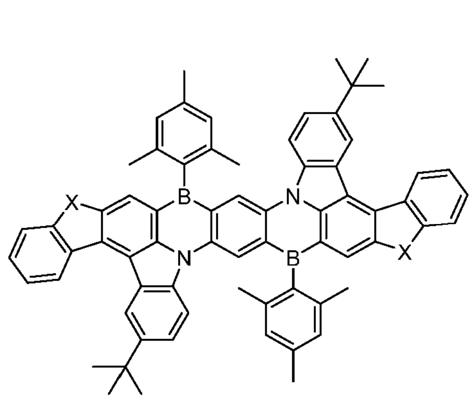

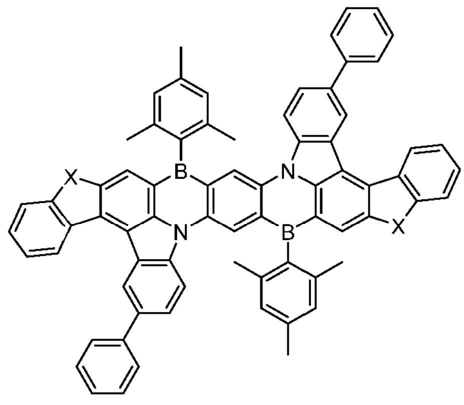

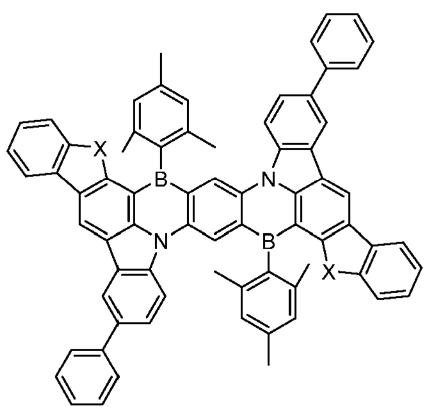

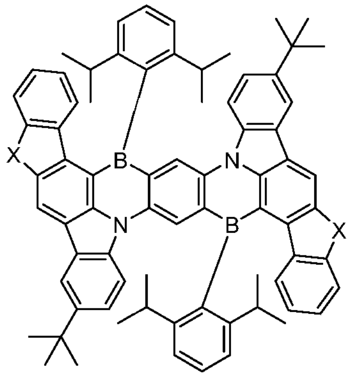

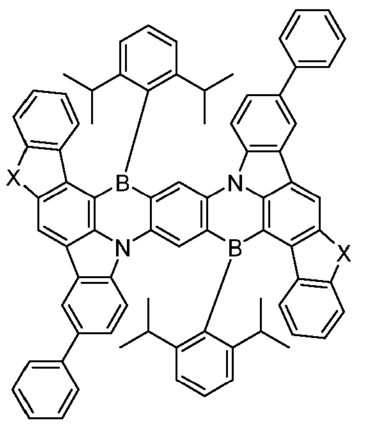

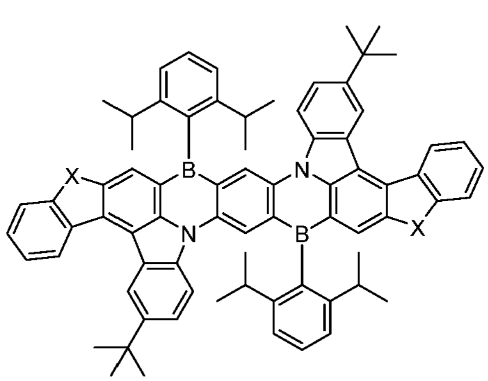

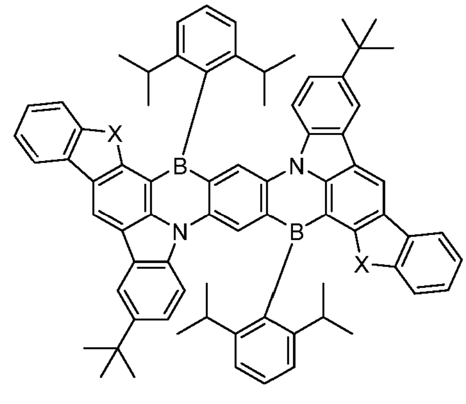

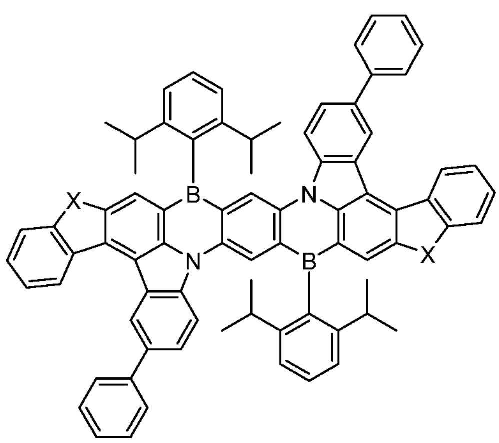

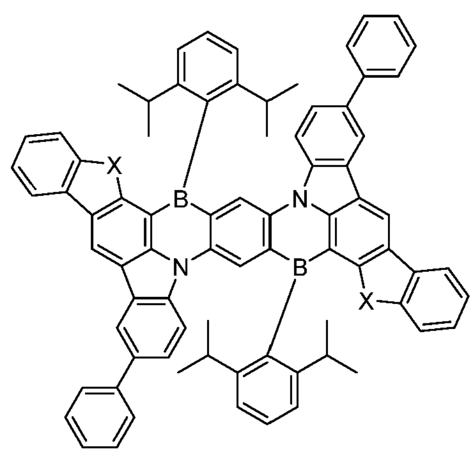

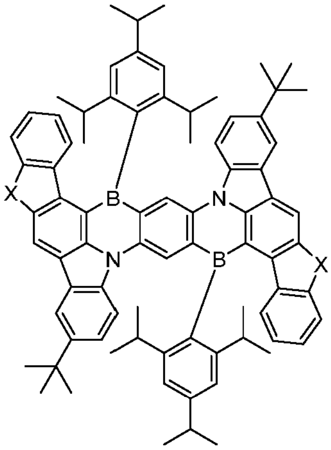

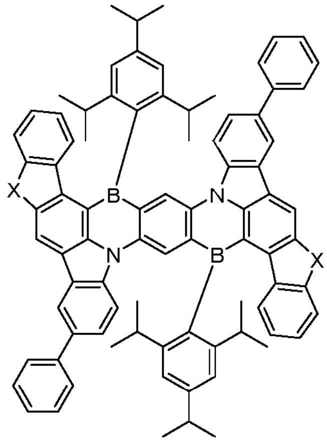

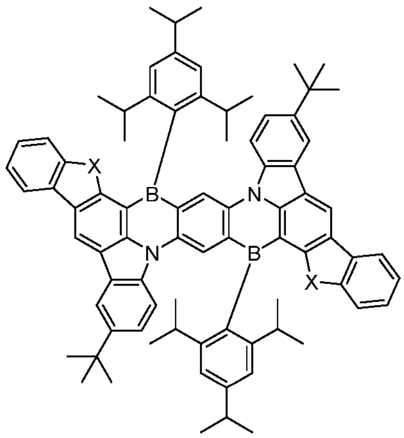

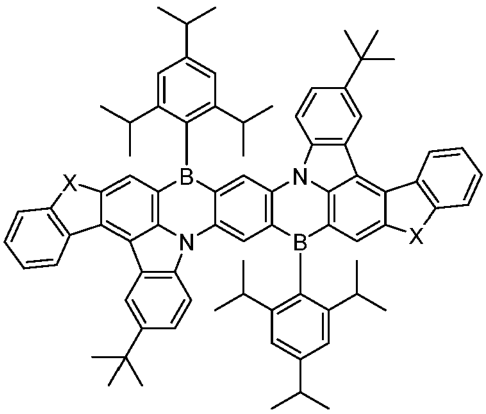

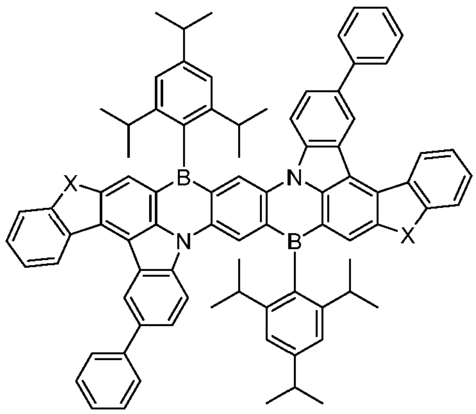

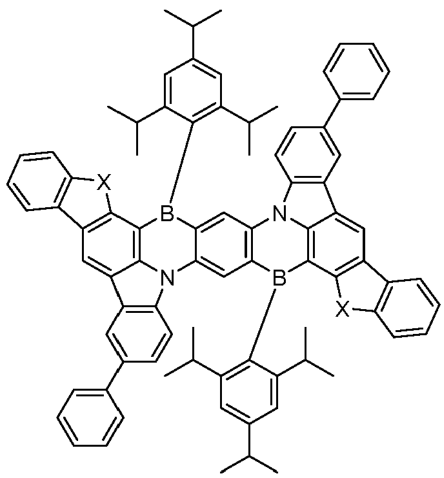

As one preferable group of compounds having the skeleton (20b), compounds represented by the following formula (20b) may be exemplified.

Formula (20b)

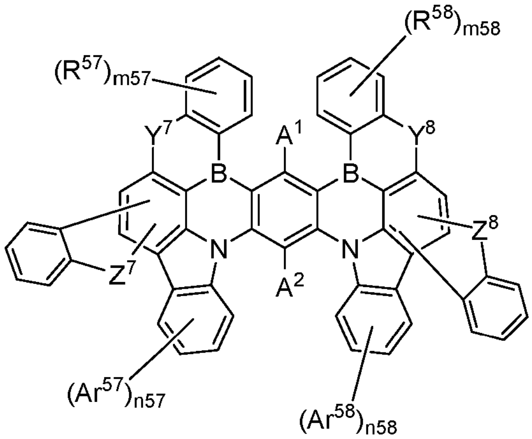

In the formula (20b), each of $Ar^{57}$ and $Ar^{58}$ independently represents a substituted or unsubstituted aryl group, or a substituted or unsubstituted heteroaryl group. Each of $R^{57}$ and $R^{58}$ independently represents a substituted or unsubstituted alkyl group. Each of m57 and m58 independently represents an integer of 0 to 4. Each of n57 and n58 independently represents an integer of 0 to 4. Each of $Y^7$ and $Y^8$ independently represents two hydrogen atoms, a single bond or $N(R^{27})$. $R^{27}$ represents a hydrogen atom, a deuterium atom, or a substituent. Each of $Z^7$ and $Z^8$ independently represents an oxygen atom or a sulfur atom. Each of $A^1$ and $A^2$ independently represents a hydrogen atom, a deuterium atom, or a substituent. In relation to details of $Ar^{57}$, $Ar^{58}$, $R^{57}$, $R^{58}$, m57, m58, n57, n58, $A^1$, and $A^2$, descriptions on $Ar^{55}$, $Ar^{56}$, $R^{55}$, $R^{56}$, m55, m56, n55, n56, $A^1$, and $A^2$ in the formula (20a) may be referred to.

Hereinafter, specific examples of the compound represented by the formula (20b) will be given. Compounds of the formula (20b) that may be used in the invention are not construed as limiting to the following specific examples. In relation to specific examples including X, it is assumed that a compound in which all X's in the molecule are oxygen atoms, and a compound in which all X's in the molecule are sulfur atoms are disclosed, respectively. A compound in which some of X's in the molecule are oxygen atoms and the rest are sulfur atoms may also be adopted.

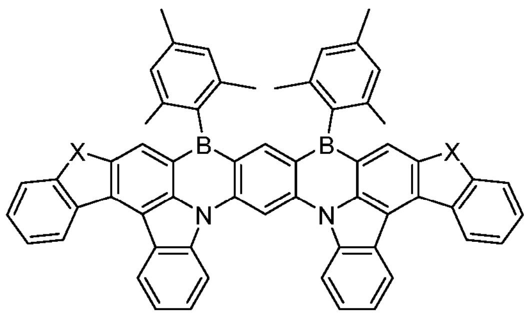

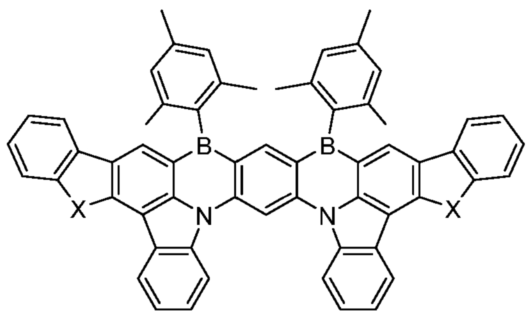

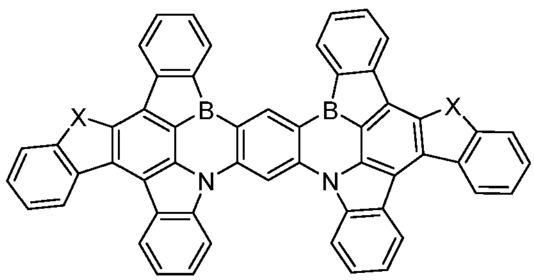

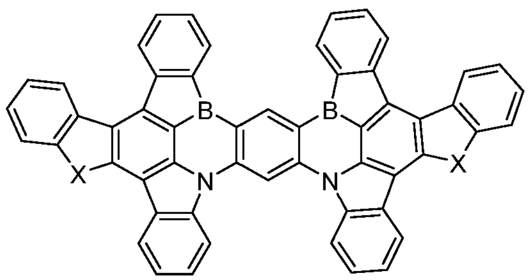

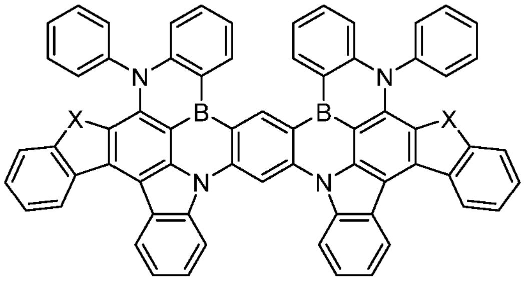

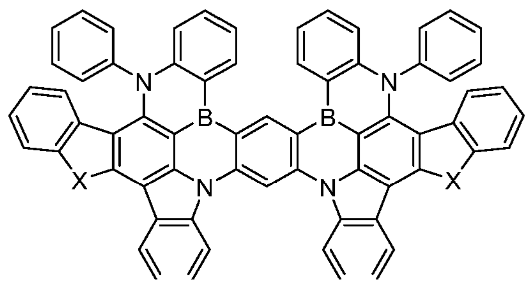

A compound in which benzofuran rings or benzothiophene rings are condensed with both of two benzene rings forming a carbazole partial structure existing in the formula (16) may be preferably mentioned. Examples of such a compound include a compound having the following skeleton (21a), and a compound having the following skeleton (21b).

Skeleton (21a)

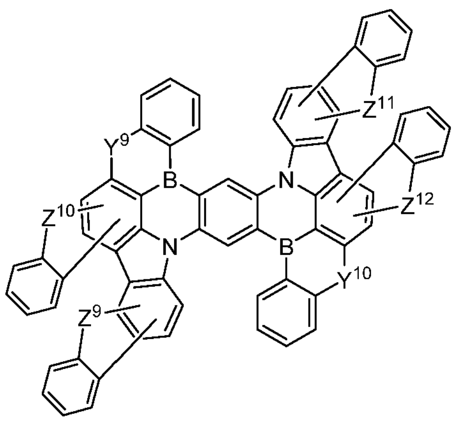

Skeleton (21b)

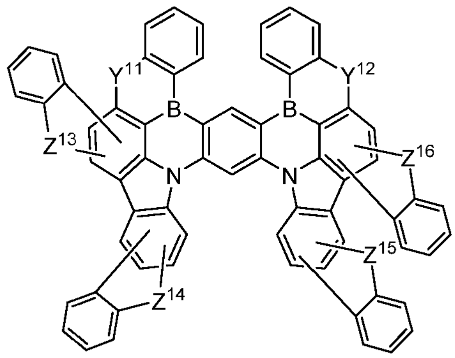

In the skeletons (21a) and (21b), each of $Y^9$ to $Y^{12}$ independently represents two hydrogen atoms, a single bond or $N(R^{27})$. Each of $Z^9$ to $Z^{16}$ independently represents an oxygen atom or a sulfur atom. It is preferable that $Z^9$ to $Z^{16}$ are the same, but they may be different. In one aspect of the invention, $Z^9$ to $Z^{16}$ are oxygen atoms. In one aspect of the invention, $Z^9$ to $Z^{16}$ are sulfur atoms. In relation to details of $Y^9$ to $Y^{12}$, corresponding descriptions for the skeletons (19a) and (19b) may be referred to. In one aspect of the invention, each hydrogen atom in the skeletons (21a) and (21b) is not substituted with a linking group together with an adjacent hydrogen atom to form a ring structure.

As one preferable group of the compounds having the skeleton (21a), a compound represented by the following formula (21a) can be exemplified.

Formula (21a)

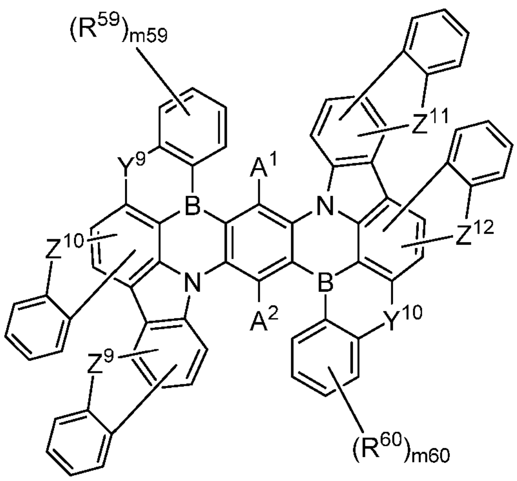

In the formula (21a), each of $R^{59}$ and $R^{60}$ independently represents a substituted or unsubstituted alkyl group. Each of m59 and m60 independently represents an integer of 0 to 4. Each of $Y^9$ and $Y^{10}$ independently represents two hydrogen atoms, a single bond or $N(R^{27})$. $R^{27}$ represents a hydrogen atom, a deuterium atom, or a substituent. Each of $Z^9$ to $Z^{12}$ independently represents an oxygen atom or a sulfur atom. Each of $A^1$ and $A^2$ independently represents a hydrogen atom, a deuterium atom, or a substituent. In relation to details of $R^{59}$, $R^{60}$, m59, m60, $Z^9$ to $Z^{12}$, $A^1$, and $A^2$, descriptions on $R^{55}$, $R^{56}$, m55, m56, $A^1$, and $A^2$ in the formula (20a) and $Z^9$ to $Z^{12}$ in the skeleton (21a) may be referred to.

Hereinafter, specific examples of the compound represented by the formula (21a) will be given. Compounds of the formula (21a) that may be used in the invention are not construed as limiting to the following specific examples. In relation to specific examples including X, it is assumed that a compound in which all X's in the molecule are oxygen atoms, and a compound in which all X's in the molecule are sulfur atoms are disclosed, respectively. A compound in which some of X's in the molecule are oxygen atoms, and the rest are sulfur atoms may also be adopted.

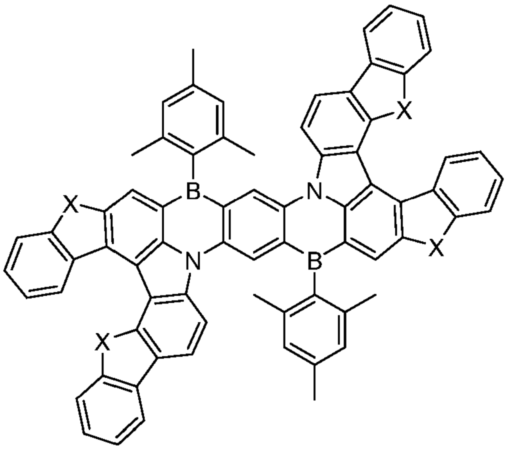

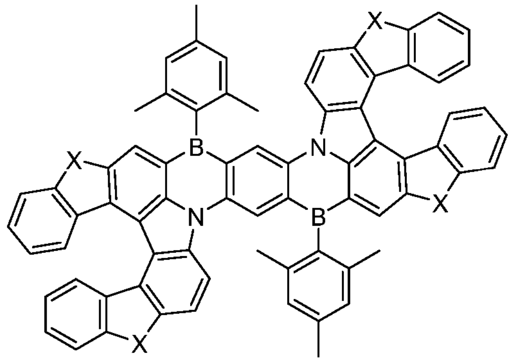

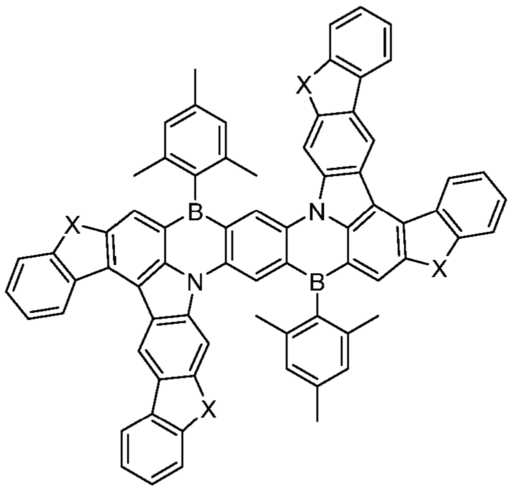

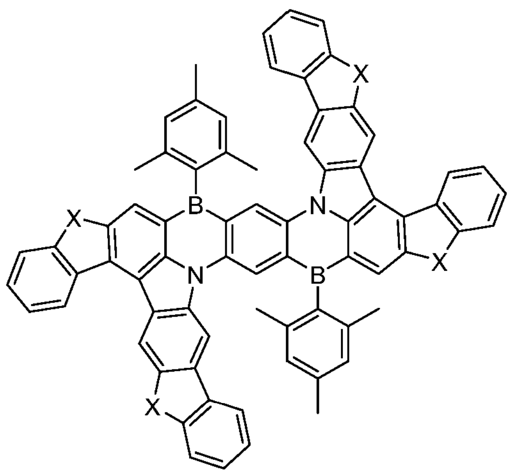

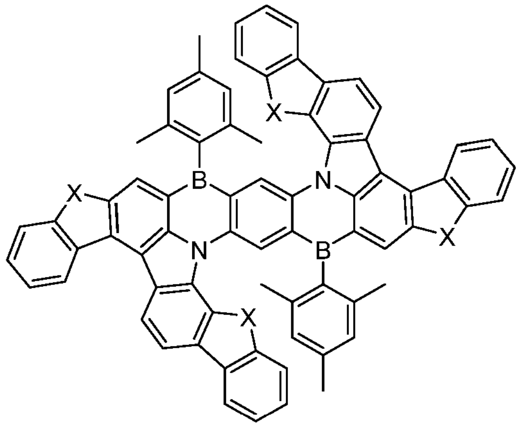

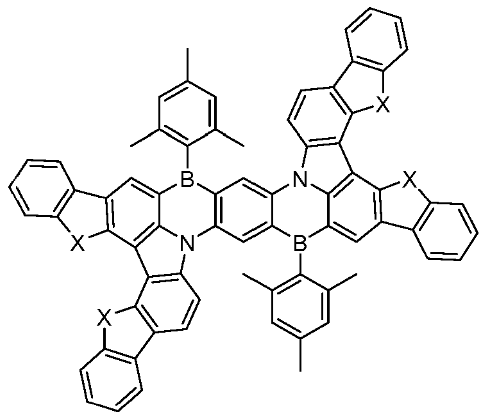

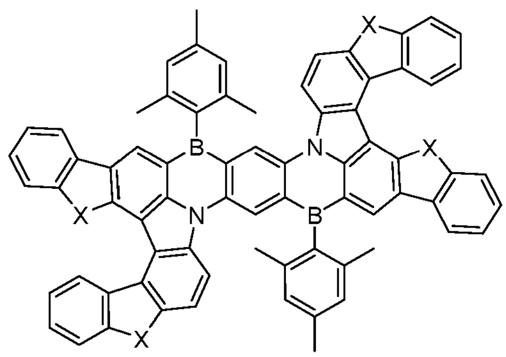

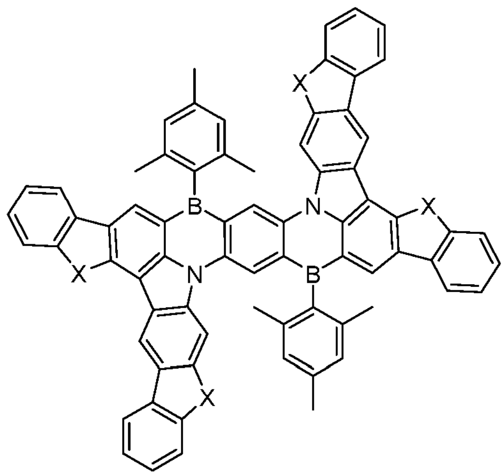

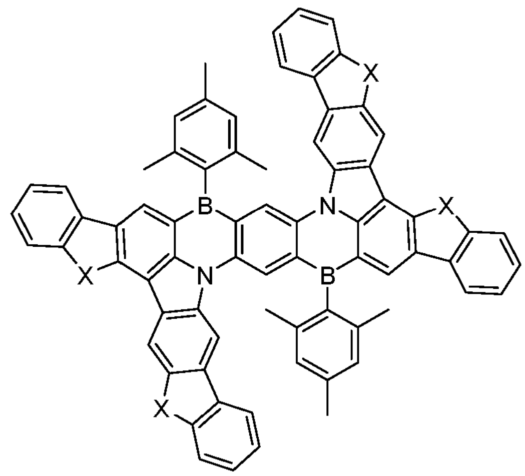

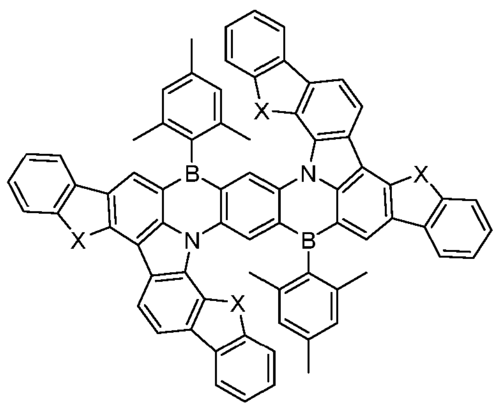

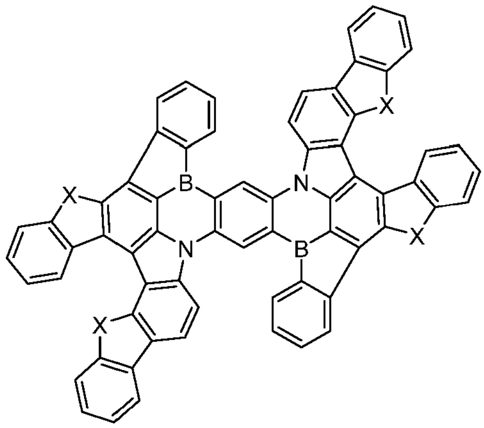

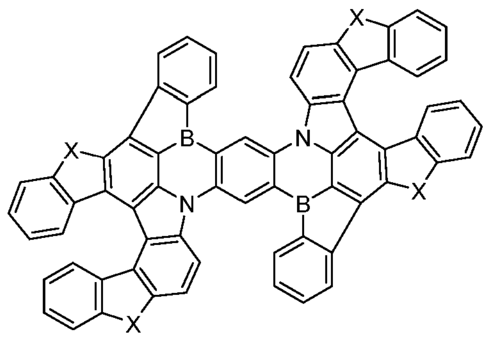

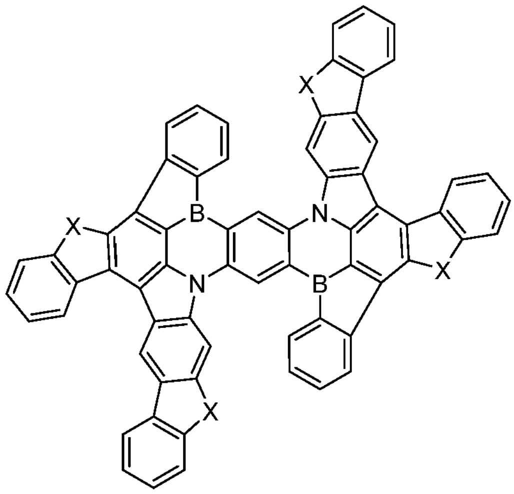

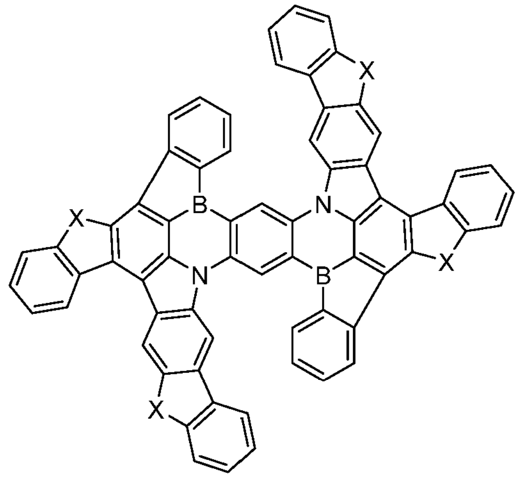

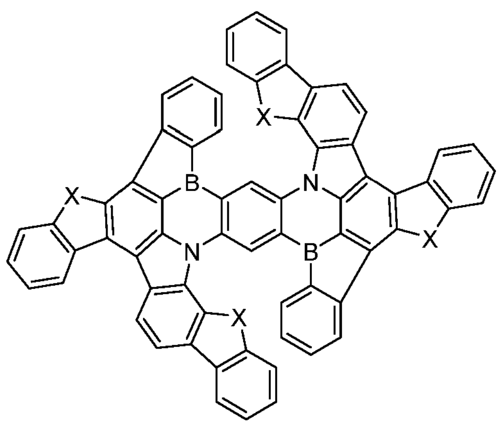

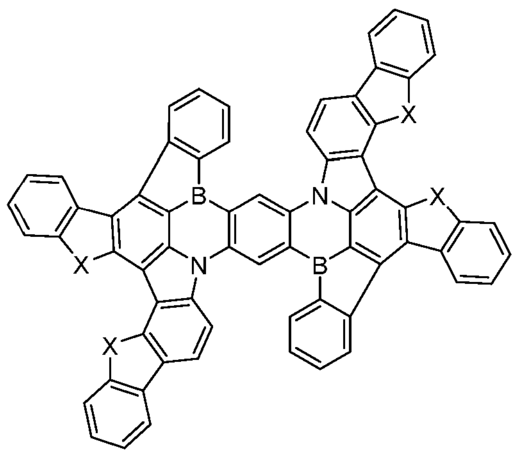

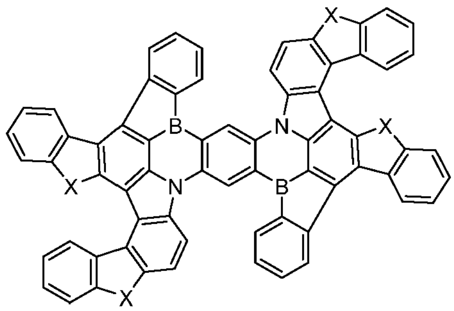

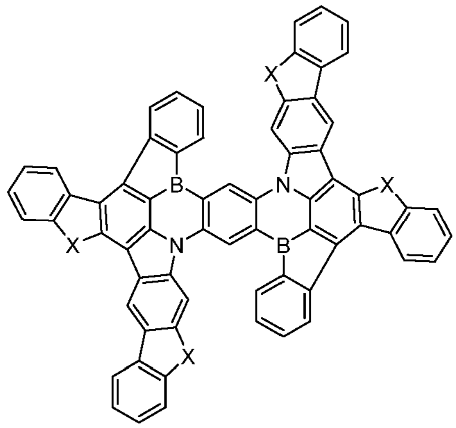

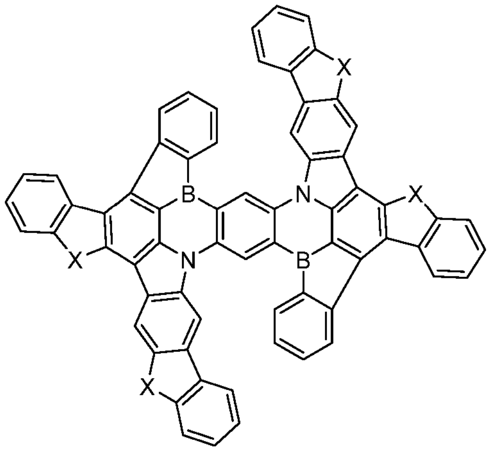

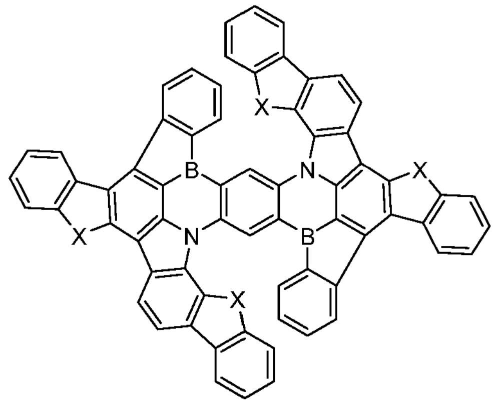

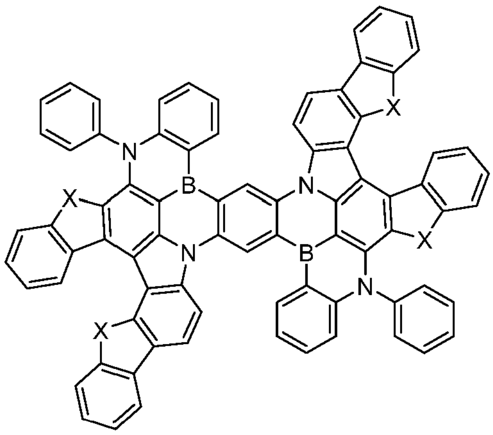

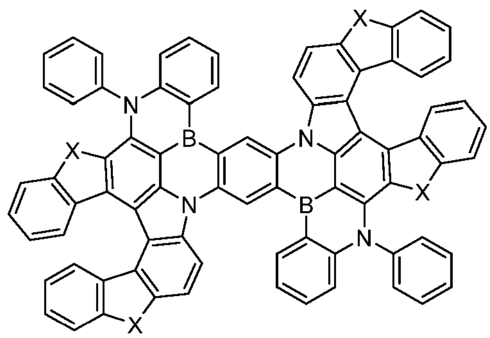

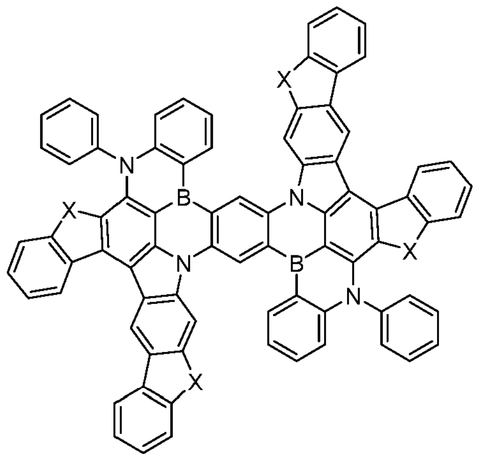
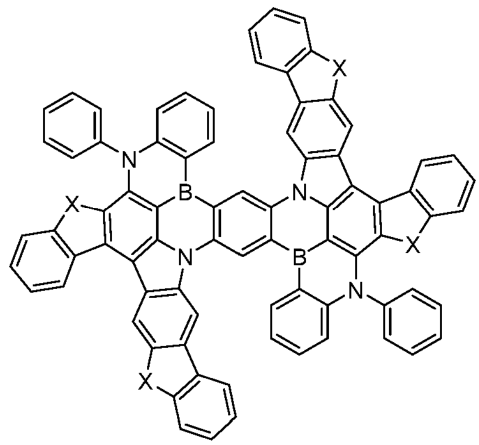

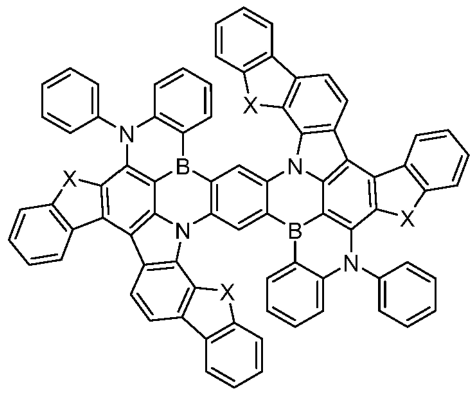
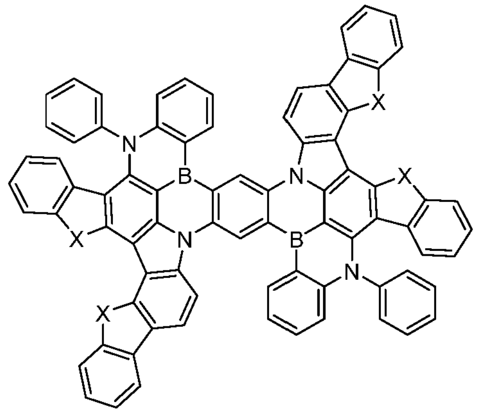

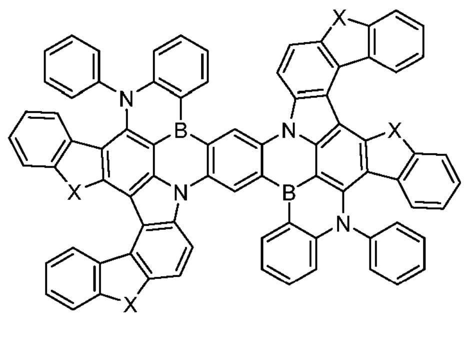
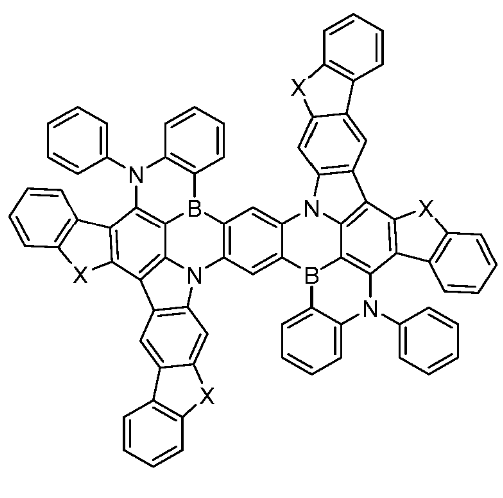

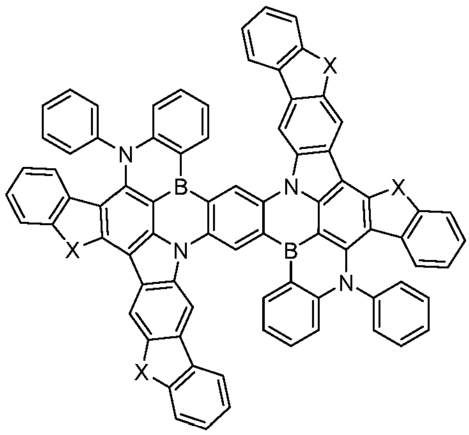

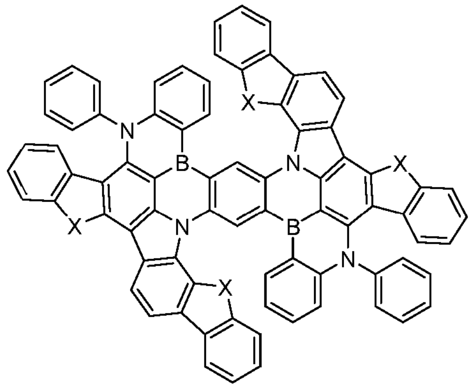

As one preferable group of compounds having the skeleton (21b), compounds represented by the following formula (21b) may be exemplified.

Formula (21b)

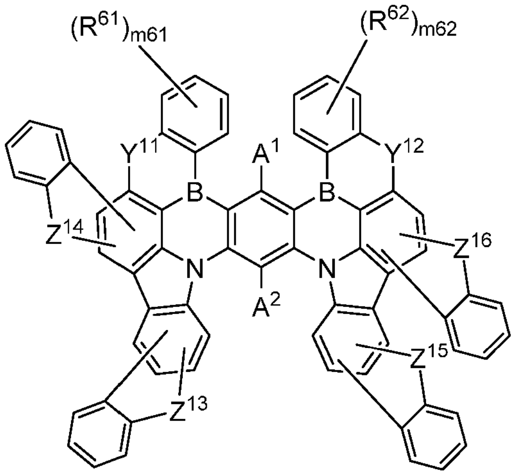

In the formula (21b), each of $R^{61}$ and $R^{62}$ independently represents a substituted or unsubstituted alkyl group. Each of m61 and m60 independently represents an integer of 0 to 4. Each of $Y^{11}$ and $Y^{12}$ independently represents two hydrogen atoms, a single bond or $N(R^{27})$. $R^{27}$ represents a hydrogen atom, a deuterium atom, or a substituent. Each of $Z^{13}$ to $Z^{16}$ independently represents an oxygen atom or a sulfur atom. Each of $A^1$ and $A^2$ independently represents a hydrogen atom, a deuterium atom, or a substituent. In relation to details of $R^{61}$, $R^{62}$, m61, m62, $Z^{13}$ to $Z^{16}$, $A^1$, and $A^2$, descriptions on $R^{59}$, $R^{60}$, m59, m60, $A^1$, and $A^2$ in the formula (21a), and $Z^{13}$ to $Z^{16}$ in the skeleton (21b) may be referred to.

Hereinafter, specific examples of the compound represented by the formula (21b) will be given. Compounds of the formula (21b) that may be used in the invention are not construed as limiting to the following specific examples. In relation to specific examples including X, it is assumed that a compound in which all X's in the molecule are oxygen atoms, and a compound in which all X's in the molecule are sulfur atoms are disclosed, respectively. A compound in which some of X's in the molecule are oxygen atoms, and the rest are sulfur atoms may also be adopted.

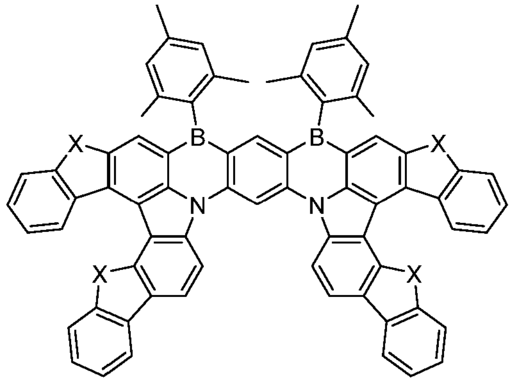

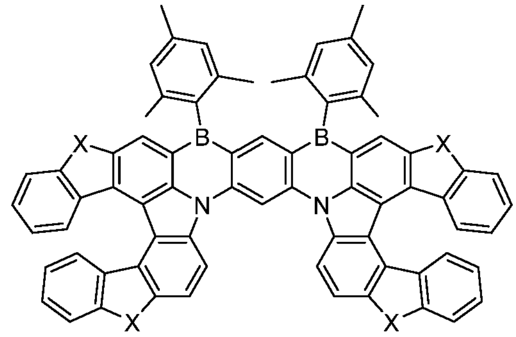

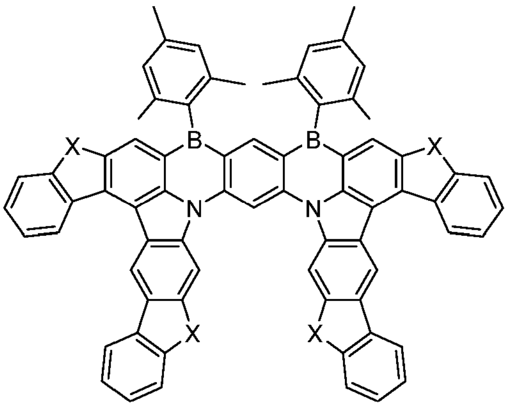

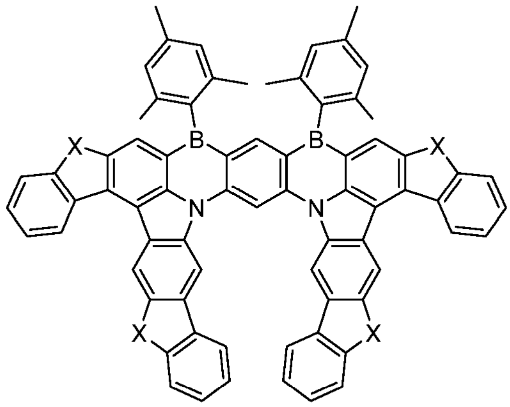

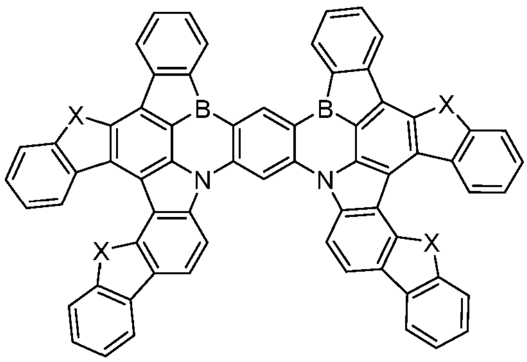

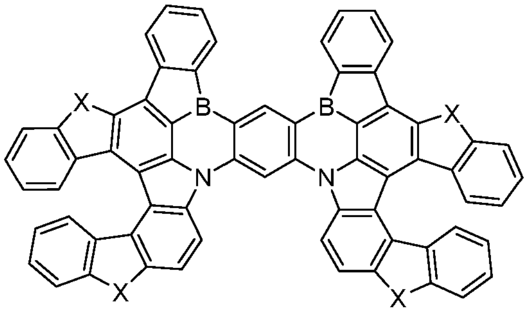

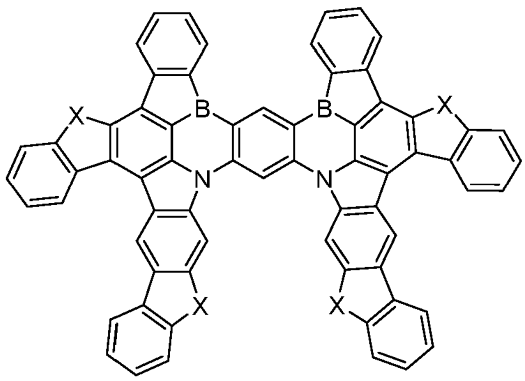

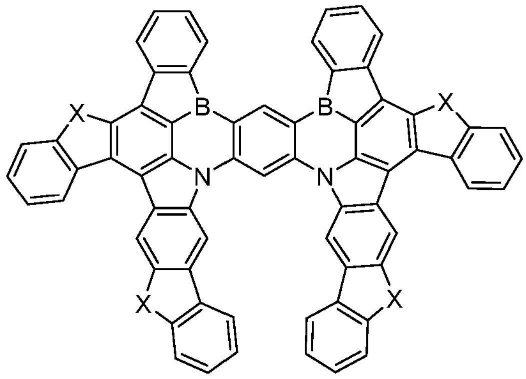

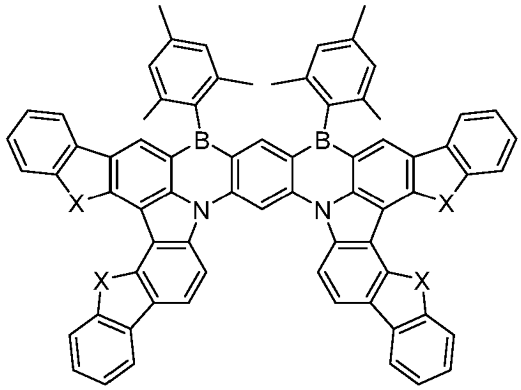

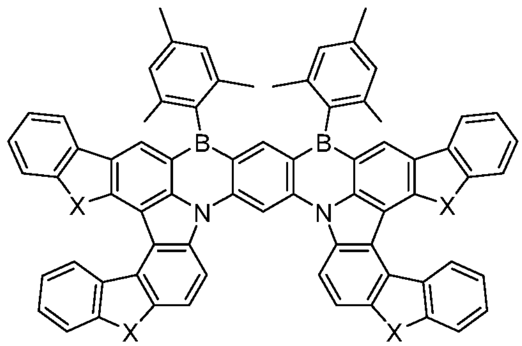

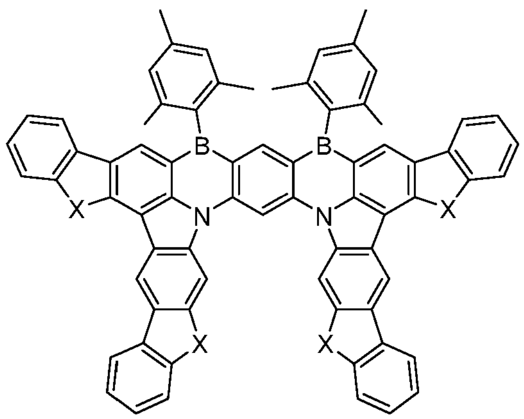

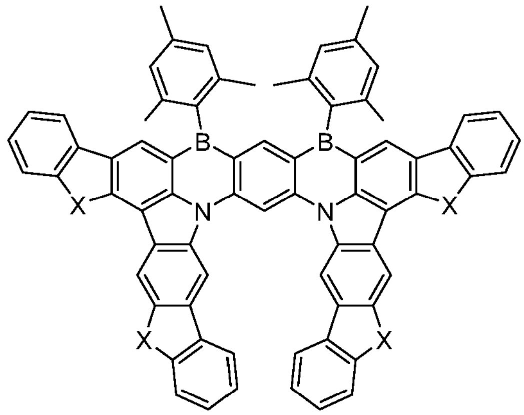

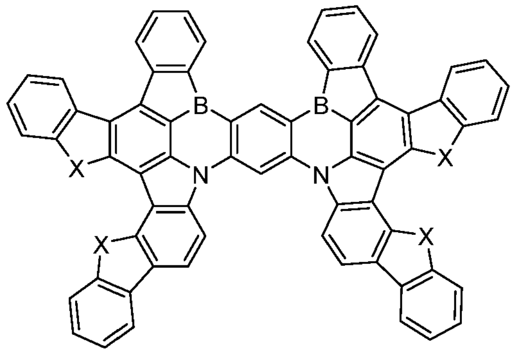

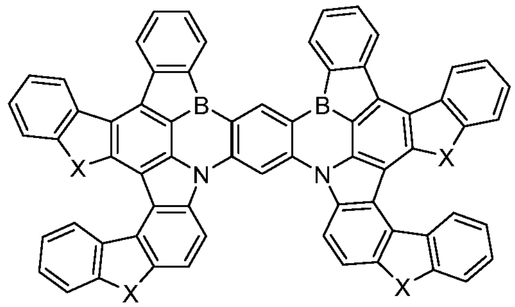

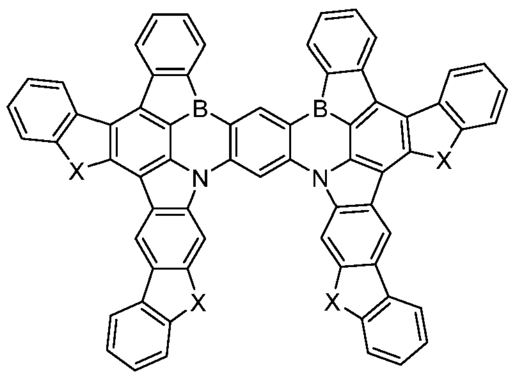

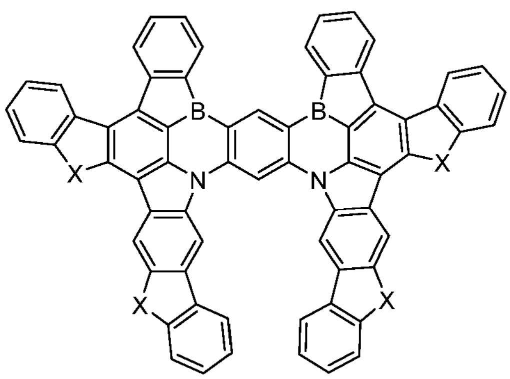

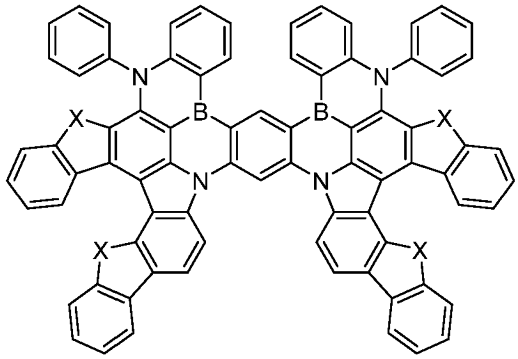

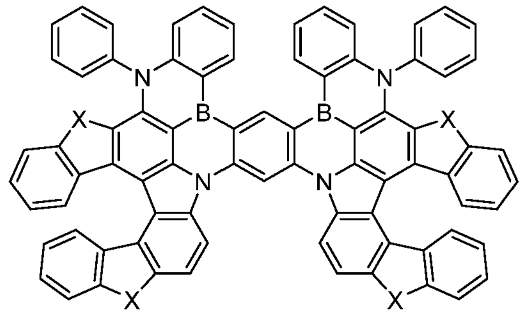

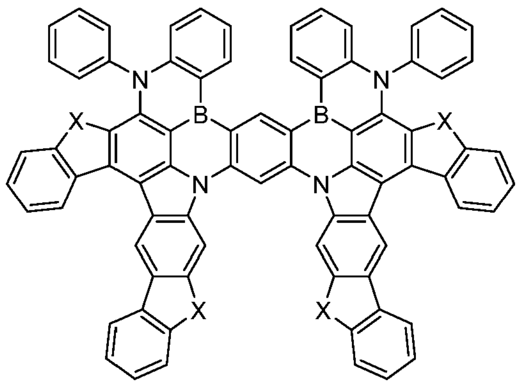

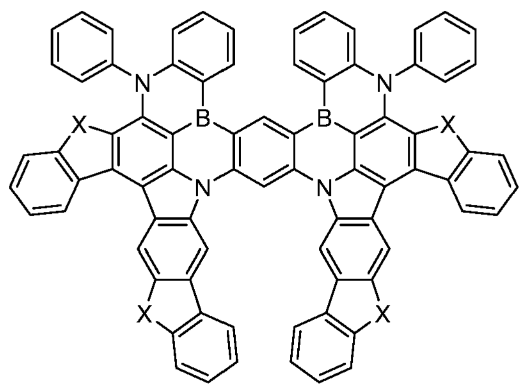

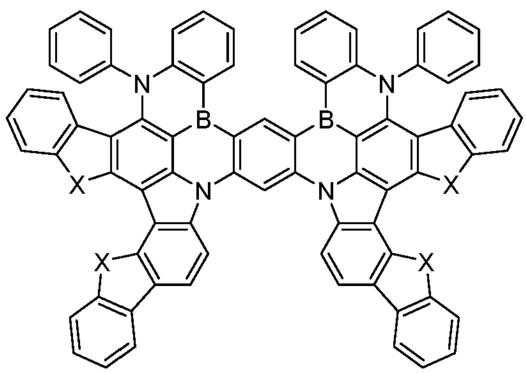

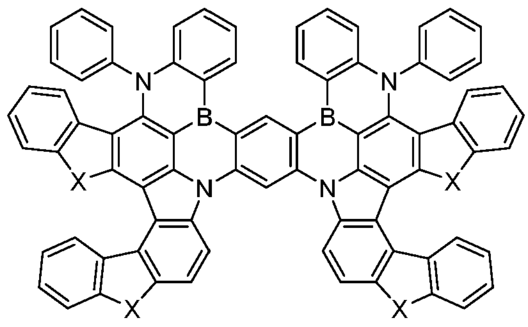

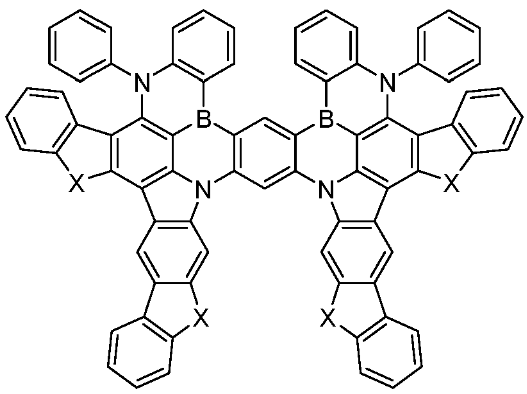

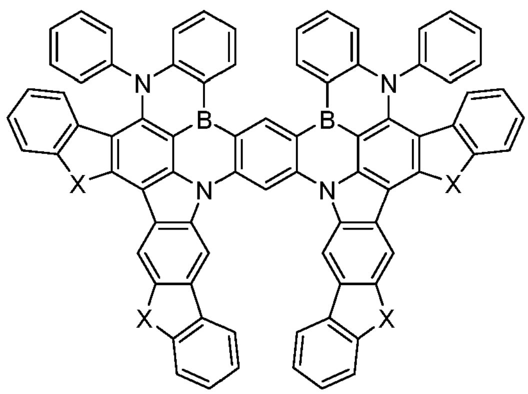

A compound in which a benzene ring is condensed with a benzene ring to which a boron atom is not directly bonded, between two benzene rings forming a carbazole partial structure existing in the formula (16), may be preferably mentioned. Examples of such a compound include a compound having the following skeleton (22a), and a compound having the following skeleton (22b).

Skeleton (22a)

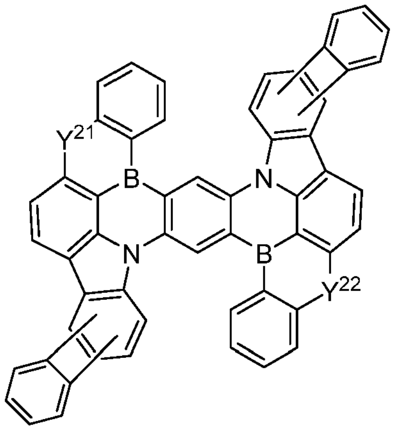

Skeleton (22b)

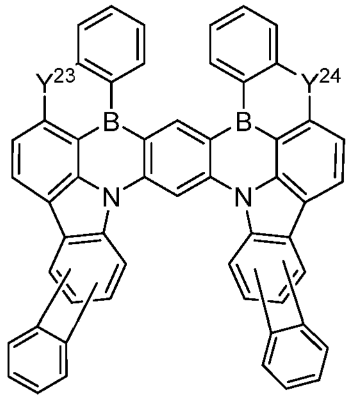

In the skeletons (22a) and (22b), each of $Y^{21}$ to $Y^{24}$ independently represents two hydrogen atoms, a single bond or $N(R^{27})$. In relation to details of $Y^{21}$ to $Y^{24}$, descriptions on $Y^1$ to $Y^4$ in the skeletons (19a) and (19b) may be referred to. In one aspect of the invention, each hydrogen atom in the skeletons (22a) and (22b) is not substituted with a linking group together with an adjacent hydrogen atom to form a ring structure.

As one preferable group of compounds having the skeleton (22a), compounds represented by the following formula (22a) may be exemplified.

Formula (22a)

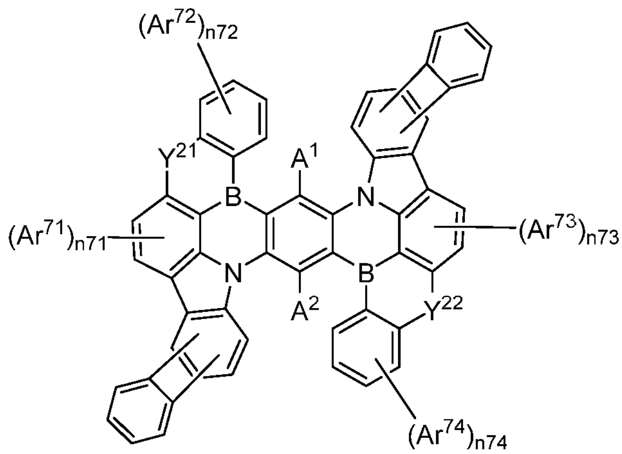

In the formula (22a), each of $Ar^{71}$ to $Ar^{74}$ independently represents a substituted or unsubstituted aryl group, or a substituted or unsubstituted heteroaryl group. Each of n71 and n73 independently represents an integer of 0 to 2. Each of n72 and n74 independently represents an integer of 0 to 4. Each of $Y^{21}$ and $Y^{22}$ independently represents two hydrogen atoms, a single bond or $N(R^{27})$. $R^{27}$ represents a hydrogen atom, a deuterium atom, or a substituent. Each of $A^1$ and $A^2$ independently represents a hydrogen atom, a deuterium atom, or a substituent.

In one aspect of the invention, n71 to n74 are integers of 0 to 2. In one aspect of the invention, n71 and n73 are the same number, and n72 and n74 are the same number. n71 to n74 may be the same number. For example, n71 to n74 may be 0. n71 to n74 may be all 1. Further, for example, n71 and n73 may be 0, and n72 and n74 may be 1. In relation to preferable groups for $Ar^{71}$ to $Ar^{74}$, $A^1$, and $A^2$, corresponding descriptions on $Ar^1$ to $Ar^4$, $A^1$, and $A^2$ in the formula (16a) may be referred to.

Hereinafter, specific examples of the compound represented by the formula (22a) will be given. Compounds of the formula (22a) that may be used in the invention are not construed as limiting to the following specific examples.

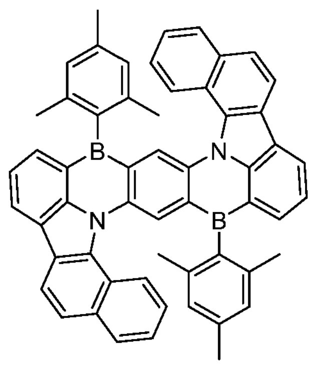

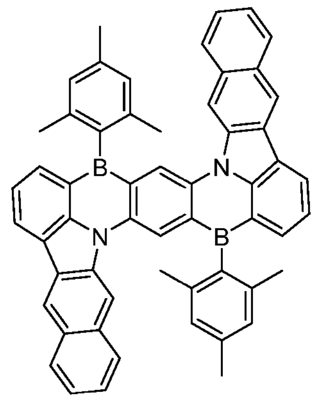

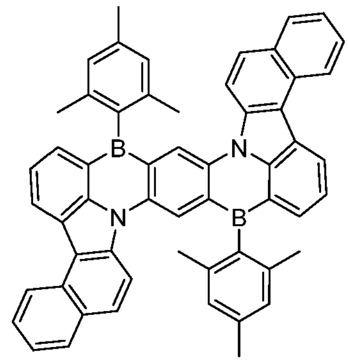

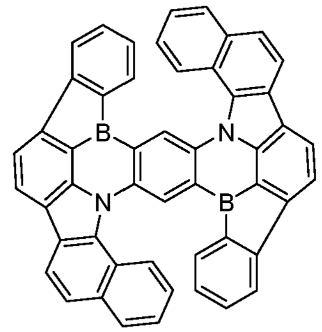

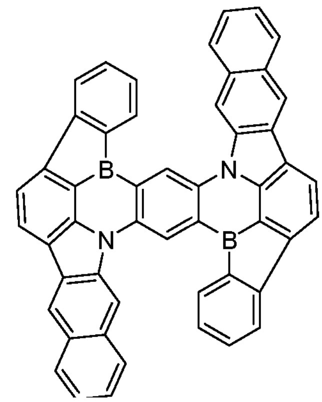

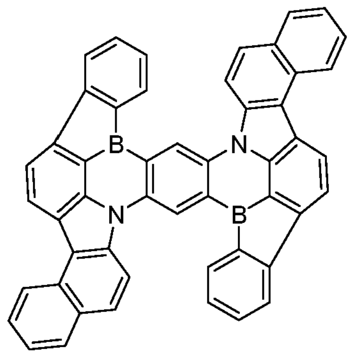

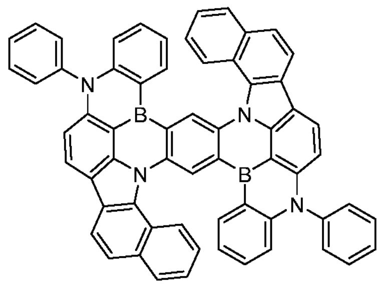

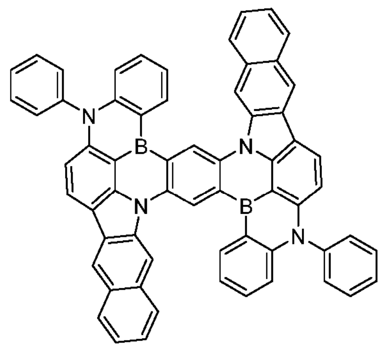

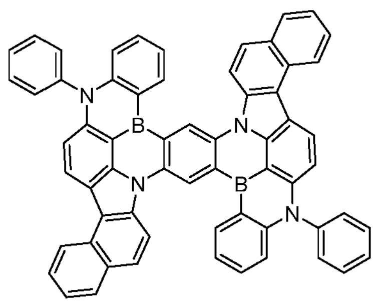

As one preferable group of compounds having the skeleton (22b), compounds represented by the following formula (22b) may be exemplified.

Formula (22b)

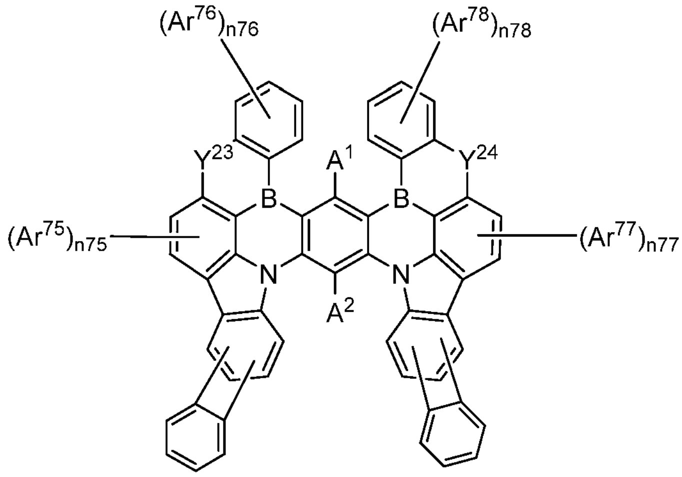

In the formula (22b), each of $Ar^{75}$ to $Ar^{78}$ independently represents a substituted or unsubstituted aryl group, or a substituted or unsubstituted heteroaryl group. Each of n75 and n77 independently represents an integer of 0 to 2. Each of n76 and n78 independently represents an integer of 0 to 4. Each of $Y^{23}$ and $Y^{24}$ independently represents two hydrogen atoms, a single bond or $N(R^{27})$. $R^{27}$ represents a hydrogen atom, a deuterium atom, or a substituent. For detailed descriptions of n75 to n78, descriptions on n71 to n74 in the formula (22a) may be referred to in this order. In relation to preferable groups for $Ar^{75}$ to $Ar^{78}$, corresponding descriptions on $Ar^1$ to $Ar^4$ in the formula (16a) may be referred to.

Hereinafter, specific examples of the compound represented by the formula (22b) will be given. Compounds of the formula (22b) that may be used in the invention are not construed as limiting to the following specific examples.

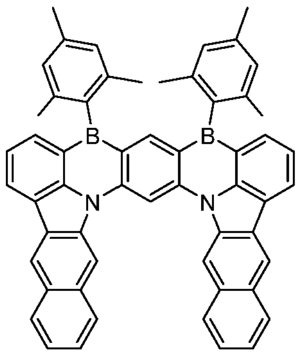

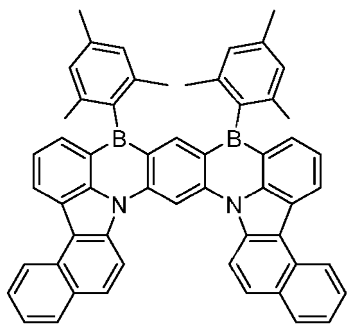

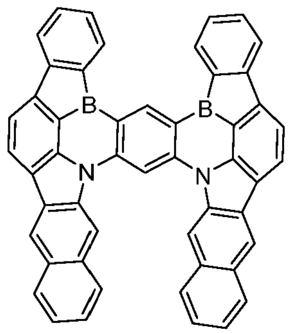

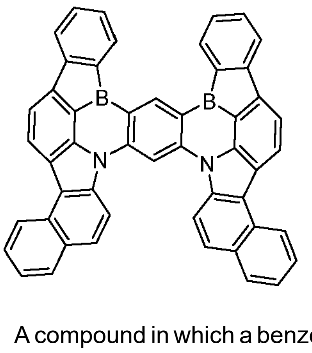

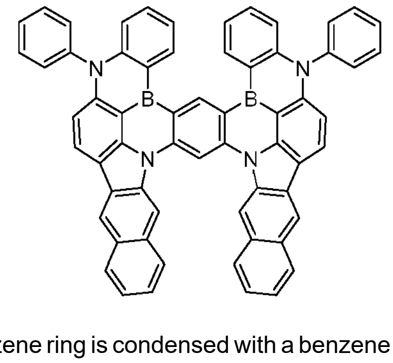

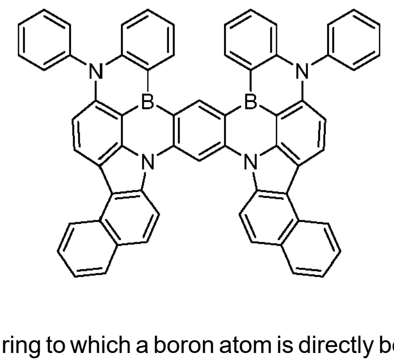

A compound in which a benzene ring is condensed with a benzene ring to which a boron atom is directly bonded, between two benzene rings forming a carbazole partial structure existing in the formula (16), may be preferably mentioned. Examples of such a compound include a compound having the following skeleton (23a), and a compound having the following skeleton (23b).

Skeleton (23a)

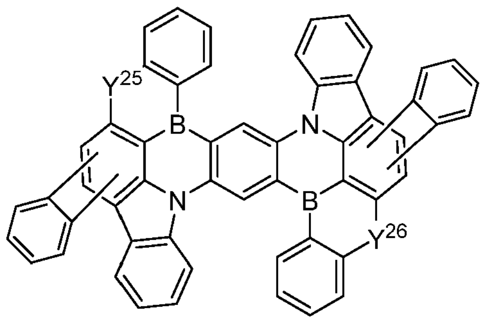

Skeleton (23b)

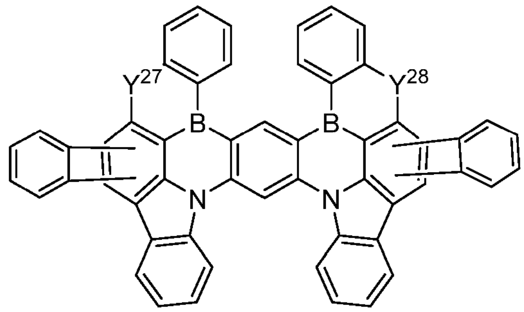

In the skeletons (23a) and (23b), each of $Y^{25}$ to $Y^{28}$ independently represents two hydrogen atoms, a single bond or $N(R^{27})$. In relation to details of $Y^{25}$ to $Y^{28}$, corresponding descriptions for the skeletons (19a) and (19b) may be referred to. In one aspect of the invention, each hydrogen atom in the skeletons (23a) and (23b) is not substituted with a linking group together with an adjacent hydrogen atom to form a ring structure.

As one preferable group of compounds having the skeleton (23a), compounds represented by the following formula (23a) may be exemplified.

Formula (23a)

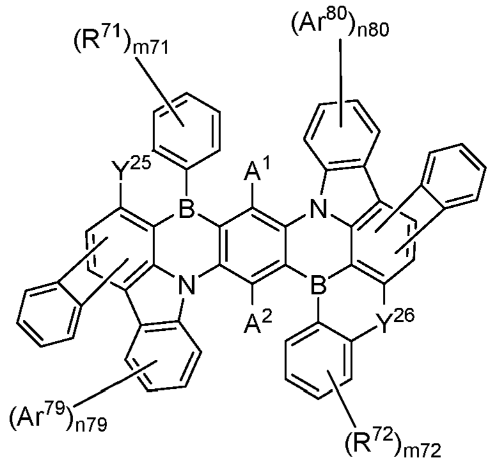

**In** the formula (23a), each of $Ar^{79}$ and $Ar^{80}$ independently represents a substituted or unsubstituted aryl group, or a substituted or unsubstituted heteroaryl group. Each of $R^{71}$ and $R^{72}$ independently represents a substituted or unsubstituted alkyl group. Each of m71 and m72 independently represents an integer of 0 to 4. Each of n79 and n80 independently represents an integer of 0 to 4. Each of $Y^{25}$ and $Y^{26}$ independently represents two hydrogen atoms, a single bond or $N(R^{27})$. $R^{27}$ represents a hydrogen atom, a deuterium atom, or a substituent. Each of $A^1$ and $A^2$ independently represents a hydrogen atom, a deuterium atom, or a substituent.

In one aspect of the invention, n79 and n80 are integers of 0 to 2. In one aspect of the invention, n79 and n80 are the same number, and for example, may be all 0, or may be all 1. In one aspect of the invention, m71 and m72 are integers of 0 to 2. In one aspect of the invention, m71 and m72 are the same number, and for example, may be all 0, or may be all 1. In relation to preferable groups for $Ar^{79}$, $Ar^{80}$, $R^{71}$, $R^{72}$, $A^1$, and $A^2$, corresponding descriptions on $Ar^1$, $Ar^3$, $R^{41}$, $R^{42}$, $A^1$, and $A^2$ in the formula (16a) may be referred to.

Hereinafter, specific examples of the compound represented by the formula (23a) will be given. Compounds of the formula (23a) that may be used in the invention are not construed as limiting to the following specific examples.

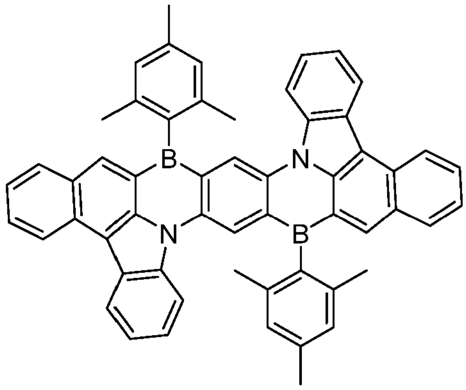

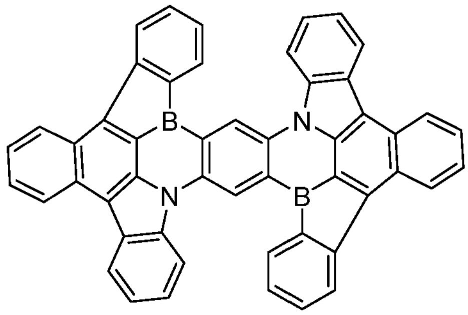

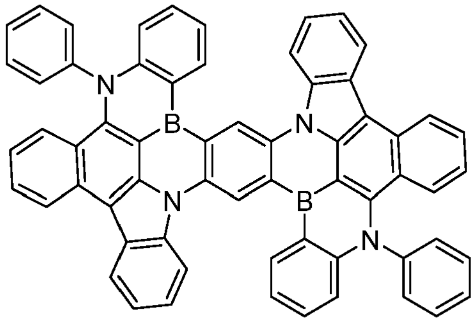

As one preferable group of compounds having the skeleton (23b), compounds represented by the following formula (23b) may be exemplified.

Formula (23b)

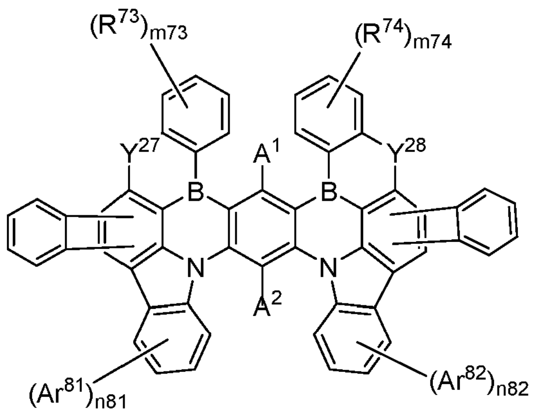

In the formula (23b), each of $Ar^{81}$ and $Ar^{82}$ independently represents a substituted or unsubstituted aryl group, or a substituted or unsubstituted heteroaryl group. Each of $R^{73}$ and $R^{74}$ independently represents a substituted or unsubstituted alkyl group. Each of m73 and m74 independently represents an integer of 0 to 4. Each of n81 and n82 independently represents an integer of 0 to 4. Each of $Y^{27}$ and $Y^{28}$ independently represents two hydrogen atoms, a single bond or $N(R^{27})$. $R^{27}$ represents a hydrogen atom, a deuterium atom, or a substituent. Each of $A^1$ and $A^2$ independently represents a hydrogen atom, a deuterium atom, or a substituent.

In relation to detailed descriptions of m73, m74, n81, and n82, descriptions on m71, m72, n79, and n80 in the formula (23a) may be referred to. In relation to preferable groups for $Ar^{81}$, $Ar^{82}$, $R^{73}$, $R^{74}$, $A^1$, and $A^2$, corresponding descriptions on $Ar^1$, $Ar^3$, $R^{41}$, $R^{42}$, $A^1$, and $A^2$ in the formula (16a) may be referred to.

Hereinafter, specific examples of the compound represented by the formula (23b) will be given. Compounds of the formula (23b) that may be used in the invention are not construed as limiting to the following specific examples.

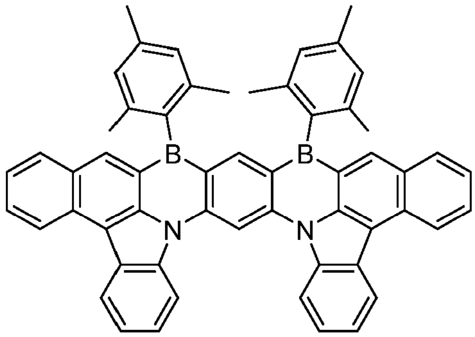

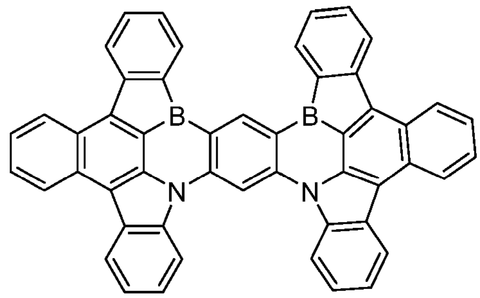

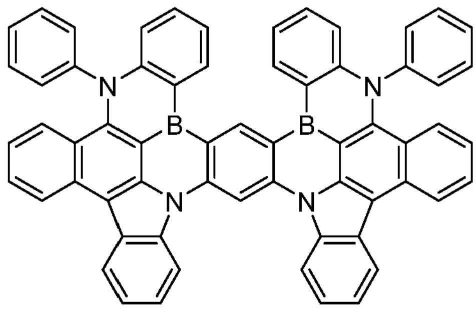

A compound in which benzene rings are condensed with both of two benzene rings forming a carbazole partial structure existing in the formula (16) may be preferably mentioned. Examples of such a compound include a compound having the following skeleton (24a), and a compound having the following skeleton (24b).

Skeleton (24a)

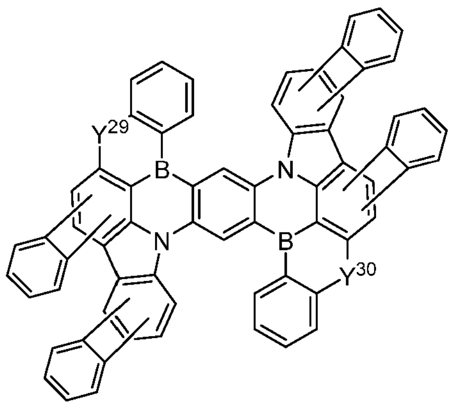

Skeleton (24b)

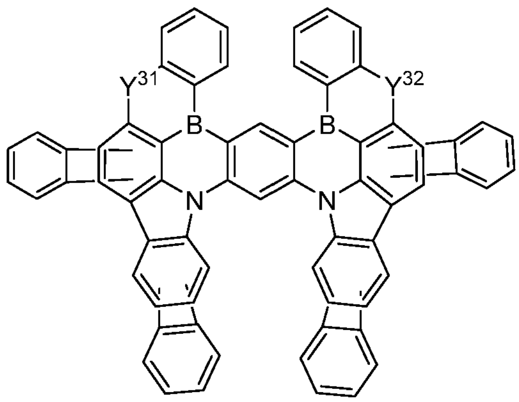

In the skeletons (24a) and (24b), each of $Y^{29}$ to $Y^{32}$ independently represents two hydrogen atoms, a single bond or $N(R^{27})$. In relation to details of $Y^{29}$ to $Y^{32}$, corresponding descriptions for the skeletons (19a) and (19b) may be referred to. In one aspect of the invention, each hydrogen atom in the skeletons (24a) and (24b) is not substituted with a linking group together with an adjacent hydrogen atom to form a ring structure.

As one preferable group of compounds having the skeleton (24a), compounds represented by the following formula (24a) may be exemplified.

Formula (24a)

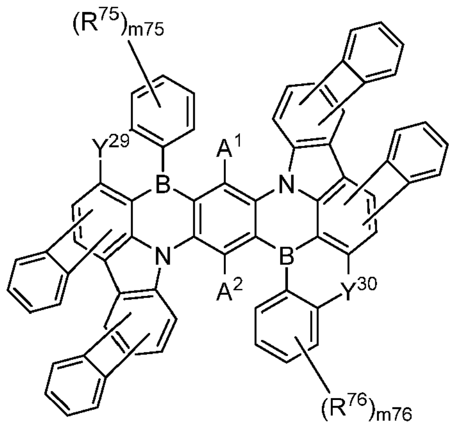

In the formula (24a), each of $R^{75}$ and $R^{76}$ independently represents a substituted or unsubstituted alkyl group. Each of m75 and m76 independently represents an integer of 0 to 4. Each of $Y^{29}$ and $Y^{30}$ independently represents two hydrogen atoms, a single bond or $N(R^{27})$. $R^{27}$ represents a hydrogen atom, a deuterium atom, or a substituent. Each of $A^1$ and $A^2$ independently represents a hydrogen atom, a deuterium atom, or a substituent. In relation to details of $R^{71}$, $R^{76}$, m75, m76, $A^1$, and $A^2$, descriptions on $R^{71}$, $R^{72}$, m71, m72, $A^1$, and $A^2$ in the formula (23a) may be referred to.

Hereinafter, specific examples of the compound represented by the formula (24a) will be given. Compounds of the formula (24a) that may be used in the invention are not construed as limiting to the following specific examples.

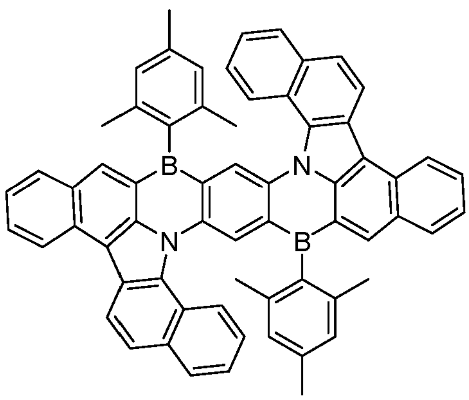

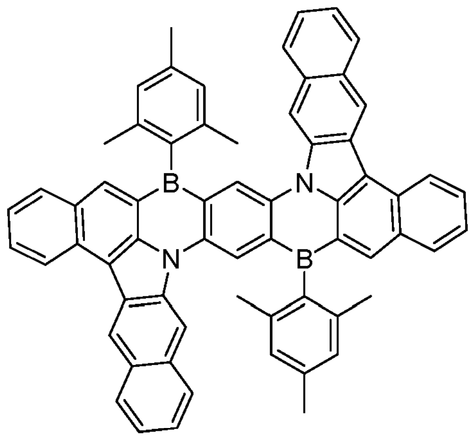

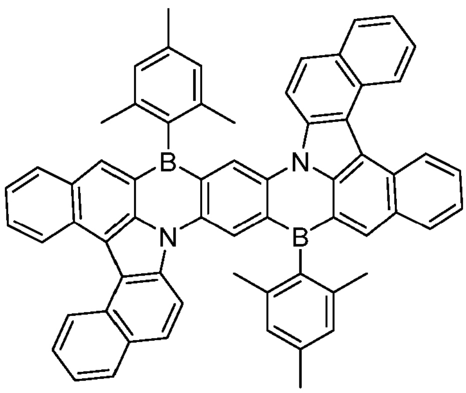

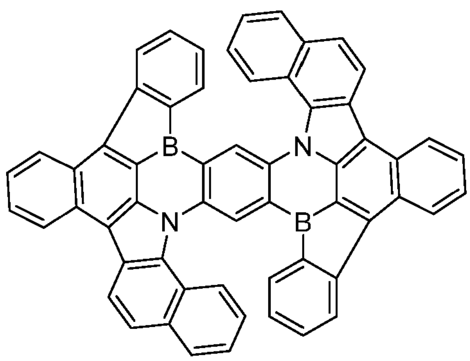

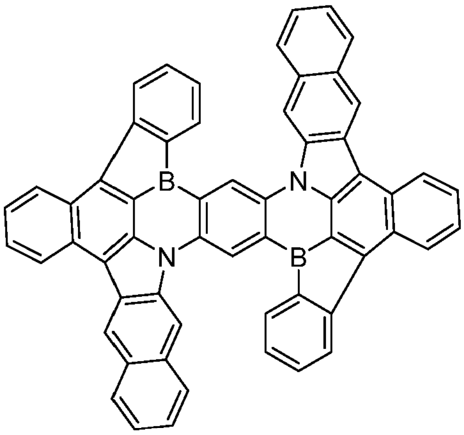

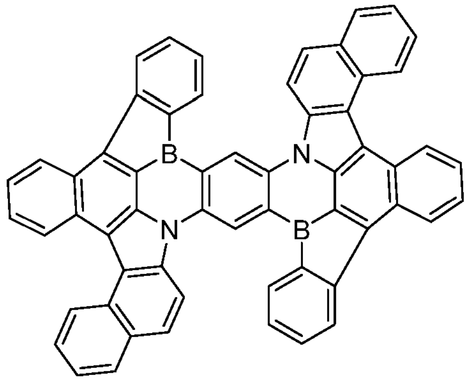

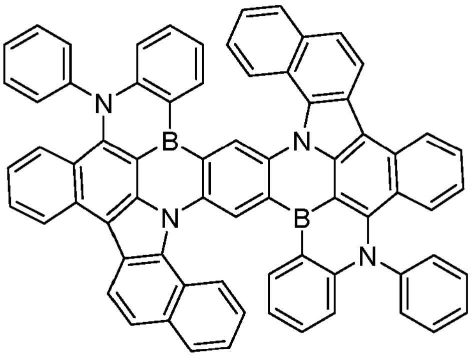

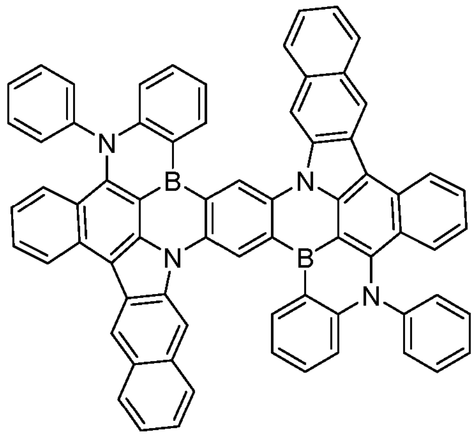

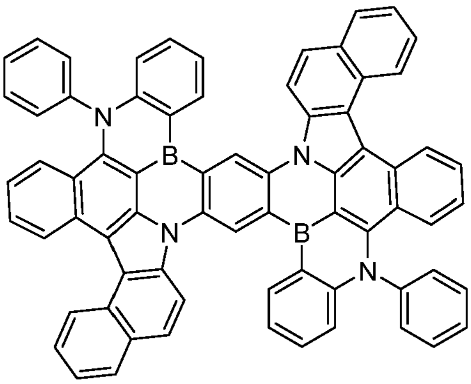

As one preferable group of compounds having the skeleton (24b), compounds represented by the following formula (24b) may be exemplified.

Formula (24b)

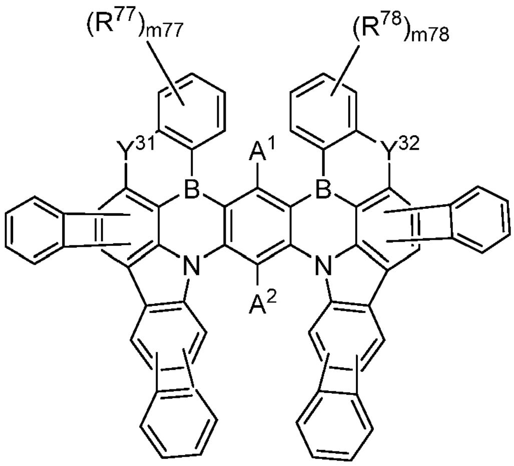

In the formula (24b), each of $R^{77}$ and $R^{78}$ independently represents a substituted or unsubstituted alkyl group. Each of m77 and m78 independently represents an integer of 0 to 4. Each of $Y^{31}$ and $Y^{32}$ independently represents two hydrogen atoms, a single bond or $N(R^{27})$. $R^{27}$ represents a hydrogen atom, a deuterium atom, or a substituent. Each of $A^1$ and $A^2$ independently represents a hydrogen atom, a deuterium atom, or a substituent. In relation to details of $R^{77}$, $R^{78}$, m77, m78, $A^1$, and $A^2$, descriptions on $R^{71}$, $R^{72}$, m71, m72, $A^1$, and $A^2$ in the formula (23a) may be referred to.

Hereinafter, specific examples of the compound represented by the formula (24b) will be given. Compounds of the formula (24b) that may be used in the invention are not construed as limiting to the following specific examples.

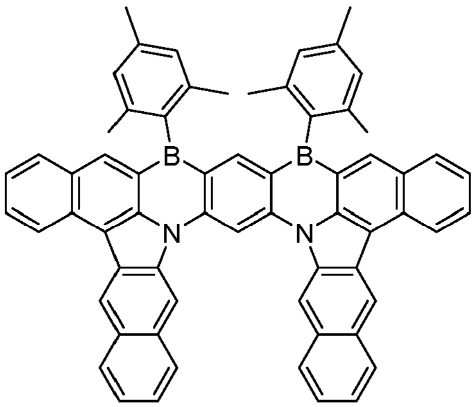

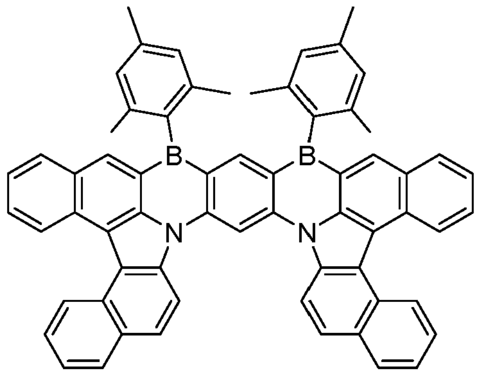

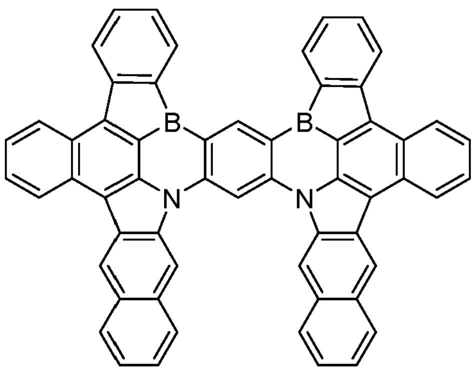

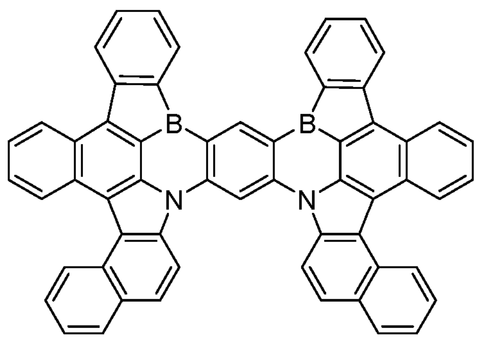

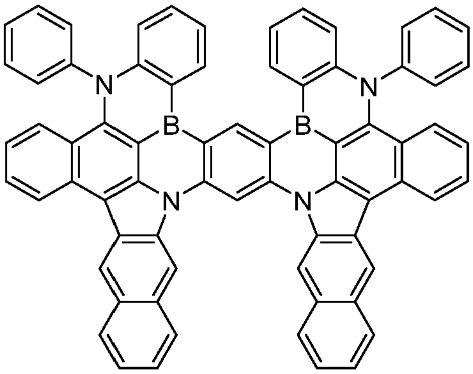

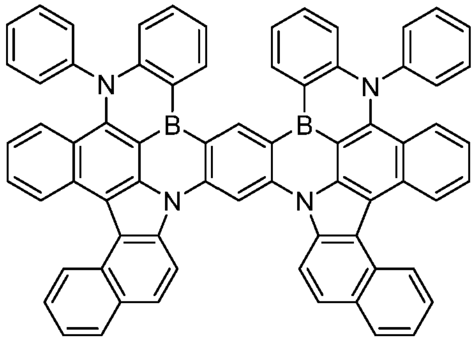

As the compound represented by the formula (16), a compound in which four or more carbazole partial structures are included in the molecule is also preferable. As an example of such a compound, a compound having the following skeleton (25) may be exemplified.

Skeleton (25)

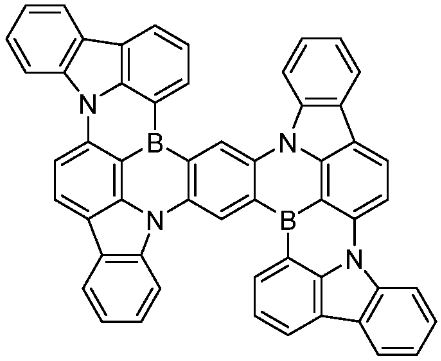

Each hydrogen atom in the skeleton (25) may be substituted with a deuterium atom or a substituent. Further, it may be substituted with a linking group together with an adjacent hydrogen atom to form a ring structure. For details, corresponding descriptions on $R^1$ to $R^{26}$, $A^1$, and $A^2$ in the formula (16) may be referred to. At least one hydrogen atom of a benzene ring forming a carbazole partial structure included in the skeleton (25) is substituted with a substituted or unsubstituted aryl group. In one aspect of the invention, each hydrogen atom in the skeleton (25) is not substituted with a linking group together with an adjacent hydrogen atom to form a ring structure.

As one preferable group of compounds having the skeleton (25), compounds represented by the following formula (25) may be exemplified.

Formula (25)

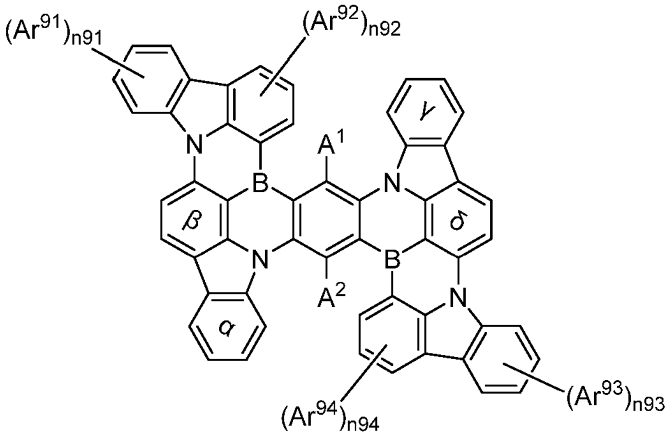

In the formula (25), each of $Ar^{91}$ to $Ar^{94}$ independently represents a substituted or unsubstituted aryl group, or a

substituted or unsubstituted heteroaryl group. Each of n91 and n93 independently represents an integer of 0 to 4, and each of n92 and n94 independently represents an integer of 0 to 3. An α ring, a β ring, a γ ring, and a δ ring may be substituted. At least one ring is substituted with a substituted or unsubstituted aryl group, is condensed with a benzene ring that may be substituted, or is condensed with a substituted or unsubstituted furan ring of benzofuran or a substituted or unsubstituted thiophene ring of thiophene. Each of $A^1$ and $A^2$ independently represents a hydrogen atom, a deuterium atom, or a substituent.

In one aspect of the invention, n91 to n94 are integers of 0 to 2. In one aspect of the invention, n91 and n93 are the same number, and n92 and n94 are the same number. n91 to n94 may be all the same number, and for example may be all 0, or may be all 1. In relation to preferable groups for $Ar^{91}$ to $Ar^{94}$, corresponding descriptions on $Ar^1$ to $Ar^4$ in the formula (16a) may be referred to. In one aspect of the invention, the α ring and the γ ring have the same substituents or have the same condensed structures, and the β ring and the δ ring have the same substituents or have the same condensed structures. In one aspect of the invention, both the β ring and the δ ring are substituted with substituted or unsubstituted aryl groups, are condensed with benzene rings that may be substituted, or are condensed with substituted or unsubstituted furan rings of benzofuran or substituted or unsubstituted thiophene rings of thiophene. In one aspect of the invention, both the α ring and the γ ring are substituted with substituted or unsubstituted aryl groups, are condensed with benzene rings that may be substituted, or are condensed with substituted or unsubstituted furan rings of benzofuran or substituted or unsubstituted thiophene rings of thiophene. In one aspect of the invention, all of the α ring, the β ring, the γ ring, and the δ ring are substituted with substituted or unsubstituted aryl groups, are condensed with benzene rings that may be substituted, or are condensed with substituted or unsubstituted furan rings of benzofuran or substituted or unsubstituted thiophene rings of thiophene. In relation to descriptions and preferable ranges of $A^1$ and $A^2$, corresponding descriptions for the formula (16) may be referred to.

Hereinafter, specific examples of the compound represented by the formula (25) will be given. Compounds of the formula (25) that may be used in the invention are not construed as limiting to the following specific examples.

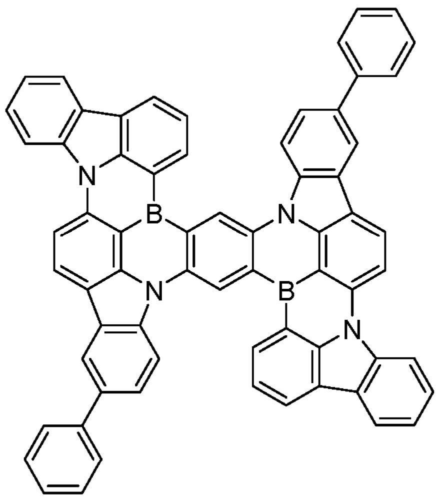

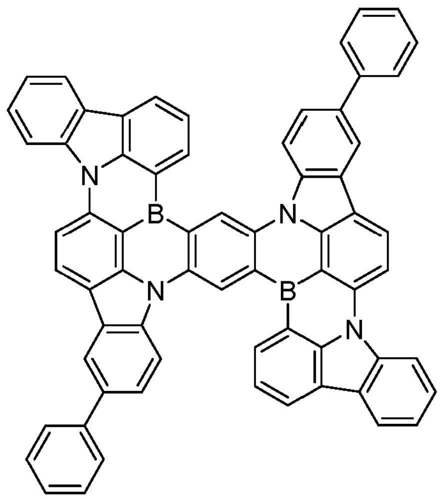

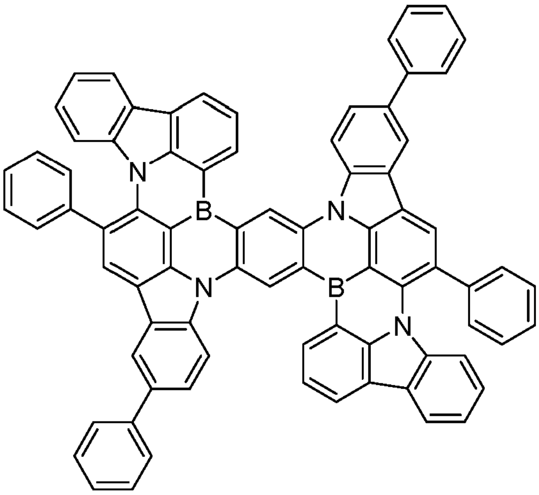
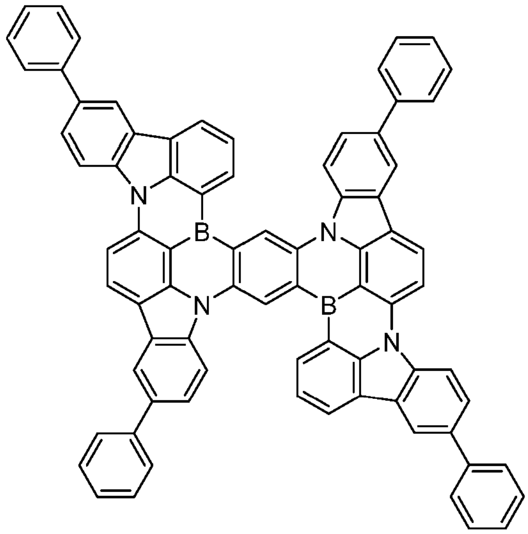
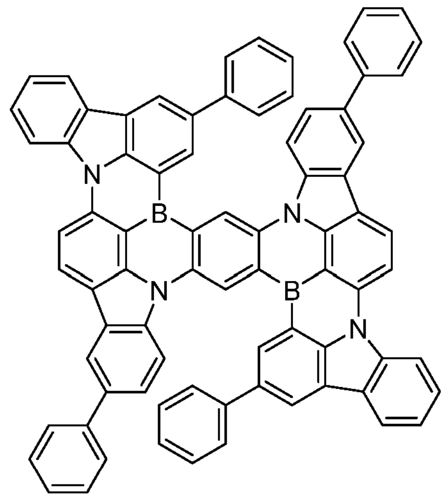
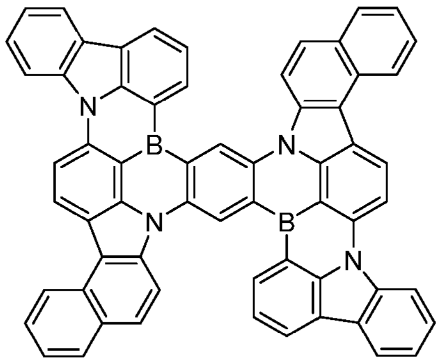
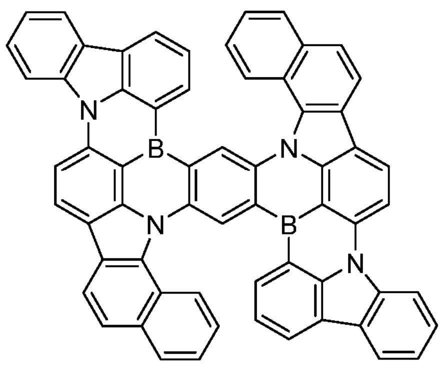
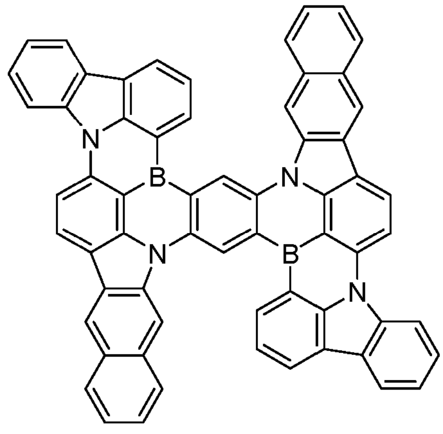

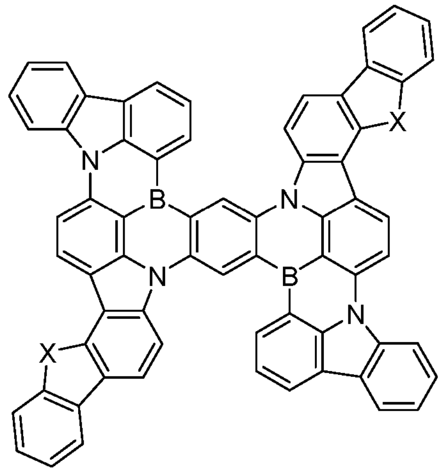

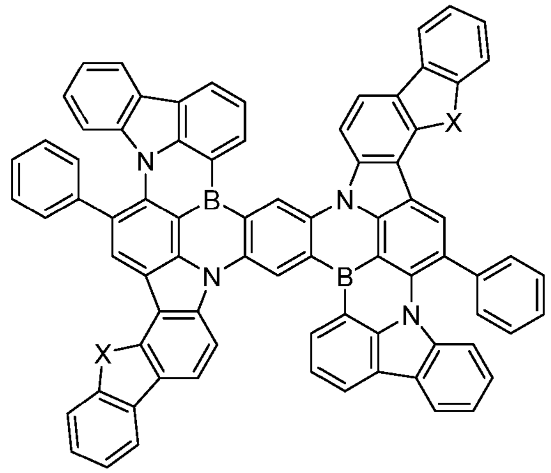

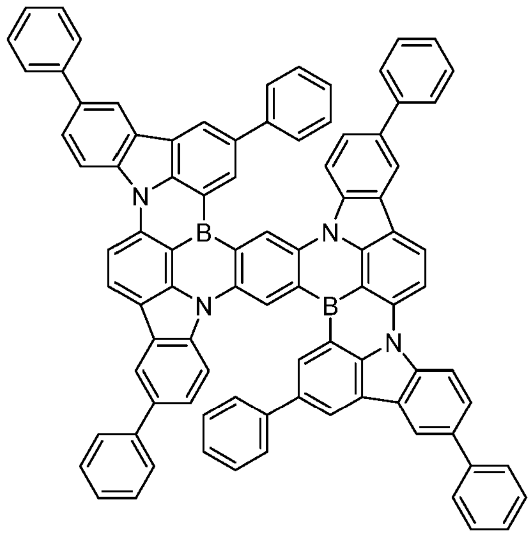

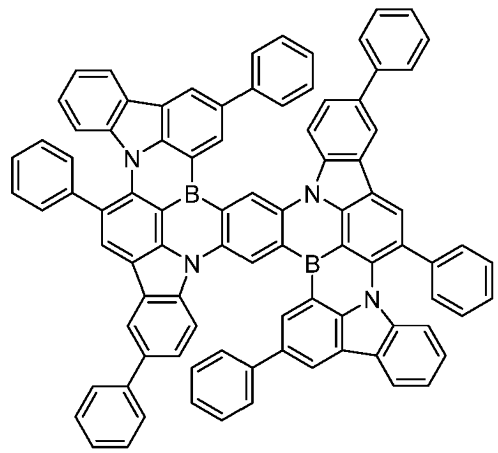

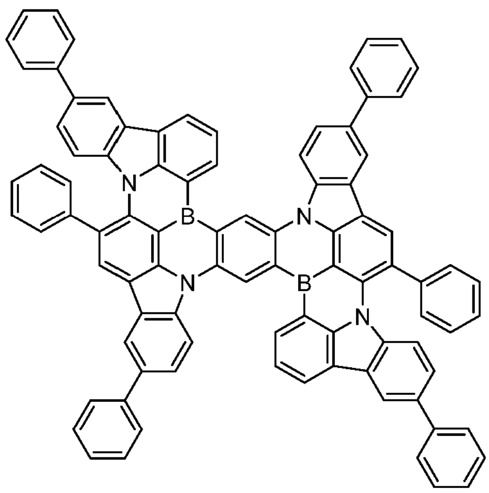

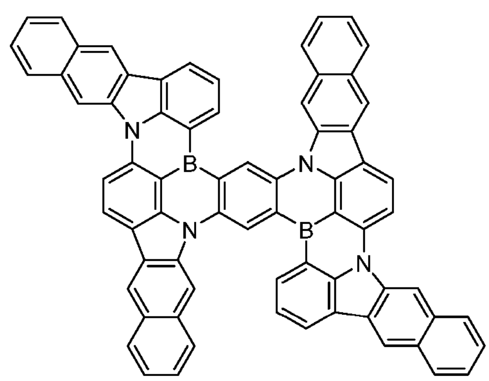

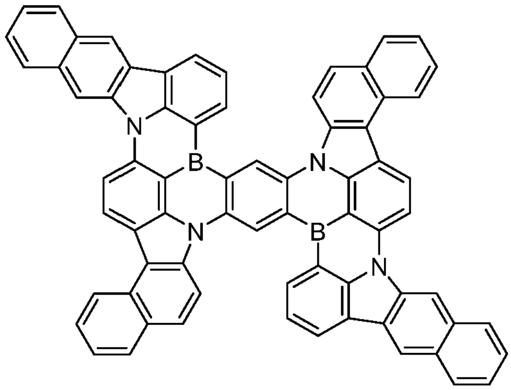

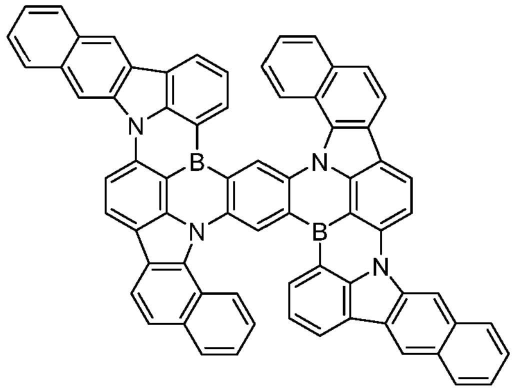

In one aspect of the invention, the skeletons (16a) to (25) are skeletons in which other rings are not further condensed. In one aspect of the invention, the skeletons (16a) to (25) are skeletons in which other rings may be further condensed. Regarding other rings mentioned herein, the above descriptions on the ring structures formed by bonding $R^1$ and $R^2$, $R^2$ and $R^3$, $R^3$ and $R^4$, $R^4$ and $R^5$, $R^5$ and $R^6$, $R^6$ and $R^7$, $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{18}$ and $R^{19}$, $R^{19}$ and $R^{20}$, $R^{20}$ and $R^{21}$, $R^{22}$ and $R^{23}$, $R^{23}$ and $R^{24}$, $R^{24}$ and $R^{25}$, and $R^{25}$ and $R^{26}$ to each other may be referred to.

In one aspect of the invention, $A^1$ and $A^2$ in the formula (16) are acceptor groups. For example, a compound having acceptor groups at positions of $A^1$ and $A^2$ and having any of the skeletons (16a) to (25) may be mentioned. In relation to descriptions and specific examples of the acceptor group, descriptions, and specific examples of the acceptor group for $A^1$ and $A^2$ in the formula (16) may be referred to.

Hereinafter, specific examples of a compound in which $A^1$ and $A^2$ are acceptor groups will be given. The compounds in which $A^1$ and $A^2$ are acceptor groups, which may be used in the invention, are not construed as limiting to the following specific examples. The following specific examples have structures in which both $A^1$ and $A^2$ are "A", and the structure of each compound is specified by individually specifying the "A".

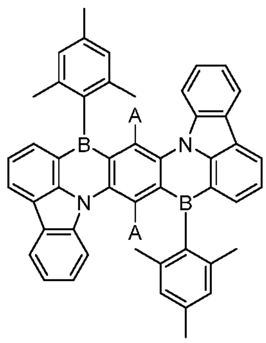

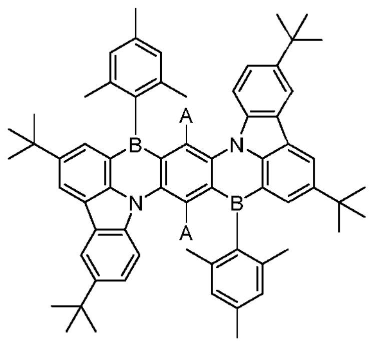

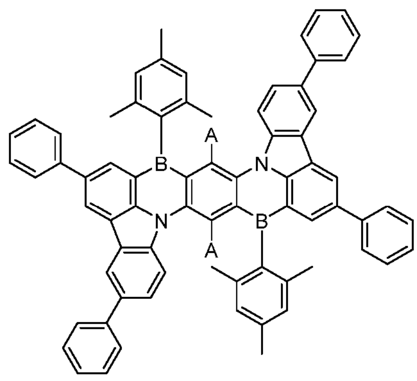

| | | |
|---|---|---|
| 1a:A=A1 | 2a:A=A1 | 3a:A=A1 |
| 1b:A=A2 | 2b:A=A2 | 3b:A=A2 |
| 1c:A=A3 | 2c:A=A3 | 3c:A=A3 |
| 1d:A=A4 | 2d:A=A4 | 3d:A=A4 |
| 1e:A=A7 | 2e:A=A7 | 3e:A=A7 |
| 1f:A=A10 | 2f:A=A10 | 3f:A=A10 |
| 1g:A=A11 | 2g:A=A11 | 3g:A=A11 |
| 1h:A=A16 | 2h:A=A16 | 3h:A=A16 |
| 1i:A=A35 | 2i:A=A35 | 3i:A=A35 |
| 1j:A=A39 | 2j:A=A39 | 3j:A=A39 |
| 1k:A=A40 | 2k:A=A40 | 3k:A=A40 |
| 1l:A=A41 | 2l:A=A41 | 3l:A=A41 |
| 1m:A=A42 | 2m:A=A42 | 3m:A=A42 |

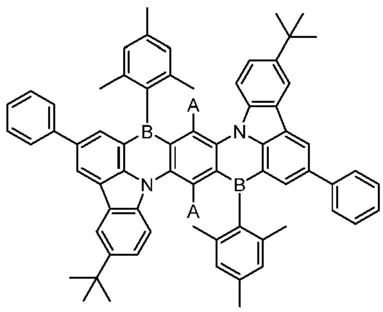

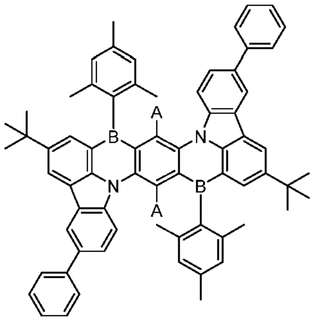

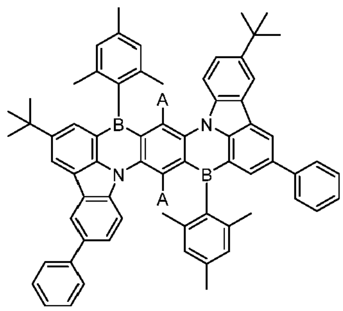

| | | |
|---|---|---|
| 4a:A=A1 | 5a:A=A1 | 6a:A=A1 |
| 4b:A=A2 | 5b:A=A2 | 6b:A=A2 |
| 4c:A=A3 | 5c:A=A3 | 6c:A=A3 |
| 4d:A=A4 | 5d:A=A4 | 6d:A=A4 |
| 4e:A=A7 | 5e:A=A7 | 6e:A=A7 |
| 4f:A=A10 | 5f:A=A10 | 6f:A=A10 |
| 4g:A=A11 | 5g:A=A11 | 6g:A=A11 |
| 4h:A=A16 | 5h:A=A16 | 6h:A=A16 |
| 4i:A=A35 | 5i:A=A35 | 6i:A=A35 |
| 4j:A=A39 | 5j:A=A39 | 6j:A=A39 |
| 4k:A=A40 | 5k:A=A40 | 6k:A=A40 |
| 4l:A=A41 | 5l:A=A41 | 6l:A=A41 |
| 4m:A=A42 | 5m:A=A42 | 6m:A=A42 |

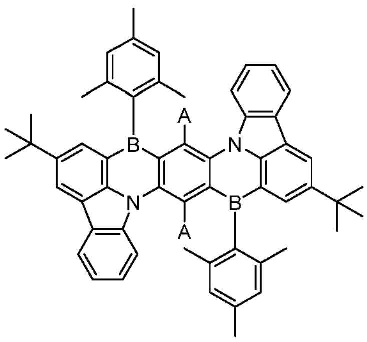

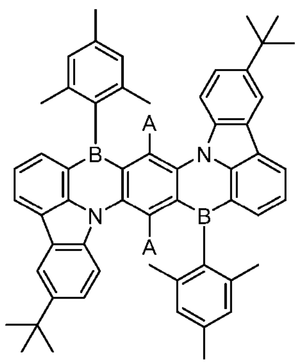

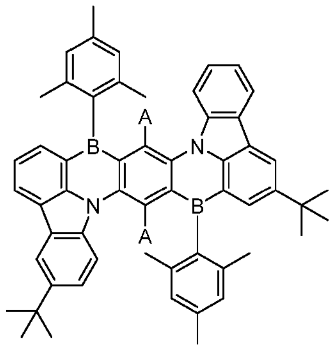

| | | |
|---|---|---|
| 7a:A=A1 | 8a:A=A1 | 9a:A=A1 |
| 7b:A=A2 | 8b:A=A2 | 9b:A=A2 |
| 7c:A=A3 | 8c:A=A3 | 9c:A=A3 |
| 7d:A=A4 | 8d:A=A4 | 9d:A=A4 |
| 7e:A=A7 | 8e:A=A7 | 9e:A=A7 |
| 7f:A=A10 | 8f:A=A10 | 9f:A=A10 |
| 7g:A=A11 | 8g:A=A11 | 9g:A=A11 |
| 7h:A=A16 | 8h:A=A16 | 9h:A=A16 |
| 7i:A=A35 | 8i:A=A35 | 9i:A=A35 |
| 7j:A=A39 | 8j:A=A39 | 9j:A=A39 |
| 7k:A=A40 | 8k:A=A40 | 9k:A=A40 |
| 7l:A=A41 | 8l:A=A41 | 9l:A=A41 |
| 7m:A=A42 | 8m:A=A42 | 9m:A=A42 |

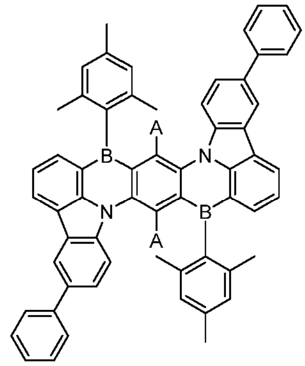

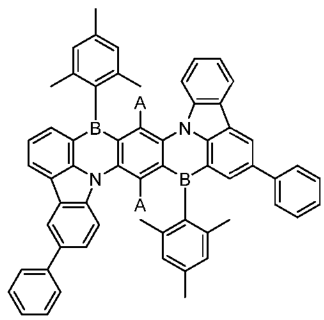

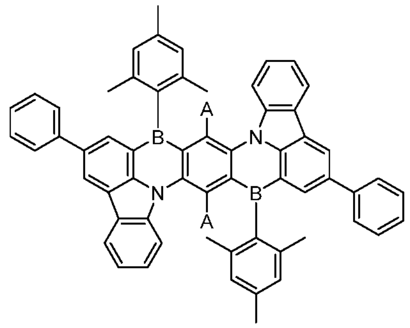

10a:A=A1
10b:A=A2
10c:A=A3
10d:A=A4
10e:A=A7
10f:A=A10
10g:A=A11
10h:A=A16
10i:A=A35
10j:A=A39
10k:A=A40
10l:A=A41
10m:A=A42

11a:A=A1
11b:A=A2
11c:A=A3
11d:A=A4
11e:A=A7
11f:A=A10
11g:A=A11
11h:A=A16
11i:A=A35
11j:A=A39
11k:A=A40
11l:A=A41
11m:A=A42

12a:A=A1
12b:A=A2
12c:A=A3
12d:A=A4
12e:A=A7
12f:A=A10
12g:A=A11
12h:A=A16
12i:A=A35
12j:A=A39
12k:A=A40
12l:A=A41
12m:A=A42

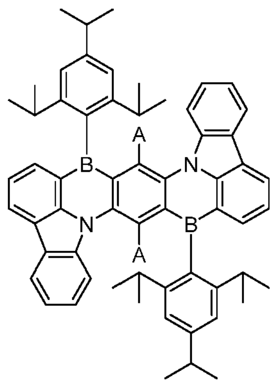

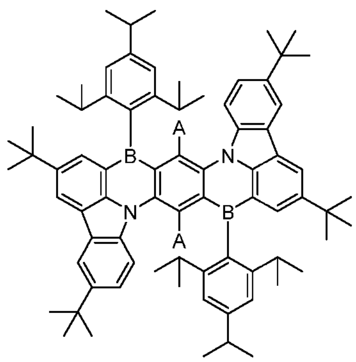

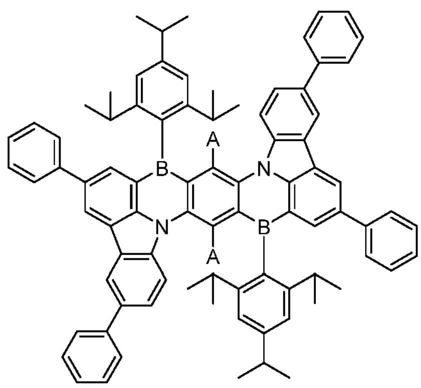

13a:A=A1
13b:A=A2
13c:A=A3
13d:A=A4
13e:A=A7
13f:A=A10
13g:A=A11
13h:A=A16
13i:A=A35
13j:A=A39
13k:A=A40
13l:A=A41
13m:A=A42

14a:A=A1
14b:A=A2
14c:A=A3
14d:A=A4
14e:A=A7
14f:A=A10
14g:A=A11
14h:A=A16
14i:A=A35
14j:A=A39
14k:A=A40
14l:A=A41
14m:A=A42

15a:A=A1
15b:A=A2
15c:A=A3
15d:A=A4
15e:A=A7
15f:A=A10
15g:A=A11
15h:A=A16
15i:A=A35
15j:A=A39
15k:A=A40
15l:A=A41
15m:A=A42

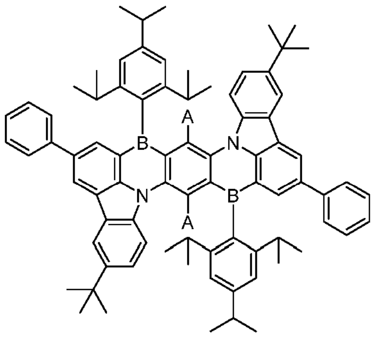
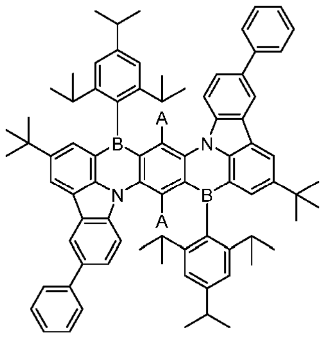
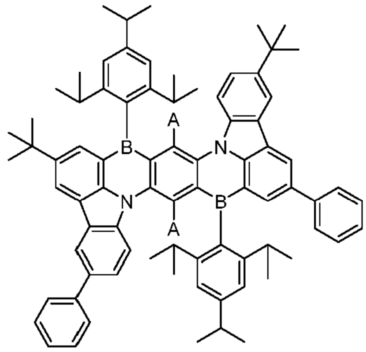

| | | |
|---|---|---|
| 16a:A=A1 | 17a:A=A1 | 18a:A=A1 |
| 16b:A=A2 | 17b:A=A2 | 18b:A=A2 |
| 16c:A=A3 | 17c:A=A3 | 18c:A=A3 |
| 16d:A=A4 | 17d:A=A4 | 18d:A=A4 |
| 16e:A=A7 | 17e:A=A7 | 18e:A=A7 |
| 16f:A=A10 | 17f:A=A10 | 18f:A=A10 |
| 16g:A=A11 | 17g:A=A11 | 18g:A=A11 |
| 16h:A=A16 | 17h:A=A16 | 18h:A=A16 |
| 16i:A=A35 | 17i:A=A35 | 18i:A=A35 |
| 16j:A=A39 | 17j:A=A39 | 18j:A=A39 |
| 16k:A=A40 | 17k: A=A40 | 18k:A=A40 |
| 16l:A=A41 | 17l:A=A41 | 18l:A=A41 |
| 16m:A=A42 | 17m:A=A42 | 18m:A=A42 |

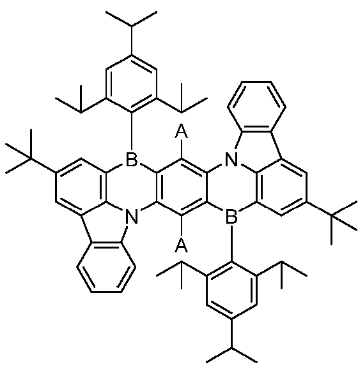
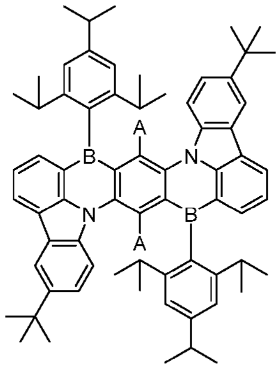
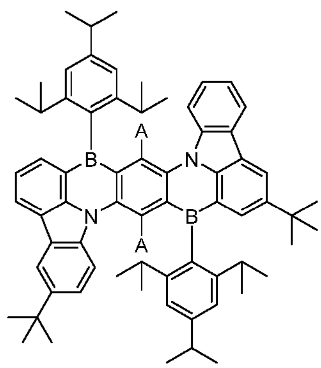

| | | |
|---|---|---|
| 19a:A=A1 | 20a:A=A1 | 21a:A=A1 |
| 19b:A=A2 | 20b:A=A2 | 21b:A=A2 |
| 19c:A=A3 | 20c:A=A3 | 21c:A=A3 |
| 19d:A=A4 | 20d:A=A4 | 21d:A=A4 |
| 19e:A=A7 | 20e:A=A7 | 21e:A=A7 |
| 19f:A=A10 | 20f:A=A10 | 21fA=A10 |
| 19g:A=A11 | 20g:A=A11 | 21g:A=A11 |
| 19h:A=A16 | 20h:A=A16 | 21h:A=A16 |
| 19i:A=A35 | 20i:A=A35 | 21i:A=A35 |
| 19j:A=A39 | 20j:A=A39 | 21j:A=A39 |
| 19k:A=A40 | 20k:A=A40 | 21k:A=A40 |
| 19l:A=A41 | 20l:A=A41 | 21l:A=A41 |
| 19m:A=A42 | 20m:A=A42 | 21m:A=A42 |

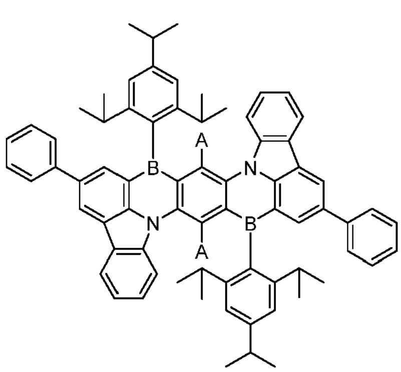 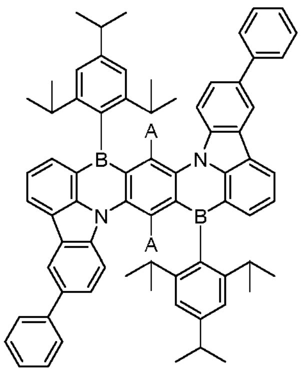 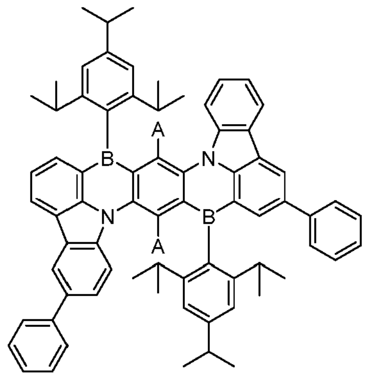

| | | |
|---|---|---|
| 22a:A=A1 | 23a:A=A1 | 24a:A=A1 |
| 22b:A=A2 | 23b:A=A2 | 24b:A=A2 |
| 22c:A=A3 | 23c:A=A3 | 24c:A=A3 |
| 22d:A=A4 | 23d:A=A4 | 24d:A=A4 |
| 22e:A=A7 | 23e:A=A7 | 24e:A=A7 |
| 22f:A=A 10 | 23f:A=A10 | 24f:A=A10 |
| 22g:A=A11 | 23g:A=A11 | 24g:A=A11 |
| 22h:A=A16 | 23h:A=A16 | 24h:A=A16 |
| 22i:A=A35 | 23i:A=A35 | 24i:A=A35 |
| 12j:A=A39 | 6j:A=A39 | 6j:A=A39 |
| 22k:A=A40 | 23k:A=A40 | 24k: A=A40 |
| 22l:A=A41 | 23l:A=A41 | 24l:A=A41 |
| 22m:A=A42 | 23m:A=A42 | 24m:A=A42 |

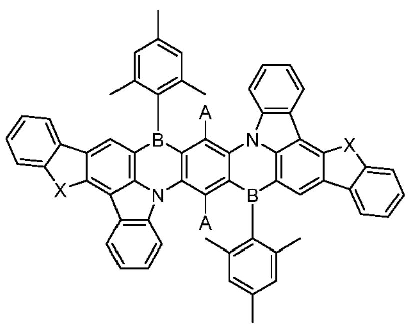 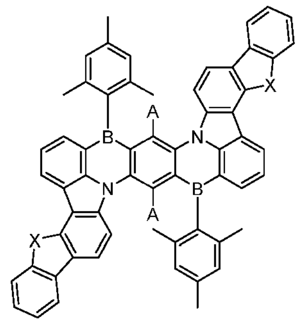 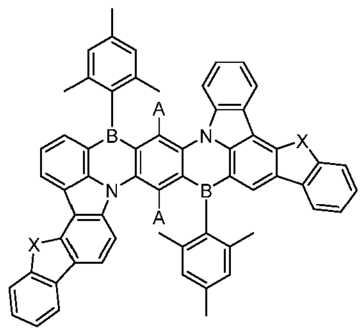

| | | |
|---|---|---|
| 25a:A=A1 | 26a:A=A1 | 27a:A=A1 |
| 25b:A=A2 | 26b:A=A2 | 27b:A=A2 |
| 25c:A=A3 | 26c:A=A3 | 27c:A=A3 |
| 25d:A=A4 | 26d:A=A4 | 27d:A=A4 |
| 25e:A=A7 | 26e:A=A7 | 27e:A=A7 |
| 25f:A=A10 | 26f:A=A10 | 27f:A=A10 |
| 25g:A=A11 | 26g:A=A11 | 27g:A=A11 |
| 25h:A=A16 | 26h:A=A16 | 27h:A=A16 |
| 25i:A=A35 | 26i:A=A35 | 27i:A=A35 |
| 25j:A=A39 | 16j:A=A39 | 27j:A=A39 |
| 25k:A=A40 | 26k: A=A40 | 27k:A=A40 |
| 25l:A=A41 | 26l:A=A41 | 27l:A=A41 |
| 25m:A=A42 | 26m:A=A42 | 27m:A=A42 |

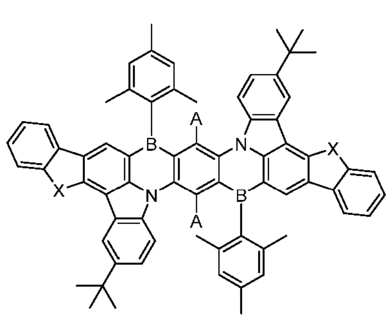
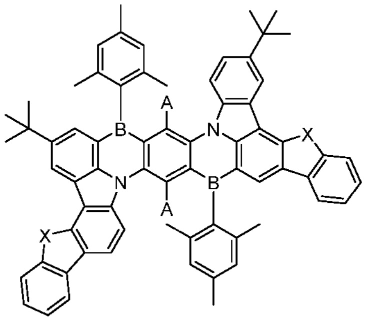
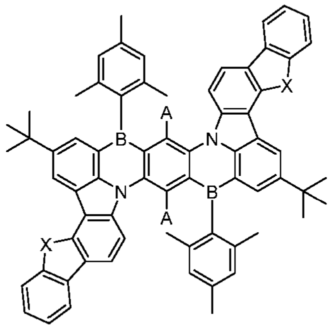

28a:A=A1 29a:A=A1 30a:A=A1
28b:A=A2 29b:A=A2 30b:A=A2
28c:A=A3 29c:A=A3 30c:A=A3
28d:A=A4 29d:A=A4 30d:A=A4
28e:A=A7 29e:A=A7 30e:A=A7
28f:A=A10 29f:A=A10 30f:A=A10
28g:A=A11 29g:A=A11 30g:A=A11
28h:A=A16 29h:A=A16 30h:A=A16
28i:A=A35 29i:A=A35 30i:A=A35
28j:A=A39 29j:A=A39 30j:A=A39
28k:A=A40 29k:A=A40 30k:A=A40
28l:A=A41 29l:A=A41 30l:A=A41
28m:A=A42 29m:A=A42 30m:A=A42

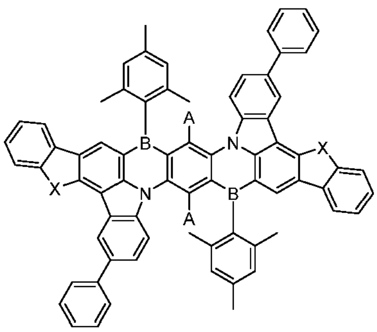
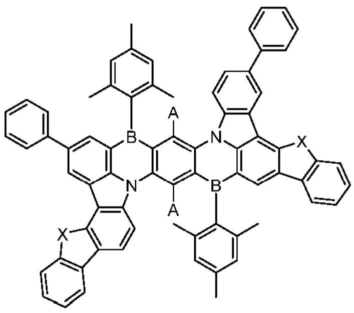
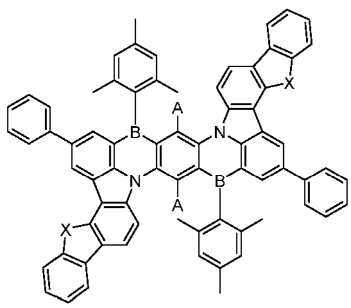

31a:A=A1 32a:A=A1 33a:A=A1
31b:A=A2 32b:A=A2 33b:A=A2
31c:A=A3 32c:A=A3 33c:A=A3
31d:A=A4 32d:A=A4 33d:A=A4
31e:A=A7 32e:A=A7 33e:A=A7
31f:A=A10 32f:A=A10 33f:A=A10
31g:A=A11 32g:A=A11 33g:A=A11
31h:A=A16 32h:A=A16 33h:A=A16
31i:A=A35 32i:A=A35 33i:A=A35
31j:A=A39 12j:A=A39 6j:A=A39
31k:A=A40 32k:A=A40 33k:A=A40
31l:A=A41 32l:A=A41 33l:A=A41
31m:A=A42 32m:A=A42 33m:A=A42

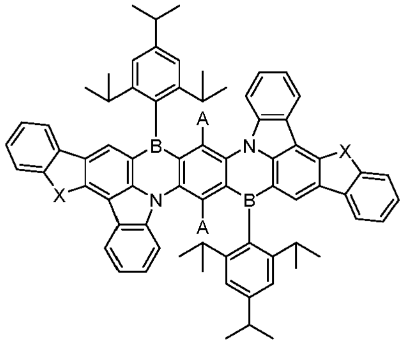
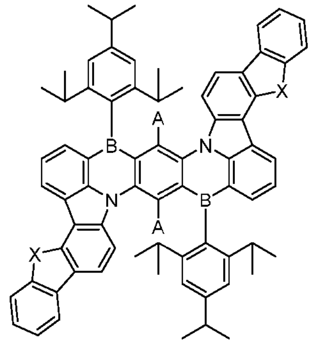
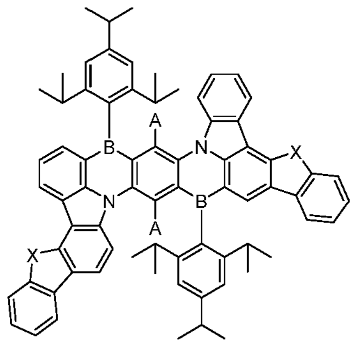

34a:A=A1
34b:A=A2
34c:A=A3
34d:A=A4
34e:A=A7
34f:A=A10
34g:A=A11
34h:A=A16
34i:A=A35
6j:A=A39
34k:A=A40
34l:A=A41
34m:A=A42

35a:A=A1
35b:A=A2
35c:A=A3
35d:A=A4
35e:A=A7
35f:A=A10
35g:A=A11
35h:A=A16
35i:A=A35
35j:A=A39
35k:A=A40
35l:A=A41
35m:A=A42

36a:A=A1
36b:A=A2
36c:A=A3
36d:A=A4
36e:A=A7
36f:A=A10
36g:A=A11
36h:A=A16
36i:A=A35
16j:A=A39
36k: A=A40
36l:A=A41
36m:A=A42

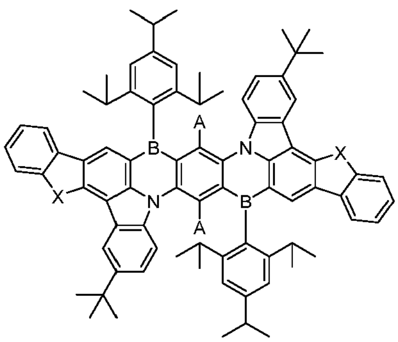
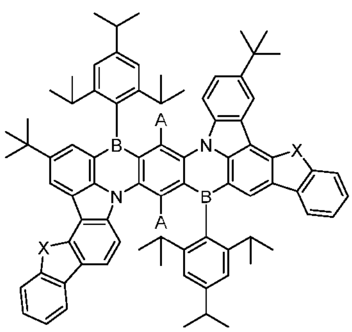
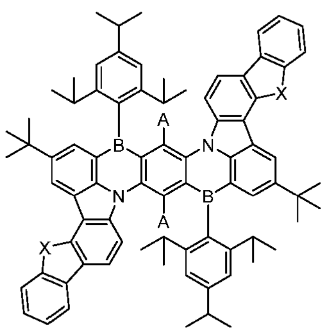

37a:A=A1
37b:A=A2
37c:A=A3
37d:A=A4
37e:A=A7
37f:A=A10
37g:A=A11
37h:A=A16
37i:A=A35
37j:A=A39
37k:A=A40
37l:A=A41
37m:A=A42

38a:A=A1
38b:A=A2
38c:A=A3
38d:A=A4
38e:A=A7
38f:A=A10
38g:A=A11
38h:A=A16
38i:A=A35
38j:A=A39
38k:A=A40
38l:A=A41
38m:A=A42

39a:A=A1
39b:A=A2
39c:A=A3
39d:A=A4
39e:A=A7
39f:A=A10
39g:A=A11
39h:A=A16
39i:A=A35
29j:A=A39
39k:A=A40
39l:A=A41
39m:A=A42

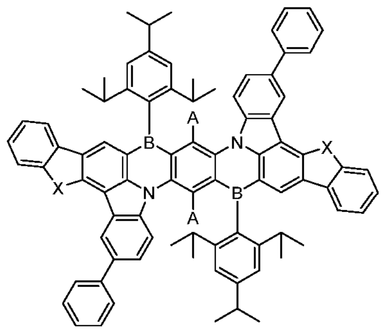
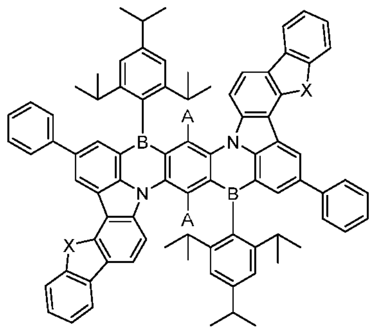
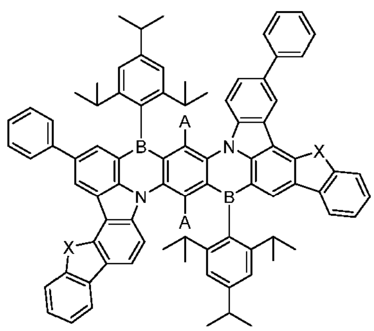

| | | |
|---|---|---|
| 40a:A=A1 | 41a:A=A1 | 42a:A=A1 |
| 40b:A=A2 | 41b:A=A2 | 42b:A=A2 |
| 40c:A=A3 | 41c:A=A3 | 42c:A=A3 |
| 40d:A=A4 | 41d:A=A4 | 42d:A=A4 |
| 40e:A=A7 | 41e:A=A7 | 42e:A=A7 |
| 40f:A=A10 | 41f:A=A10 | 42f:A=A10 |
| 40g:A=A11 | 41g:A=A11 | 42g:A=A11 |
| 40h:A=A16 | 41h:A=A16 | 42h:A=A16 |
| 40i:A=A35 | 41i:A=A35 | 42i:A=A35 |
| 40j:A=A39 | 41j:A=A39 | 12j:A=A39 |
| 40k:A=A40 | 41k:A=A40 | 42k:A=A40 |
| 40l:A=A41 | 41l:A=A41 | 42l:A=A41 |
| 40m:A=A42 | 41m:A=A42 | 42m:A=A42 |

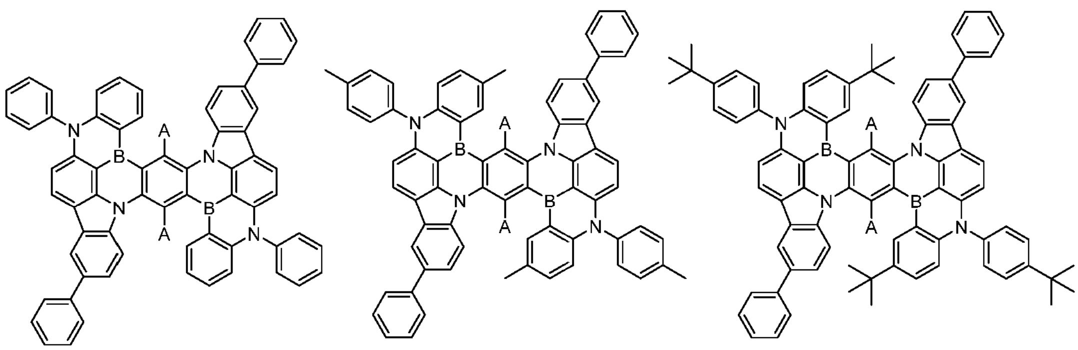

| | | |
|---|---|---|
| 43a:A=A1 | 44a:A=A1 | 45a:A=A1 |
| 43b:A=A2 | 44b:A=A2 | 45b:A=A2 |
| 43c:A=A3 | 44c:A=A3 | 45c:A=A3 |
| 43d:A=A4 | 44d:A=A4 | 45d:A=A4 |
| 43e:A=A7 | 44e:A=A7 | 45e:A=A7 |
| 43f:A=A10 | 44f:A=A10 | 45f:A=A10 |
| 43g:A=A11 | 44g:A=A11 | 45g:A=A11 |
| 43h:A=A16 | 44h:A=A16 | 45h:A=A16 |
| 43i:A=A35 | 44i:A=A35 | 45i:A=A35 |
| 43j:A=A39 | 6j:A=A39 | 45j:A=A39 |
| 43k:A=A40 | 44k:A=A40 | 45k:A=A40 |
| 43l:A=A41 | 44l:A=A41 | 45l:A=A41 |
| 43m:A=A42 | 44m:A=A42 | 45m:A=A42 |

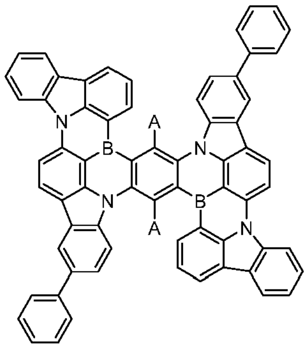

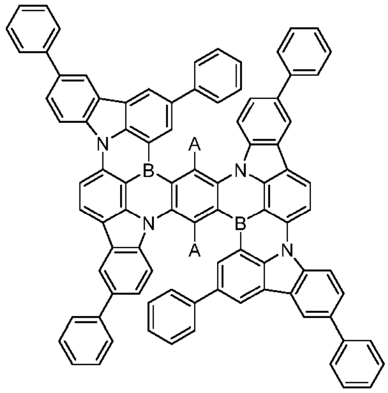

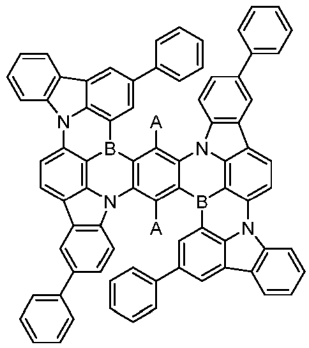

46a:A=A1
46b:A=A2
46c:A=A3
46d:A=A4
46e:A=A7
46f:A=A10
46g:A=A11
46h:A=A16
46i:A=A35
46j:A=A39
46k:A=A40
46l:A=A41
46m:A=A42

47a:A=A1
47b:A=A2
47c:A=A3
47d:A=A4
47e:A=A7
47f:A=A10
47g:A=A11
47h:A=A16
47i:A=A35
47j:A=A39
47k:A=A40
47l:A=A41
47m:A=A42

48a:A=A1
48b:A=A2
48c:A=A3
48d:A=A4
48e:A=A7
48f:A=A10
48g:A=A11
48h:A=A16
48i:A=A35
48j:A=A39
48k:A=A40
48l:A=A41
48m:A=A42

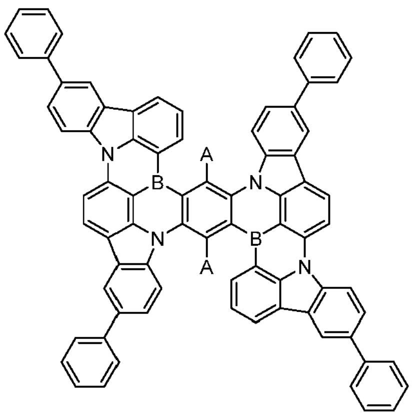

49a:A=A1
49b:A=A2
49c:A=A3
49d:A=A4
49e:A=A7
49f:A=A10
49g:A=A11
49h:A=A16
49i:A=A35
29j:A=A39
49k:A=A40
49l:A=A41
49m:A=A42

In one aspect of the invention, as for the compound represented by the formula (16), a compound having a rotationally symmetric structure is selected. In one aspect of the invention, as the compound represented by the formula (16), a compound having an axially symmetric structure is selected. In one aspect of the invention, as the compound represented by the formula (16), a compound having an asymmetric structure is selected.

Specific examples of a compound having an asymmetric skeleton will be given below. The compounds having asymmetric skeletons or the compounds having asymmetric structures, which may be used in the invention, are not construed as limiting to the following specific examples. **In** relation to specific examples including X, it is assumed that a compound in which all X's in the molecule are oxygen atoms, and a compound in which all X's in the molecule are sulfur atoms are disclosed, respectively. A compound in which some of X's in the molecule are oxygen atoms, and the rest are sulfur atoms may also be adopted.

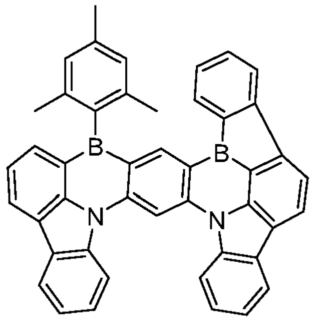
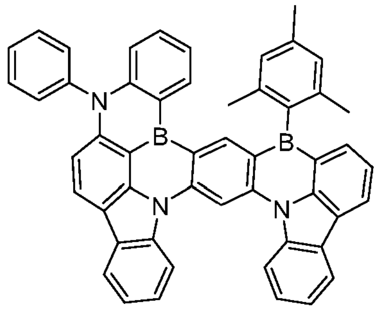
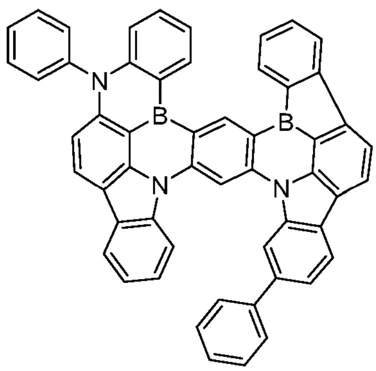

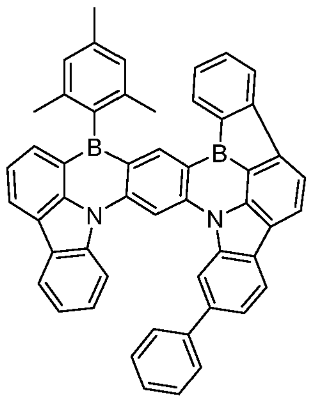
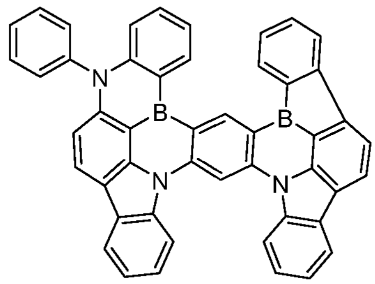
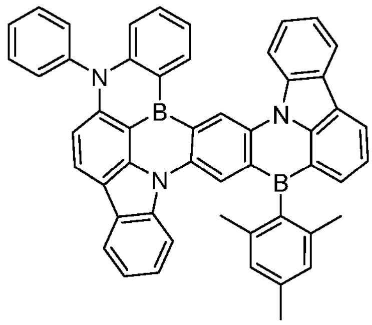

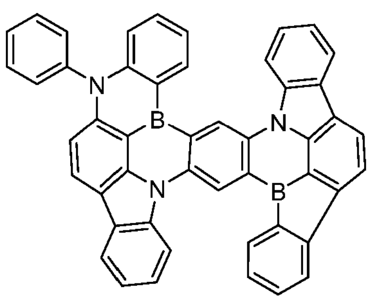
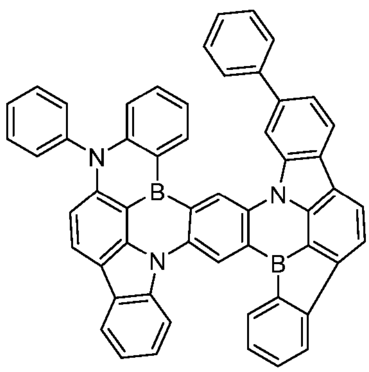
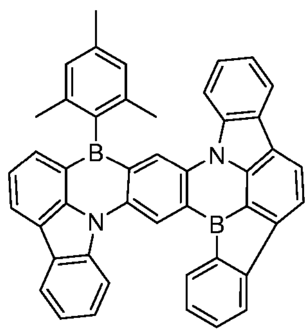

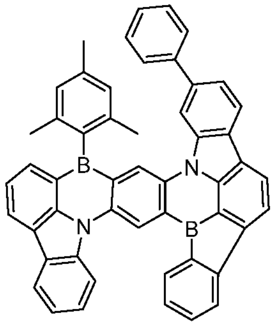

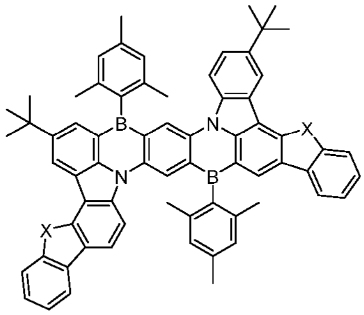

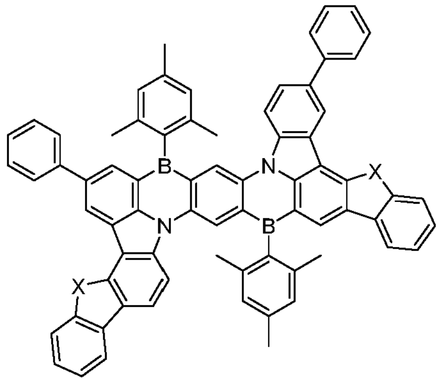

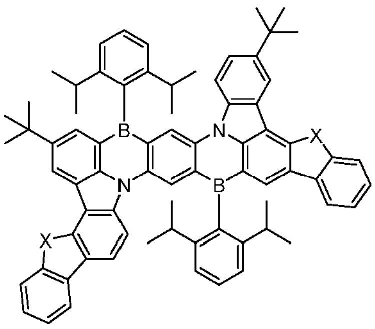

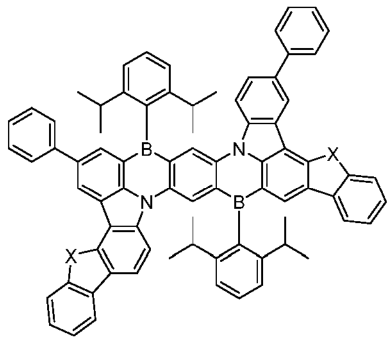

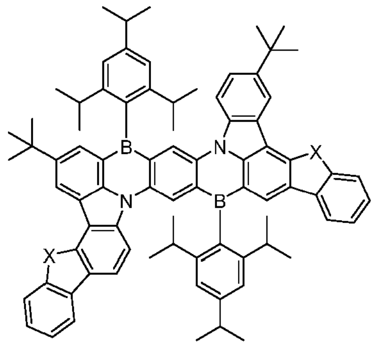

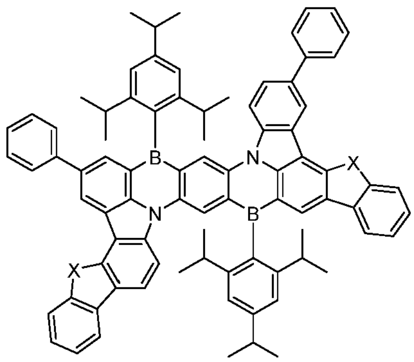

In a particularly preferable aspect of the invention, as the third organic compound, a compound in which at least one of a tert-butyl group and a phenyl group is introduced into the following skeleton (26a) or skeleton (26b) is selected.

Skeleton (26a)

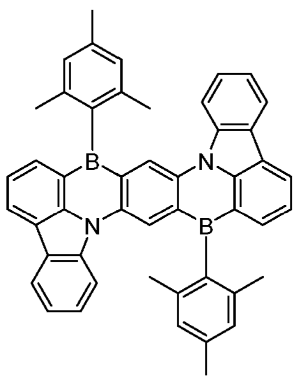

Skeleton (26b)

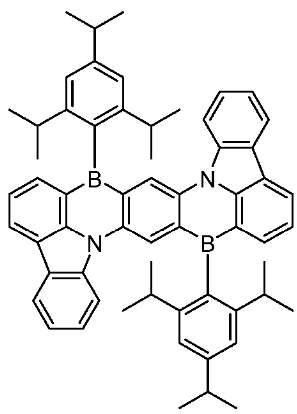

Hereinafter, specific examples of the compound in which at least one of the tert-butyl group and the phenyl group

is introduced into the skeleton (26a) or the skeleton (26b) will be given. The compound of the formula (16) that can be used in the invention is not construed as limiting to the following specific examples.

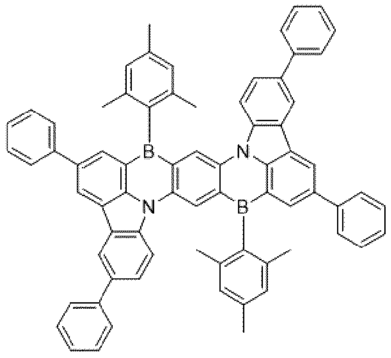

F101

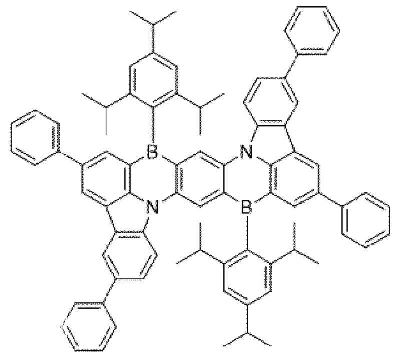

F102

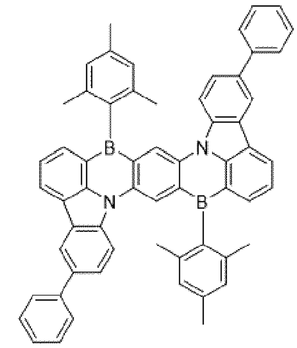

F103

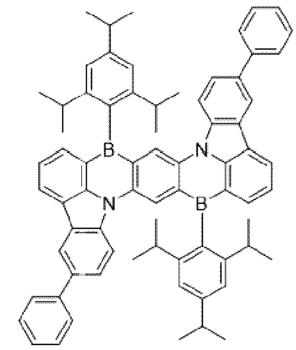

F104

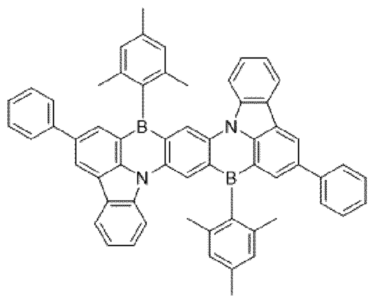

F105

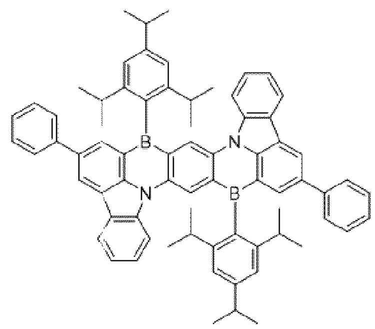

F106

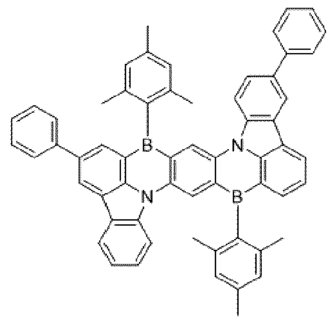

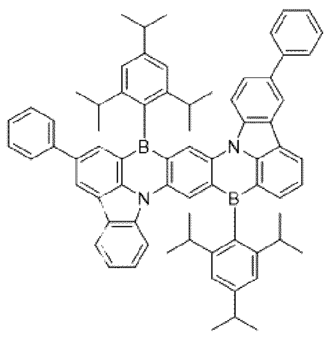

F107

F108

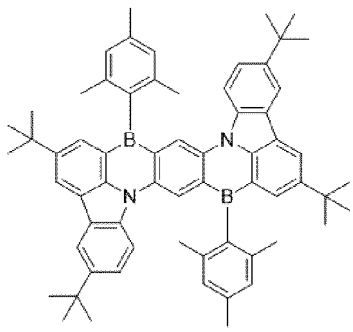

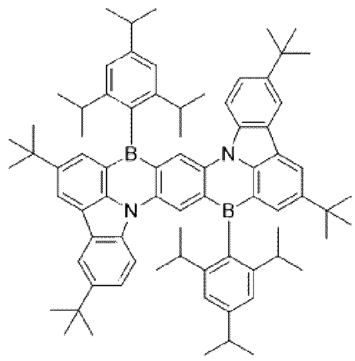

F109

F110

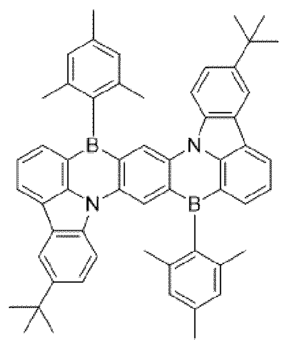

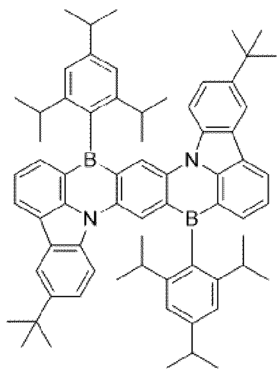

F111

F112

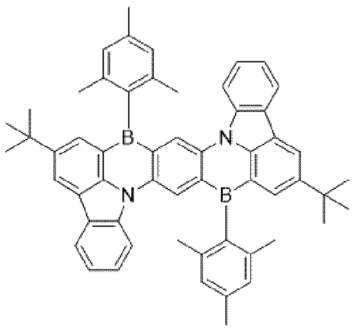

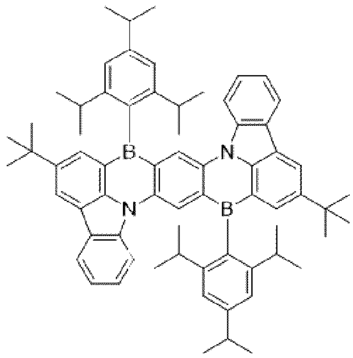

**F113** **F114**

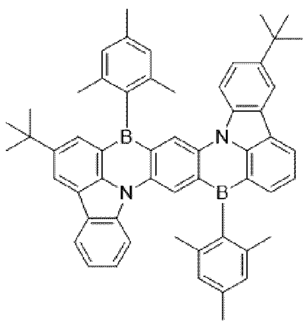

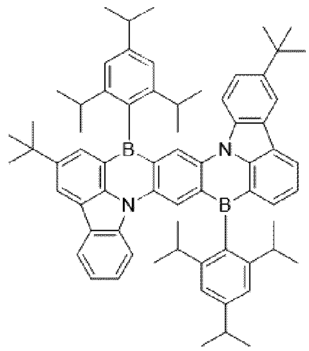

**F115** **F116**

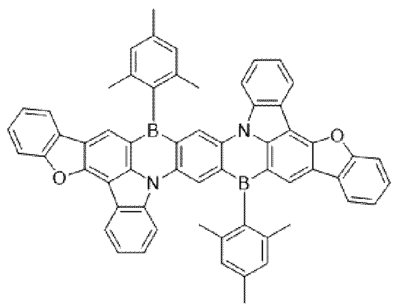

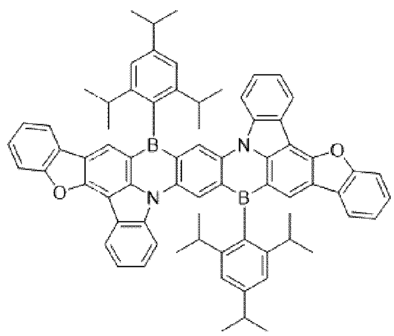

**F117** **F118**

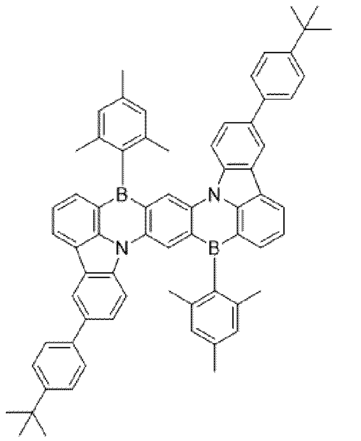

**F119**

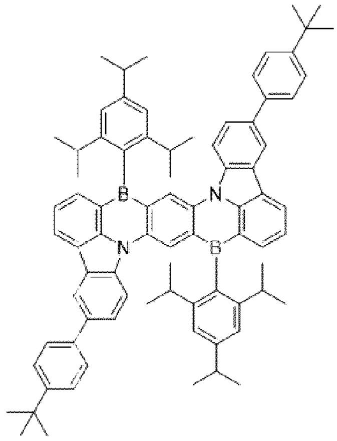

**F120**

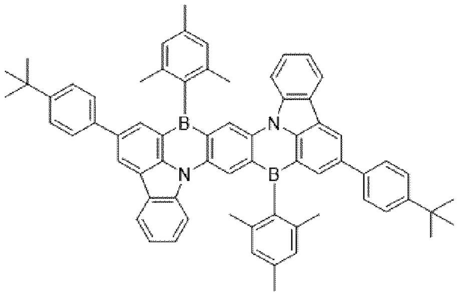

**F121**

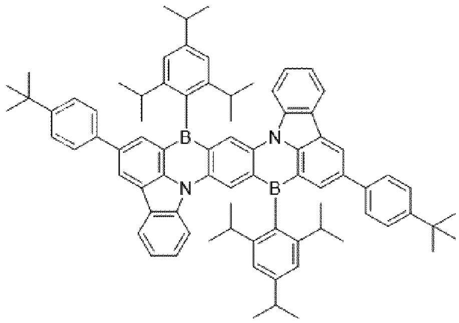

**F122**

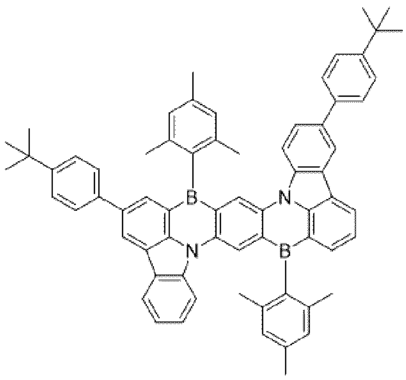

**F123**

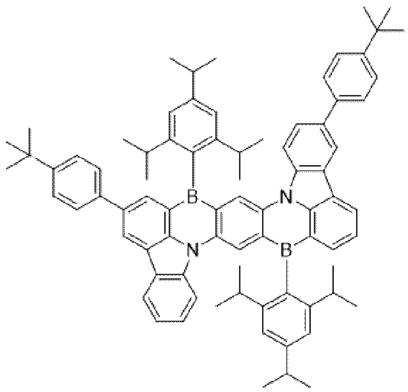

**F124**

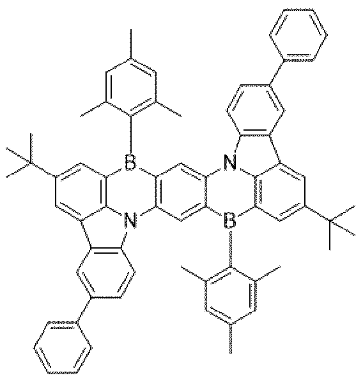

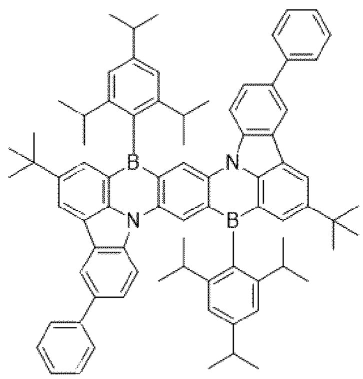

F125 F126

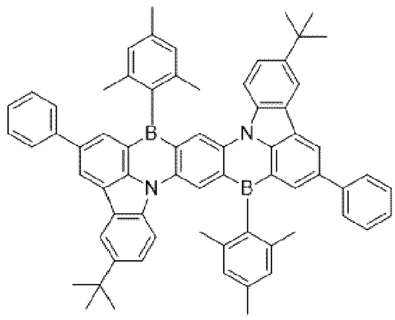

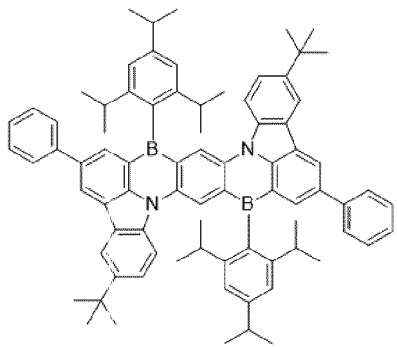

F127 F128

The molecular weight of the compound represented by the formula (16) is preferably 1500 or less, more preferably 1200 or less, further preferably 1000 or less, still further preferably 900 or less, for example, when there is an intention to form and use a film of an organic layer containing the compound represented by the formula (16) through a vapor deposition method. The lower limit value of the molecular weight is the molecular weight of the smallest compound in the compound group represented by the formula (16). It is preferably 624 or more.

The compound represented by the formula (16) may be formed into a film through a coating method regardless of the molecular weight. When the coating method is used, it is possible to form a film even if the compound has a relatively large molecular weight. The compound represented by the formula (16) has an advantage of ease of dissolution in an organic solvent. Thus, for the compound represented by the formula (16), it is easy to apply a coating method, and moreover it is also easy to increase the purity through purification.

Through an application of the invention, the use of a compound including a plurality of structures represented by the formula (16) in the molecule, as a light-emitting material, may be taken into consideration.

For example, when a polymerizable group exists in advance in the structure represented by the formula (16), the use of a polymer obtained by polymerizing the polymerizable group as the light-emitting material may be taken into consideration. Specifically, when a monomer including a polymerizable functional group is prepared in any of the structures represented by the formula (16), and this is polymerized alone, or is copolymerized with another monomer so as to obtain a polymer having repeating units, the use of the polymer as the light-emitting material may be taken into consideration. Alternatively, when a dimer or a trimer is obtained by coupling compounds represented by the formula (16) with each other, the use of these as the light-emitting material may also be taken into consideration.

As examples of the polymer having the repeating unit including the structure represented by the formula (16), polymers including the structures represented by the following formulas may be mentioned.

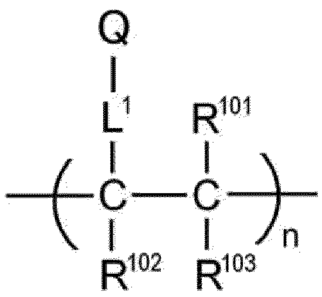

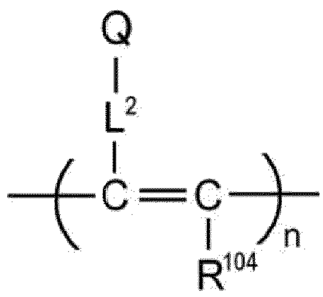

**In** the above formulas, Q represents a group including the structure represented by the formula (16), and $L^1$ and $L^2$ represent linking groups. The number of carbon atoms in the linking group is preferably 0 to 20, more preferably 1 to 15, further preferably 2 to 10. The linking group preferably has a structure represented by $-X^{11}-L^{11}-$. Here, $X^{11}$ represents an oxygen atom or a sulfur atom, and an oxygen atom is preferable. $L^{11}$ represents a linking group, and is preferably a substituted or unsubstituted alkylene group, or a substituted or unsubstituted arylene group, more preferably a substituted or unsubstituted alkylene group having 1 to 10 carbon atoms, or a substituted or unsubstituted phenylene group.

Each of $R^{101}$, $R^{102}$, $R^{103}$ and $R^{104}$ independently represents a substituent. It is preferably a substituted or unsubstituted alkyl group having 1 to 6 carbon atoms, a substituted or unsubstituted alkoxy group having 1 to 6 carbon atoms, or a halogen atom, more preferably an unsubstituted alkyl group having 1 to 3 carbon atoms, an unsubstituted alkoxy group having 1 to 3 carbon atoms, a fluorine atom, or a chlorine atom, further preferably an unsubstituted alkyl group having 1 to 3 carbon atoms, or an unsubstituted alkoxy group having 1 to 3 carbon atoms.

The linking group represented by $L^1$ and $L^2$ may be bonded to any position of the structure which is represented by the formula (16) and constitutes Q. Two or more linking groups may be linked to one Q to form a crosslinked structure or a network structure.

As specific structural examples of the repeating unit, structures represented by the following formulas may be mentioned.

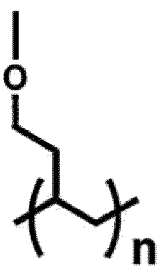

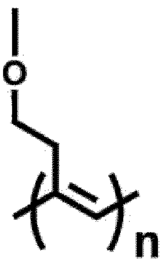

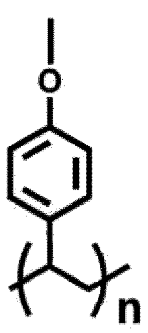

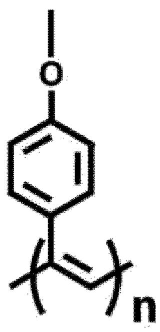

In synthesizing a polymer having repeating units including these formulas, a hydroxy group is introduced at any position of the structure represented by the formula (16), and the following compound is reacted using the hydroxy group as a linker so that a polymerizable group may be introduced, and the polymerizable group may be polymerized.

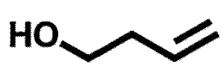

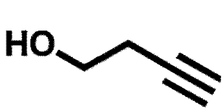

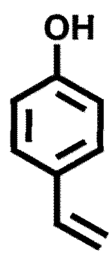

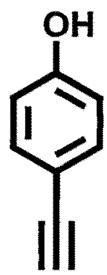

The polymer including the structure represented by the formula (16) in the molecule may be a polymer composed of only repeating units having the structure represented by the formula (16), or may be a polymer including repeating units having another structure. Further, the repeating units having the structure represented by the formula (16), which are included in the polymer, may be of a single type, or two or more types. As a repeating unit not having the structure represented by the formula (16), those derived from monomers used in a general copolymerization may be mentioned. For

example, a repeating unit derived from a monomer having an ethylenically unsaturated bond such as ethylene, or styrene may be mentioned.

It is preferable that the compound represented by the formula (16) does not include a metal atom. The metal atom mentioned herein does not include a boron atom. For example, as the compound represented by the formula (16), a compound including an atom selected from the group consisting of a carbon atom, a hydrogen atom, a deuterium atom, a nitrogen atom, an oxygen atom, a sulfur atom, and a boron atom may be selected. For example, as the compound represented by the formula (16), a compound including an atom selected from the group consisting of a carbon atom, a hydrogen atom, a deuterium atom, a nitrogen atom, an oxygen atom, and a boron atom may be selected. For example, as the compound represented by the formula (16), a compound including an atom selected from the group consisting of a carbon atom, a hydrogen atom, a deuterium atom, a nitrogen atom, a sulfur atom, and a boron atom may be selected. For example, as the compound represented by the formula (16), a compound including an atom selected from the group consisting of a carbon atom, a hydrogen atom, a deuterium atom, a nitrogen atom, and a boron atom may be selected. For example, as the compound represented by the formula (16), a compound including an atom selected from the group consisting of a carbon atom, a hydrogen atom, a nitrogen atom, an oxygen atom, a sulfur atom, and a boron atom may be selected.

**In** the present specification, the "alkyl group" may take any of linear, branched, and cyclic shapes. Further, two or more types of the linear portion, the cyclic portion, and the branched portion may be mixed. The number of carbon atoms of the alkyl group may be, for example, one or more, two or more, or four or more. Further, the number of carbon atoms may be 30 or less, 20 or less, 10 or less, 6 or less, or 4 or less. Specific examples of the alkyl group include a methyl group, an ethyl group, a n-propyl group, an isopropyl group, a n-butyl group, an isobutyl group, a tert-butyl group, a n-pentyl group, an isopentyl group, a n-hexyl group, an isohexyl group, a 2-ethylhexyl group, a n-heptyl group, an isoheptyl group, a n-octyl group, an isooctyl group, a n-nonyl group, an isononyl group, a n-decanyl group, an isodecanyl group, a cyclopentyl group, a cyclohexyl group, and a cycloheptyl group. The alkyl group as a substituent may be further substituted with an aryl group.

The "alkenyl group" may take any of linear, branched, and cyclic shapes. Further, two or more types of the linear portion, the cyclic portion, and the branched portion may be mixed. The number of carbon atoms of the alkenyl group may be, for example, two or more, or four or more. Further, the number of carbon atoms may be 30 or less, 20 or less, 10 or less, 6 or less, or 4 or less. Specific examples of the alkenyl group include an ethenyl group, a n-propenyl group, an isopropenyl group, a n-butenyl group, an isobutenyl group, a n-pentenyl group, an isopentenyl group, a n-hexenyl group, an isohexenyl group, and a 2-ethyl hexenyl group. The alkenyl group as a substituent may be further substituted with a substituent.

The "aryl group" and the "heteroaryl group" may be monocycles, or may be condensed rings in which two or more rings are condensed. **In** the case of the condensed ring, the number of rings for condensation is preferably two to six, and, for example, may be selected from two to four. Specific examples of the ring include a benzene ring, a pyridine ring, a pyrimidine ring, a triazine ring, a naphthalene ring, an anthracene ring, a phenanthrene ring, a triphenylene ring, a quinoline ring, a pyrazine ring, a quinoxaline ring, and a naphthiridine ring, and these may be condensed to form a ring. Specific examples of the aryl group or the heteroaryl group include a phenyl group, a 1-naphthyl group, a 2-naphthyl group, a 1-anthrasenyl group, a 2-anthrasenyl group, a 9-anthrasenyl group, a 2-pyridyl group, a 3-pyridyl group, and a 4-pyridyl group. The number of ring skeleton forming atoms of the aryl group is preferably 6 to 40, more preferably 6 to 20, and may be selected in a range of 6 to 14, or selected in a range of 6 to 10. The number of ring skeleton forming atoms of the heteroaryl group is preferably 4 to 40, more preferably 5 to 20, and may be selected in a range of 5 to 14, or selected in a range of 5 to 10. The "arylene group" and the "heteroaryl group" may be those obtained by changing the valence in the descriptions for the aryl group and the heteroaryl group, from 1 to 2.

The "substituent group A" in the present specification means one group selected from the group consisting of a hydroxy group, a halogen atom (e.g., a fluorine atom, a chlorine atom, a bromine atom, an iodine atom), an alkyl group (e.g., 1 to 40 carbon atoms), an alkoxy group (e.g., 1 to 40 carbon atoms), an alkylthio group (e.g., 1 to 40 carbon atoms), an aryl group (e.g., 6 to 30 carbon atoms), an aryl oxy group (e.g., 6 to 30 carbon atoms), an arylthio group (e.g., 6 to 30 carbon atoms), a heteroaryl group (e.g., 5 to 30 ring skeleton forming atoms), a heteroaryl oxy group (e.g., 5 to 30 ring skeleton forming atoms), a heteroarylthio group (e.g., 5 to 30 ring skeleton forming atoms), an acyl group (e.g., 1 to 40 carbon atoms), an alkenyl group (e.g., 1 to 40 carbon atoms), an alkynyl group (e.g., 1 to 40 carbon atoms), an alkoxycarbonyl group (e.g., 1 to 40 carbon atoms), an aryl oxycarbonyl group (e.g., 1 to 40 carbon atoms), a heteroaryl oxycarbonyl group (e.g., 1 to 40 carbon atoms), a silyl group (e.g., a trialkyl silyl group having 1 to 40 carbon atoms) and a nitro group, or a group formed by combining two or more thereof.

The "substituent group B" in the present specification means one group selected from the group consisting of an alkyl group (e.g., 1 to 40 carbon atoms), an alkoxy group (e.g., 1 to 40 carbon atoms), an aryl group (e.g., 6 to 30 carbon atoms), an aryl oxy group (e.g., 6 to 30 carbon atoms), a heteroaryl group (e.g., 5 to 30 ring skeleton forming atoms), a heteroaryl oxy group (e.g., 5 to 30 ring skeleton forming atoms), and a diaryl aminoamino group (e.g., 0 to 20 carbon atoms), or a group formed by combining two or more thereof.

The "substituent group C" in the present specification means one group selected from the group consisting of an alkyl group (e.g., 1 to 20 carbon atoms), an aryl group (e.g., 6 to 22 carbon atoms), a heteroaryl group (e.g., 5 to 20 ring

skeleton forming atoms), and a diaryl amino group (e.g., 12 to 20 carbon atoms), or a group formed by combining two or more thereof.

The "substituent group D" in the present specification means one group selected from the group consisting of an alkyl group (e.g., 1 to 20 carbon atoms), an aryl group (e.g., 6 to 22 carbon atoms) and a heteroaryl group (e.g., 5 to 20 ring skeleton forming atoms), or a group formed by combining two or more thereof.

The "substituent group E" in the present specification means one group selected from the group consisting of an alkyl group (e.g., 1 to 20 carbon atoms) and an aryl group (e.g., 6 to 22 carbon atoms), or a group formed by combining two or more thereof.

In the present specification, in the case of the description of "substituent" or "substituted or unsubstituted", the substituent may be selected from, for example, the substituent group A, may be selected from the substituent group B, may be selected from the substituent group C, may be selected from the substituent group D, or may be selected from the substituent group E.

In an embodiment not forming part of the invention, as the third organic compound, the following compound can also be used.

In a preferred aspect of the invention, a compound represented by the following formula (27) is used as the third organic compound.

Formula (27)

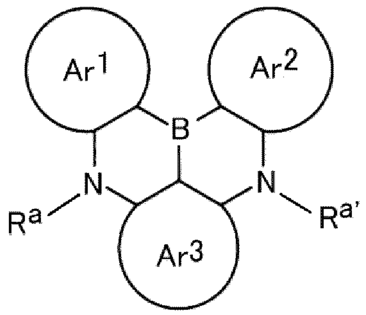

In the formula (27), each of $Ar^1$ to $Ar^3$ is independently an aryl ring or a heteroaryl ring, and in such rings, at least one hydrogen atom may be substituted, or a ring may be condensed. When the hydrogen atom is substituted, it is preferable that the hydrogen atom is substituted with one group selected from the group consisting of a deuterium atom, an aryl group, a heteroaryl group, and an alkyl group, or a group formed by combining two or more thereof. Further, when the ring is condensed, it is preferable that a benzene ring or a heteroaromatic ring (for example, a furan ring, a thiophene ring, a pyrrole ring, or the like) is condensed. Each of $R^a$ and $R^{a'}$ independently represents a substituent, and is preferably one group selected from the group consisting of a deuterium atom, an aryl group, a heteroaryl group, and an alkyl group, or a group formed by combining two or more thereof. $R^a$ and $Ar^1$, $Ar^1$ and $Ar^2$, $Ar^2$ and $R^{a'}$, $R^{a'}$ and $Ar^3$, and $Ar^3$ and $R^a$ may be bonded to each other to form ring structures.

It is preferable that the compound represented by the formula (27) has at least one carbazole structure. For example, one benzene ring constituting the carbazole structure may be a ring represented by $Ar^1$, one benzene ring constituting the carbazole structure may be a ring represented by $Ar^2$, or one benzene ring constituting the carbazole structure may be a ring represented by $Ar^3$. Further, a carbazolyl group may be bonded to one or more of $Ar^1$ to $Ar^3$. For example, a substituted or unsubstituted carbazole-9-yl group may be bonded to the ring represented by $Ar^3$.

A condensed aromatic ring structure such as anthracene, pyrene, and perylene may be bonded to $Ar^1$ to $Ar^3$. Further, the rings represented by $Ar^1$ to $Ar^3$ may be one ring constituting the condensed aromatic ring structure. Further, at least one of $R^a$ and $R^{a'}$ may be a group having a condensed aromatic ring structure.

There may be a plurality of skeletons represented by the formula (27) in the compound. For example, the skeleton may have a structure in which the skeletons represented by the formula (27) are bonded to each other via a single bond or a linking group. Further, a structure having a multiple resonance effect in which benzene rings are linked by a boron atom, a nitrogen atom, an oxygen atom, and a sulfur atom may be further added to the skeleton represented by the formula (27).

In an embodiment not forming part of the invention, as the third organic compound, a compound having a BODIPY (4,4-difluoro-4-bora-3a,4a-diaza-s-indacene) structure is used. For example, a compound represented by the following formula (28) is used.

Formula (28)

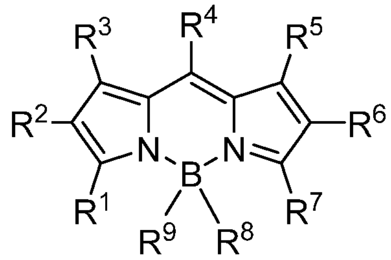

In the formula (28), each of $R^1$ to $R^7$ is independently a hydrogen atom, a deuterium atom, or a substituent. It is preferable that at least one of $R^1$ to $R^7$ is a group represented by the following formula (29).

Formula (29)

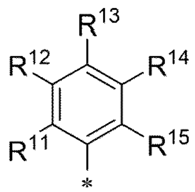

In the formula (29), each of $R^{11}$ to $R^{15}$ independently represents a hydrogen atom, a deuterium atom, or a substituent, and * represents a bond position.

The group represented by the formula (29) may be one, may be two, or may be three of $R^1$ to $R^7$ in the formula (28). Further, the group may be at least four of $R^1$ to $R^7$ in the formula (28), and for example, can be four or five of $R^1$ to $R^7$ in the formula (28). In a preferred aspect of the invention, one of $R^1$ to $R^7$ is the group represented by the formula (29). In a preferred aspect of the invention, at least $R^1$, $R^3$, $R^5$, and $R^7$ are the group represented by the formula (29). In a preferred aspect of the invention, only $R^1$, $R^3$, $R^4$, $R^5$, and $R^7$ are the group represented by the formula (29). In a preferred aspect of the invention, $R^1$, $R^3$, $R^4$, $R^5$, and $R^7$ are the group represented by the formula (29), and $R^2$ and $R^4$ are a hydrogen atom, a deuterium atom, an unsubstituted alkyl group (e.g., 1 to 10 carbon atoms), or an unsubstituted aryl group (e.g., 6 to 14 carbon atoms). In one aspect of the invention, all of $R^1$ to $R^7$ are the group represented by the formula (29).

In a preferred aspect, $R^1$ and $R^7$ are the same. In a preferred aspect of the invention, $R^3$ and $R^5$ are the same. In a preferred aspect of the invention, $R^2$ and $R^6$ are the same. **In** a preferred aspect of the invention, $R^1$ and $R^7$ are the same, $R^3$ and $R^5$ are the same, and $R^1$ and $R^3$ are different from each other. In a preferred aspect of the invention, $R^1$, $R^3$, $R^5$, and $R^7$ are the same. In a preferred aspect of the invention, $R^1$, $R^4$, and $R^7$ are the same, which are different from $R^3$ or $R^5$. **In** a preferred aspect of the invention, $R^3$, $R^4$, and $R^5$ are the same, which are different from $R^1$ or $R^7$. In a preferred aspect of the invention, all of $R^1$, $R^3$, $R^5$, and $R^7$ are different from $R^4$.

As the substituent that may be possessed by $R^{11}$ to $R^{15}$ in the formula (29), for example, the group of the substituent group A can be selected. It is preferable that the substituent that may be possessed by $R^{11}$ to $R^{15}$ is one group selected from the group consisting of a substituted or unsubstituted alkyl group (e.g., 1 to 40 carbon atoms), a substituted or unsubstituted alkoxy group (e.g., 1 to 40 carbon atoms), a substituted or unsubstituted aryl group (e.g., 6 to 30 carbon atoms), a substituted or unsubstituted aryl oxy group (e.g., 6 to 30 carbon atoms), and a substituted or unsubstituted amino group (e.g., 0 to 20 carbon atoms), or a group formed by combining two or more thereof (hereinafter, such a group will be referred to as a "group of a substituent group C"). In the substituent group C, it is preferable to select an unsubstituted alkyl group having 1 to 20 carbon atoms, an unsubstituted alkoxy group having 1 to 20 carbon atoms, an unsubstituted aryl group having 6 to 14 carbon atoms, an aryl oxy group having 6 to 14 carbon atoms, or an unsubstituted diaryl amino group having 5 to 20 ring skeleton forming atoms (hereinafter, such a group will be referred to as a "group of a substituent group D"). As the substituted amino group mentioned herein, a substituted diamino group is preferable, and each of two substituents with respect to an amino group is independently preferably a substituted or unsubstituted aryl group, a substituted or unsubstituted heteroaryl group, or a substituted or unsubstituted alkyl group, and particularly preferably a substituted or unsubstituted aryl group (a diaryl amino group). As the substituent that may be possessed by two aryl groups of the diaryl amino group, the group of the substituent group A can be selected, the group of the substituent group B can be selected, or the group of the substituent group C can be selected. Two aryl groups of the diaryl amino group may be bonded to each other via a single bond or a linking group, and for the linking group mentioned herein, the description on the linking group in $R^{33}$ and $R^{34}$ can be referred to. As a specific example of the diaryl amino group, for example, a substituted or unsubstituted carbazole-9-yl group can be adopted. As the substituted or unsubstituted carbazole-9-yl group, for example,

a group in which $L^{11}$ in the formula (6) is a single bond can be given.

In a preferred aspect, only $R^{13}$ in the formula (29) is a substituent, and $R^{11}$, $R^{12}$, $R^{14}$, and $R^{15}$ are hydrogen atoms. In a preferred aspect of the invention, only $R^{11}$ in the formula (29) is a substituent, and $R^{12}$, $R^{13}$, $R^{14}$, and $R^{15}$ are hydrogen atoms. In a preferred aspect of the invention, only $R^{11}$ and $R^{13}$ in the formula (29) are substituents, and $R^{12}$, $R^{14}$, and $R^{15}$ are hydrogen atoms.

$R^1$ to $R^7$ of the formula (28) may include a group in which all of $R^{11}$ to $R^{15}$ in the formula (29) are hydrogen atoms (that is, a phenyl group). For example, $R^2$, $R^4$, and $R^6$ may be phenyl groups.

In the formula (28), it is preferable that each of $R^8$ and $R^9$ is independently one group selected from the group consisting of a hydrogen atom, a deuterium atom, a halogen atom, an alkyl group (e.g., 1 to 40 carbon atoms), an alkoxy group (e.g., 1 to 40 carbon atoms), an aryl oxy group (e.g., 6 to 30 carbon atoms) and a cyano group, or a group formed by combining two or more thereof. In a preferable embodiment of the invention, $R^8$ and $R^9$ are the same. **In** a preferable embodiment of the invention, $R^8$ and $R^9$ are halogen atoms, and are particularly preferably fluorine atoms.

In one aspect , it is preferable that the total number of substituted or unsubstituted alkoxy groups, substituted or unsubstituted aryl oxy groups, and substituted or unsubstituted amino groups present in $R^1$ to $R^9$ of the formula (28) is 3 or more, and for example, a compound in which the total number is 3 can be adopted, or a compound in which the total number is 4 can be adopted. It is more preferable that the total number of substituted or unsubstituted alkoxy groups, substituted or unsubstituted aryl oxy groups, and substituted or unsubstituted amino groups present in $R^1$ to $R^7$ of the formula (28) is 3 or more, and for example, a compound in which the total number is 3 can be adopted, or a compound in which the total number is 4 can be adopted. Here, an alkoxy group, an aryl oxy group, and an amino group may not be present in $R^8$ and $R^9$. It is further preferable that the total number of substituted or unsubstituted alkoxy groups, substituted or unsubstituted aryl oxy groups, and substituted or unsubstituted amino groups present in $R^1$, $R^3$, $R^4$, $R^5$, and $R^7$ of the formula (28) is 3 or more, and for example, a compound in which the total number is 3 can be adopted, or a compound in which the total number is 4 can be adopted. Here, an alkoxy group, an aryl oxy group, and an amino group may not be present in $R^2$, $R^6$, $R^8$, and $R^9$. In a preferred aspect of the invention, there are three or more substituted or unsubstituted alkoxy groups. In a preferred aspect of the invention, there are four or more substituted or unsubstituted alkoxy groups. In a preferred aspect of the invention, there are one or more substituted or unsubstituted alkoxy groups and two or more substituted or unsubstituted aryl oxy groups. In a preferred aspect of the invention, there are two or more substituted or unsubstituted alkoxy groups and one or more substituted or unsubstituted amino groups. In a preferred aspect of the invention, a substituted or unsubstituted alkoxy group or a substituted or unsubstituted aryl oxy is present in each of $R^1$, $R^4$, and $R^7$. In a preferred aspect of the invention, a substituted or unsubstituted alkoxy group is present in each of $R^1$, $R^4$, and $R^7$.

In one aspect, the total number of substituents having a Hammett $\sigma p$ value of less than -0.2 present in $R^1$ to $R^9$ of the formula (28) is 3 or more. Examples of the substituent having a Hammett $\sigma p$ value of less than -0.2 include a methoxy group (-0.27), an ethoxy group (-0.24), a n-propoxy group (-0.25), an isopropoxy group (-0.45), and a n-butoxy group (-0.32). On the other hand, a fluorine atom (0.06), a methyl group (-0.17), an ethyl group (-0.15), a tert-butyl group (-0.20), a n-hexyl group (-0.15), a cyclohexyl group (-0.15), and the like are not the substituent having a Hammett $\sigma p$ value of less than -0.2.

In one aspect, a compound in which the number of substituents having a Hammett $\sigma p$ value of less than -0.2 present in $R^1$ to $R^9$ of the formula (28) is 3 can be adopted, or a compound in which the number is 4 can be adopted. It is more preferable that the number of substituents having a Hammett $\sigma p$ value of less than -0.2 present in $R^1$ to $R^7$ of the formula (28) is 3 or more, and for example, a compound in which the number is 3 can be adopted, or a compound in which the number is 4 can be adopted. Here, the substituent having a Hammett $\sigma p$ value of less than -0.2 may not be present in $R^8$ and $R^9$. It is further preferable that the number of substituents having a Hammett $\sigma p$ value of less than -0.2 present in $R^1$, $R^3$, $R^4$, $R^5$, and $R^7$ of the formula (28) is 3 or more, and for example, a compound in which the number is 3 can be adopted, or a compound in which the number is 4 can be adopted. Here, the substituent having a Hammett $\sigma p$ value of less than -0.2 may not be present in $R^2$, $R^6$, $R^8$, and $R^9$. In a preferred aspect of the invention, the substituent having a Hammett $\sigma p$ value of less than -0.2 is present in each of $R^1$, $R^4$, and $R^7$.

Hereinafter, reference compounds which can also be used as the third organic compound will be given. **In** the structural formulae of the exemplary compounds, t-Bu represents a tertiary butyl group.

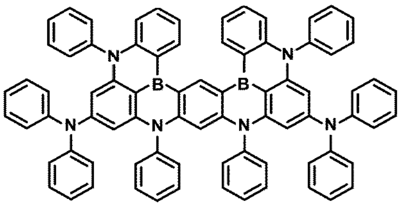

F1

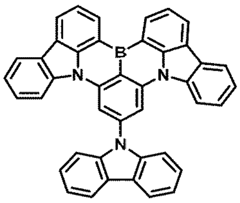

F2

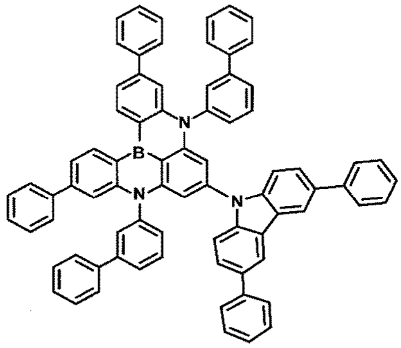

F3

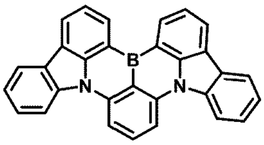

F4

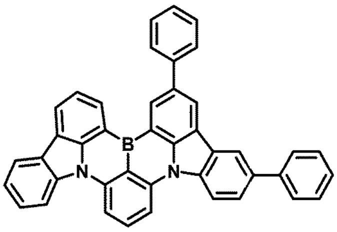

F5

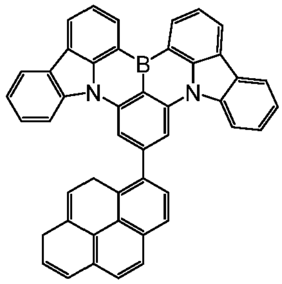

F6

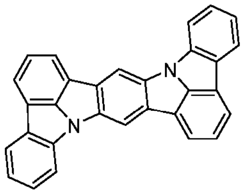

F7

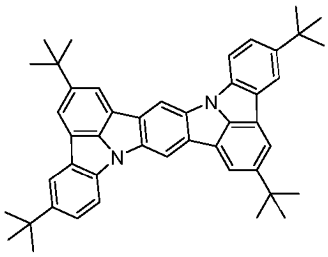

F8

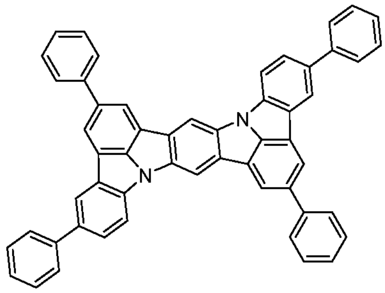

F9

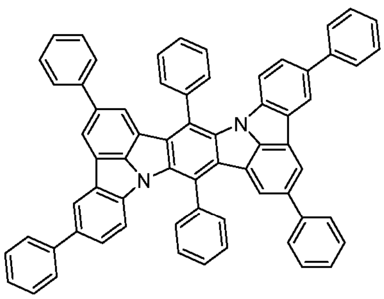

F10

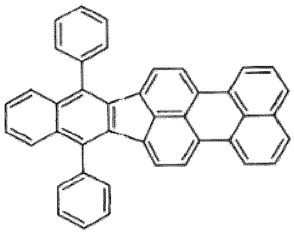

F11

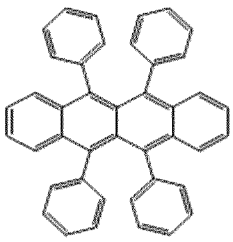

F12

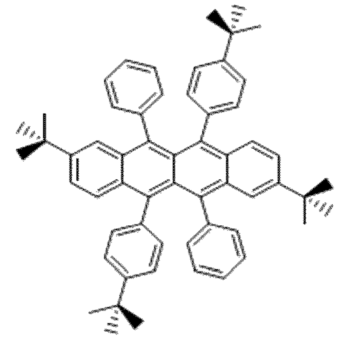

**F13**

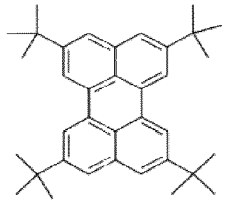

**F14**

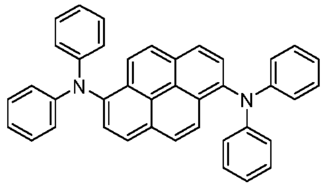

**F15**

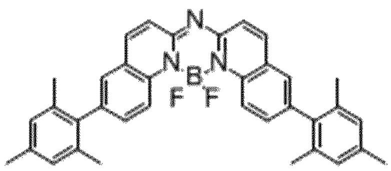

**F16**

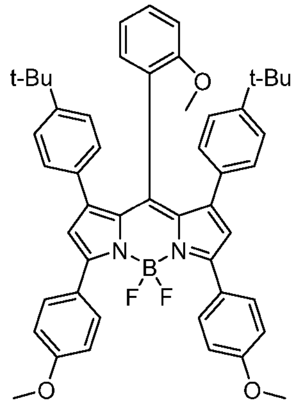

**F17**

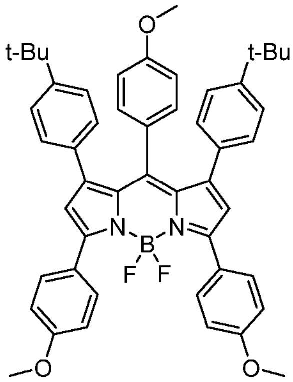

**F18**

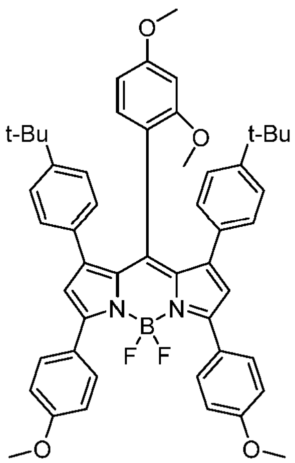

**F19**

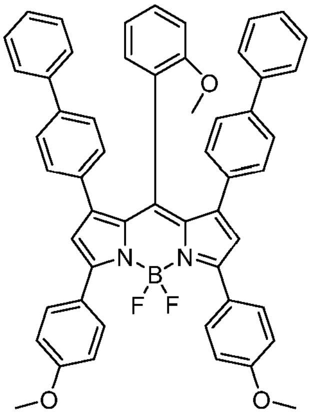

**F20**

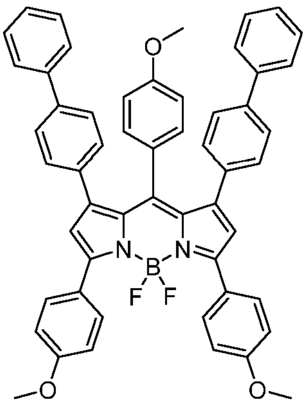

**F21**

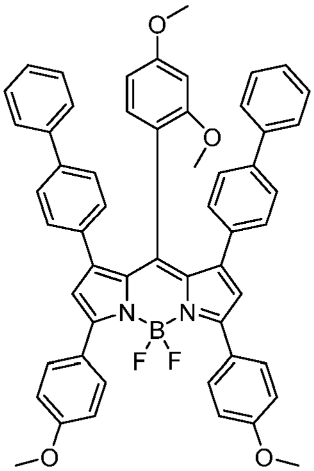

**F22**

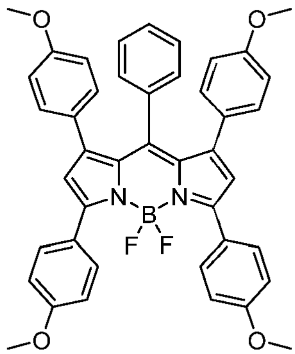

**F23**

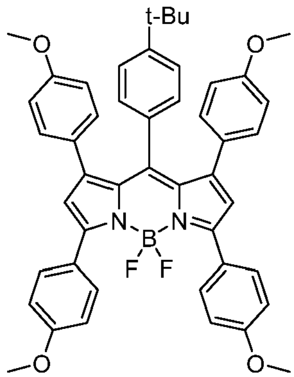

**F24**

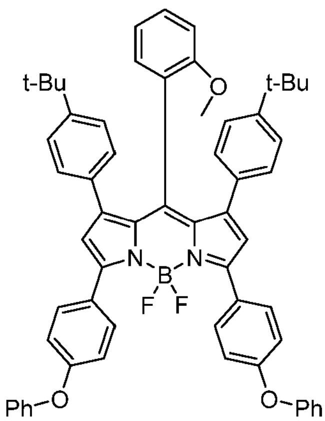

**F25**

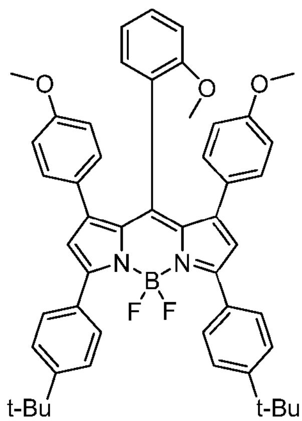

**F26**

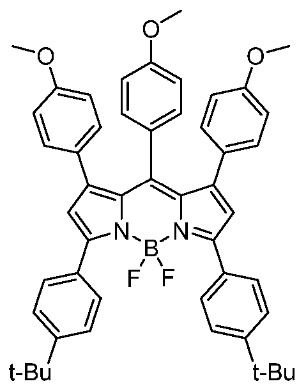

**F27**

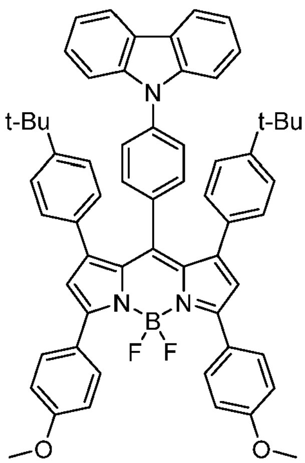

**F28**

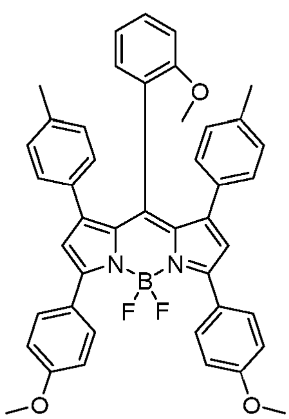

**F29**

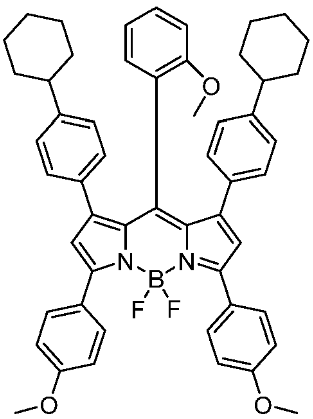

**F30**

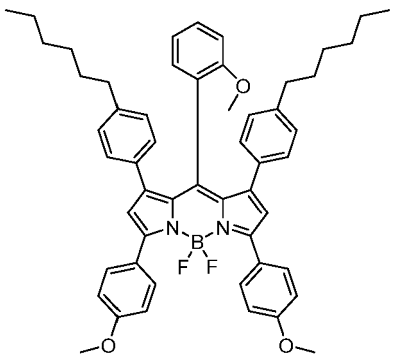

F31

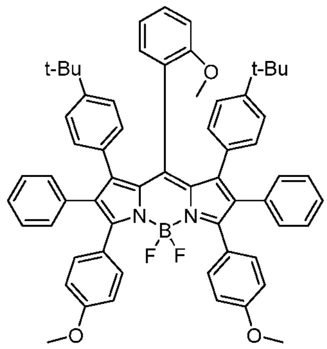

F32

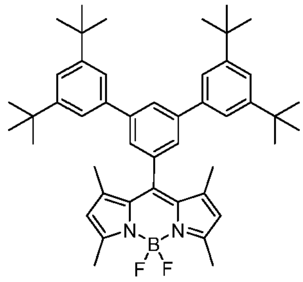

F33

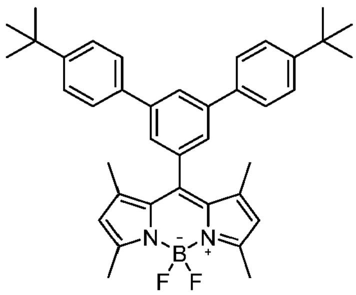

F34

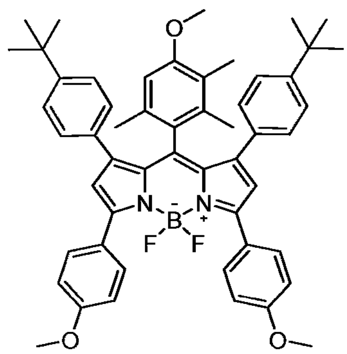

F35

Examples of the derivatives of the exemplary compounds include a compound in which at least one hydrogen atom is substituted with a deuterium atom, an alkyl group, an aryl group, a heteroaryl group, and a diaryl amino group.

Further, compounds, as described in WO2015/022974, paragraphs 0220 to 0239, WO2019/111971, paragraphs 0066 and 0117, and WO2021/015177, paragraphs 0196 to 0255, can also be used as the third organic compound of the invention.

(Light-emitting layer)

The light-emitting layer of the organic electroluminescence device of the invention is made of the first organic compound, the second organic compound, and the third organic compound, which satisfy the conditions (a) and (b). In a preferable aspect of the invention, as the second organic compound, a compound T132 is used, and as the third organic compound, a compound selected from the group consisting of compounds F101 to F128 is used. The light-emitting layer may not be made of a compound accepting charges or energy other than the first organic compound, the second organic compound, and the third organic compound, or a metal element other than boron. Further, the light-emitting layer may be made of only a compound containing atoms selected from the group consisting of a carbon atom, a hydrogen atom, a deuterium atom, a nitrogen atom, a boron atom, an oxygen atom, and a sulfur atom. For example, the light-emitting layer may be made of only a compound containing atoms selected from the group consisting of a carbon atom, a hydrogen atom, a deuterium atom, a nitrogen atom, a boron atom, and an oxygen atom. For example, the light-emitting layer may be made of only a compound containing atoms selected from the group consisting of a carbon atom, a hydrogen atom, a deuterium atom, a nitrogen atom, a boron atom, and a sulfur atom. For example, the light-emitting layer may be made of only a compound containing atoms selected from the group consisting of a carbon atom, a hydrogen atom, a deuterium atom, a nitrogen atom, and a boron atom. For example, the light-emitting layer may be made of only a compound containing atoms selected from the group consisting of a carbon atom, a hydrogen atom, a deuterium atom, a nitrogen atom, an oxygen atom, and a sulfur atom. For example, the light-emitting layer may be made of only a compound containing atoms selected from the group consisting of a carbon atom, a hydrogen atom, a deuterium atom, and a nitrogen atom. The light-emitting layer may be made of the first organic compound containing atoms selected from the group consisting of a carbon atom, a hydrogen atom, a nitrogen atom, and an oxygen atom, the second organic compound containing atoms selected from the group consisting of a carbon atom, a hydrogen atom, a deuterium atom, a nitrogen atom, an oxygen atom, and a sulfur atom, and the third organic compound containing atoms selected from the group consisting of a carbon atom, a hydrogen atom, a deuterium atom, a nitrogen atom, a boron atom, an oxygen atom, and a sulfur atom. Further, the light-emitting layer

may be made of the first organic compound containing atoms selected from the group consisting of a carbon atom, a hydrogen atom, a nitrogen atom, and an oxygen atom, the second organic compound containing atoms selected from the group consisting of a carbon atom, a hydrogen atom, a deuterium atom, and a nitrogen atom, and the third organic compound containing atoms selected from the group consisting of a carbon atom, a hydrogen atom, a deuterium atom, a nitrogen atom, and a boron atom.

The light-emitting layer may be formed by co-evaporating the first organic compound, the second organic compound, and the third organic compound, or may be formed by a coating method using a solution in which the first organic compound, the second organic compound, and the third organic compound are dissolved. When the light-emitting layer is formed by the co-evaporation, two or more of the first organic compound, the second organic compound, and the third organic compound may be mixed in advance and put in a crucible or the like, as an evaporation source, and the light-emitting layer may be formed by the co-evaporation using the evaporation source. For example, the first organic compound and the second organic compound may be mixed in advance to prepare one evaporation source, and the co-evaporation may be performed by using the evaporation source and an evaporation source of the third organic compound to form the light-emitting layer.

In the following, the constituent members and the other layers than a light-emitting layer of the organic electroluminescent device are described.

Substrate:

In some embodiments, the organic electroluminescent device of the invention is supported by a substrate, wherein the substrate is not particularly limited and may be any of those that have been commonly used in an organic electroluminescent device, for example those formed of glass, transparent plastics, quartz, and silicon.

Anode:

In some embodiments, the anode of the organic electroluminescent device is made of a metal, an alloy, an electroconductive compound, or a combination thereof. In some embodiments, the metal, alloy, or electroconductive compound has a large work function (4 eV or more). In some embodiments, the metal is Au. In some embodiments, the electroconductive transparent material is selected from CuI, indium tin oxide (ITO), $SnO_2$, and ZnO. In some embodiments, an amorphous material capable of forming a transparent electroconductive film, such as IDIXO ($In_2O_3$-ZnO), is used. In some embodiments, the anode is a thin film. In some embodiments, the thin film is made by vapor deposition or sputtering. In some embodiments, the film is patterned by a photolithography method. In some embodiments, when the pattern may not require high accuracy (for example, approximately 100 μm or more), the pattern may be formed with a mask having a desired shape on vapor deposition or sputtering of the electrode material. In some embodiments, when a material can be applied as a coating material, such as an organic electroconductive compound, a wet film forming method, such as a printing method and a coating method is used. In some embodiments, when the emitted light goes through the anode, the anode has a transmittance of more than 10%, and the anode has a sheet resistance of several hundred Ohm per square or less. In some embodiments, the thickness of the anode is from 10 to 1,000 nm. In some embodiments, the thickness of the anode is from 10 to 200 nm. In some embodiments, the thickness of the anode varies depending on the material used.

Cathode:

In some embodiments, the cathode is made of an electrode material such as a metal having a small work function (4 eV or less) (referred to as an electron injection metal), an alloy, an electroconductive compound, or a combination thereof. In some embodiments, the electrode material is selected from sodium, a sodium-potassium alloy, magnesium, lithium, a magnesium-cupper mixture, a magnesium-silver mixture, a magnesium-aluminum mixture, a magnesium-indium mixture, an aluminum-aluminum oxide ($Al_2O_3$) mixture, indium, a lithium-aluminum mixture, and a rare earth metal. In some embodiments, a mixture of an electron injection metal and a second metal that is a stable metal having a larger work function than the electron injection metal is used. In some embodiments, the mixture is selected from a magnesium-silver mixture, a magnesium-aluminum mixture, a magnesium-indium mixture, an aluminum-aluminum oxide ($Al_2O_3$) mixture, a lithium-aluminum mixture, and aluminum. In some embodiments, the mixture increases the electron injection property and the durability against oxidation. In some embodiments, the cathode is produced by forming the electrode material into a thin film by vapor deposition or sputtering. In some embodiments, the cathode has a sheet resistance of several hundred Ohm per square or less. In some embodiments, the thickness of the cathode ranges from 10 nm to 5 μm. In some embodiments, the thickness of the cathode ranges from 50 to 200 nm. In some embodiments, for transmitting the emitted light, any one of the anode and the cathode of the organic electroluminescent device is transparent or translucent. In some embodiments, the transparent or translucent electroluminescent devices enhance the light emission luminance.

In some embodiments, the cathode is formed with an electroconductive transparent material, as described for the anode, to form a transparent or translucent cathode. In some embodiments, a device comprises an anode and a cathode, both being transparent or translucent.

Injection Layer:

An injection layer is a layer between the electrode and the organic layer. In some embodiments, the injection layer decreases the driving voltage and enhances the light emission luminance. In some embodiments, the injection layer includes a hole injection layer and an electron injection layer. The injection layer can be positioned between the anode and the light-emitting layer or the hole transport layer, and between the cathode and the light-emitting layer or the electron transport layer. In some embodiments, an injection layer is present. In some embodiments, no injection layer is present.

Preferred compound examples for use as a hole injection material are shown below.

$MoO_3$,

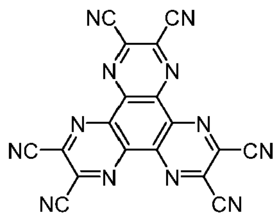
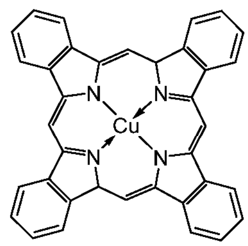
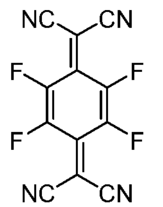
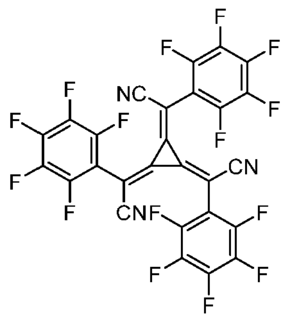

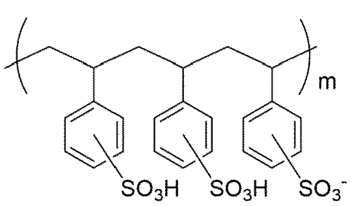
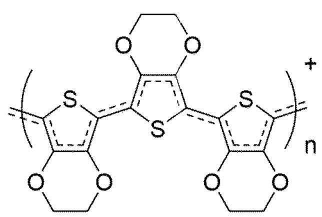

Next, preferred compound examples for use as an electron injection material are shown below. LiF, CsF,

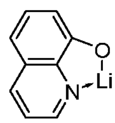

Barrier Layer:

A barrier layer is a layer capable of inhibiting charges (electrons or holes) and/or excitons present in the light-emitting layer from being diffused outside the light-emitting layer. In some embodiments, the electron barrier layer is between the light-emitting layer and the hole transport layer, and inhibits electrons from passing through the light-emitting layer toward the hole transport layer. In some embodiments, the hole barrier layer is between the light-emitting layer and the electron transport layer, and inhibits holes from passing through the light-emitting layer toward the electron transport layer. In some embodiments, the barrier layer inhibits excitons from being diffused outside the light-emitting layer. In some embodiments, the electron barrier layer and the hole barrier layer are exciton barrier layers. As used herein, the term "electron barrier layer" or "exciton barrier layer" includes a layer that has the functions of both electron barrier layer and of an exciton barrier layer.

Hole Barrier Layer:

A hole barrier layer acts as an electron transport layer. In some embodiments, the hole barrier layer inhibits holes

from reaching the electron transport layer while transporting electrons. In some embodiments, the hole barrier layer enhances the recombination probability of electrons and holes in the light-emitting layer. The material for the hole barrier layer may be the same materials as the ones described for the electron transport layer.

Preferred compound examples for use for the hole barrier layer are shown below.

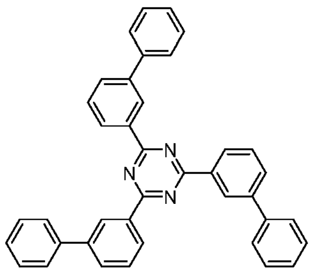
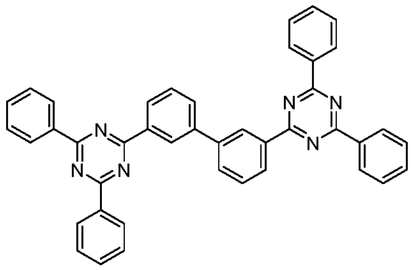
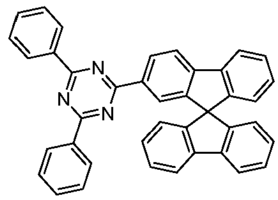
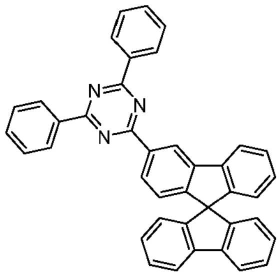
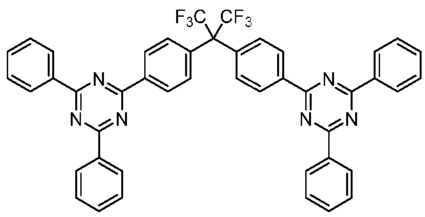
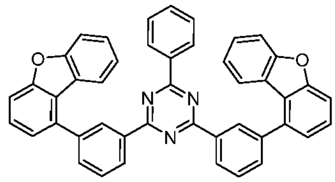
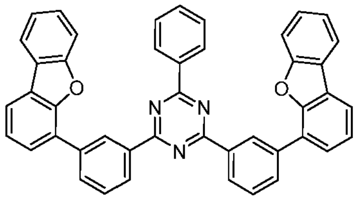
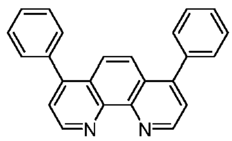
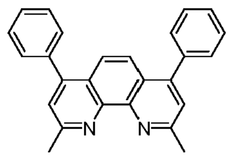

Electron Barrier Layer:

An electron barrier layer transports holes. In some embodiments, the electron barrier layer inhibits electrons from reaching the hole transport layer while transporting holes. In some embodiments, the electron barrier layer enhances the recombination probability of electrons and holes in the light-emitting layer. The materials for use for the electron barrier layer may be the same materials as those mentioned herein above for the hole transport layer.

Preferred compound examples for use as the electron barrier material are shown below.

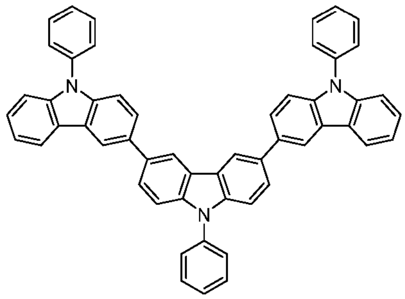
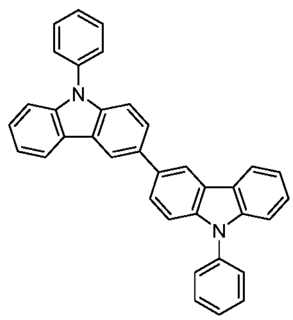
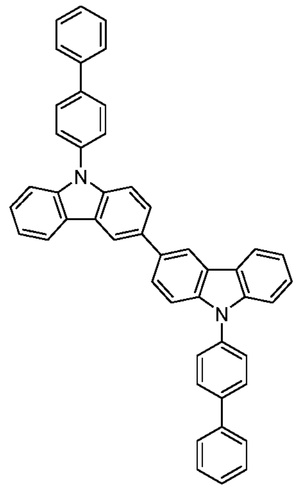

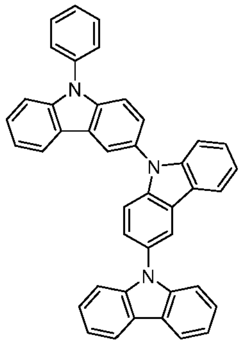

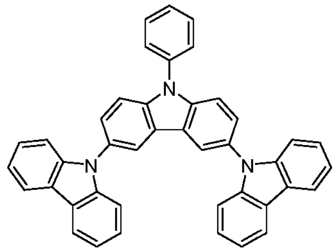

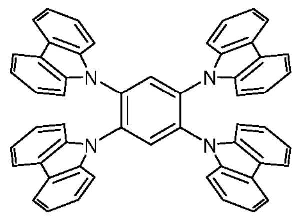

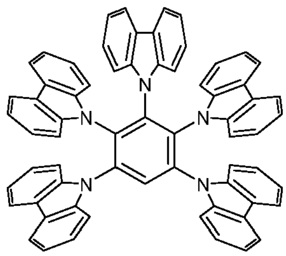

Exciton Barrier Layer:

An exciton barrier layer inhibits excitons generated through recombination of holes and electrons in the light-emitting layer from being diffused to the charge transport layer. In some embodiments, the exciton barrier layer enables effective confinement of excitons in the light-emitting layer. In some embodiments, the light emission efficiency of the device is enhanced. In some embodiments, the exciton barrier layer is adj acent to the light-emitting layer on any of the side of the anode and the side of the cathode, and on both the sides. In some embodiments, when the exciton barrier layer is on the side of the anode, the layer can be between the hole transport layer and the light-emitting layer and adjacent to the light-emitting layer. In some embodiments, when the exciton barrier layer is on the side of the cathode, the layer can be between the light-emitting layer and the cathode and adjacent to the light-emitting layer. In some embodiments, a hole injection layer, an electron barrier layer, or a similar layer is between the anode and the exciton barrier layer that is adjacent to the light-emitting layer on the side of the anode. In some embodiments, a hole injection layer, an electron barrier layer, a hole barrier layer, or a similar layer is between the cathode and the exciton barrier layer that is adjacent to the light-emitting layer on the side of the cathode. In some embodiments, the exciton barrier layer comprises excited singlet energy and excited triplet energy, at least one of which is higher than the excited singlet energy and the excited triplet energy of the light-emitting material, respectively.

Hole Transport Layer:

The hole transport layer comprises a hole transport material. In some embodiments, the hole transport layer is a single layer. In some embodiments, the hole transport layer comprises a plurality of layers.

In some embodiments, the hole transport material has one of injection or transport property of holes and barrier property of electrons. In some embodiments, the hole transport material is an organic material. In some embodiments, the hole transport material is an inorganic material. Examples of known hole transport materials that may be used herein include but are not limited to a triazole derivative, an oxadiazole derivative, an imidazole derivative, a carbazole derivative, an indolocarbazole derivative, a polyaryl alkane derivative, a pyrazoline derivative, a pyrazolone derivative, a phenylene diamine derivative, an arylamine derivative, an amino-substituted chalcone derivative, an oxazole derivative, a styryl anthracene derivative, a fluorenone derivative, a hydrazone derivative, a stilbene derivative, a silazane derivative, an aniline copolymer and an electroconductive polymer oligomer, particularly a thiophene oligomer, or a combination thereof. In some embodiments, the hole transport material is selected from a porphyrin compound, an aromatic tertiary amine compound, and a styryl amine compound. In some embodiments, the hole transport material is an aromatic tertiary amine compound. Preferred compound examples for use as the hole transport material are shown below.

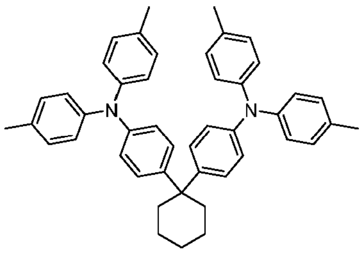
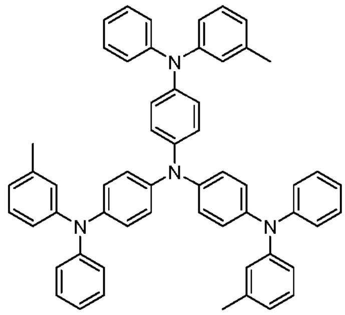
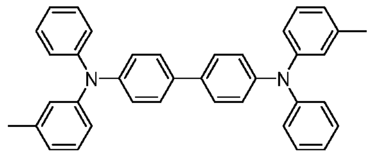

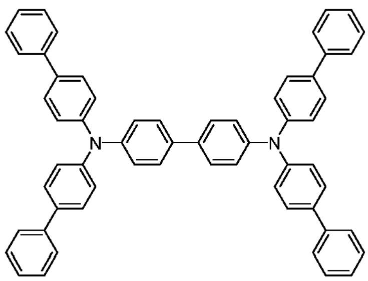
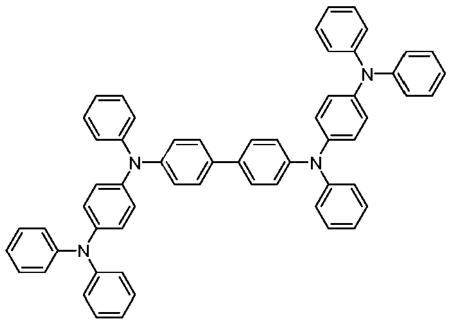

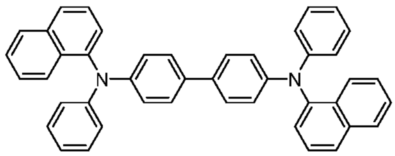
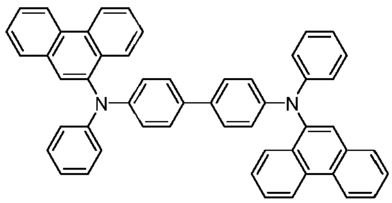

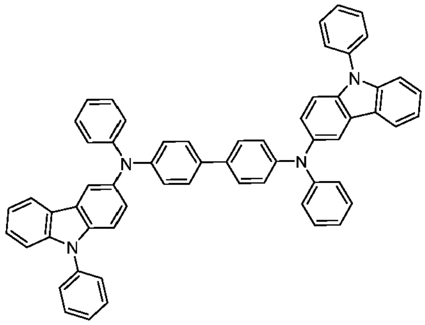
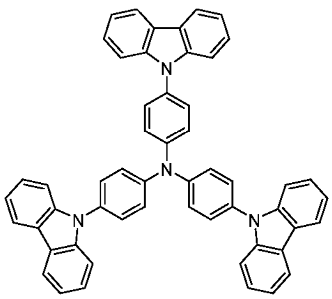
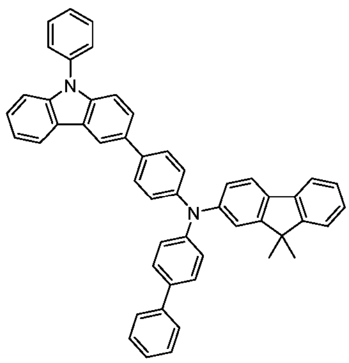

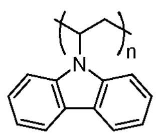
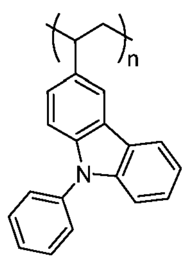
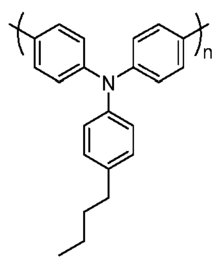

Electron Transport Layer:

The electron transport layer comprises an electron transport material. In some embodiments, the electron transport layer is a single layer. In some embodiments, the electron transport layer comprises a plurality of layers.

In some embodiments, the electron transport material needs only to have a function of transporting electrons,

which are injected from the cathode, to the light-emitting layer. In some embodiments, the electron transport material also functions as a hole barrier material. Examples of the electron transport layer that may be used herein include but are not limited to a nitro-substituted fluorene derivative, a diphenyl quinone derivative, a thiopyran dioxide derivative, carbodiimide, a fluorenylidene methane derivative, anthraquinodimethane, an anthrone derivatives, an oxadiazole derivative, an azole derivative, an azine derivative, or a combination thereof, or a polymer thereof. In some embodiments, the electron transport material is a thiadiazole derivative, or a quinoxaline derivative. In some embodiments, the electron transport material is a polymer material. Preferred compound examples for use as the electron transport material are shown below.

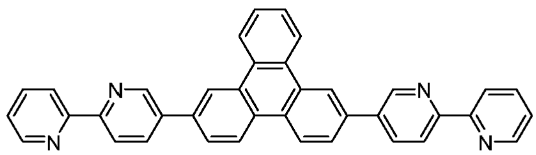

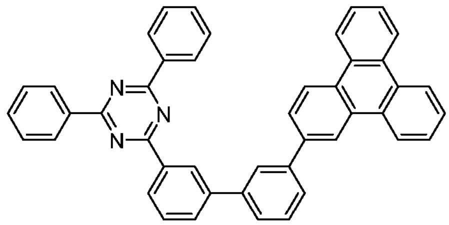

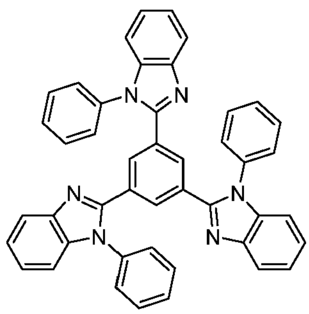

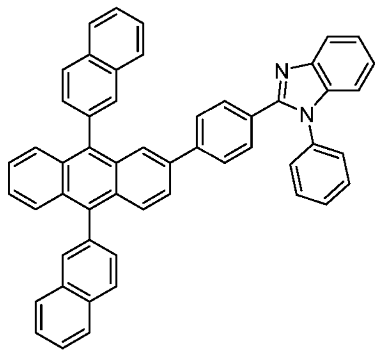

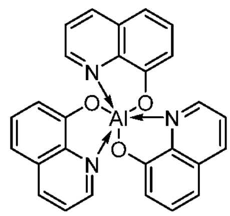

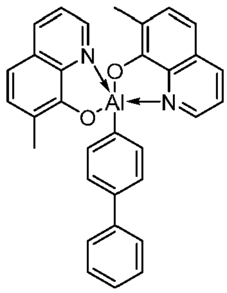

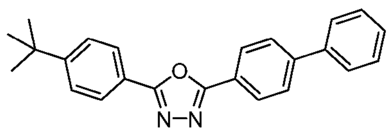

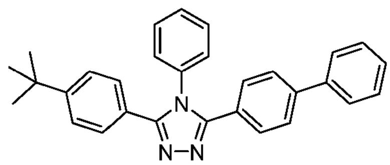

Preferred examples of compounds usable as materials that can be added to each organic layer are shown below. For example, the addition of a compound as a stabilizing material may be taken into consideration.

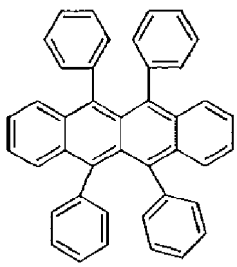

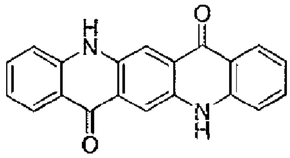

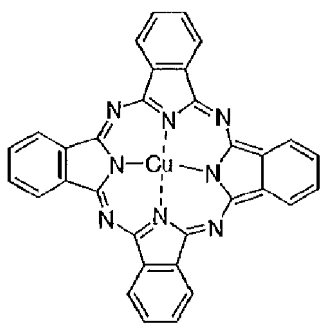

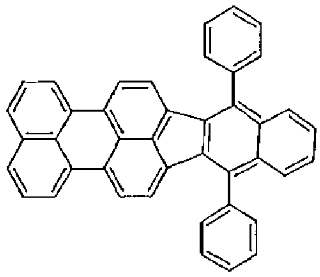
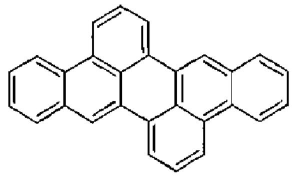

Herein under preferred materials for use in an organic electroluminescent device are specifically shown. However, the materials usable in the invention should not be limitatively interpreted by the following exemplary compounds. Compounds that are exemplified as materials having a specific function can also be used as materials having any other function.

Devices:

In some embodiments, the light-emitting layers are incorporated into a device. For example, the device includes, but is not limited to an OLED bulb, an OLED lamp, a television screen, a computer monitor, a mobile phone, and a tablet.

In some embodiments, an electronic device comprises an OLED comprising an anode, a cathode, and at least one organic layer comprising a light-emitting layer between the anode and the cathode.

In some embodiments, compositions described herein may be incorporated into various light-sensitive or light-activated devices, such as OLEDs or photovoltaic devices. In some embodiments, the composition may be useful in facilitating charge transfer or energy transfer within a device and/or as a hole transport material. The device may be, for example, an organic light-emitting diode (OLED), an organic integrated circuit (OIC), an organic field-effect transistor (O-FET), an organic thin-film transistor (O-TFT), an organic light-emitting transistor (O-LET), an organic solar cell (O-SC), an organic optical detector, an organic photoreceptor, an organic field-quench device (O-FQD), a light-emitting electrochemical cell (LEC) or an organic laser diode (O-laser).

Bulbs or Lamps:

In some embodiments, an electronic device comprises an OLED comprising an anode, a cathode, and at least one organic layer comprising a light-emitting layer between the anode and the cathode.

In some embodiments, a device comprises OLEDs that differ in color. In some embodiments, a device comprises an array comprising a combination of OLEDs. In some embodiments, the combination of OLEDs is a combination of three colors (e.g., RGB). In some embodiments, the combination of OLEDs is a combination of colors that are not red, green, or blue (for example, orange and yellow green). In some embodiments, the combination of OLEDs is a combination of two, four, or more colors.

In some embodiments, a device is an OLED light comprising,

a circuit board having a first side with a mounting surface and an opposing second side, and defining at least one aperture;

at least one OLED on the mounting surface, the at least one OLED configured to emanate light, comprising an anode, a cathode, and at least one organic layer comprising a light-emitting layer between the anode and the cathode;

a housing for the circuit board; and

at least one connector arranged at an end of the housing, the housing and the connector defining a package adapted for installation in a light fixture.

In some embodiments, the OLED light comprises a plurality of OLEDs mounted on a circuit board such that light emanates in a plurality of directions. In some embodiments, a portion of the light emanated in a first direction is deflected to emanate in a second direction. In some embodiments, a reflector is used to deflect the light emanated in a first direction.

Displays or Screens:

In some embodiments, the light-emitting layer in the invention can be used in a screen or a display. In some embodiments, the compounds in the invention are deposited onto a substrate using a process including, but not limited to, vacuum evaporation, deposition, vapor deposition, or chemical vapor deposition (CVD). In some embodiments, the substrate is a photoplate structure useful in a two-sided etch that provides a unique aspect ratio pixel. The screen (which

may also be referred to as a mask) is used in a process in the manufacturing of OLED displays. The corresponding artwork pattern design facilitates a very steep and narrow tie-bar between the pixels in the vertical direction and a large, sweeping bevel opening in the horizontal direction. This allows the close patterning of pixels needed for high definition displays while optimizing the chemical deposition onto a TFT backplane.

The internal patterning of the pixel allows the construction of a 3-dimensional pixel opening with varying aspect ratios in the horizontal and vertical directions. Additionally, the use of imaged "stripes" or halftone circles within the pixel area inhibits etching in specific areas until these specific patterns are undercut and fall off the substrate. At that point, the entire pixel area is subjected to a similar etch rate but the depths are varying depending on the halftone pattern. Varying the size and spacing of the halftone pattern allows etching to be inhibited at different rates within the pixel allowing for a localized deeper etch needed to create steep vertical bevels.

A preferred material for the deposition mask is invar. Invar is a metal alloy that is cold rolled into long thin sheet in a steel mill. Invar cannot be electrodeposited onto a rotating mandrel as the nickel mask. A preferred and more cost feasible method for forming the open areas in the mask used for deposition is through a wet chemical etching.

In some embodiments, a screen or display pattern is a pixel matrix on a substrate. In some embodiments, a screen or display pattern is fabricated using lithography (e.g., photolithography and e-beam lithography). In some embodiments, a screen or display pattern is fabricated using a wet chemical etch. In further embodiments, a screen or display pattern is fabricated using plasma etching.

Methods of Manufacturing Devices:

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light-emitting layer, a counter electrode, and an encapsulation layer, and then is cut from the mother panel.

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light-emitting layer, a counter electrode, and an encapsulation layer, and then is cut from the mother panel.

In another aspect, provided herein is a method of manufacturing an organic light-emitting diode (OLED) display, the method comprising:

forming a barrier layer on a base substrate of a mother panel;
forming a plurality of display units in units of cell panels on the barrier layer;
forming an encapsulation layer on each of the display units of the cell panels; and
applying an organic film to an interface portion between the cell panels.

In some embodiments, the barrier layer is an inorganic film formed of, for example, SiNx, and an edge portion of the barrier layer is covered with an organic film formed of polyimide or acryl. In some embodiments, the organic film helps the mother panel to be softly cut in units of the cell panel.

In some embodiments, the thin film transistor (TFT) layer EL device, a gate electrode, and a source/drain electrode. Each of the plurality of display units may include a thin film transistor (TFT) layer, a planarization film formed on the TFT layer, and a light-emitting unit formed on the planarization film, wherein the organic film applied to the interface portion is formed of a same material as a material of the planarization film and is formed at a same time as the planarization film is formed. In some embodiments, a light-emitting unit is connected to the TFT layer with a passivation layer and a planarization film therebetween and an encapsulation layer that covers and protects the light-emitting unit. In some embodiments of the method of manufacturing, the organic film contacts neither the display units nor the encapsulation layer.

Each of the organic film and the planarization film may include any one of polyimide and acryl. In some embodiments, the barrier layer may be an inorganic film. In some embodiments, the base substrate may be formed of polyimide. The method may further include, before the forming of the barrier layer on one surface of the base substrate formed of polyimide, attaching a carrier substrate formed of a glass material to another surface of the base substrate, and before the cutting along the interface portion, separating the carrier substrate from the base substrate. In some embodiments, the OLED display is a flexible display.

In some embodiments, the passivation layer is an organic film disposed on the TFT layer to cover the TFT layer. In some embodiments, the planarization film is an organic film formed on the passivation layer. In some embodiments, the planarization film is formed of polyimide or acryl, like the organic film formed on the edge portion of the barrier layer. In some

embodiments, the planarization film and the organic film are simultaneously formed when the OLED display is manufactured. In some embodiments, the organic film may be formed on the edge portion of the barrier layer such that a portion of the organic film directly contacts the base substrate and a remaining portion of the organic film contacts the barrier layer while surrounding the edge portion of the barrier layer.

In some embodiments, the light-emitting layer includes a pixel electrode, a counter electrode, and an organic light-emitting layer disposed between the pixel electrode and the counter electrode. In some embodiments, the pixel electrode is connected to the source/drain electrode of the TFT layer.

In some embodiments, when a voltage is applied to the pixel electrode through the TFT layer, an appropriate voltage is formed between the pixel electrode and the counter electrode, and thus the organic light-emitting layer emits light, thereby forming an image. Hereinafter, an image forming unit including the TFT layer and the light-emitting unit is referred to as a display unit.

In some embodiments, the encapsulation layer that covers the display unit and prevents penetration of external moisture may be formed to have a thin film encapsulation structure in which an organic film and an inorganic film are alternately stacked. In some embodiments, the encapsulation layer has a thin film encapsulation structure in which a plurality of thin films are stacked. In some embodiments, the organic film applied to the interface portion is spaced apart from each of the plurality of display units. In some embodiments, the organic film is formed such that a portion of the organic film directly contacts the base substrate and a remaining portion of the organic film contacts the barrier layer while surrounding an edge portion of the barrier layer.

In some embodiments, the OLED display is flexible and uses the soft base substrate formed of polyimide. In some embodiments, the base substrate is formed on a carrier substrate formed of a glass material, and then the carrier substrate is separated.

In some embodiments, the barrier layer is formed on a surface of the base substrate opposite to the carrier substrate. In some embodiments, the barrier layer is patterned according to a size of each of the cell panels. For example, while the base substrate is formed over the entire surface of a mother panel, the barrier layer is formed according to a size of each of the cell panels, and thus a groove is formed at an interface portion between the barrier layers of the cell panels. Each of the cell panels can be cut along the groove.

In some embodiments, the method of manufacture further comprises cutting along the interface portion, wherein a groove is formed in the barrier layer, wherein at least a portion of the organic film is formed in the groove, and wherein the groove does not penetrate into the base substrate. In some embodiments, the TFT layer of each of the cell panels is formed, and the passivation layer which is an inorganic film and the planarization film which is an organic film are disposed on the TFT layer to cover the TFT layer. At the same time as the planarization film formed of, for example, polyimide or acryl is formed, the groove at the interface portion is covered with the organic film formed of, for example, polyimide or acryl. This is to prevent cracks from occurring by allowing the organic film to absorb an impact generated when each of the cell panels is cut along the groove at the interface portion. That is, if the entire barrier layer is entirely exposed without the organic film, an impact generated when each of the cell panels is cut along the groove at the interface portion is transferred to the barrier layer, thereby increasing the risk of cracks. However, in some embodiments, since the groove at the interface portion between the barrier layers is covered with the organic film and the organic film absorbs an impact that would otherwise be transferred to the barrier layer, each of the cell panels may be softly cut and cracks may be prevented from occurring in the barrier layer. In some embodiments, the organic film covering the groove at the interface portion and the planarization film are spaced apart from each other. For example, if the organic film and the planarization film are connected to each other as one layer, since external moisture may penetrate into the display unit through the planarization film and a portion where the organic film remains, the organic film and the planarization film are spaced apart from each other such that the organic film is spaced apart from the display unit.

In some embodiments, the display unit is formed by forming the light-emitting unit, and the encapsulation layer is disposed on the display unit to cover the display unit. As such, once the mother panel is completely manufactured, the carrier substrate that supports the base substrate is separated from the base substrate. In some embodiments, when a laser beam is emitted toward the carrier substrate, the carrier substrate is separated from the base substrate due to a difference in a thermal expansion coefficient between the carrier substrate and the base substrate.

In some embodiments, the mother panel is cut in units of the cell panels. In some embodiments, the mother panel is cut along an interface portion between the cell panels by using a cutter. In some embodiments, since the groove at the interface portion along which the mother panel is cut is covered with the organic film, the organic film absorbs an impact during the cutting. In some embodiments, cracks may be prevented from occurring in the barrier layer during the cutting.

In some embodiments, the methods reduce a defect rate of a product and stabilize its quality.

Another aspect is an OLED display including: a barrier layer that is formed on a base substrate; a display unit that is formed on the barrier layer; an encapsulation layer that is formed on the display unit; and an organic film that is applied to an edge portion of the barrier layer.

## Examples

The features of the invention will be described more specifically with reference to Examples given below. The materials, processes, procedures and the like shown below may be appropriately modified unless they deviate from the substance of the invention. Accordingly, the scope of the invention is not construed as being limited to the specific examples shown below. Herein under the luminescent performance was evaluated using a source meter (available from Keithley Instruments Corporation: 2400 series), a semiconductor parameter analyzer (available from Agilent Corporation: E5273A), an optical power meter device (available from Newport Corporation: 1930C), an optical spectroscope (available from Ocean Optics Corporation: USB2000), a spectroradiometer (available from Topcon Corporation: SR-3), and a streak camera (available from Hamamatsu Photonics K.K., Model C4334), and an orientation value was measured using a molecular orientation characteristic measurement system (available from Hamamatsu Photonics K.K., C14234-01).

<First to Third Organic Compounds Used in Examples and Comparative Examples>

First to third organic compounds used in each of the following examples and comparative examples will be given below.

First Organic Compound

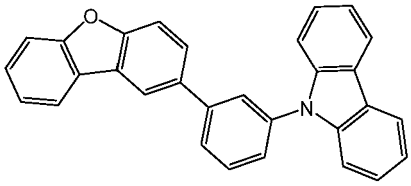

**H1**

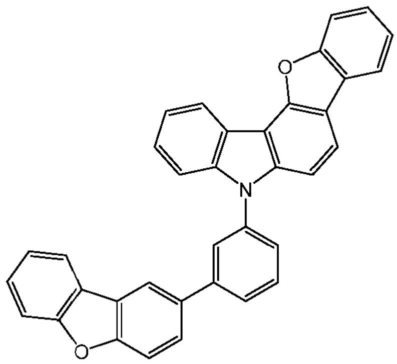

**H41**

Second Organic Compound (Delayed fluorescence material)

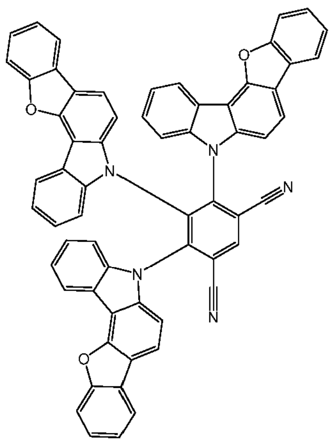

**T55**

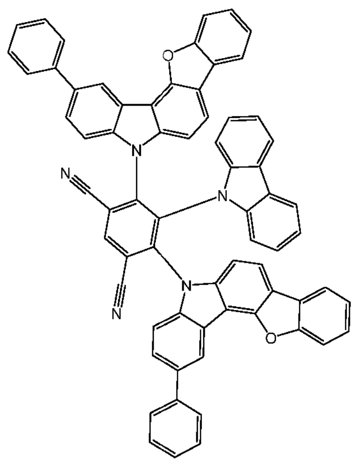

**T132**

Third Organic Compound (Fluorescence material)

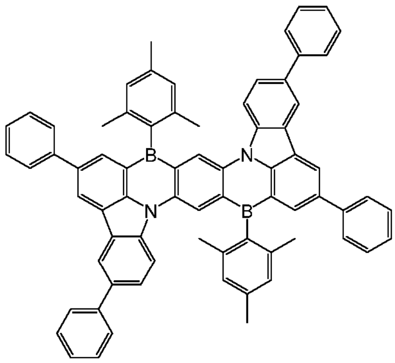

**F101**

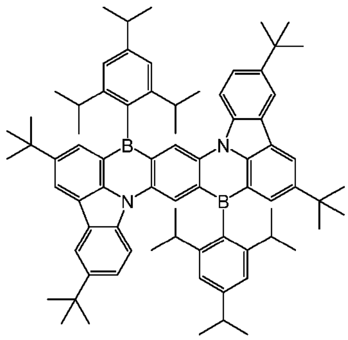

**F110**

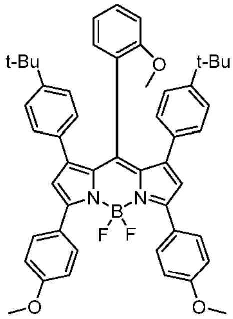

**F13**

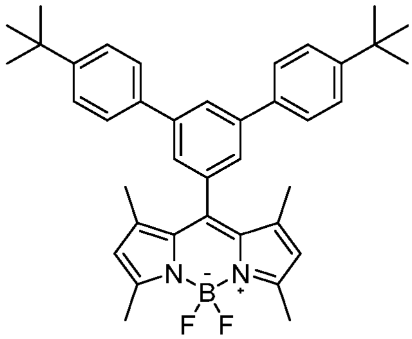

**F30**

HOMO energy $E_{HOMO}$ and LUMO energy $E_{LUMO}$ of the compounds are shown in Table 1 described below. Further, lowest excited singlet energy $E_{S1}$ and lowest excited triplet energy $E_{T1}$ measured for some compounds are also shown.

Table 1

| | | $E_{LUMO}$ | $E_{HOMO}$ | $E_{S1}$ | $E_{T1}$ |
|---|---|---|---|---|---|
| First organic compound | Compound H1 | -2.63 | -6.13 | -3.69 | -2.83 |
| | Compound H41 | -2.50 | -5.87 | -3.50 | -2.70 |
| Second organic compound | Compound T55 | -3.55 | -5.98 | -2.43 | -2.24 |
| | Compound T132 | -3.46 | -5.92 | -2.44 | - |
| Third organic compound | Compound F101 | -3.70 | -5.93 | -2.34 | - |
| | Compound F110 | -3.57 | -5.91 | -2.44 | - |
| | Compound F13 | -3.79 | -5.76 | - | - |
| | Compound F30 | -3.65 | -5.95 | - | - |
| (unit eV) | | | | | |

(Examples 1 to 10 and Comparative Examples 5 to 7, 9, 10, and 12)

On a glass substrate with a thickness of 2 mm on which an anode made of indium·tin oxide (ITO) was formed with a film thickness of 50 nm, each thin film was laminated through a vacuum evaporation method at a vacuum degree of $1\times10^{-6}$ Pa. First, HATCN was deposited with a thickness of 5 nm on ITO to form a hole injection layer, and NPD was

deposited with a thickness of 60 nm to form a hole transport layer. Subsequently, EB1 was deposited with a thickness of 5 nm to form an electron block layer. Next, the first organic compound, the second organic compound, and the third organic compound were co-evaporated from different evaporation sources to have a composition shown in Table 2 or Table 3, and a light-emitting layer was formed with a thickness of 40 nm. Next, HB1 was deposited with a thickness of 10 nm to form a hole block layer, and then, ET1 was deposited with a thickness of 30 nm to form an electron transport layer. Further, Liq was deposited with a thickness of 2 nm to form an electron injection layer, and then, aluminum (Al) was deposited with a thickness of 100 nm to form a cathode. Accordingly, an organic electroluminescence device was prepared. It was checked that in all of the devices prepared herein, the formula (a) was satisfied, and the largest component of light emitted from the device was fluorescence from the third organic compound.

(Comparative Examples 1 to 4, 8, and 11)

An organic electroluminescence device was prepared as with Example 1 except that when forming the light-emitting layer, the light-emitting layer having a composition shown in Table 2 was formed by the co-evaporation of the first organic compound and the second organic compound without using the evaporation source of the third organic compound.

(Composition of light-emitting layer and evaluation results)

For each of the devices prepared in Examples 1 to 6 and Comparative Examples 1 to 10, the composition of the light-emitting layer, and measurement results of an external quantum efficiency EQE and a light-emitting maximum wavelength are shown in Table 2. In the composition of the light-emitting layer, the compositional ratio of the third organic compound in Examples 1 to 4 and Comparative Examples 5 to 7, 9, and 10 was represented by a ratio (% by weight) to the total weight of the first organic compound and the second organic compound, and the compositional ratio of the other organic compound was represented by a ratio (% by weight) to the total weight of the organic compounds configuring the light-emitting layer. Further, in Table 2, "-" represents that the third organic compound is not added.

Table 2

| Example No. | Composition of light-emitting layer | | | | | | Orientation value S of third organic compound | Evaluation Results | |
|---|---|---|---|---|---|---|---|---|---|
| | First organic compound | | Second organic compound | | Third organic compound | | | EQE (%) | Light-emitting maximum wavelength (nm) |
| | Compound | Content (wt%) | Compound | Content (wt%) | Compound | Content (wt%) | | | |
| Comparative Example 1 | H1 | 55.0 | T132 | 45.0 | - | | - | 15.37 | 536 |
| Example 1 | H1 | 55.0 | T132 | 45.0 | F110 | 0.5 | -0.42 | 17.30 | 529 |
| Example 2 | H1 | 55.0 | T132 | 45.0 | F110 | 1.0 | -0.43 | 18.93 | 529 |
| Comparative Example 2 | H1 | 65.0 | T132 | 35.0 | - | | - | 16.97 | 532 |
| Example 3 | H1 | 65.0 | T132 | 35.0 | F110 | 0.5 | -0.39 | 19.82 | 528 |
| Example 4 | H1 | 65.0 | T132 | 35.0 | F110 | 1.0 | -0.39 | 20.96 | 529 |
| Comparative Example 3 | H1 | 65.0 | T132 | 35.0 | - | | - | 17.50 | 539 |
| Example 5 | H1 | 64.5 | T132 | 35.0 | F101 | 0.5 | -0.31 | 19.00 | 542 |
| Example 6 | H1 | 64.0 | T132 | 35.0 | F101 | 1.0 | -0.34 | 20.20 | 544 |
| Comparative Example 4 | H1 | 55.0 | T132 | 45.0 | - | | - | 15.10 | 540 |
| Comparative Example 5 | H1 | 55.0 | T132 | 45.0 | F30 | 0.3 | 0.12 | 13.90 | 523 |
| Comparative Example 6 | H1 | 55.0 | T132 | 45.0 | F30 | 0.5 | 0.13 | 12.90 | 523 |
| Comparative Example 7 | H1 | 55.0 | T132 | 45.0 | F30 | 1.0 | 0.13 | 11.50 | 523 |
| Comparative Example 8 | H1 | 65.0 | T132 | 35.0 | - | | - | 16.2 | 540 |
| Comparative Example 9 | H1 | 65.0 | T132 | 35.0 | F30 | 0.5 | 0.12 | 15.0 | 524 |
| Comparative Example 10 | H1 | 65.0 | T132 | 35.0 | F30 | 1.0 | 0.13 | 12.5 | 524 |
| Comparative Example 11 | H41 | 60.0 | T55 | 40.0 | - | | - | 13.17 | 554 |
| Comparative Example 12 | H41 | 59.5 | T55 | 40.0 | F13 | 0.5 | -0.29 | 10.40 | 634 |

As shown in Table 2, in the devices of Examples 1 to 6 in which an orientation value S of the third organic compound is -0.3 or less, a high external quantum efficiency was exhibited compared to the devices of Comparative Examples 1 to 3 in which the third organic compound is not contained in the light-emitting layer. Further, in the devices of Examples 2, 4, and 6 in which the concentration the third organic compound is high, a high external quantum efficiency was obtained compared to the devices of Examples 1, 3, and 5. In contrast, in the devices of Comparative Examples 5 to 7, 9 to 10, and 12 in which the orientation value S of the third organic compound is greater than -0.3, the external quantum efficiency decreased compared to the devices of Comparative Examples 4, 8, and 11 having the same configuration except that the third organic compound is not contained in the light-emitting layer. Further, from the results of Comparative Examples 5 to 7 and Comparative Examples 9 to 10, it was found that there is a tendency that the external quantum efficiency decreases as the concentration of the third organic compound increases. From the results described above, it was checked that even in the case of a device of which the LUMO energy of the third organic compound is lower than the LUMO energy of the second organic compound, the external quantum efficiency can be improved by increasing the concentration of the third organic compound when orientation value S of the third organic compound is -0.3 or less.

The composition of the light-emitting layer of each of the devices prepared in Examples 7 to 10 and evaluation results of durability are shown in Table 3. In Table 3, "LT95%" is a relative value obtained by measuring time (T95%) until a luminance is 95% of the initial luminance when continuously driving each of the devices at a current density of 12.6 $mA/cm^2$, and dividing the value of T95% by T95% of the device prepared in Example 7. A larger value of LT95% indicates excellent durability.

Table 3

| Example No. | Composition of light-emitting layer | | | | | | Orientation value S of third organic compound | LT95% |
|---|---|---|---|---|---|---|---|---|
| | First organic compound | | Second organic compound | | Third organic compound | | | |
| | Compound | Content (wt%) | Compound | Content (wt%) | Compound | Content (wt%) | | |
| Example 7 | H1 | 79.2 | T132 | 20.0 | F110 | 0.8 | -0.40 | 1 |
| Example 8 | H1 | 74.2 | T132 | 25.0 | F110 | 0.8 | -0.41 | 1.5 |
| Example 9 | H1 | 69.2 | T132 | 30.0 | F110 | 0.8 | -0.40 | 2.0 |
| Example 10 | H1 | 64.2 | T132 | 35.0 | F110 | 0.8 | -0.39 | 2.2 |

In the devices of Examples 7 to 10, the concentration of the second organic compound in the light-emitting layer was changed, but the light-emitting maximum wavelength was the same as 529 nm, and the external quantum efficiency was the same as about 22%. Further, Table 3 shows that the durability of the device tends to be improved by increasing the concentration of the second organic compound.

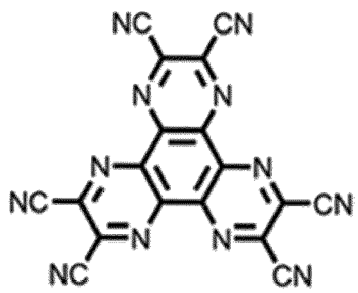

HATCN

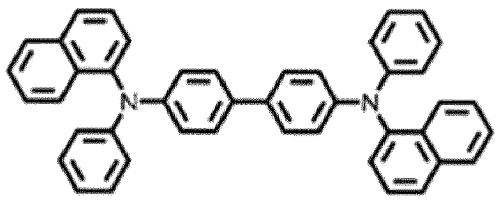

NPD

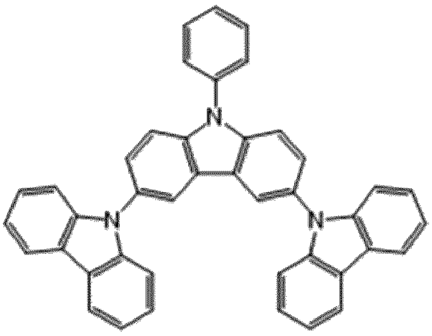

EB1

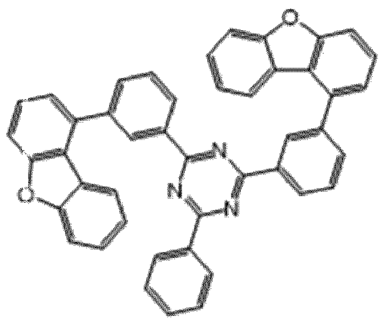

HB1

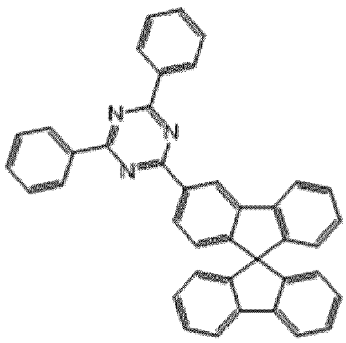

ET1

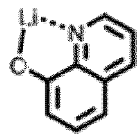

Liq

Industrial Applicability

According to the invention, in the organic electroluminescence device containing the first organic compound, the second organic compound that is the delayed fluorescence material, and the third organic compound emitting the fluorescence in the light-emitting layer, even when the LUMO energy of the first organic compound is lower than the LUMO energy of the second organic compound, the external quantum efficiency can be improved by increasing the concentration of the second organic compound. Accordingly, it is possible to expand the range of choices for the LUMO energy of the third organic compound, and thus, to increase the degree of freedom in material design of the organic electroluminescence device. Therefore, the invention has high industrial applicability.

## Claims

**1.** An organic electroluminescence device comprising: an anode; a cathode; and at least one organic layer including a light-emitting layer between the anode and the cathode, wherein the light-emitting layer contains a first organic compound, a second organic compound, and a third organic compound, and satisfies the following formulae (a) and (b),

the second organic compound is a delayed fluorescence material,
**characterized in that**
the third organic compound is a compound represented by the following formula (16),

Formula (16)

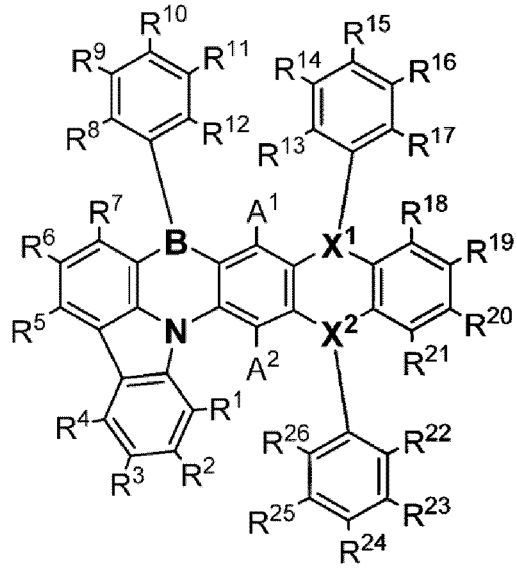

wherein in the formula (16), one of $X^1$ and $X^2$ is a nitrogen atom, and the other is a boron atom; each of $R^1$ to $R^{26}$, $A^1$, and $A^2$ independently represents a hydrogen atom, a deuterium atom, or a substituent, $R^1$ and $R^2$, $R^2$ and $R^3$, $R^3$ and $R^4$, $R^4$ and $R^5$, $R^5$ and $R^6$, $R^6$ and $R^7$, $R^7$ and $R^8$, $R^8$ and $R^9$, $R^9$ and $R^{19}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{17}$ and $R^{18}$, $R^{18}$ and $R^{19}$, $R^{19}$ and $R^{20}$, $R^{20}$ and $R^{21}$, $R^{21}$ and $R^{22}$, $R^{22}$ and $R^{23}$, $R^{23}$ and $R^{24}$, $R^{24}$ and $R^{25}$, and $R^{25}$ and $R^{26}$ may be bonded to each other to form ring structures; here, when $X^1$ is a nitrogen atom, then $R^{17}$ and
$R^{18}$ are bonded to each other to form a single bond and to form a pyrrole ring, and when $X^2$ is a nitrogen atom, then $R^{21}$ and $R^{22}$ are bonded to each other to form a single bond and to form a pyrrole ring; here, when $X^1$ is a nitrogen atom, $R^7$ and $R^8$ and $R^{21}$ and $R^{22}$ are bonded via nitrogen atoms to form 6-membered rings, and $R^{17}$ and $R^{18}$ are bonded to each other to form a single bond, then
at least one of $R^1$ to $R^6$ is a substituted or unsubstituted aryl group, or any of $R^1$ and $R^2$, $R^2$ and $R^3$, $R^3$ and $R^4$, $R^4$ and $R^5$, and $R^5$ and $R^6$ is bonded to each other to form an aromatic ring or a heteroaromatic ring, and the largest component of light emitted from the device is fluorescence from the third organic compound,

$$E_{LUMO}(2) > E_{LUMO}(3) \quad \text{Formula (a)}$$

$$S \leq -0.3 \quad \text{Formula (b)}$$

here,
$E_{LUMO}(2)$ represents LUMO energy obtained by photo-electron spectroscopy in air of the second organic compound,
$E_{LUMO}(3)$ represents LUMO energy obtained by photo-electron spectroscopy in air of the third organic compound, and
S represents an orientation value of the third organic compound in the light-emitting layer.

**2.** The organic electroluminescence device according to claim 1, wherein a concentration of the third organic compound in the light-emitting layer is greater than 0.3% by weight.

**3.** The organic electroluminescence device according to any one of claims 1 or 2, wherein a concentration of the second organic compound in the light-emitting layer is 25% by weight or more.

**4.** The organic electroluminescence device according to any one of claims 1 to 3, wherein the second organic compound has a structure in which 1 to 2 cyano groups and at least one donor group are bonded to a benzene ring.

**5.** The organic electroluminescence device according to claim 4, wherein the donor group has a structure in which a substituted or unsubstituted benzofuran ring is condensed with a benzene ring constituting a carbazole-9-yl group.

6. The organic electroluminescence device according to claim 5, wherein the donor group is a substituted or unsubstituted SH-benzofuro[3,2-c]carbazole-5-yl group.

7. The organic electroluminescence device according to any one of claims 4 to 6, wherein three or more donor groups are bonded to the benzene ring.

8. The organic electroluminescence device according to any one of claims 1 to 7, wherein the first organic compound, the second organic compound, and the third organic compound satisfy (a1) described below,

$$E_{LUMO}(1) > E_{LUMO}(2) > E_{LUMO}(3) \quad \text{Formula (a1)}$$

here,
$E_{LUMO}(1)$ represents LUMO energy obtained by photo-electron spectroscopy in air of the first organic compound,
$E_{LUMO}(2)$ represents LUMO energy obtained by photo-electron spectroscopy in air of the second organic compound,
$E_{LUMO}(3)$ represents LUMO energy obtained by photo-electron spectroscopy in air of the third organic compound, and
S represents an orientation value of the third organic compound in the light-emitting layer.

**Patentansprüche**

1. Organische elektrolumineszierende Vorrichtung, umfassend: eine Anode; eine Kathode; und mindestens eine organische Schicht, die eine Licht emittierende Schicht zwischen der Anode und der Kathode einschließt,

wobei die Licht emittierende Schicht eine erste organische Verbindung, eine zweite organische Verbindung, und eine dritte organische Verbindung enthält, und die folgenden Formeln (a) und (b) erfüllt,
wobei die zweite organische Verbindung ein Material mit verzögerter Fluoreszenz ist, **dadurch gekennzeichnet, dass**
die dritte Verbindung eine Verbindung ist, dargestellt durch die folgende Formel (16),

Formel (16)

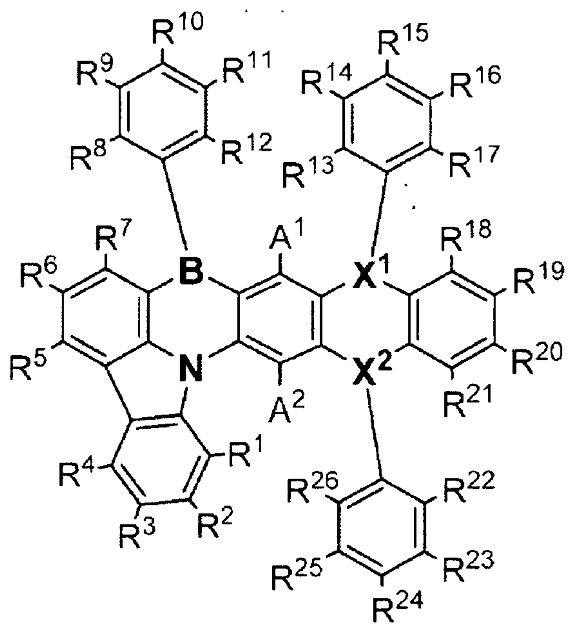

wobei in der Formel (16) eines von $X^1$ und $X^2$ ein Stickstoffatom und das andere ein Boratom ist; jedes von $R^1$ bis $R^{26}$, $A^1$, und $A^2$ unabhängig voneinander ein Wasserstoffatom, ein Deuterium-Atom oder einen Substituenten darstellt; $R^1$ und $R^2$, $R^2$ und $R^3$, $R^3$ und $R^4$, $R^4$ und $R^5$, $R^5$ und $R^6$, $R^6$ und $R^7$, $R^7$ und $R^8$, $R^8$ und $R^9$, $R^9$ und $R^{10}$, $R^{10}$

und $R^{11}$, $R^{11}$ und $R^{12}$, $R^{13}$ und $R^{14}$, $R^{14}$ und $R^{15}$, $R^{15}$ und $R^{16}$, $R^{16}$ und $R^{17}$, $R^{17}$ und $R^{18}$, $R^{18}$ und $R^{19}$, $R^{19}$ und $R^{20}$, $R^{20}$ und $R^{21}$,
$R^{21}$ und $R^{22}$, $R^{22}$ und $R^{23}$, $R^{23}$ und $R^{24}$, $R^{24}$ und $R^{25}$ und $R^{25}$ und $R^{26}$ miteinander verbunden sein können, um Ringstrukturen zu bilden; hier, wenn $X^1$ ein Stickstoffatom ist, dann sind $R^{17}$ und $R^{18}$ miteinander verbunden, um eine Einzelbindung und einen Pyrrolring zu bilden, und wenn $X^2$ ein Stickstoffatom ist, dann sind $R^{21}$ und $R^{22}$ miteinander verbunden, um eine Einzelbindung und einen Pyrrolring zu bilden; hier, wenn $X^1$ ein Stickstoffatom ist, $R^7$ und $R^8$ und $R^{21}$ und $R^{22}$ über Stickstoffatome miteinander verbunden sind, um 6-gliedrige Ringe zu bilden, und $R^{17}$ und $R^{18}$ miteinander verbunden sind, um eine Einzelbindung zu bilden, dann ist mindestens einer von $R^1$ bis $R^6$ eine substituierte oder nicht substituierte Arylgruppe, oder irgendeines von $R^1$ und $R^2$, $R^2$ und $R^3$, $R^3$ und $R^4$, $R^4$ und $R^5$ und $R^5$ und $R^6$ sind miteinander verbunden, um einen aromatischen Ring oder einen heteroaromatischen Ring zu bilden, und der größte Anteil des von der Vorrichtung emittierten Lichts Fluoreszenz aus der dritten organischen Verbindung ist,

$$E_{LUMO}(2) > E_{LUMO}(3) \qquad \text{Formel (a)}$$

$$S \leq -0{,}3 \qquad \text{Formel (b)}$$

hier stellt $E_{LUMO}(2)$ die LUMO-Energie dar, die mittels Photoelektronenspektroskopie in Luft für die zweite Verbindung erhalten wurde,
$E_{LUMO}(3)$ stellt die LUMO-Energie dar, die mittels Photoelektronenspektroskopie in Luft für die dritte Verbindung erhalten wurde, und
S stellt einen Orientierungswert der dritten organischen Verbindung in der Licht emittierenden Schicht dar.

**2.** Organische elektrolumineszierende Vorrichtung nach Anspruch 1, wobei eine Konzentration der dritten organischen Verbindung in der Licht emittierenden Schicht größer als 0,3 Gewichts-% ist.

**3.** Organische elektrolumineszierende Vorrichtung nach einem der Ansprüche 1 oder 2, wobei eine Konzentration der zweiten organischen Verbindung in der Licht emittierenden Schicht 25 Gewichts-% oder mehr beträgt.

**4.** Organische elektrolumineszierende Vorrichtung nach einem der Ansprüche 1 bis 3, wobei die zweite organische Verbindung eine Struktur aufweist, in der 1 bis 2 Cyanogruppen und mindestens eine Donorgruppe an einen Benzolring gebunden sind.

**5.** Organische elektrolumineszierende Vorrichtung nach Anspruch 4, wobei die Donorgruppe eine Struktur aufweist, in der ein substituierter oder nicht substituierter Benzofuranring mit einem Benzolring kondensiert ist und eine Carbazol-9-yl-Gruppe bildet.

**6.** Organische elektrolumineszierende Vorrichtung nach Anspruch 5, wobei die Donorgruppe eine substituierte oder nicht substituierte 5H-Benzofuro[3,2-c]carbazol-5-yl-Gruppe ist.

**7.** Organische elektrolumineszierende Vorrichtung nach einem der Ansprüche 4 bis 6, wobei drei oder mehr Donorgruppen an den Benzolring gebunden sind.

**8.** Organische elektrolumineszierende Vorrichtung nach einem der Ansprüche 1 bis 7, wobei die erste organische Verbindung, die zweite organische Verbindung, und die dritte organische Verbindung, wie unten beschrieben, (al) erfüllen,

$$E_{LUMO}(1) > E_{LUMO}(2) > E_{LUMO}(3) \qquad \text{Formel (a1)}$$

hier stellt $E_{LUMO}(1)$ die LUMO-Energie dar, die mittels Photoelektronenspektroskopie in Luft für die erste Verbindung erhalten wurde,
$E_{LUMO}(2)$ stellt die LUMO-Energie dar, die mittels Photoelektronenspektroskopie in Luft für die zweite Verbindung erhalten wurde,
$E_{LUMO}(3)$ stellt die LUMO-Energie dar, die mittels Photoelektronenspektroskopie in Luft für die dritte Verbindung erhalten wurde, und
S stellt einen Orientierungswert der dritten organischen Verbindung in der Licht emittierenden Schicht dar.

## Revendications

1. Dispositif d'électroluminescence organique comprenant : une anode ; une cathode ; et au moins une couche organique incluant une couche émettrice de lumière entre l'anode et la cathode,

dans lequel la couche émettrice de lumière contient un premier composé organique, un deuxième composé organique, et un troisième composé organique, et satisfait aux formules (a) et (b) suivantes,
le deuxième composé organique est un matériau à fluorescence retardée,
**caractérisé en ce que**
le troisième composé organique est un composé représenté par la formule (16), suivante,

formule (16)

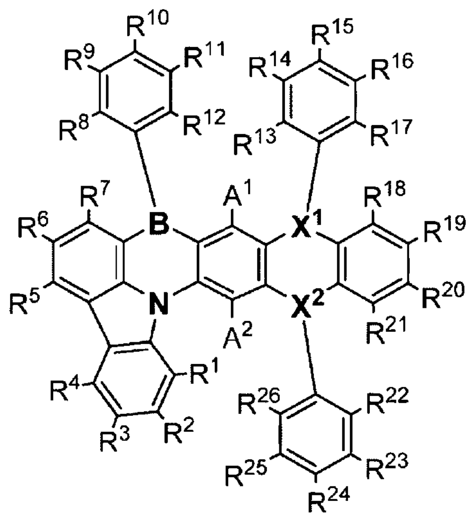

où, dans la formule (16), l'un de $X^1$ et $X^2$ est un atome d'azote, et l'autre est un atome de bore ; chacun de $R^1$ à $R^{26}$, $A^1$, et $A^2$ représente indépendamment un atome d'hydrogène, un atome de deutérium, ou un substituant ; $R^1$ et $R^2$, $R^2$ et $R^3$, $R^3$ et $R^4$, $R^4$ et $R^5$, $R^5$ et $R^6$, $R^6$ et $R^7$, $R^7$ et $R^8$, $R^8$ et $R^9$, $R^9$ et $R^{10}$, $R^{10}$ et $R^{11}$, $R^{11}$ et $R^{12}$, $R^{13}$ et $R^{14}$, $R^{14}$ et $R^{15}$, $R^{15}$ et $R^{16}$, $R^{16}$ et $R^{17}$, $R^{17}$ et $R^{18}$, $R^{18}$ et $R^{19}$, $R^{19}$ et $R^{20}$, $R^{20}$ et $R^{21}$, $R^{21}$ et $R^{22}$, $R^{22}$ et $R^{23}$, $R^{23}$ et $R^{24}$, $R^{24}$ et $R^{25}$, et $R^{25}$ et $R^{26}$ peuvent être liés les uns aux autres pour former des structures cycliques ; où ici, quand $X^1$ est un atome d'azote, alors $R^{17}$ et $R^{18}$ sont liés l'un à l'autre pour former une liaison simple et pour former un cycle pyrrole, et quand $X^2$ est un atome d'azote, alors $R^{21}$ et $R^{22}$ sont liés l'un à l'autre pour former une liaison simple et pour former un cycle pyrrole ; où ici, quand $X^1$ est un atome d'azote, $R^7$ et $R^8$ et $R^{21}$ et $R^{22}$ sont liés par l'intermédiaire d'atomes d'azote pour former des cycles à 6 chaînons, et $R^{17}$ et $R^{18}$ sont liés l'un à l'autre pour former une liaison simple, alors l'un au moins de $R^1$ à $R^6$ est un groupe aryle substitué ou non substitué, ou l'un quelconque de $R^1$ et $R^2$, $R^2$ et $R^3$, $R^3$ et $R^4$, $R^4$ et $R^5$, et $R^5$ et $R^6$ sont liés l'un à l'autre pour former un cycle aromatique ou un cycle hétéroaromatique, et
la plus grande composante de lumière émise depuis le dispositif est une fluorescence depuis le troisième composé organique,

$$E_{LUMO}(2) > E_{LUMO}(3) \qquad \text{formule (a)}$$

$$S \leq -0,3 \qquad \text{formule (b)}$$

où ici,
$E_{LUMO}(2)$ représente une énergie LUMO obtenue par spectroscopie photo-électronique dans l'air du deuxième composé organique,
$E_{LUMO}(3)$ représente une énergie LUMO obtenue par spectroscopie photo-électronique dans l'air du troisième composé organique, et
S représente une valeur d'orientation du troisième composé organique dans la couche émettrice de lumière.

**2.** Dispositif d' électroluminescence organique selon la revendication 1, dans lequel une concentration du troisième composé organique dans la couche émettrice de lumière est supérieure à 0,3 % en poids.

**3.** Dispositif d'électroluminescence organique selon l'une quelconque des revendications 1 ou 2, dans lequel une concentration du deuxième composé organique dans la couche émettrice de lumière est de 25 % en poids ou plus.

**4.** Dispositif d'électroluminescence organique selon l'une quelconque des revendications 1 à 3, dans lequel le deuxième composé organique a une structure dans laquelle 1 à 2 groupes cyano et au moins un groupe donneur sont liés à un cycle benzénique.

**5.** Dispositif d' électroluminescence organique selon la revendication 4, dans lequel le groupe donneur a une structure dans laquelle un cycle benzofurane substitué ou non substitué est condensé avec un cycle benzénique constituant un groupe carbazole-9-yl.

**6.** Dispositif d' électroluminescence organique selon la revendication 5, dans lequel le groupe donneur est un groupe 5H-benzofuro[3,2-c]carbazole-5-yl substitué ou non substitué.

**7.** Dispositif d'électroluminescence organique selon l'une quelconque des revendications 4 à 6, dans lequel trois ou plus groupes donneur sont liés au cycle benzénique.

**8.** Dispositif d'électroluminescence organique selon l'une quelconque des revendications 1 à 7, dans lequel le premier composé organique, le deuxième composé organique, et le troisième composé organique satisfont à (a1) décrite ci-dessous,

$$E_{LUMO}(1) > E_{LUMO}(2) > E_{LUMO}(3) \qquad \text{formule (a1)}$$

où ici,

$E_{LUMO}(1)$ représente une énergie LUMO obtenue par spectroscopie photo-électronique dans l'air du premier composé organique,
$E_{LUMO}(2)$ représente une énergie LUMO obtenue par spectroscopie photo-électronique dans l'air du deuxième composé organique,
$E_{LUMO}(3)$ représente une énergie LUMO obtenue par spectroscopie photo-électronique dans l'air du troisième composé organique, et
S représente une valeur d'orientation du troisième composé organique dans la couche émettrice de lumière.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015179809 A
- WO 2020039930 A
- WO 2013154064 A
- WO 2015080183 A
- WO 2015129715 A
- JP 2017119663 A
- JP 2017119664 A
- WO 2013081088 A
- WO 2021188860 A
- WO 2013011954 A
- WO 2013011955 A
- JP 2013256490 A
- JP 2013116975 A
- WO 2013133359 A
- WO 2013161437 A
- JP 2014009352 A
- JP 2014009224 A
- JP 2017222623 A
- JP 2017226838 A
- JP 2018100411 A
- WO 2018047853 A
- JP 2013253121 A
- WO 2014034535 A
- WO 2014115743 A
- WO 2014122895 A
- WO 2014126200 A
- WO 2014136758 A
- WO 2014133121 A
- WO 2014136860 A
- WO 2014196585 A
- WO 2014189122 A
- WO 2014168101 A
- WO 2015008580 A
- WO 2014203840 A
- WO 2015002213 A
- WO 2015016200 A
- WO 2015019725 A
- WO 2015072470 A
- WO 2015108049 A
- WO 2015080182 A
- WO 2015072537 A
- JP 2015129240 A
- WO 2015129714 A
- WO 2015133501 A
- WO 2015136880 A
- WO 2015137244 A
- WO 2015137202 A
- WO 2015137136 A
- WO 2015146541 A
- WO 2015159541 A
- WO 2015022974 A
- WO 2019111971 A
- WO 2021015177 A


### Non-patent literature cited in the description


- *Scientific Reports*, 2017, vol. 7, 8405
- **HANSCH, C.** *Chem. Rev.*, 1991, vol. 91, 165-195
- *Appl. Phys. Let*, 2011, vol. 98, 083302